# FASCICULE DE BREVET EUROPEEN

(11) **EP 2 255 011 B1**
(45) Date de publication et mention de la délivrance du brevet: **26.11.2014**
(21) Numéro de dépôt: 08873435.5
(22) Date de dépôt: 29.12.2008
(51) Int. Cl.: C12Q 1/68

(54) **TYPAGE ET SOUS-TYPAGE MOLECULAIRE DE SALMONELLA PAR IDENTIFICATION DES SEQUENCES NUCLEOTIDIQUES VARIABLES DES LOCI CRISPR**
MOLEKULARE TYPISIERUNG UND SUBTYPISIERUNG VON SALMONELLA DURCH IDENTIFIZIERUNG DER VARIABLEN NUKLEOTIDSEQUENZEN DES CRISPR-LOCUS
MOLECULAR TYPING AND SUBTYPING OF SALMONELLA BY IDENTIFICATION OF THE VARIABLE NUCLEOTIDE SEQUENCES OF THE CRISPR LOCI

(30) Priorité: 28.12.2007 FR 0709188
(43) Date de publication de la demande: 01.12.2010
(73) Titulaire: Institut Pasteur, 75015 Paris (FR)
(72) Inventeur: WEILL, François-Xavier, 75015 Paris (FR); FABRE-BERLAND, Laetitia, 78760 Pontchartrain (FR); GUIBERT, Véronique, 78540 Vernouillet (FR); DIANCOURT, Laure, 95220 Herblay (FR); BRISSE, Sylvain, 92140 Clamart (FR)
(74) Mandataire: Rançon, Xavier Lucien Abel
(86) Numéro de dépôt international: PCT/IB2008/004004
(87) Numéro de publication internationale: WO 2009/115861

(56) Documents cités:
- WO-A-99/51771
- WO-A-2007/136815
- WITONSKI D ET AL: "Variable-number tandem repeats that are useful in genotyping isolates of Salmonella enterica subsp. enterica serovars Typhimurium and Newport." JOURNAL OF CLINICAL MICROBIOLOGY NOV 2006, vol. 44, no. 11, novembre 2006 (2006-11), pages 3849-3854, XP002490212 ISSN: 0095-1137
- LINDSTEDT BJORN-ARNE ET AL: "DNA fingerprinting of Salmonella enterica subsp. enterica serovar typhimurium with emphasis on phage type DT104 based on variable number of tandem repeat loci." JOURNAL OF CLINICAL MICROBIOLOGY APR 2003, vol. 41, no. 4, avril 2003 (2003-04), pages 1469-1479, XP004563766 ISSN: 0095-1137
- LIU YICHUN ET AL: "Molecular typing of Salmonella enterica serovar typhi isolates from various countries in Asia by a multiplex PCR assay on variable-number tandem repeats." JOURNAL OF CLINICAL MICROBIOLOGY SEP 2003, vol. 41, no. 9, septembre 2003 (2003-09), pages 4388-4394, XP002490214 ISSN: 0095-1137
- RAMISSE VINCENT ET AL: "Variable number of tandem repeats in Salmonella enterica subsp. enterica for typing purposes." JOURNAL OF CLINICAL MICROBIOLOGY DEC 2004, vol. 42, no. 12, décembre 2004 (2004-12), pages 5722-5730, XP002490215 ISSN: 0095-1137
- POURCEL C ET AL: "CRISPR elements in Yersinia pestis acquire new repeats by preferential uptake of bacteriophage DNA, and provide additional tools for evolutionary studies" MICROBIOLOGY, SOCIETY FOR GENERAL MICROBIOLOGY, READING, GB, vol. 151, 1 mars 2005 (2005-03-01), pages 653-663, XP002460396 ISSN: 1350-0872 cité dans la demande
- NAKATA A ET AL: "Unusual nucleotide arrangement with repeated sequences in the Escherichia coli K-12 chromosome." JOURNAL OF BACTERIOLOGY JUN 1989, vol. 171, no. 6, juin 1989 (1989-06), pages 3553-3556, XP002490216 ISSN: 0021-9193
- JANSEN R ET AL: "IDENTIFICATION OF A NOVEL FAMILY OF SEQUENCE REPEATS AMONG PROKARYOTES" OMICS A JOURNAL OF INTEGRATIVE BIOLOGY, MARY ANN LIEBERT, NEW YORK, NY, US, vol. 6, no. 1, 1 janvier 2002 (2002-01-01), pages 23-33, XP008066327 ISSN: 1536-2310
- GODDE J S ET AL: "The Repetitive DNA Elements Called CRISPRs and Their Associated Genes: Evidence of Horizontal Transfer Among Prokaryotes" JOURNAL OF MOLECULAR EVOLUTION, SPRINGER-VERLAG, NE, vol. 62, no. 6, 11 avril 2006 (2006-04-11), pages 718-729, XP019421382 ISSN: 1432-1432
- GRISSA IBTISSEM ET AL: "CRISPRFinder: a web tool to identify clustered regularly interspaced short palindromic repeats." NUCLEIC ACIDS RESEARCH JUL 2007, vol. 35, no. Web Server issue, 31 mai 2007 (2007-05-31), pages W52-W57, XP002490217 ISSN: 1362-4962
- GROENEN P M ET AL: "Nature of DNA polymorphism in the direct repeat cluster of Mycobacterium tuberculosis; Application for strain differentiation by a novel typing method" MOLECULAR MICROBIOLOGY, BLACKWELL SCIENTIFIC, OXFORD, vol. 10, no. 5, 1 janvier 1993 (1993-01-01), pages 1057-1065, XP002091621 ISSN: 0950-382X
- GRIMONT P.; WEILL F.-X.: "Centre National de Référence des Salmonella, Rapport d'activité annuel" [Online] 2005, EDITIONS PASTEUR , INSTITUT PASTEUR, PARIS, FRANCE , XP002490218 Extrait de l'Internet: URL:http://www.pasteur.fr/sante/clre/cadre cnr/salmcnr/salmcnr-actualites.html> [extrait le 2008-07-23] page 28, alinéa 2 page 43
- SCHOULS LEO M ET AL: "Comparative genotyping of Campylobacter jejuni by amplified fragment length polymorphism, multilocus sequence typing, and short repeat sequencing: strain diversity, host range, and recombination.", JOURNAL OF CLINICAL MICROBIOLOGY JAN 2003 LNKD- PUBMED:12517820, vol. 41, no. 1, January 2003 (2003-01), pages 15-26, ISSN: 0095-1137
- DATABASE EMBL [Online] 22 April 2002 (2002-04-22), "Sequence 70 from patent US 6355428.", retrieved from EBI accession no. EMBL:AR199361 Database accession no. AR199361 -& US 6 355 428 B1 (SCHROTH GARY P [US] ET AL) 12 March 2002 (2002-03-12)
- DATABASE EMBL [Online] 6 April 2006 (2006-04-06), "Sequence 23 from patent US 6986992.", retrieved from EBI accession no. EMBL:AR789767 Database accession no. AR789767 -& US 6 986 992 B2 (CHUI BUENA [US] ET AL) 17 January 2006 (2006-01-17)
- DATABASE EMBL [Online] 29 November 2002 (2002-11-29), "Sequence 512 from Patent WO02055741.", retrieved from EBI accession no. EMBL:AX572472 Database accession no. AX572472 -& WO 02/055741 A2 (INNOGENETICS NV [BE]; DE SMET KOENRAAD [BE]; STUYVER LIEVEN [BE]) 18 July 2002 (2002-07-18)

## Description

La présente invention est relative à la détection et à l'identification des bactéries du genre *Salmonella,* ainsi qu'à des réactifs de diagnostic, tels que des amorces et sondes oligonucléotidiques, permettant le typage et le sous-typage moléculaire de ces bactéries.

Les salmonelloses sont la première cause de diarrhées bactériennes d'origine alimentaire dans les pays industrialisés. L'Institut de Veille Sanitaire a estimé en 2004, qu'annuellement en France, 30 000 à 40 000 cas confirmés de salmonellose entraînent 100 à 500 décès. Du fait d'un vaste réservoir animal, les salmonelloses animales et humaines représentent également un coût économique important pour l'industrie agroalimentaire. La recherche de *Salmonella* est donc réalisée tout au long de la chaîne alimentaire, de l'élevage jusqu'au produit alimentaire fini. En cas de prélèvement de *Salmonella* positif, un typage plus précis de la salmonelle est réalisé par sérotypage.

Le sérotypage des *Salmonella* est actuellement basé sur la reconnaissance d'antigènes bactériens (un antigène de paroi et un ou deux antigènes flagellaires) à l'aide d'une batterie d'anticorps polyclonaux préparés chez le lapin. Plus de 2500 sérotypes ont déjà été décrits chez *Salmonella.* Le sérotypage est une technique longue (environ 3 à 6 jours pour obtenir un résultat) et coûteuse (prix élevé des sérums et coût important de la main-d'oeuvre car il s'agit d'une méthode manuelle). En outre, afin de pouvoir sérotyper l'ensemble des 2500 sérotypes, il faut pouvoir disposer d'environ 200 sérums de lapin (dont une grande partie n'est pas commercialisée). Le sérotypage est important non seulement dans le domaine médical car il permet de détecter les épidémies, mais aussi dans le domaine alimentaire car il permet de tracer les souches à l'origine de contamination des aliments.

Par ailleurs, du fait de la prédominance de certains sérotypes de *Salmonella* (Typhimurium et Enteridis représentent à eux seuls 70% des *Salmonella* détectées en France chaque année), il est nécessaire d'utiliser des méthodes plus discriminantes pour tracer une souche particulière appartenant à ces sérotypes. Ces méthodes dites de sous-typage, mettant en oeuvre par exemple l'électrophorèse en champ pulsé, la lysotypie, ou l'étude par VNTR (acronyme pour « Variable Number of Tandem Repeat », Lindstedt et al., 2003, J. Clin. Microbiol. 14, 1469-1479), sont lourdes sur le plan technique et nécessitent plusieurs jours supplémentaires de manipulation.

La présence d'une famille de séquences répétées d'ADN génomique appelée CRISPR (acronyme pour «Clustered Regularly Interspaced Short Palindromic Repeats ») a été identifiée au sein de nombreux organismes procaryotes par Jansen et al. (2002, Mol. Microbiol. 43, 1565-1575). Un locus CRISPR est caractérisé par des séquences répétées (ou DR) non-contiguës, généralement de 21 à 37 paires de bases (pb), séparées par des séquences uniques, généralement de 20 à 40 paires de bases appelées séquences variables (ou bien espaceurs ou « spacers »). Le nombre de séquences répétées au sein d'un locus CRISPR, le nombre de loci CRISPR (1 à 2 chez *Salmonella*), ou bien la présence de loci CRISPR peuvent varier au sein d'une même espèce bactérienne (van Embden et al., 2000, J. Bacteriol. 182, 2393-2401).

Les loci CRISPR ont alors été utilisés pour typer des souches bactériennes par la technique appelée spoligotypage ou « typage oligonucléotidique des espaceurs du locus CRISPR » (Kamerbeek et al., 1997, J. Clin. Microbiol. 43, 907-914 ; Hoe et al., 1999, Emerg. Infect. Dis. 5, 254-263). Cette technique consiste à amplifier le locus CRISPR par PCR grâce à des amorces oligonucléotidiques complémentaires de régions d'ADN génomiques situées uniquement dans les séquences répétées (DR) des loci CRISPR. La présence ou l'absence des séquences variables (espaceurs) dans le produit d'amplification est alors déterminée à l'aide d'une puce à ADN sur laquelle sont fixées des sondes aptes à s'hybrider à ces séquences variables.

Le typage et le sous-typage moléculaire par spoligotypage ont ainsi été décrits pour différentes espèces bactériennes, telles que *Mycobacterium tuberculo-sis* (Kamerbeek *et al.,* 1997, cité ci-dessus ; Song et al., 2007, J. Microbiol. Methods, 68, 430-433) et *Corynebacterium diphteriae* (Mokrousov et al., 2005, J. Clin. Microbiol. 43, 1662-1668).

Le principe d'une méthode de différenciation de souches bactériennes du genre *Salmonella* par spoligotypage a été décrit d'une manière très générale dans la Demande Internationale WO 99/51771. Cependant, cette Demande ne divulgue pas les séquences nucléotidiques des séquences variables susceptibles d'être utilisées pour détecter les différents types ou sous-types de *Salmonella.*

Pourcel et al. (2005, Microbiology 151, 653-663) ont par ailleurs décrit une méthode d'analyse phylogénétique de souches bactériennes de l'espèce *Yersinia pestis* basée sur l'étude du polymorphisme des loci CRISPR de ces souches. Cette méthode comprend l'amplification par PCR des loci CRISPR de souches d' *Y*. *pestis* à l'aide d'amorces oligonucléotidiques complémentaires d'une séquence d'ADN génomique adjacente aux loci CRISPR d'*Y. pestis* et *Y. pseudotuberculosis,* puis l'analyse de l'arrangement des séquences variables contenues dans ces loci.

La mise en oeuvre de la technique de spoligotypage présente plusieurs inconvénients. En effet, étant donné que des régions des séquences répétées des loci CRISPR servent de brin complémentaire auxquelles les amorces oligonucléotidiques s'hybrident lors de la réaction PCR, le produit d'amplification contient, par conséquent, un mélange de fragments d'ADN de différentes tailles comprenant d'une à toutes les séquences variables (espaceurs) d'un locus CRISPR. De fait, l'identification de l'ordre des espaceurs et de la présence de nouveaux espaceurs dans un locus CRISPR ne peut être réalisée par cette méthode car le séquençage de ce mélange de fragments d'ADN amplifiés s'avère impossible. En outre, les amorces oligonucléotidiques étant spécifiques d'une séquence répétée d'un sérotype donné, il est non seulement nécessaire de disposer de plusieurs couples d'amorces pour identifier différents sérotypes, mais aussi impossible de détecter par hybridation des sérotypes présentant des séquences répétées différentes de celles pour lesquelles ont été conçues les amorces.

La présente invention s'est donnée pour but de pourvoir à une méthode qui réponde mieux aux besoins de la pratique que les méthodes de l'art antérieur, notamment en ce que le typage et/ou le sous-typage moléculaire des bactéries du genre *Salmonella* soient rapides et facilement automatisables.

Ainsi, les Inventeurs ont conçu des amorces oligonucléotidiques de manière à amplifier par PCR des fragments de la séquence génomique de différentes souches de *Salmonella ;* ces fragments comprennent l'un des deux ou les deux loci CRISPR. Ces amorces s'hybrident, non pas à une région des séquences répétées situées à l'intérieur des loci CRISPR mais à une séquence génomique située à l'extérieur de ces loci. Les Inventeurs ont ensuite séquencé les fragments d'ADN amplifiés de façon à identifier les différentes séquences variables (espaceurs) dans chaque locus CRISPR et ainsi déterminer leurs compositions en séquences variables.

Les Inventeurs ont ainsi analysé 564 souches de *Salmonella* appartenant à 86 sérotypes des deux espèces et six sous-espèces de *Salmonella* (tels qu'identifiés par les méthodes classiques de sérotypage), dont ceux qui sont retrouvés le plus fréquemment chez l'Homme, les animaux et dans les aliments. Ces sérotypes appartiennent aux deux espèces de *Salmonella : S. enterica* (et ses six sous-espèces : *arizonae, diarizonae, enterica, houtenae, indica, salamae*) et *S. bongori.* A partir des loci CRISPR de ces souches, les Inventeurs ont identifié environ 2150 séquences variables différentes, d'une taille moyenne de 34 nucléotides.

De manière surprenante les Inventeurs ont observé que la présence (de 1 à 30 par locus CRISPR) ou l'absence de certaines séquences variables ainsi que leur arrangement dans les loci CRISPR étaient caractéristiques d'un sérotype. De même, quand plusieurs souches d'un même sérotype étaient étudiées, la composition en séquences variables pouvait varier entre les souches (perte de certaines séquences variables ou acquisition de nouvelles séquences variables). Les Inventeurs ont en outre montré que cette variabilité au sein d'un même sérotype était concordante avec les méthodes traditionnelles de sous-typage.

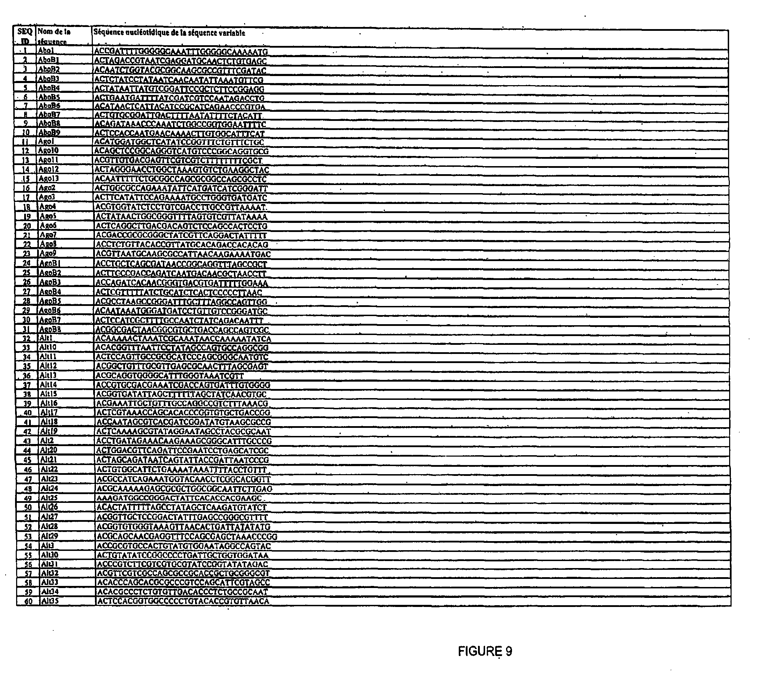

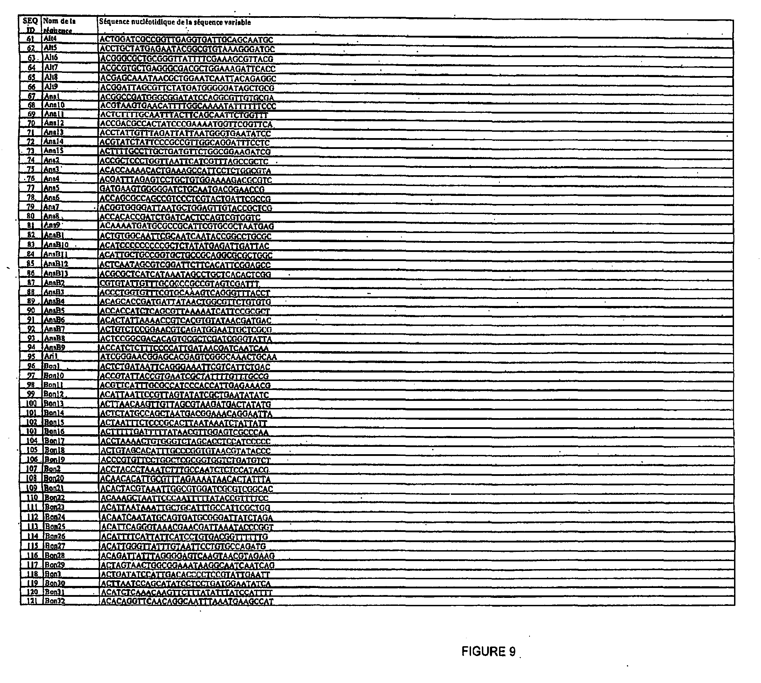

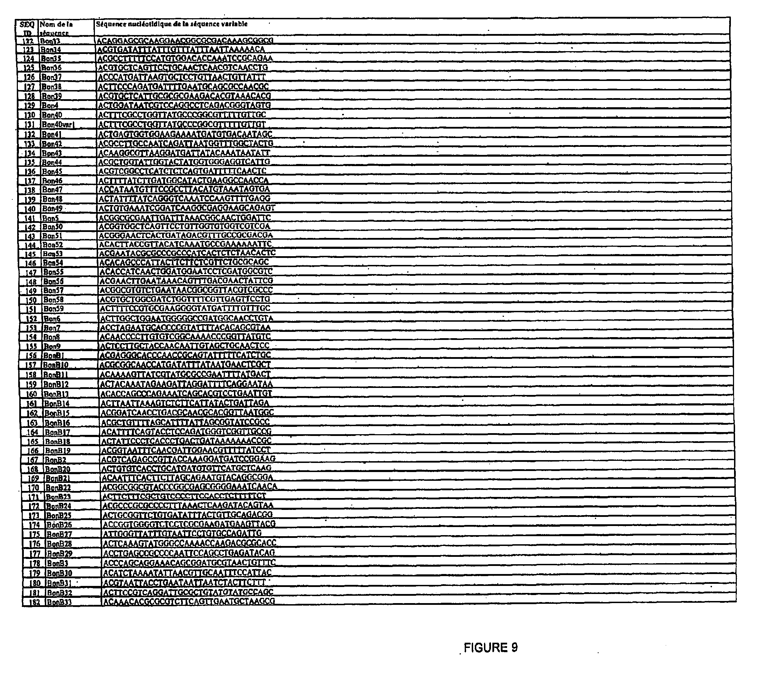

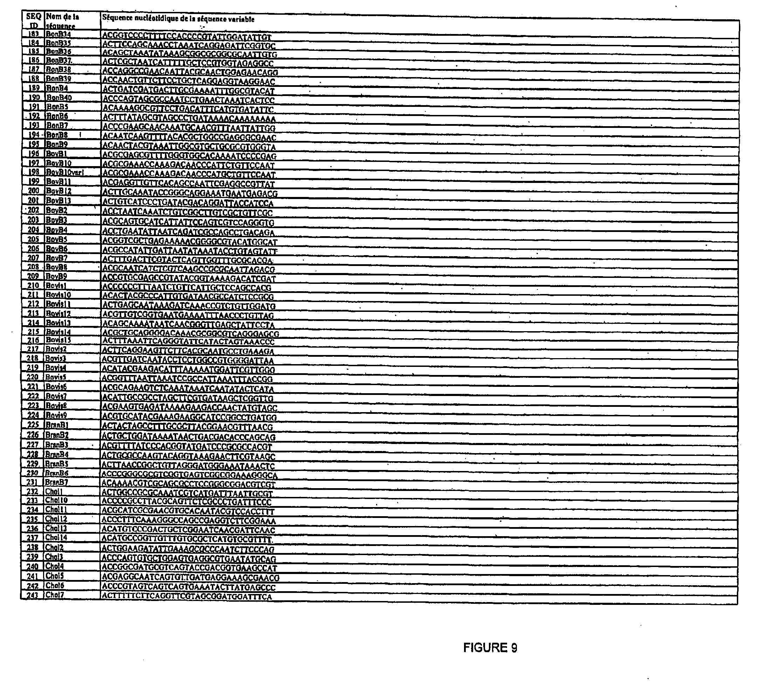

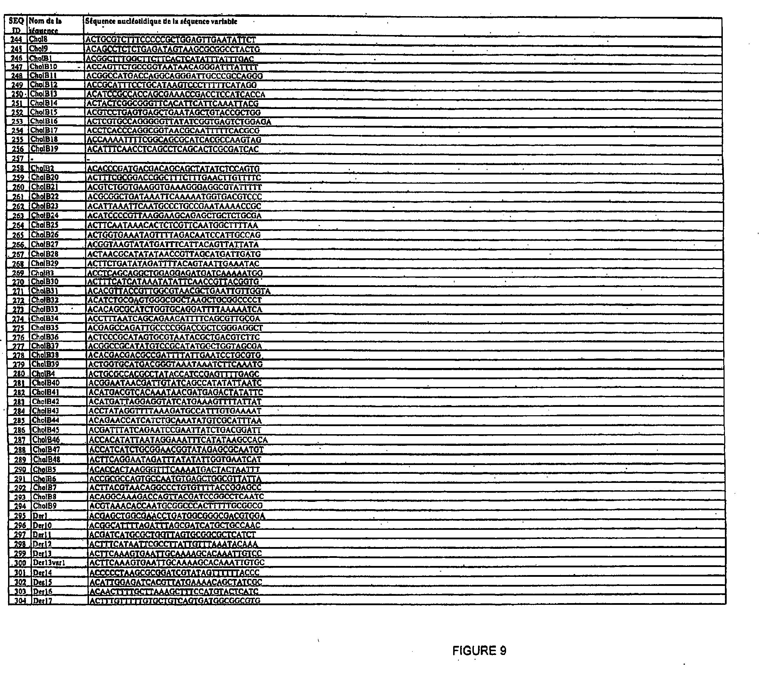

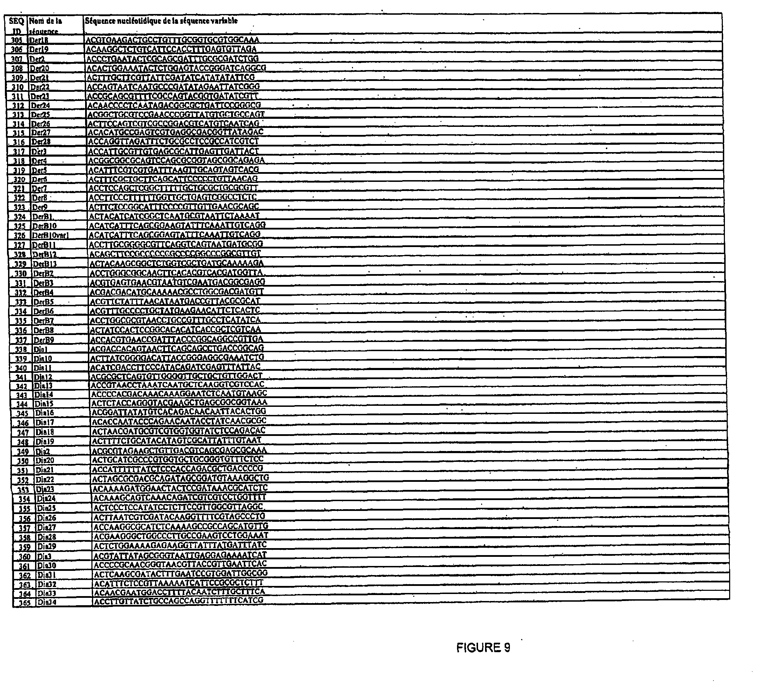

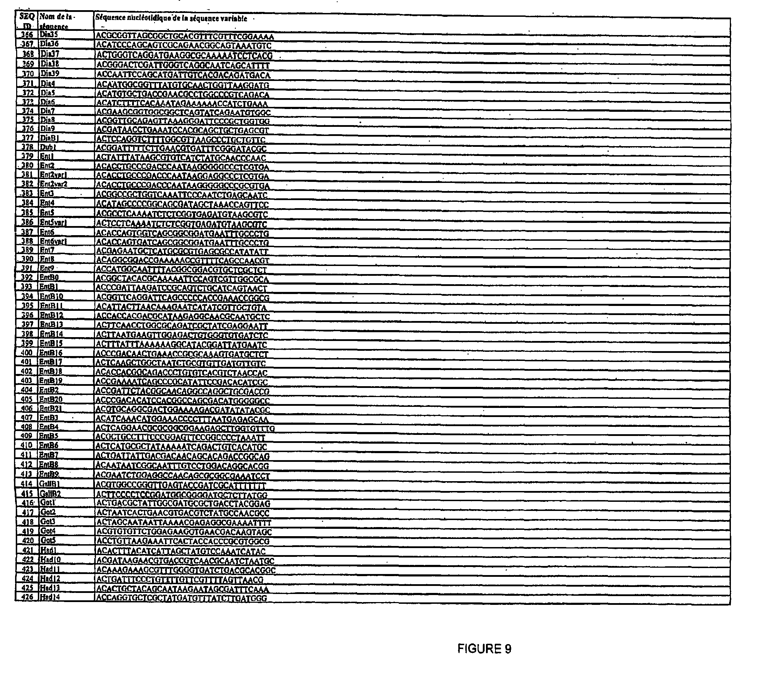

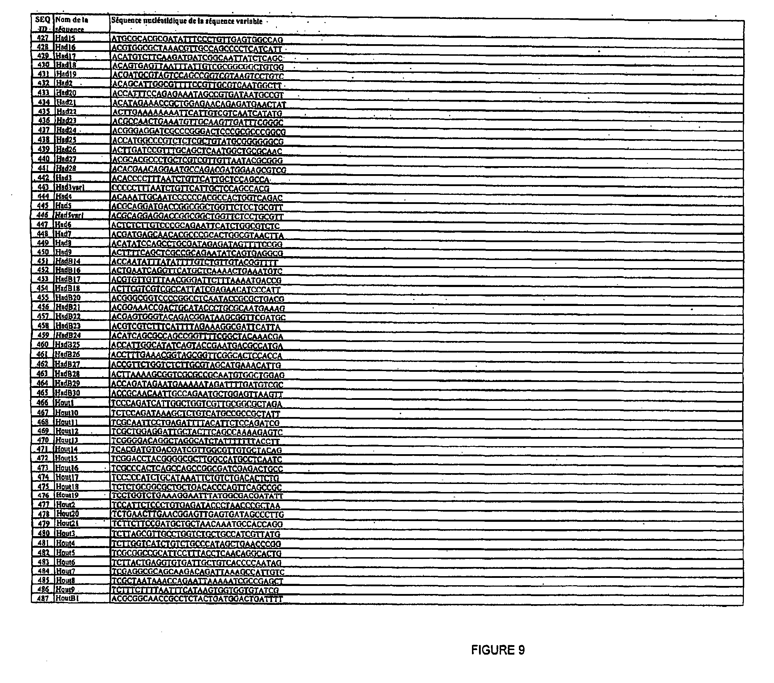

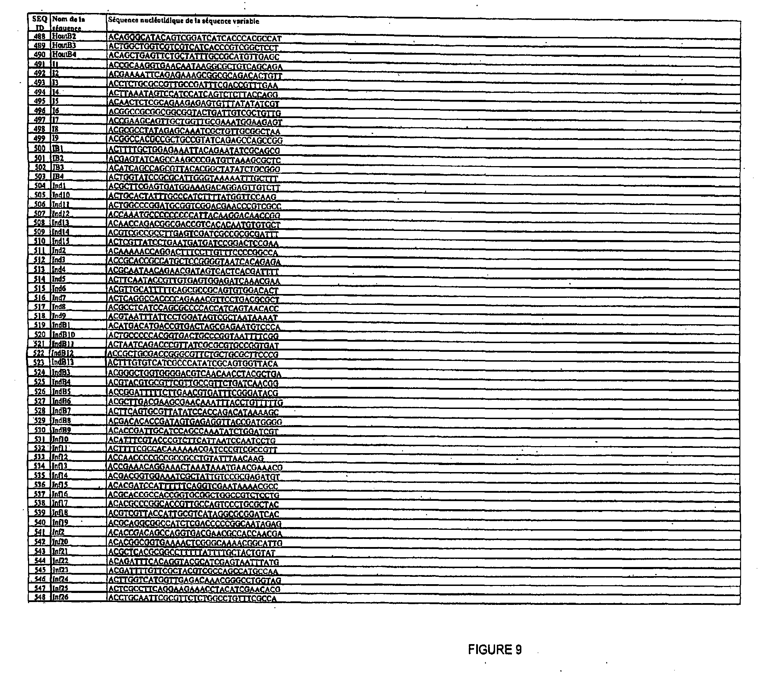

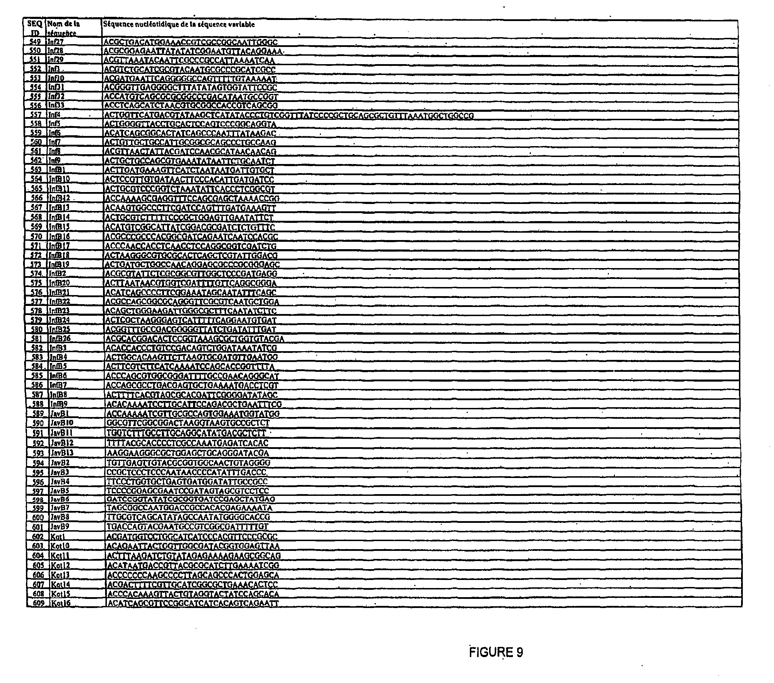

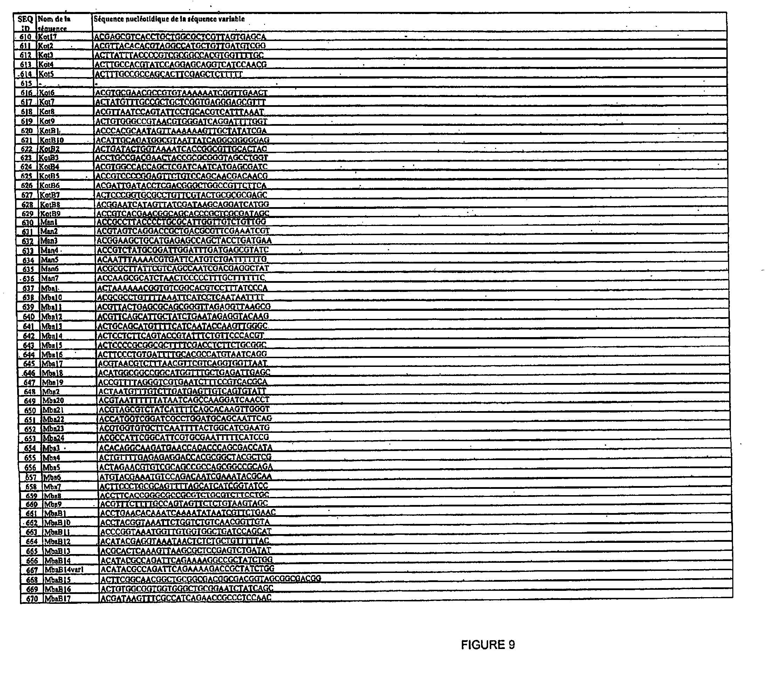

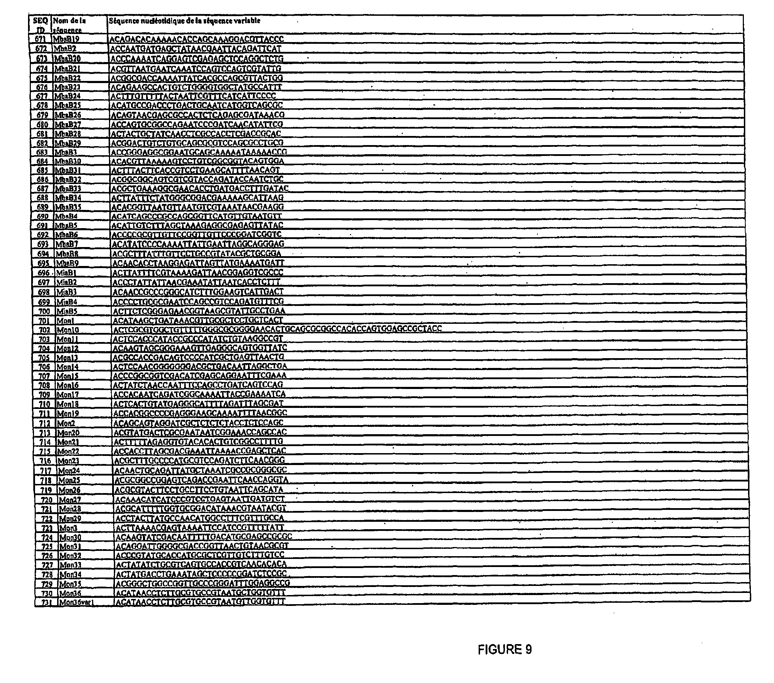

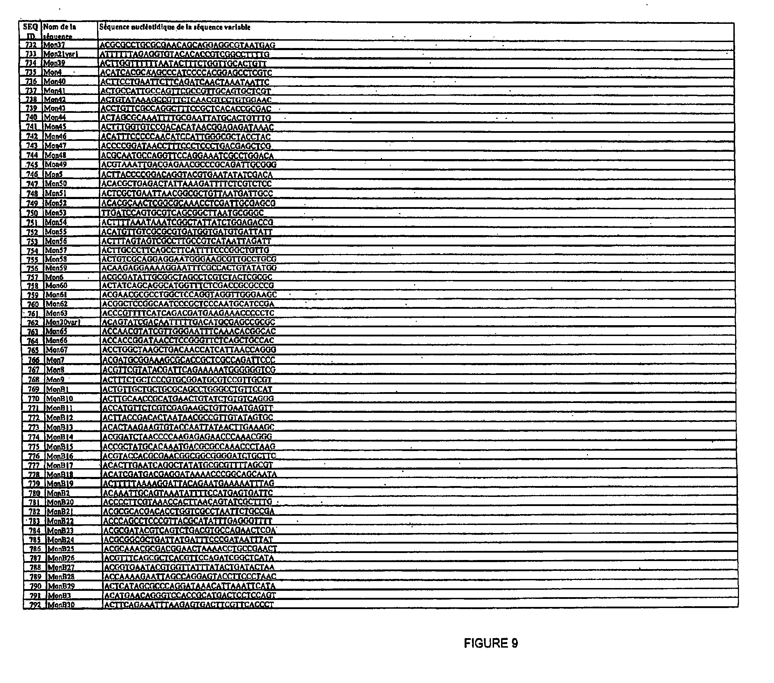

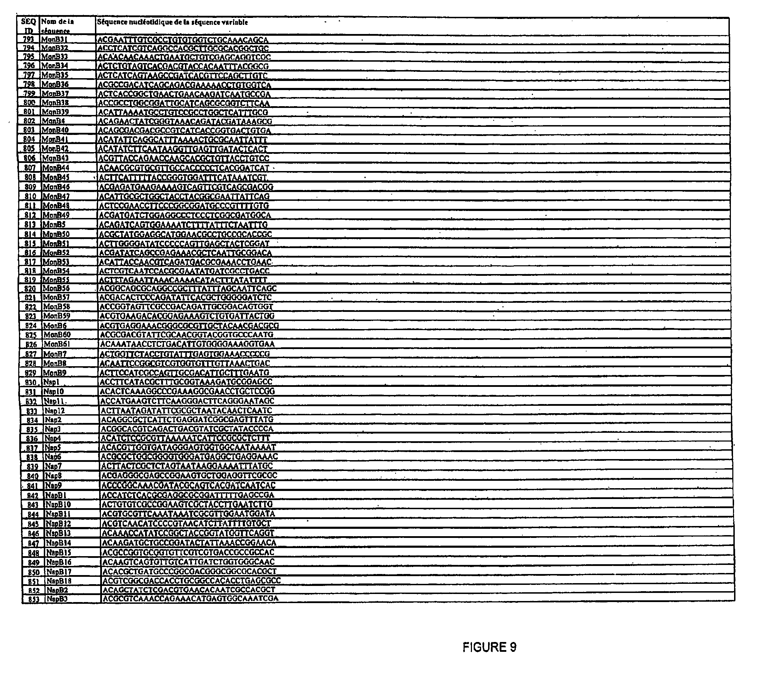

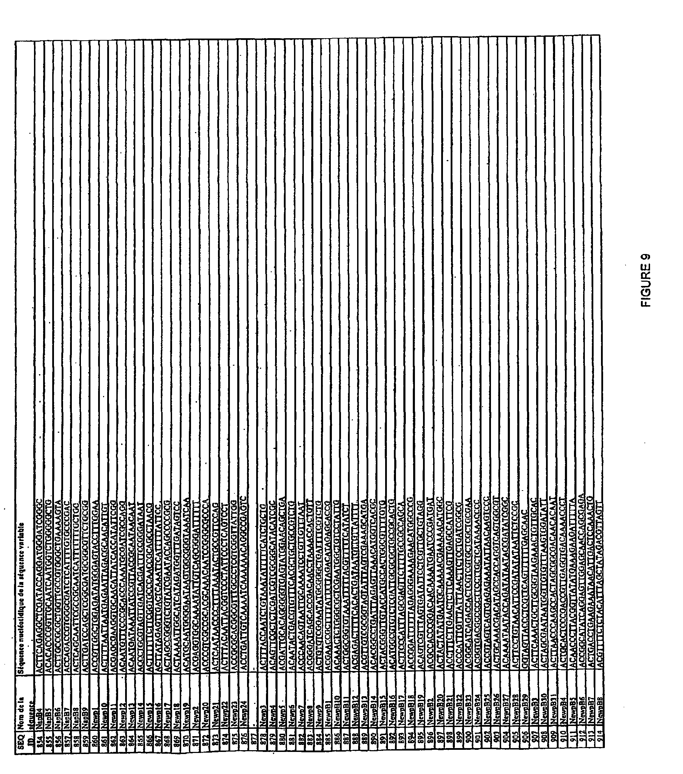

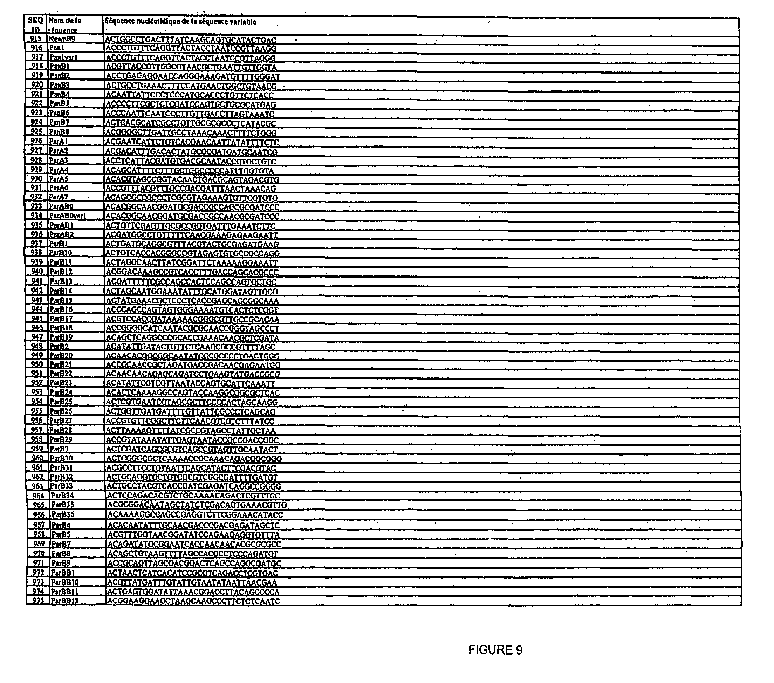

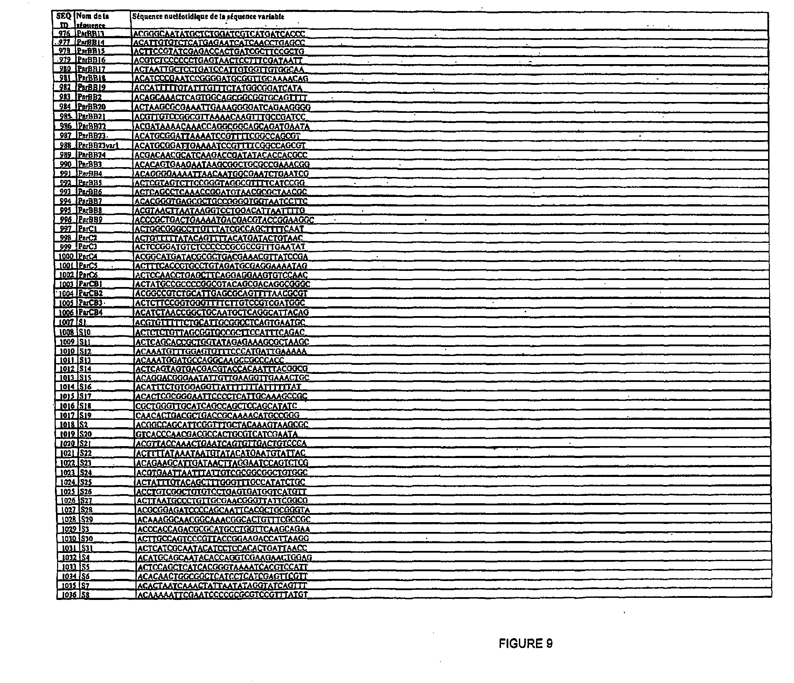

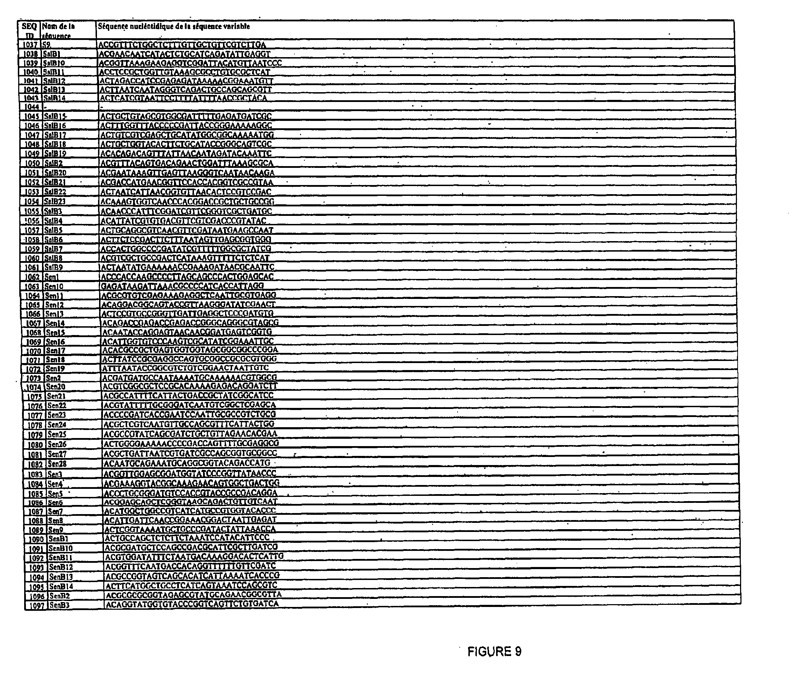

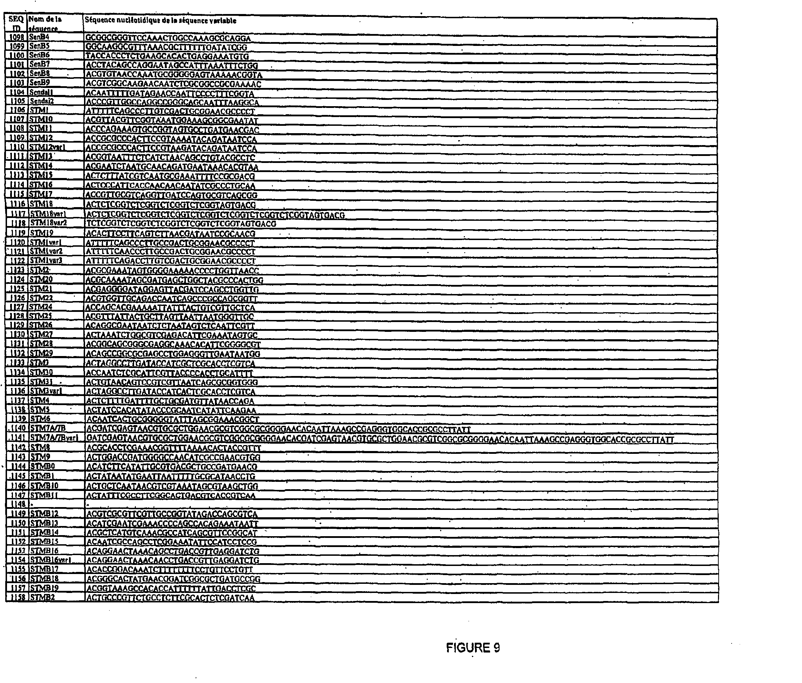

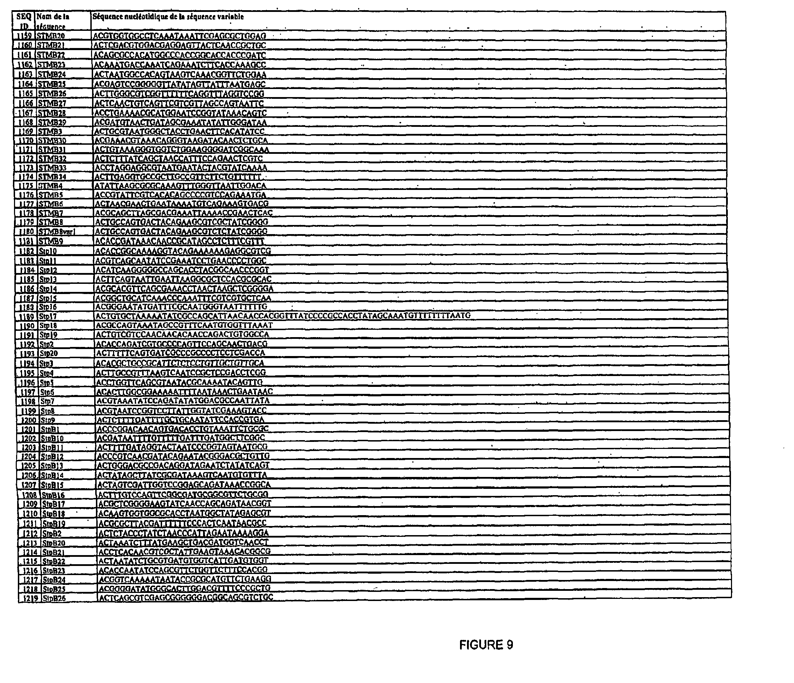

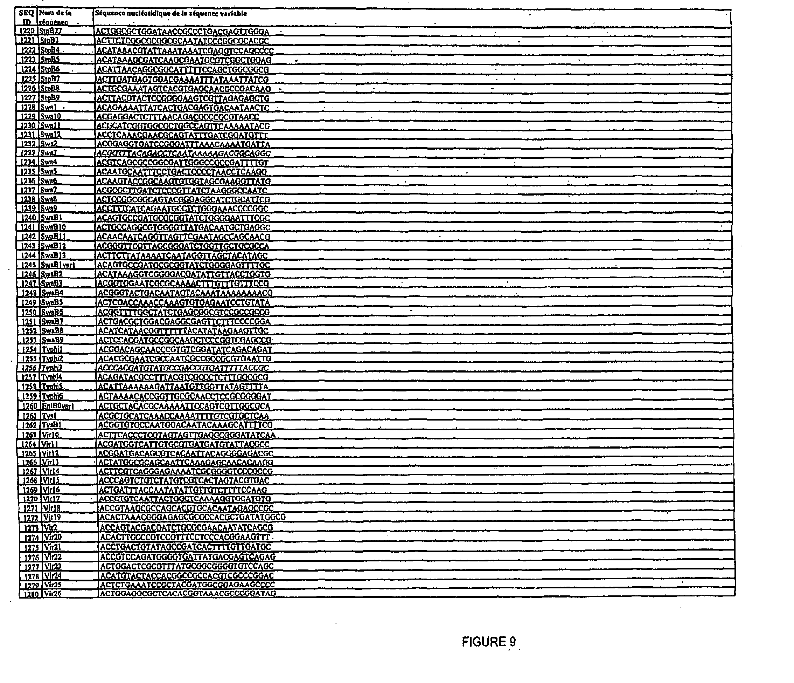

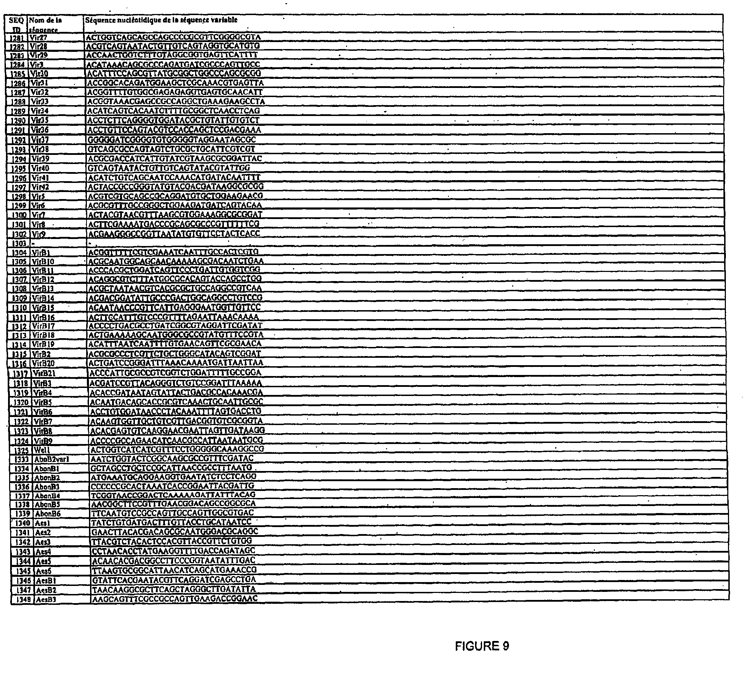

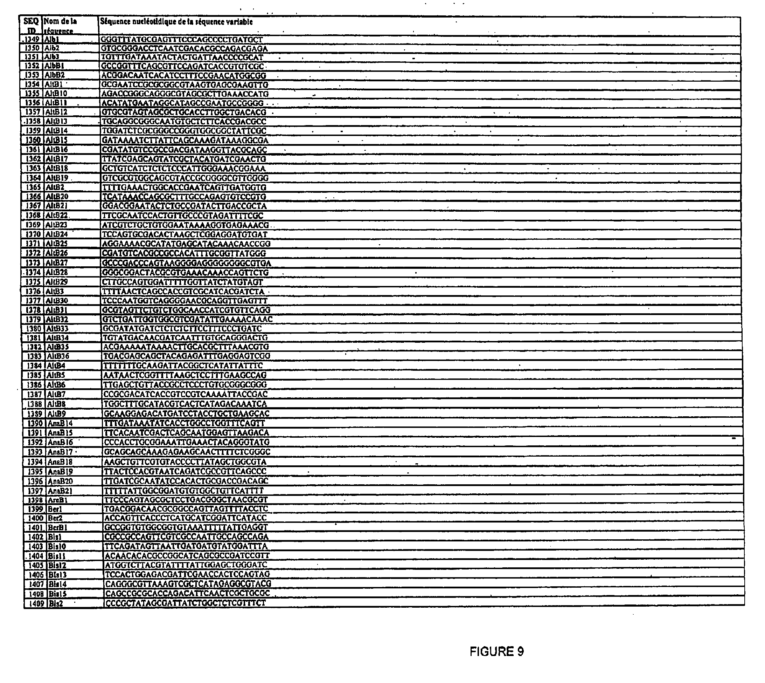

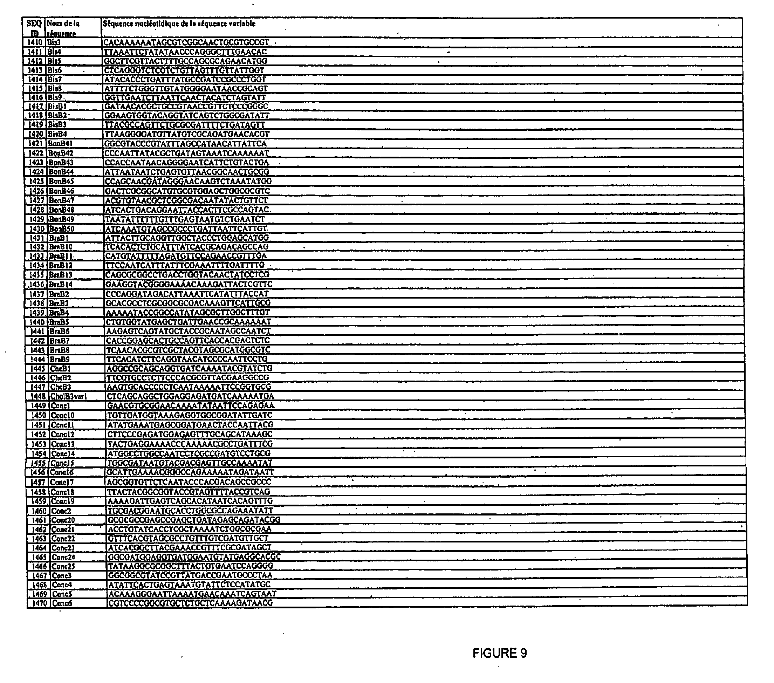

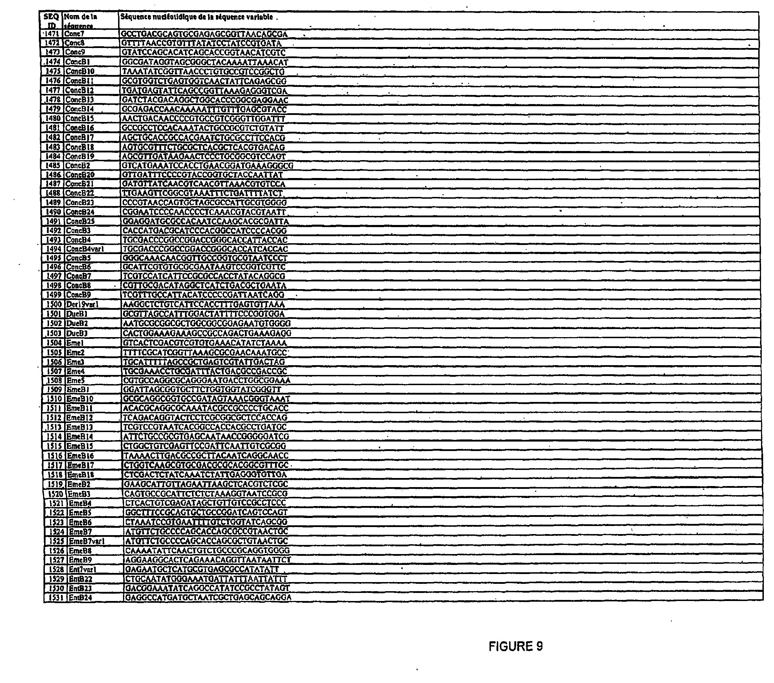

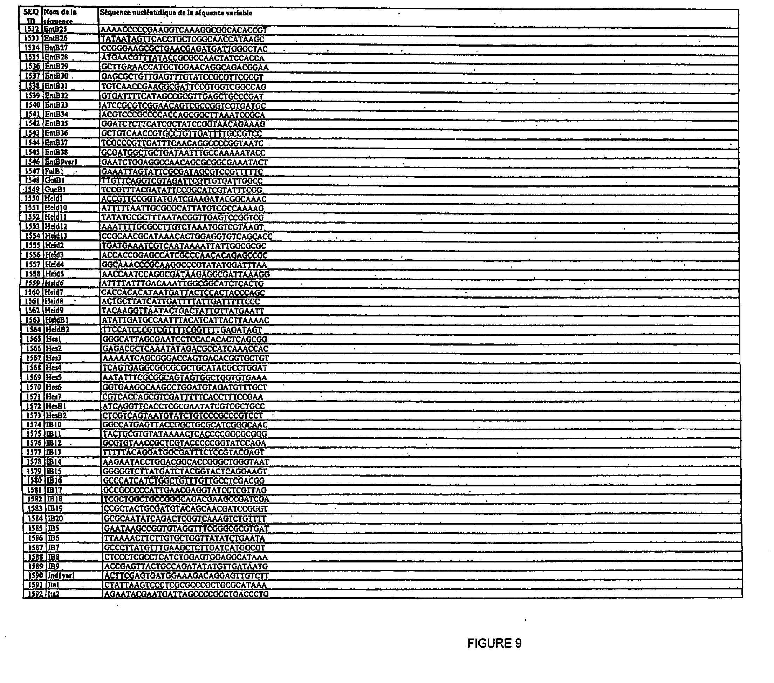

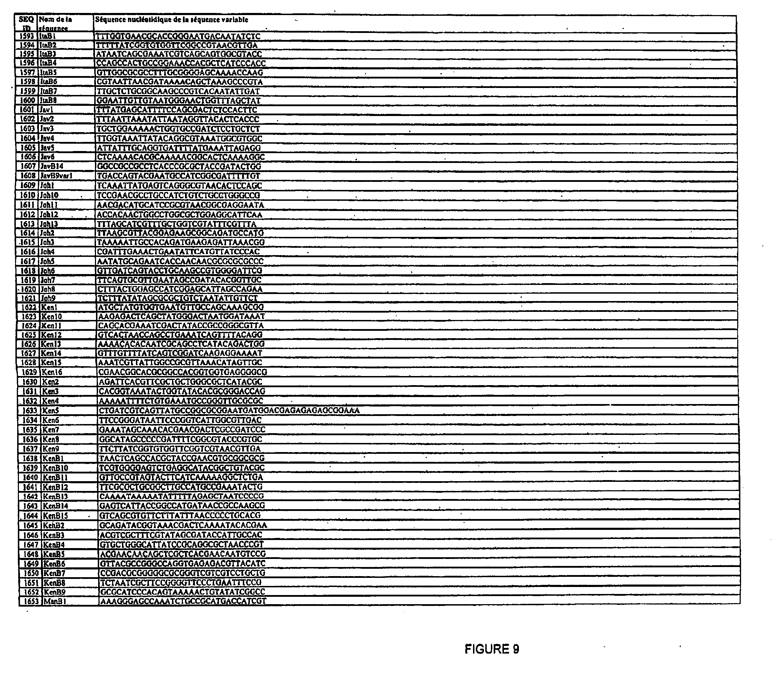

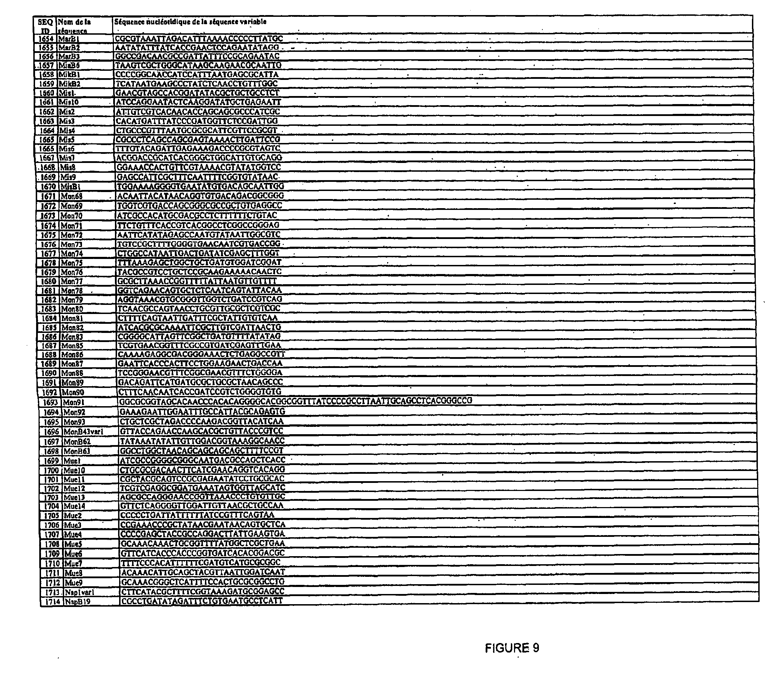

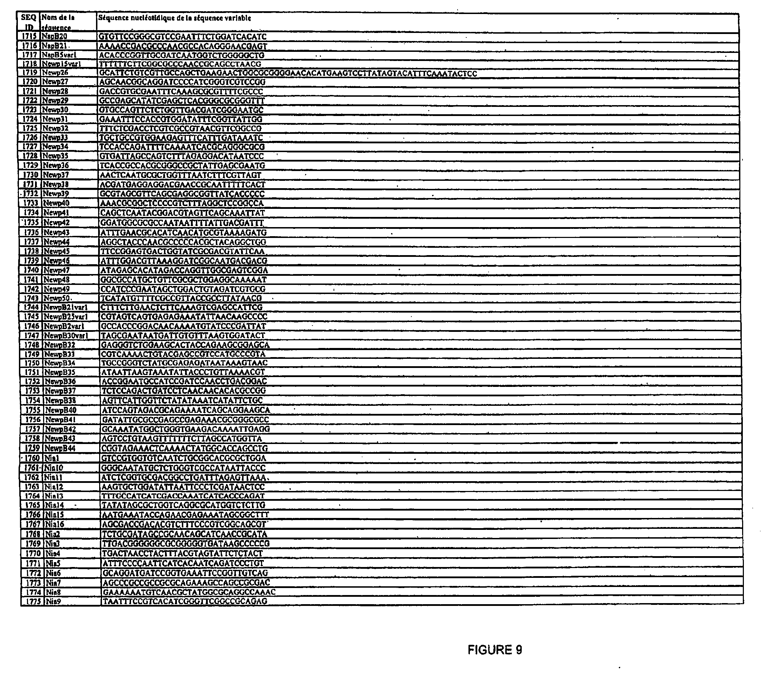

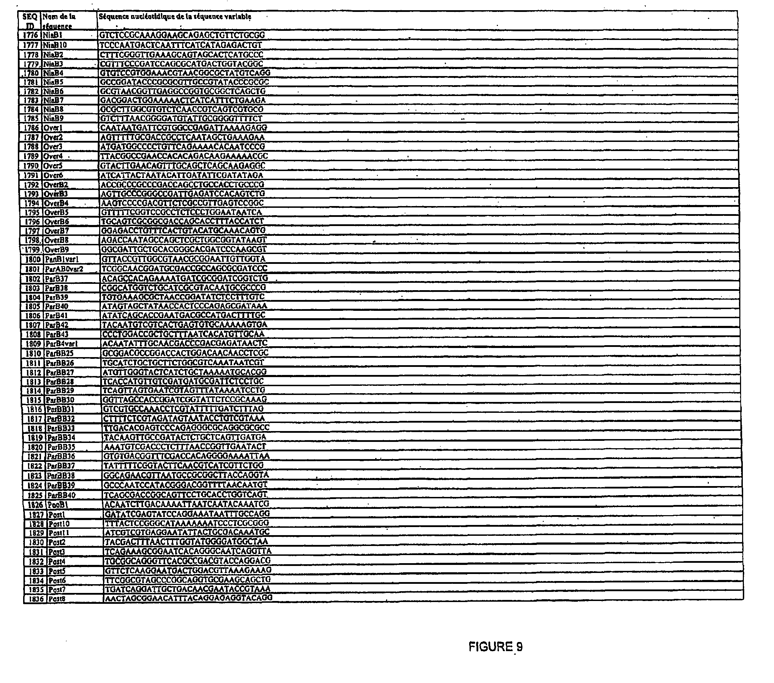

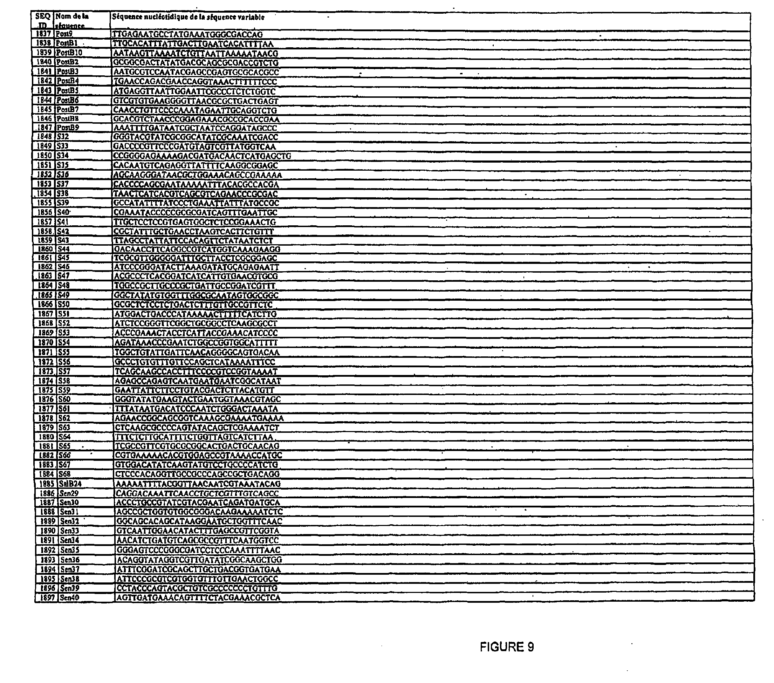

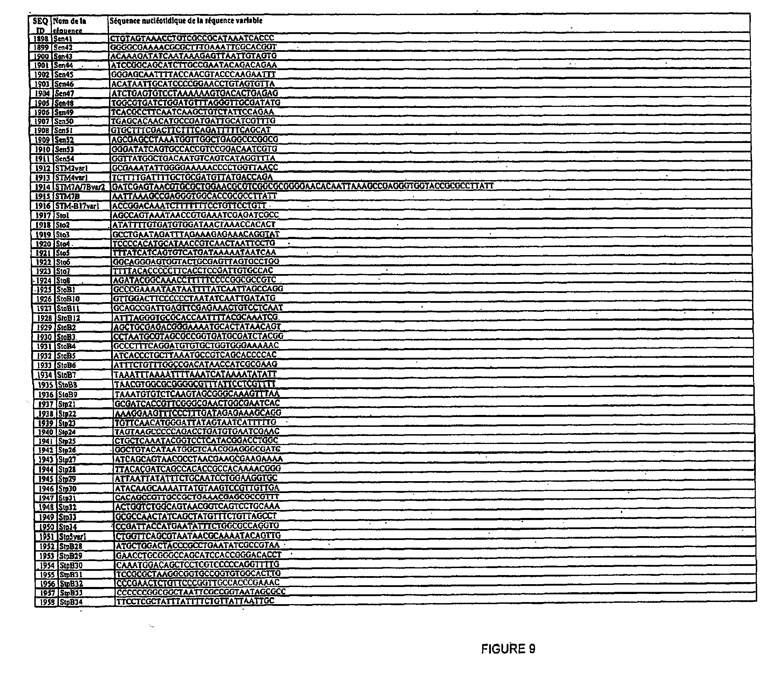

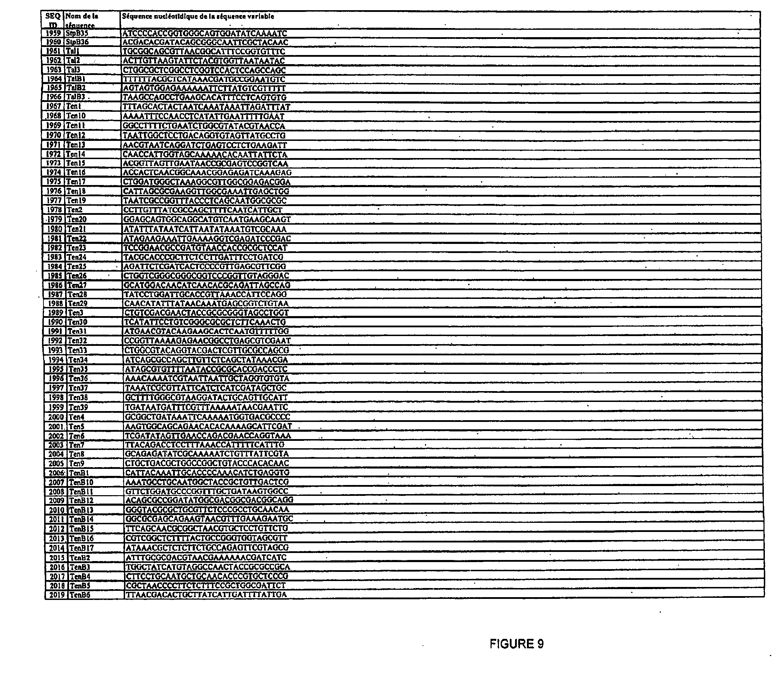

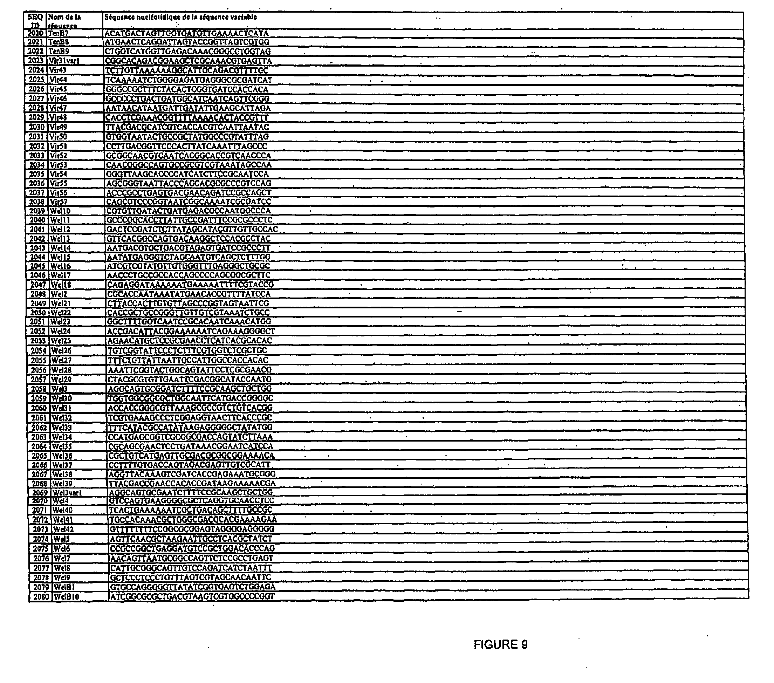

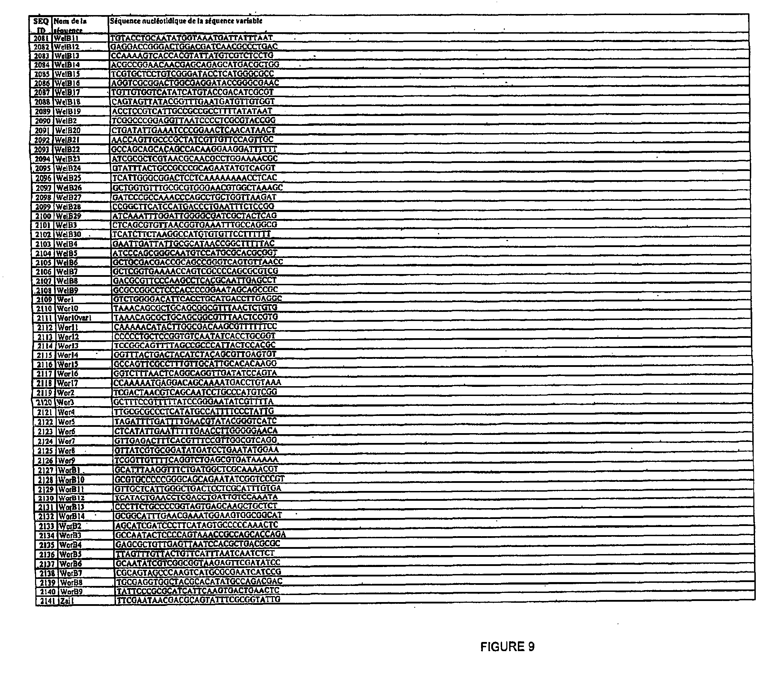

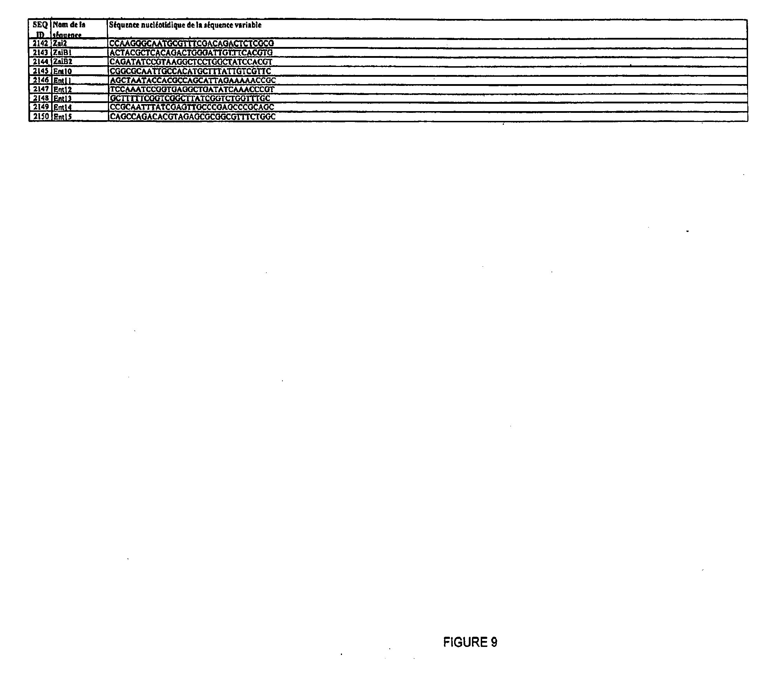

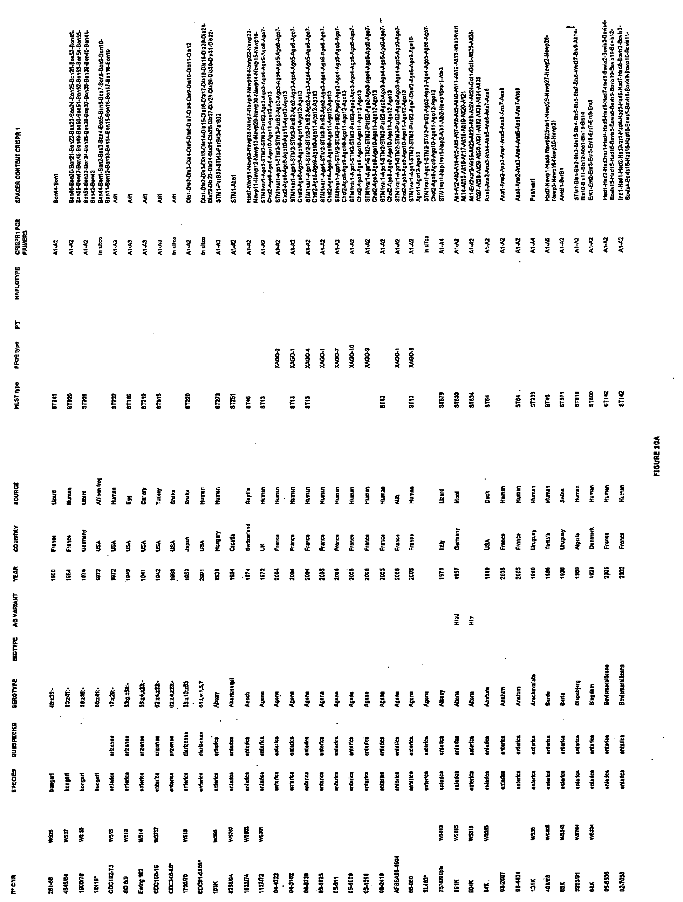

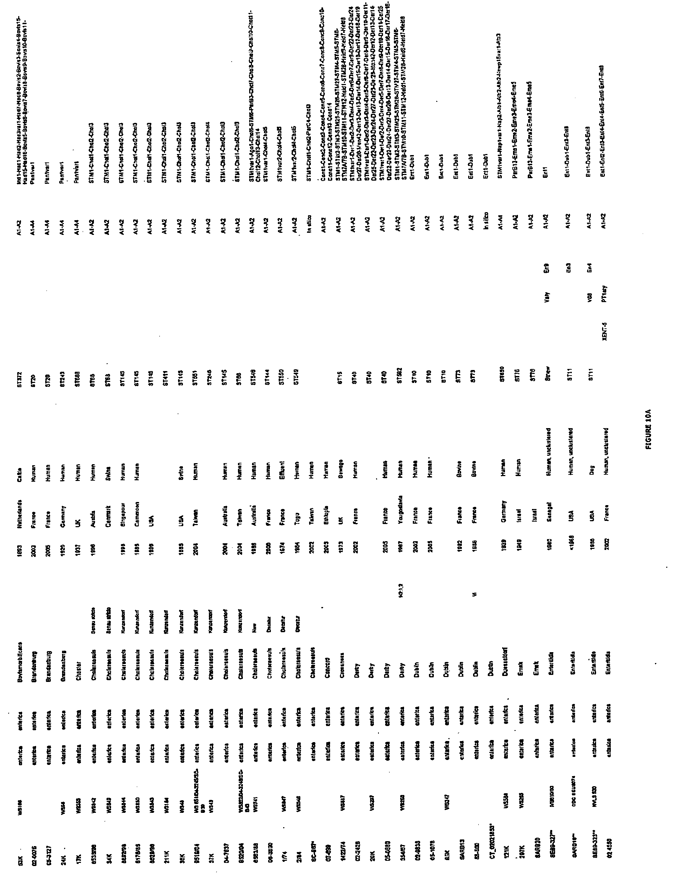

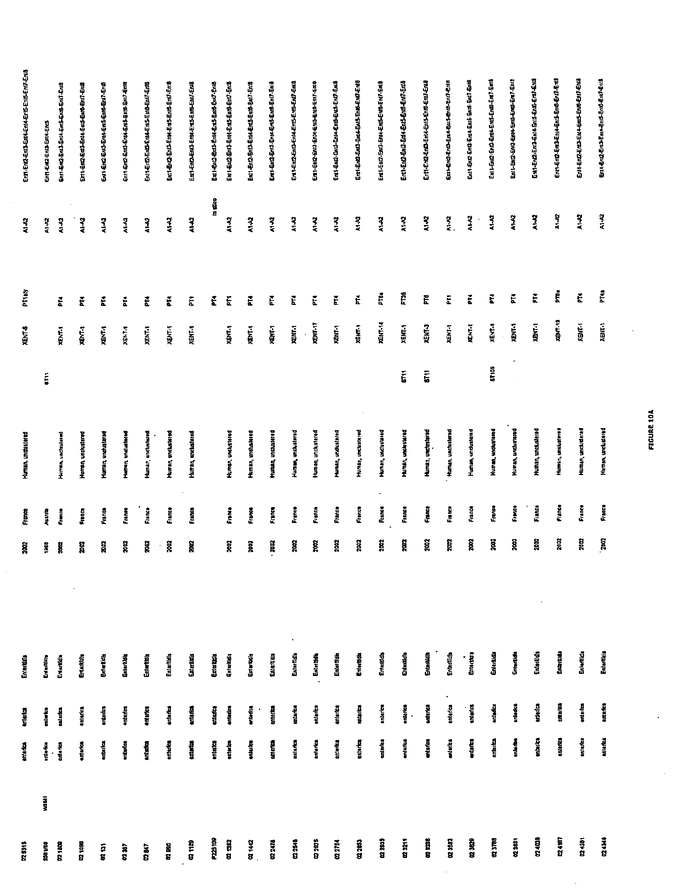

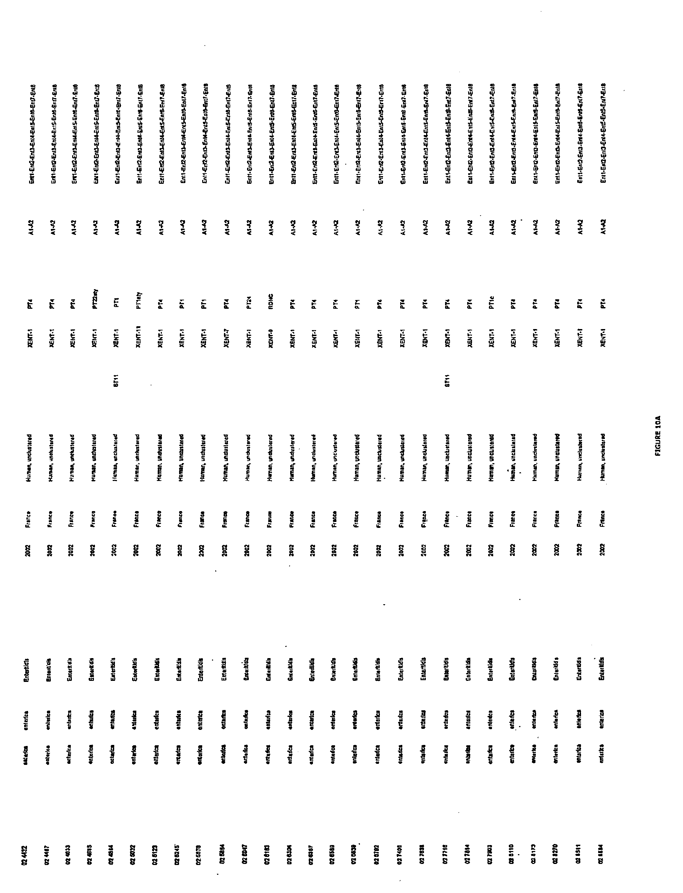

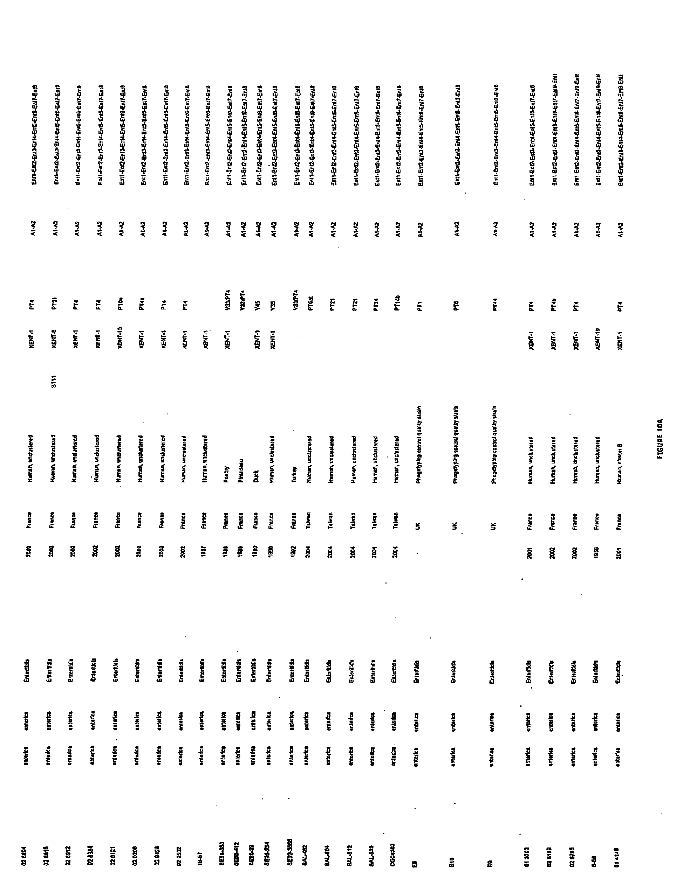

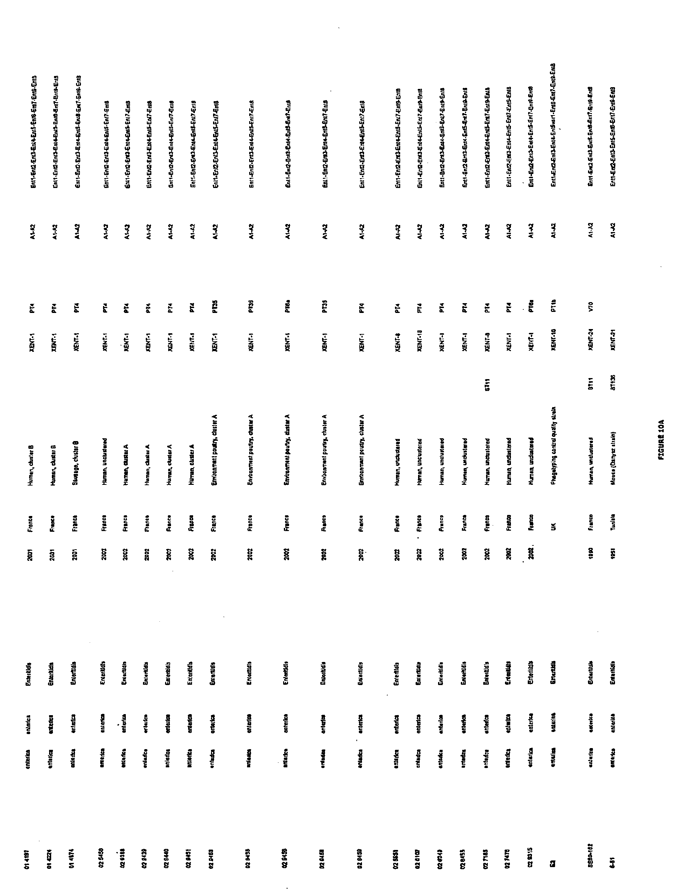

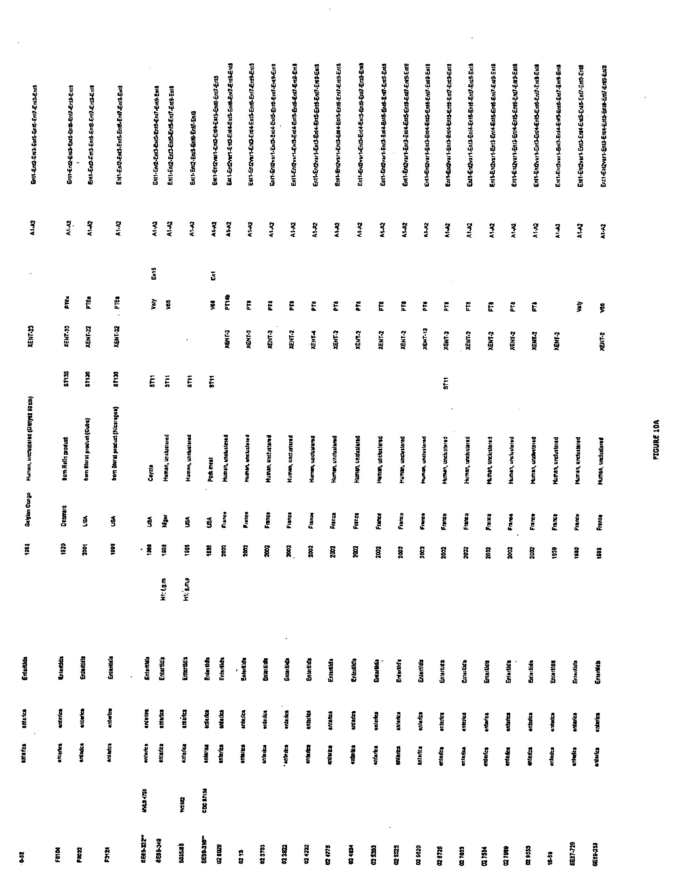

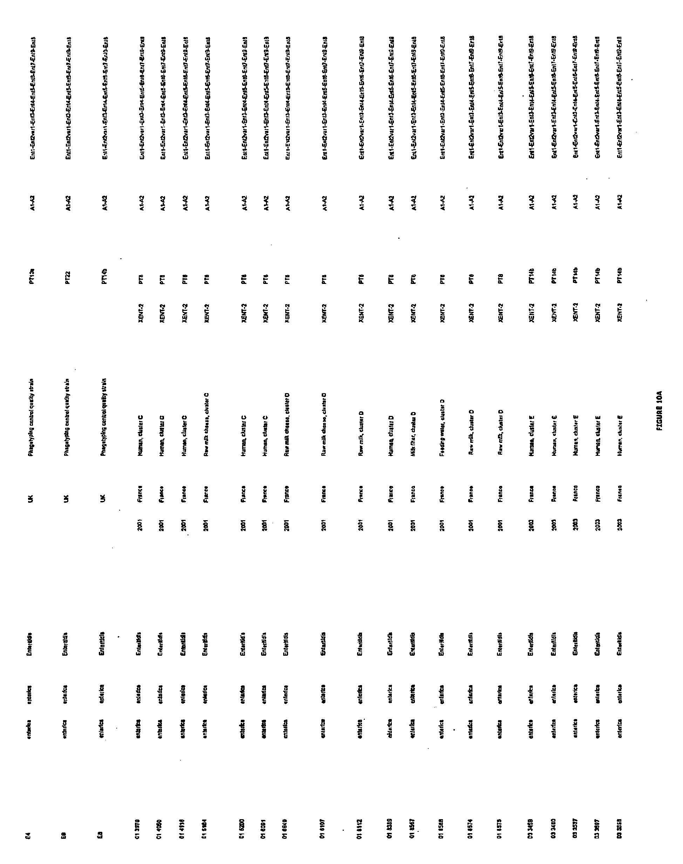

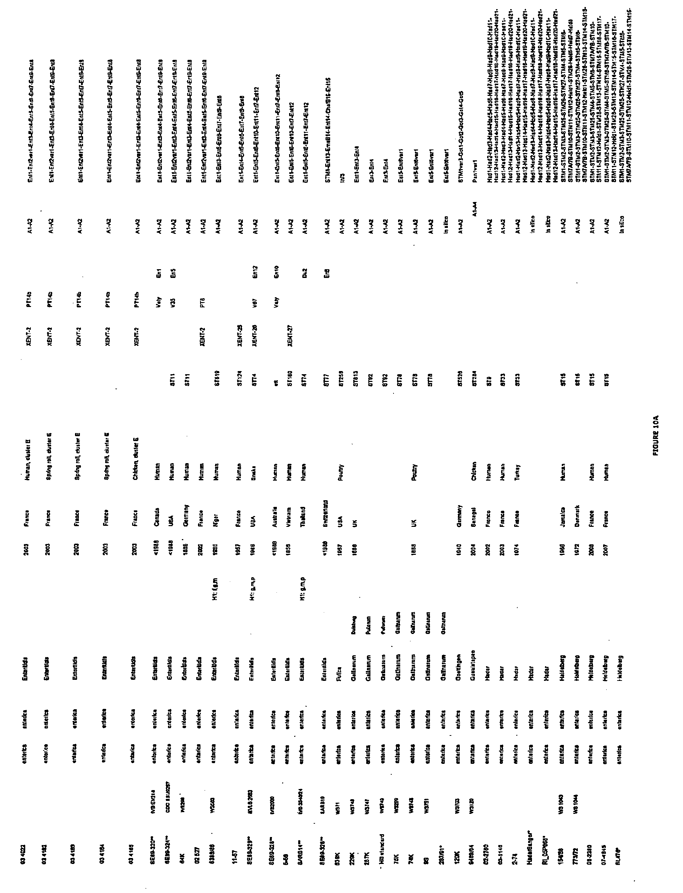

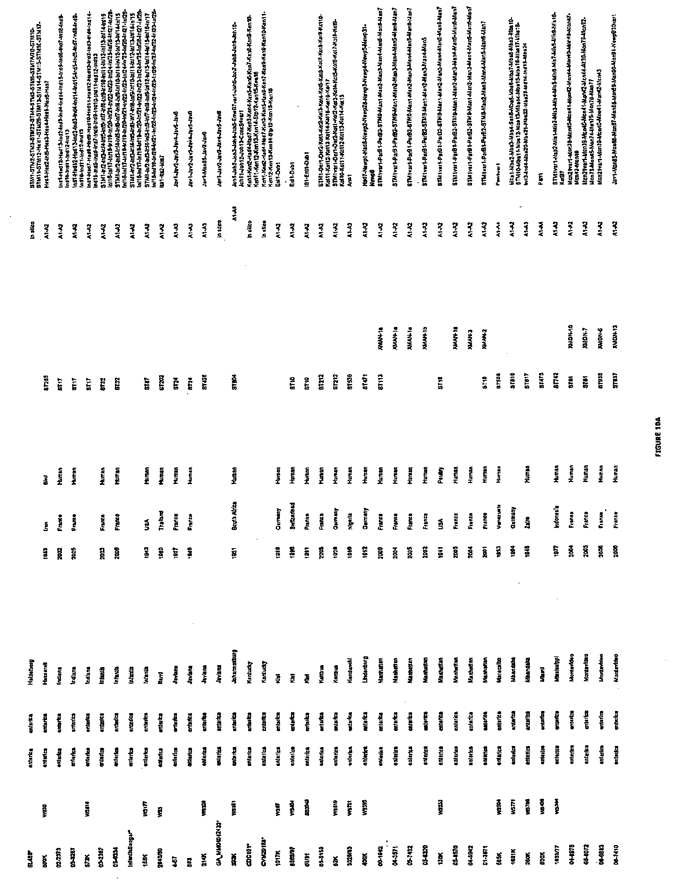

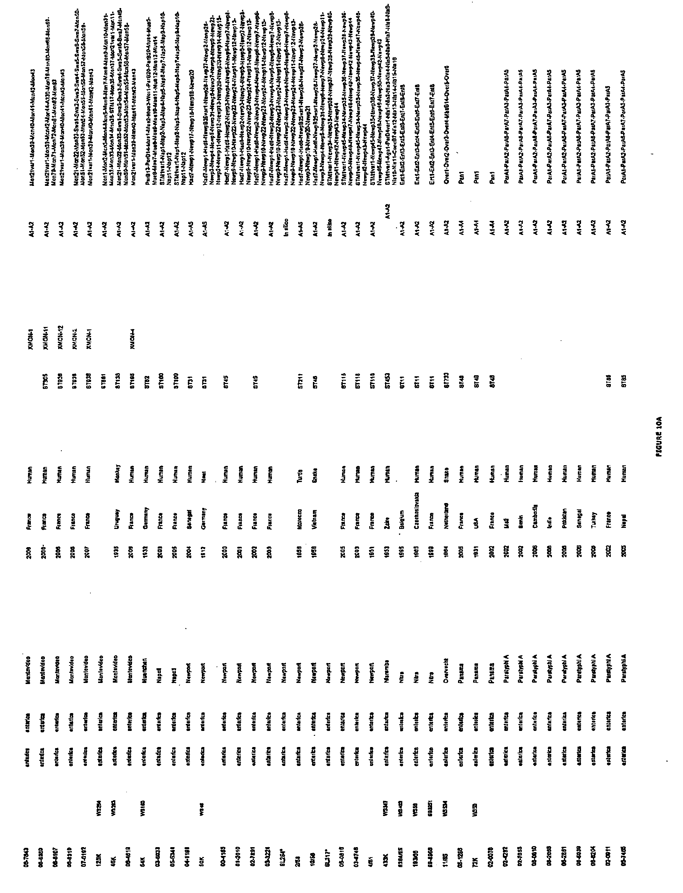

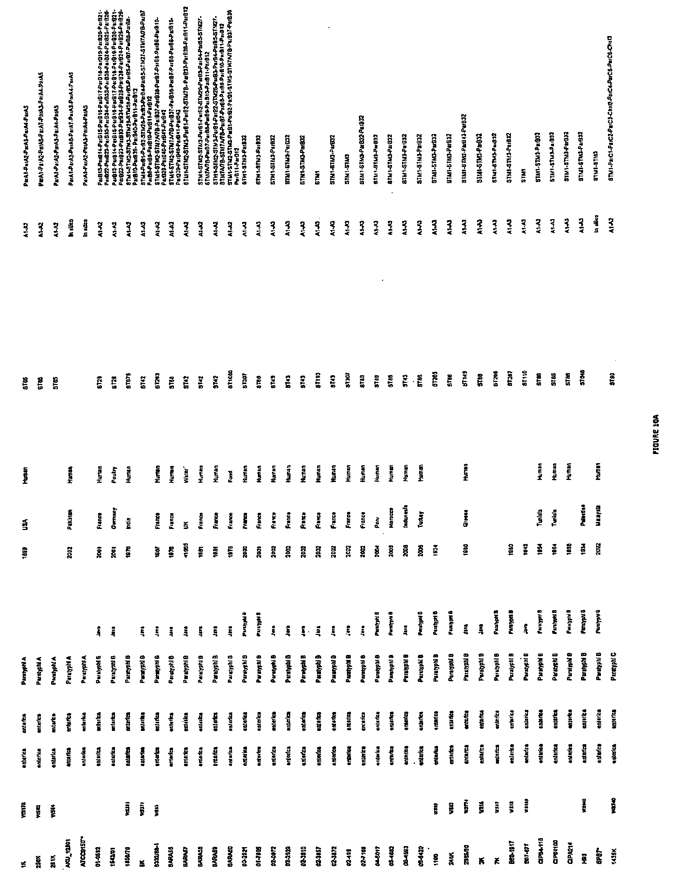

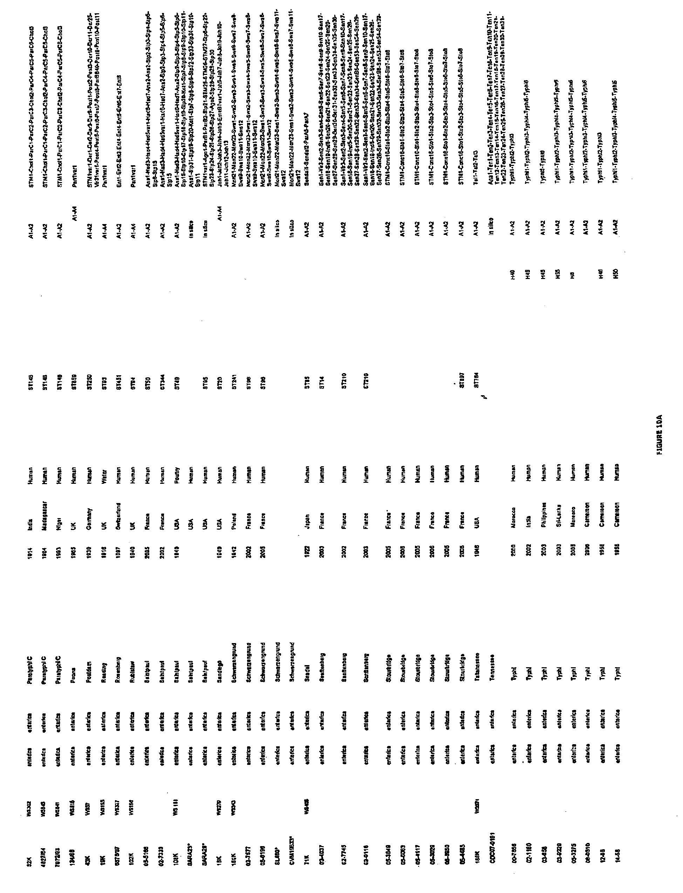

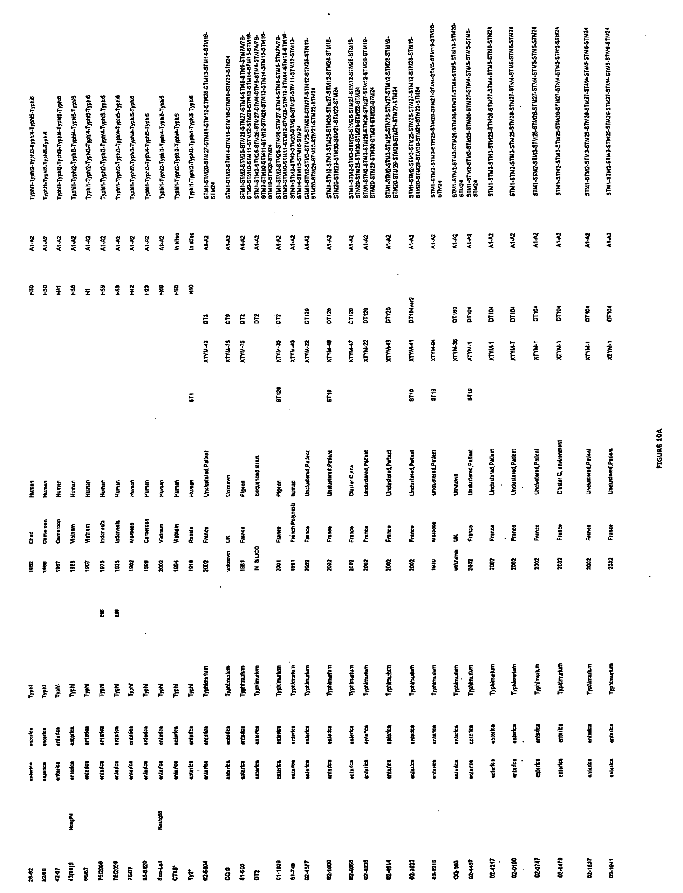

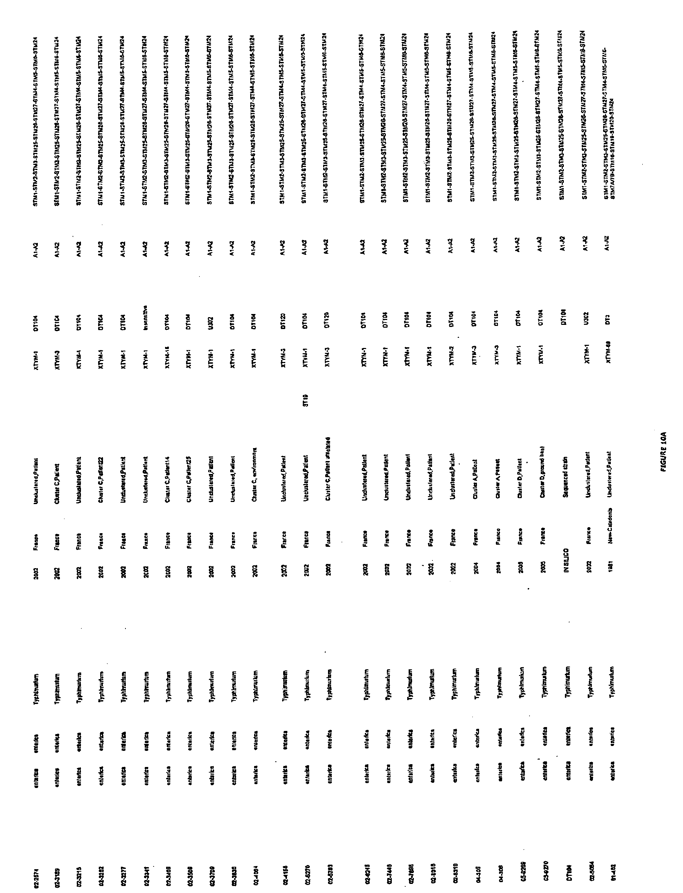

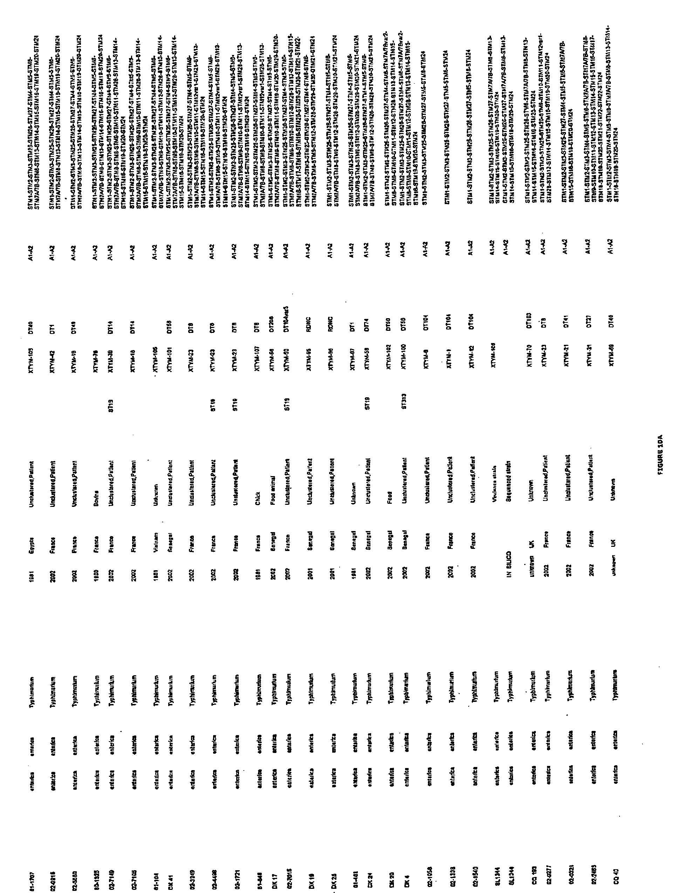

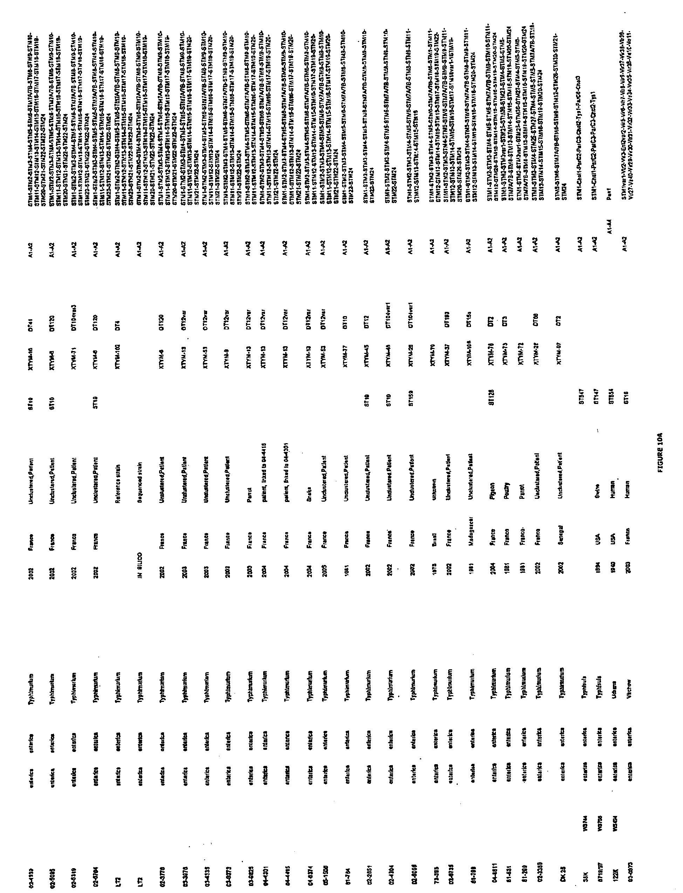

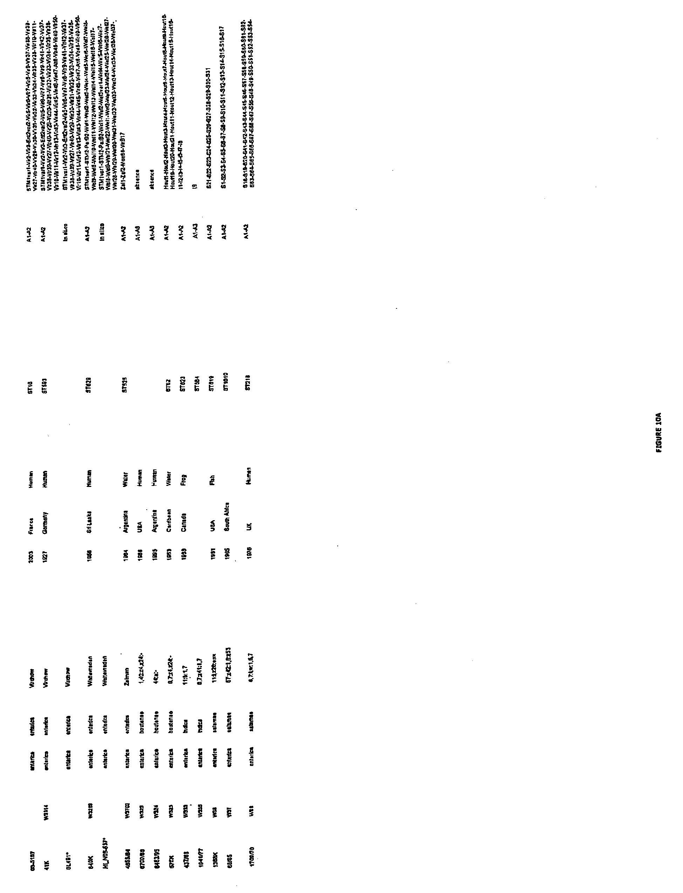

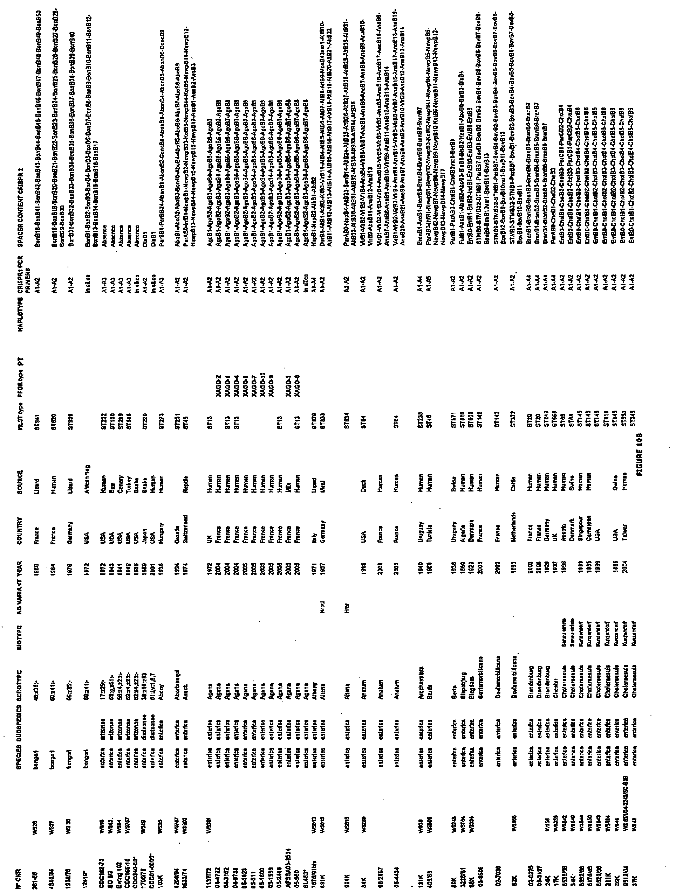

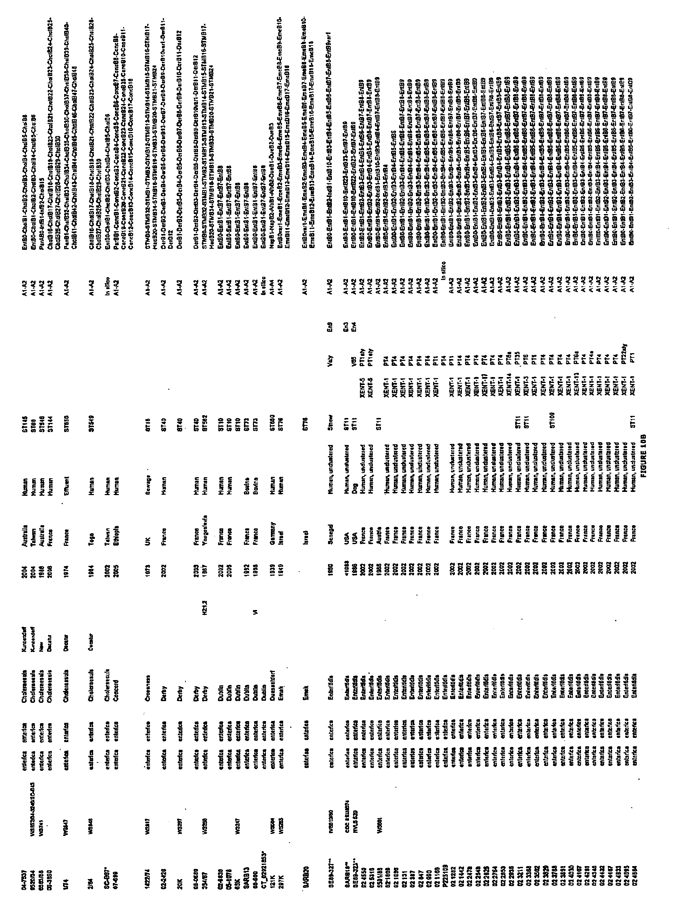

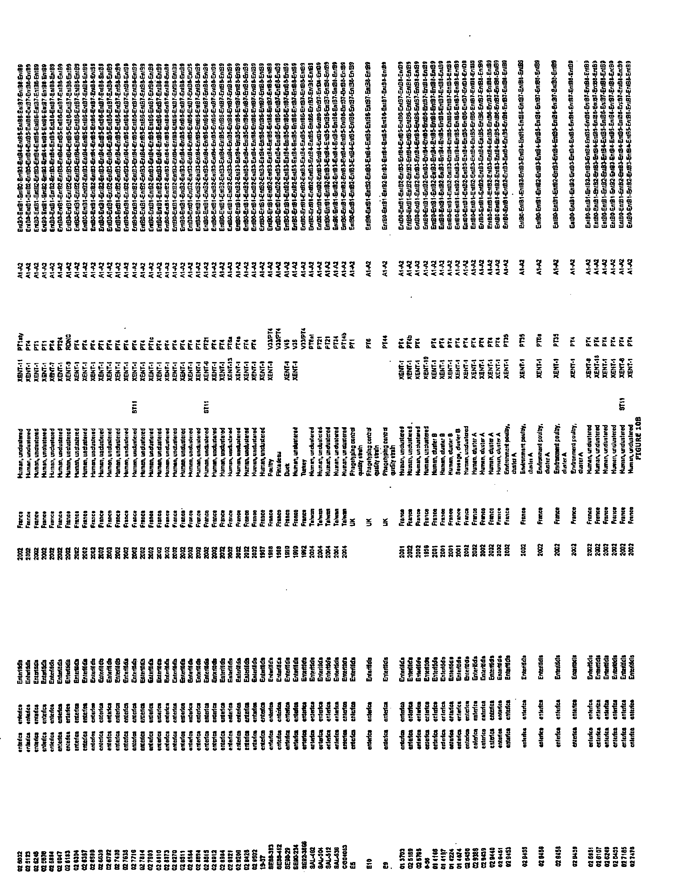

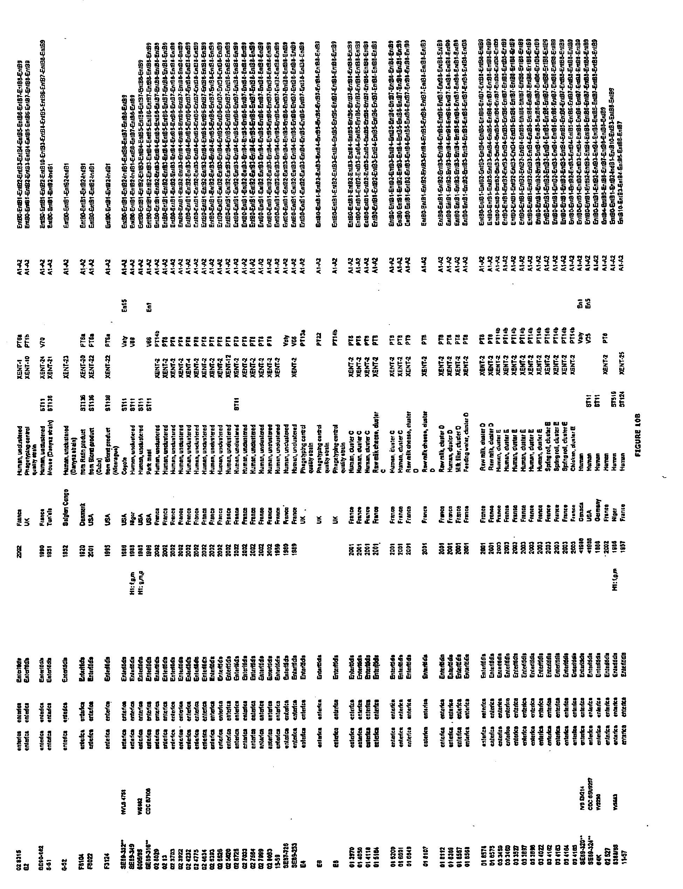

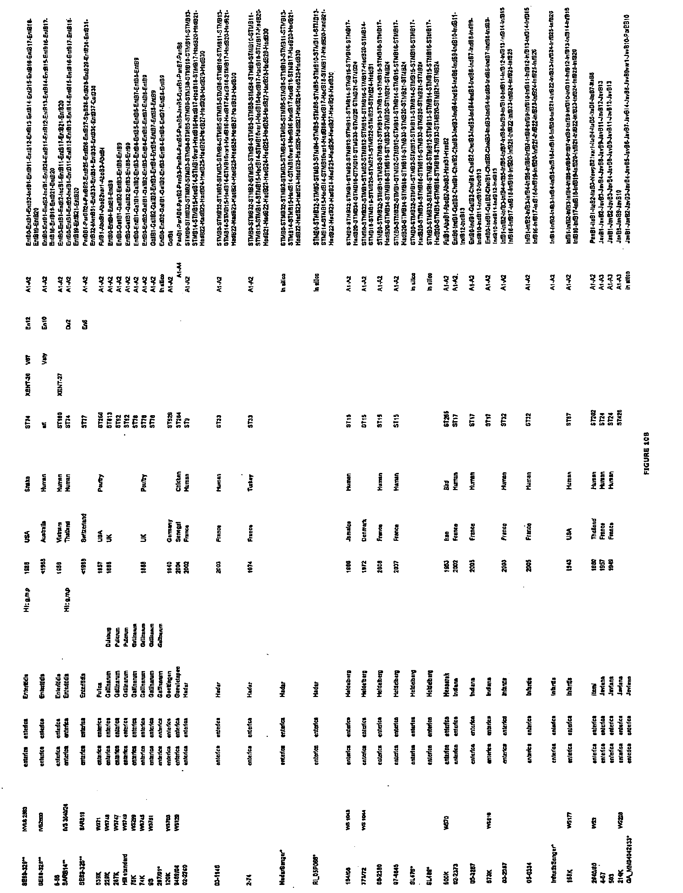

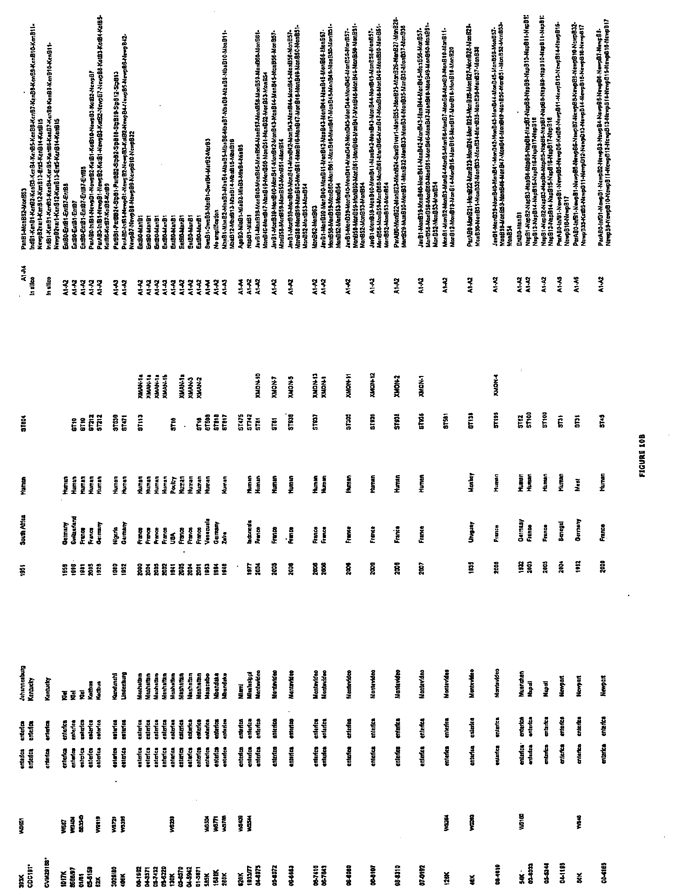

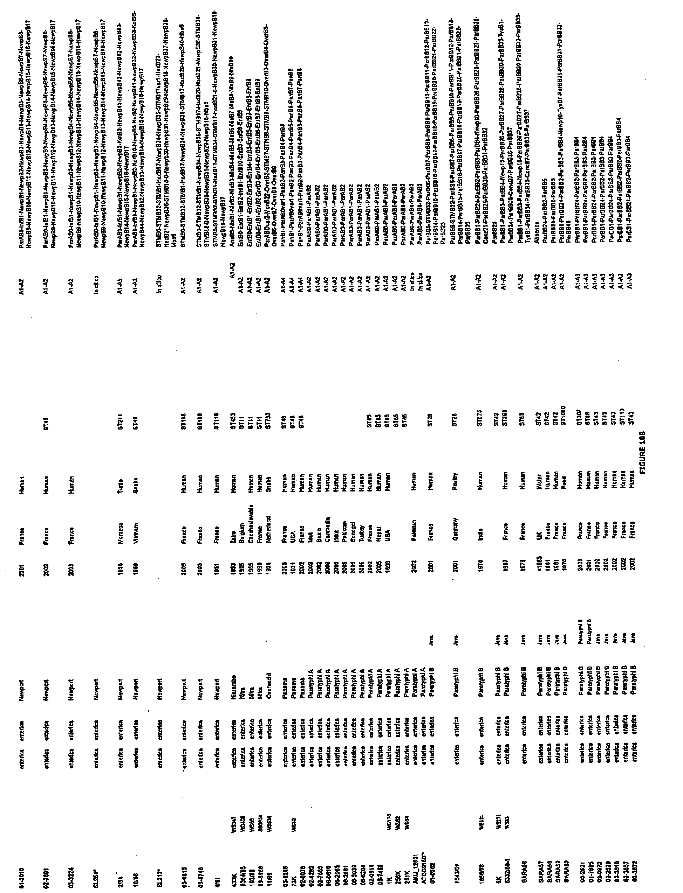

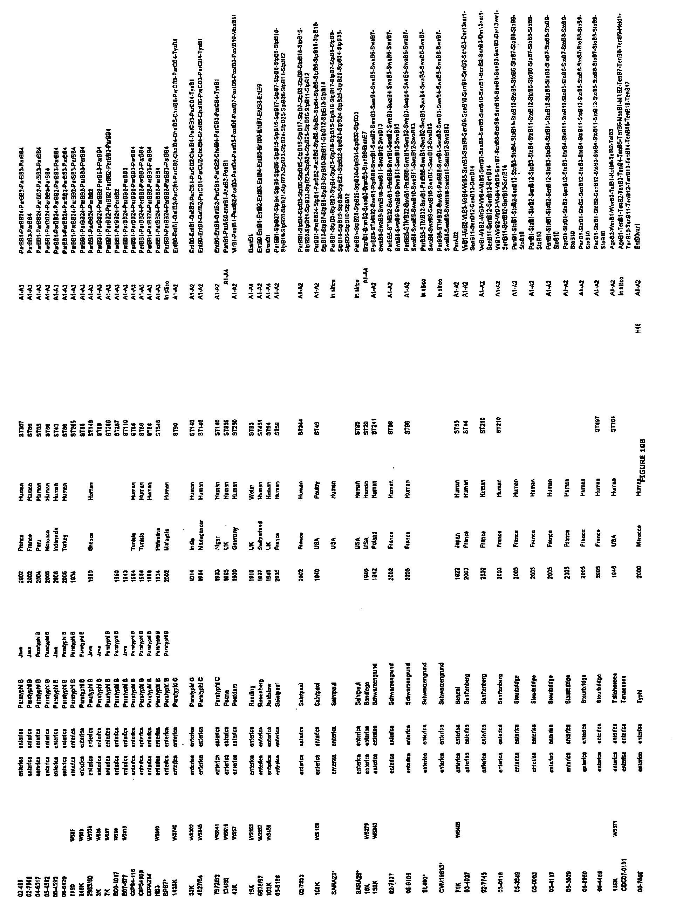

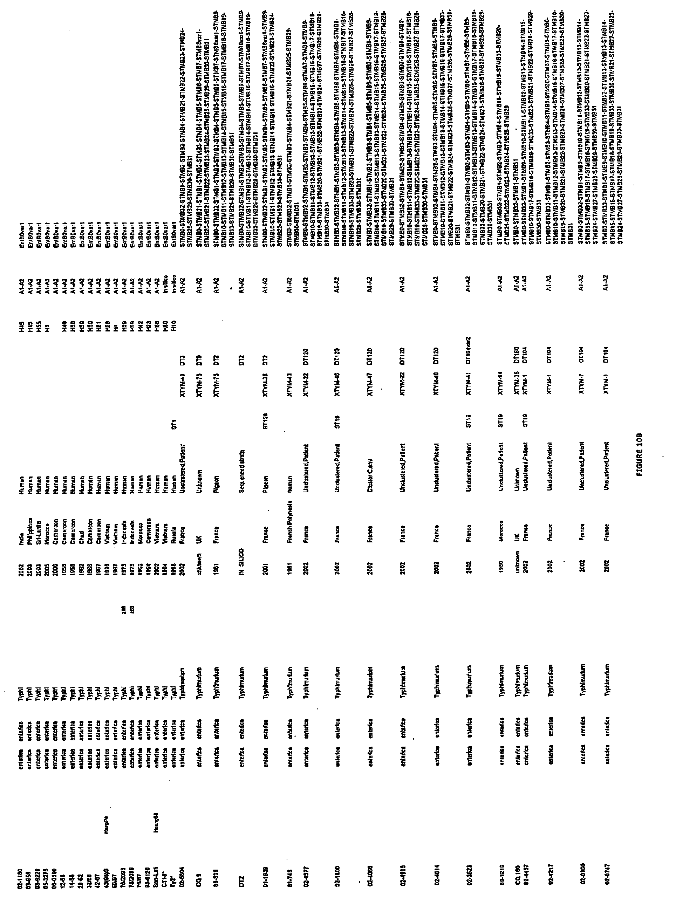

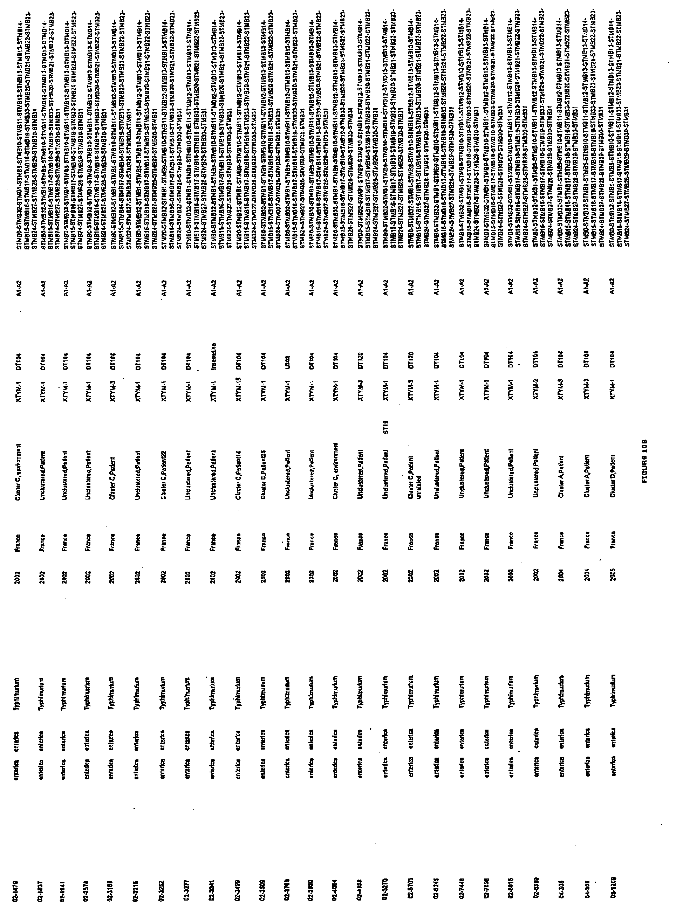

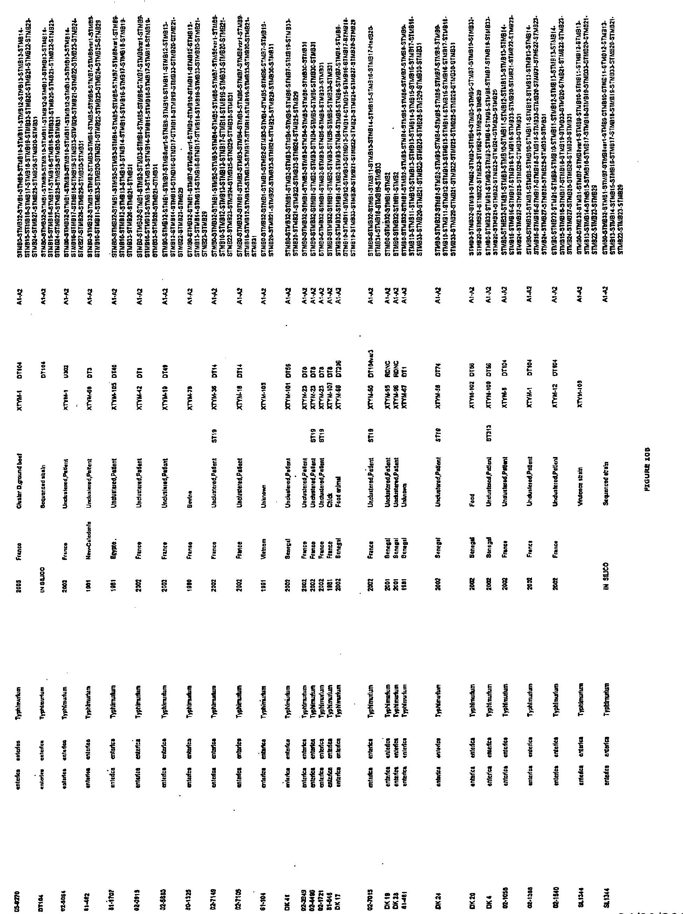

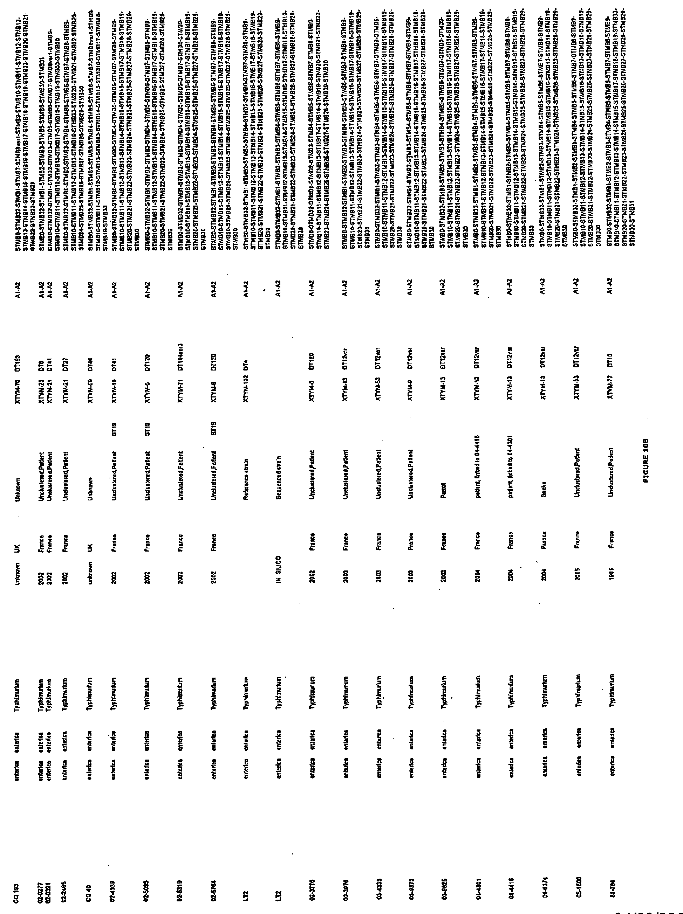

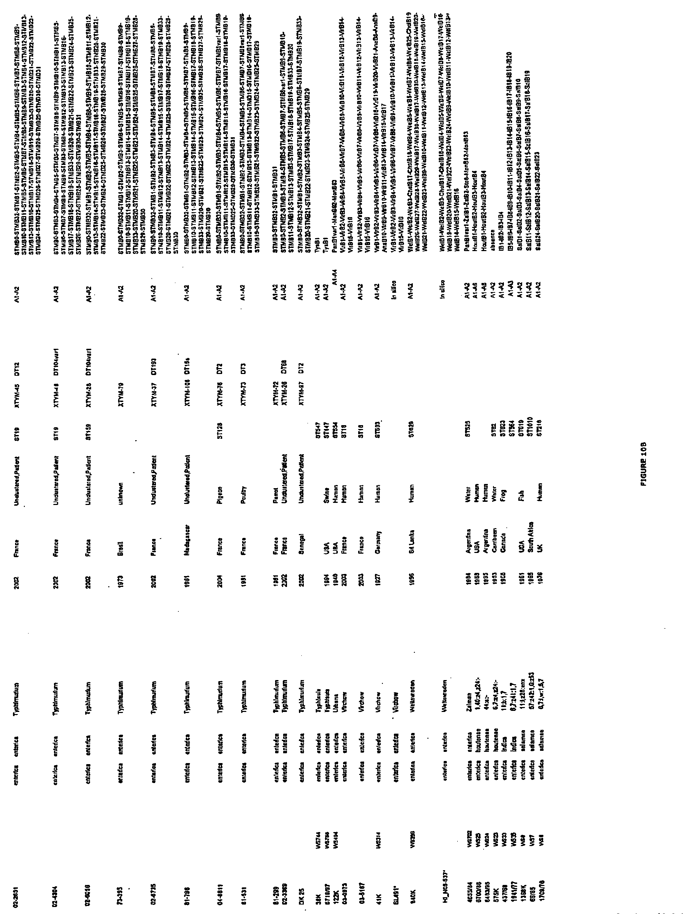

La présente invention a donc pour objet une méthode *in vitro* de typagé et/ou de sous-typage moléculaire d'une bactérie du genre *Salmonella,* à partir d'un échantillon, laquelle méthode est caractérisée en ce qu'elle comprend au moins les étapes suivantes :
(a) l'amplification d'un fragment d'acide nucléique d'une bactérie du genre *Salmonella,* ledit fragment comprenant le locus CRISPR1 et/ou le locus CRISPR2, à l'aide d'au moins un ensemble d'amorces, lesquelles amorces présentent une taille inférieure ou égale à 50 nucléotides, lesdits ensembles d'amorces étant choisis dans le groupe constitué par :
   - un ensemble d'amorces « A » apte à amplifier un fragment d'acide nucléique comprenant le locus CRISPR1, comprenant au moins une amorce sens A1 constituée d'une séquence oligonucléotidique présentant au moins 70%, de préférence, 80%, 85%, 95%, de préférence encore 99% d'identité avec, ou identique à un fragment de la séquence génomique d'une bactérie du genre *Salmonella* situé dans une région de 1000 pb en position 5' du locus CRISPR1, ledit fragment de la séquence génomique situé en position 5' du locus CRISPR1 étant de même taille que ladite amorce A1, et au moins une amorce antisens A2 constituée d'une séquence oligonucléotidique présentant 70%, de préférence, 80%, 85%, 95%, de préférence encore 99% d'identité avec, ou identique à un fragment de la séquence génomique complémentaire de la séquence génomique d'une bactérie du genre *Salmonella* situé en position 3' du locus CRISPR1 et en position 5' du locus CRISPR2, de préférence dans une région de 1000 pb en postion 3' du locus CRISPR1, ledit fragment de la séquence génomique complémentaire étant de même taille que ladite amorce A2 ;
   - un ensemble d'amorces « B » apte à amplifier un fragment d'acide nucléique comprenant le locus CRISPR2, comprenant au moins une amorce sens B1 constitué d'une séquence oligonucléotidique présentant au moins 70%, de préférence, 80%, 85%, 95%, de préférence encore 99% d'identité avec, ou identique à un fragment de la séquence génomique d'une bactérie du genre *Salmonella* situé en position 3' du locus CRISPR1 et en position 5' du locus CRISPR2, de préférence dans une région de 1000 pb en position 5' du locus CRISPR2, ledit fragment de la séquence génomique situé en position 3' du locus CRISPR1 et en position 5' du locus CRISPR2 étant de même taille que ladite amorce B1, et au moins une amorce antisens B2 constituée d'une séquence oligonucléotidique présentant au moins 70%, de préférence 80%, 85%, 95%, de préférence encore 99% d'identité avec, ou identique à un fragment de la séquence génomique complémentaire de la séquence génomique d'une bactérie du genre *Salmonella* situé dans une région de 1000 pb en position 3' du locus CRISPR2, ledit fragment de la séquence génomique complémentaire étant de même taille que ladite amorce B2 ;
   - un ensemble d'amorces « C » apte à amplifier un fragment d'acide nucléique comprenant le locus CRISPR1 et le locus CRISPR2, comprenant au moins une amorce sens A1 telle que définie ci-dessus et au moins un amorce antisens B2 telle que définie ci-dessus ;
(b) la détermination de la composition en séquences variables du locus CRISPR1 et/ou du locus CRISPR2 compris dans les fragments d'acide nucléique amplifiés à l'étape (a) ; et
(c) la comparaison de ladite composition en séquences variables des loci CRISPR1 et/ou CRISPR2 avec une base de référence fournissant la composition en séquences variables des loci CRISPR1 et CRISPR2 de types et sous-types de bactéries du genre *Salmonella* listée aux figures 10 et 9, ou une partie de cette base.

On entend par « typage moléculaire » la détermination du sérotype (ou sérovar) d'une bactérie du genre *Salmonella* appartenant aux deux espèces et six sous-espèces telles que décrites ci-dessus, par caractérisation d'une partie de son génome (c'est-à-dire les loci CRISPR1 et/ou CRISPR2).

On entend par «sous-typage moléculaire » la détermination du sous-type d'une souche bactérienne appartenant à un sérotype de *Salmonella* donné, par caractérisation d'une partie de son génome (c'est-à-dire les loci CRISPR1 et/ou CRISPR2). L'identification du sous-type d'une souche bactérienne de *Salmonella* est importante, par exemple pour reconnaître les foyers d'infection, détecter une transmission croisée de *Salmonella,* déterminer la source de l'infection ou reconnaître plus particulièrement les souches virulentes de *Salmonella.*

On entend par « locus CRISPR » une séquence d'ADN génomique composée d'une série de séquences nucléotidiques répétées (dénommées DR) d'une taille d'environ 21 à 37 paires de bases, espacées par des séquences nucléotidiques variables (espaceurs) d'une taille d'environ 20 à 40 paires de bases. Les bactéries du genre *Salmonella* possèdent un ou deux loci CRISPR. Le Tableau 1 suivant montre la localisation des loci CRISPR1 et CRISPR2 de quelques souches de bactéries du genre *Salmonella :*

| Souches et numéros d'accession dans la base de données GenBank référençant le génome des souches | Localisation du locus CRISPR1 dans le génome des bactéries, en réference au numéro d'accession dans la base de données . GenBank | Localisation du locus CRISPR2 dans le génome des bactéries, en réference au numéro d'accession dans la base de données GenBank |
|---|---|---|
| *S. enterica subsp enterica* serotype : | | |
| Typhimurium strain LT2 | nucléotides 1183-2719 | nucléotides 18852 (AE008834) - 863 (AE008835) |
| AE008834 (GI : 16421485) et AE008835 (GI : 16421501), en date du 9 août 2005 | | |
| Typhi souche CT18 | nucléotides 224182-224567 | nucléotides 241124-241212 |
| AL627276 (en date du 14 novembre 2006 ; GI : 16503805) | | |
| Typhi souche Ty2 | nucléotides 2912041-2912461 | nucléotides 2929018-2929106 |
| AE014613 (en date du 17 janvier 2006 ; GI:29140506) | | |
| Paratyphi A souche ATCC 9150 | nucléotides 2889569-2889902 | nucléotides 2906454-2906664 |
| CP000026 (en daté du 8 avril 2005 ; GI:56126533) | | |
| Paratyphi A souche AKU_12601 | nucléotides 2885105-2885559 | nucléotides 2902112-2902322 |
| FM200053 (en date du 23 octobre 2008 ; GI:197092687) | | |
| Enteritidis souche P225109 | nucléotides 2961370-2961886 | nucléotides 2978039-2978677 |
| AM933172 (en date du 23 octobre 2008 ; GI:206707319) | | |
| Gallinarum souche 287/91 | nucléotides 2952175-2952327 | nucléotides 2968479-2969117 |
| AM933173 (en date du 8 octobre 2008 ; GI:205271127) | | |
| Dublin souche CT_02021853 | nucléotides 3121101-3121251 | nucléotides 3137410-33137742 |
| CP001144 (en date du 5 septembre 2008 ; GI:197936256) | | |
| Choleraesuis souche SC-B67 | nucléotides 3031533-3031805 | nucléotides 3049245-3049698 |
| AE017220 (en date du 11 janvier 2006 ; GI:62126203) | | |
| Newport souche SL254 | nucléotides 3054859-3056473 | nucléotides 3073143-3074328 |
| CP001113 (en date du 25 juillet 2008 ; GI:194400866 | | |
| Agona souche SL483 | nucléotides 2988105 à 2989231 | nucléotides 3005518 à 006033 |
| CP001138 (en date du 22 août 2008 ; GI:197211055) | | |
| Heidelberg souche SL476 | nucléotides 3051217-3052879 | nucléotides 3069138-3070263 |
| CP001120 (en date du 25 juillet 2008 ; GI:194405610) | | |
| Schwarzengrund souche CVM19633 | nucléotides 2981949-2982709 | nucléotides 2999470-3000534 |
| CP001127 (en date du 30 juillet 2008 ; GI:194709404) | | |
| Paratyphi B souche SPB7 | nucléotides 3041329-3041479 | nucléotides 3050805-3051137 |
| CP000886 (en date du 15 janvier 2008 ; GI:161361677) | | |

| *S*. *enterica* subsp *arizonae* serotype | | |
|---|---|---|
| 62:z4,z23:- souche CDC346-86 | nucléotides 25560-25471 | nucléotides 17801-1773 |
| CP000880 (en date du 15 janvier 2008 ; GI:160863331) | | |

Dans le cadre de l'exposé de la présente invention, le locus CRISPR1 est défini comme étant situé en position 5' par rapport au locus CRISPR2.

Sauf précision contraire, les pourcentages d'identité indiqués dans le cadre de l'exposé de la présente invention sont calculés sur une fenêtre de comparaison constituée par la totalité de la séquence de l'amorce comme séquence de référence. Le pourcentage de nucléotides identiques peut être calculé par l'homme du métier en utilisant un programme informatique de comparaison de séquences.

La base de référence selon la présente invention indique la composition en séquences variables des loci CRISPR1 et/ou CRISPR2 des 564 souches de *Salmonella* analysées par les Inventeurs ou d'une partie de ces souches. Ladite base de référence inclut notamment les souches de *Salmonella* d'intérêt clinique et alimentaire actuellement connues. Bien entendu, ladite base de référence peut être complétée par la composition en séquences variables de nouvelles souches bactériennes de *Salmonella.*

De manière avantageuse, la méthode selon la présente invention permet non seulement d'obtenir un fragment d'ADN génomique comprenant l'ensemble d'un ou des deux loci CRISPR d'une bactérie du genre *Salmonella,* de manière à déterminer la composition en séquences variables de ces loci pour pouvoir la comparer à ladite base de référence et ainsi déterminer le type et/ou le sous-type moléculaire de la bactérie, mais aussi d'identifier de nouvelles souches de *Salmonella* en déterminant la composition en séquences variables de ces loci CRISPR.

L'identité de la composition en séquences variables déterminée à l'étape (b) de la méthode selon l'invention avec une composition figurant dans ladite base de référence est ainsi indicative du type et/ou du sous-type de la bactérie du genre *Salmonella* présente dans l'échantillon.

Par « détermination de la composition en séquences variables du locus CRISPR1 et/ou du locus CRISPR2 », on entend la détermination du nombre, de la séquence nucléotidique et optionnellement de l'ordre des espaceurs dans le locus CRISPR1 et/ou CRISPR2 de *Salmonella.*

Dans un mode de mise en oeuvre particulier, les bactéries du genre *Salmonella* pour lesquelles la méthode de typage et/ou de sous-typage moléculaire selon l'invention est réalisée, sont choisies dans le groupe constitué par :
i) l'espèce *S. enterica*
   - sous-espèce *enterica ;* en particulier les sérotypes Abony, Abortusequi, Aesch, Agona, Albany, Altona, Anatum, Arechavalata, Bardo, Berta, Bispebjerg, Blegdam, Bovismorbificans, Brandenburg, Chester, Choleraesuis variété Decatur, Choleraesuis, Choleraesuis variété Kunzendorf, Choleraesuis sensu stricto, Concord, Crossness, Derby, Dublin, Duesseldorf, Emek, Enteritidis, Fulica, Gallinarum variété Duisburg, Gallinarum variété Gallinarum, Gallinarum variété Pullorum, Goettingen, Gueuletapee, Hadar, Heidelberg, Hessarek, Indiana, Infantis, Itami, Javiana, Johannesburg, Kentucky, Kiel, Kottbus, Kundunchi, Lindenburg, Manhattan, Maracaibo, Mbandaka, Miami, Mississippi, Montevideo, Muenchen, Napoli, Newport, Niarembe, Nitra, Overvecht, Panama, Paratyphi A, Paratyphi B, Paratyphi C, Poona, Postdam, Reading, Rosenberg, Rubislaw, Saintpaul, Sandiego, Schwarzengrund, Sendai, Senftenberg, Stourbridge, Tallahassee, Tennessee, Typhi, Typhimurium, Typhisuis, Urbana, Virchow, Weltevreden, Zaiman,
   - sous-espèce *salamae* ; en particulier les sérotypes 11:1,z28:e,n,x ; 57:z42:1,6:Rz53 ;
   - sous-espèce *arizonae ;* en particulier les sérotypes 62:z4,z23:- ; 53:g,z51:- ; 56:z4,z23:- ; 17:z29:- ;
   - sous-espèce *diarizonae ;* en particulier les sérotypes 38:z10:z53 ; 61:1,v:1,5,7 ;
   - sous-espèce *houtenae ;* en particulier les sérotypes 6,7:z4,z24:- ; 44:a:- ; 1,40:z4,z24:- ;
   - sous-espèce *indica ;* en particulier les sérotypes 11:b:1,7 ; 6,7:z41:1,7 ;
ii) l'espèce *bongori ;* en particulier les sérotypes 60:z41:- ; 66:z35:- ; 48:z35:- ; 66:z41:-.

Les termes « acide nucléique » et « ADN » sont équivalents et comprennent les acides nucléiques simple ou double brin.

Selon un mode de mise en oeuvre avantageux de la méthode conforme à l'invention, le fragment d'acide nucléique à amplifier à l'étape (a) ci-dessus, présente une taille comprise entre 400 à 20 000 pb.

Selon un mode de mise en oeuvre de la méthode conforme à l'invention, ladite amplification peut être réalisée par toute méthode connue de l'Homme du métier. De préférence, l'amplification peut être réalisée par une méthode sélectionnée dans le groupe constitué par : la réaction en chaîne par polymérase (PCR), réaction en chaîne par ligase (LCR), « nucleic acid sequence-based amplification » (NASBA), « cycling probe technology » (CPT), PCR nichée et PCR multiplexe.

Selon un mode de mise en oeuvre avantageux de la méthode conforme à l'invention, ledit fragment de la séquence génomique d'une bactérie du genre *Salmonella* situé en position 5' du locus CRISPR1, et/ou ledit fragment de la séquence génomique complémentaire de la séquence génomique d'une bactérie du genre *Salmonella* situé en position 3' du locus CRISPR2, se situent à une distance du locus CRISPR1 ou CRISPR2 inférieure à 500 pb, de préférence inférieure à 100 pb.

Le terme « amorce » désigne un oligonucléotide simple brin ou double brin, de préférence simple brin pour une efficacité optimale. Une amorce (oligonucléotidique), une fois hybridée à une séquence d'acide nucléique simple brin, dite « matrice », est un substrat d'au moins une ADN polymérase (c'est-à-dire qu'une amorce hybridée à une séquence d'acide nucléique possède la propriété de fixer à son extrémité 3'OH au moins une ADN polymérase). En présence de nucléotides adéquats (A, C, G, T), d'une ADN polymérase (par exemple la Taq polymérase), d'un tampon adéquat (comprenant des co-facteurs ou des composés affectant le pH ou la force ionique du milieu réactionnel par exemple) et à une température adéquate, l'extrémité 3'OH d'une amorce peut être allongée, conduisant ainsi à la synthèse d'un brin complémentaire de la séquence matrice sur laquelle est hybridée ladite amorce. Les amorces, selon la présente invention, présentent de préférence une longueur inférieure ou égale à 50 nucléotides, c'est-à-dire inférieure ou égale à 40, 30, 20, 15 ou 10 nucléotides. De préférence encore, les amorces selon la présente invention présentent une longueur comprise entre 15 et 30 nucléotides, de préférence entre 18 et 25 nucléotides. Les amorces selon la présente invention peuvent être avantageusement marquées.

Au sens de la présente invention, deux molécules d'acide nucléique sont « complémentaires » lorsque chacune des bases en positions successives de l'extrémité 5' de la première molécule d'acide nucléique est appariée au résidu correspondant dans la seconde molécule, partant de l'extrémité 3', selon les règles d'appariement des paires de base (*i.e.* A et T, C et G). Dans des conditions appropriées connues de l'Homme du métier, deux simples brins complémentaires d'ADN se réassocient pour former une molécule d'ADN double brin.

Selon un mode de mise en oeuvre particulier, l'ensemble d'amorces permettant d'amplifier le fragment d'acide nucléique comprenant le locus CRISPR1 et/ou le locus CRISPR2 est sélectionnée dans le groupe constitué par :
- l'ensemble d'amorces, apte à amplifier le locus CRISPR1, constitué de l'amorce de séquence SEQ ID NO : 1326 (SALCRISP1-FB) associée à au moins une des amorces de séquence SEQ ID NOs : 1327 (SALCRISP1-RB), 1328 (AriParaB-R), 1329 (BrPanCR1-R), 2151 (50K) et 2152 (HoutWS24R),
- l'ensemble d'amorces, apte à amplifier le locus CRISPR2, constitué de l'amorce de séquence SEQ ID NO : 1330 (SALCRISP2-FB), associée à au moins une des amorces de séquence SEQ ID NOs : 1331 (SALCRISP2-RA) et 1332 (SALCRISP2-RB), et
- l'ensemble d'amorces, apte à amplifier simultanément les deux loci CRISPR1 et CRISPR2, constitué de l'amorce de séquence SEQ ID NO : 1326, associée à au moins une des deux amorces de séquence 1331 et 1332 (les amorces de séquence SEQ ID NOs : 1326 et 1331 se situant à moins de 100 bp des loci CRISPR1 et CRISPR2 respectivement et l'amorce de séquence SEQ ID NO :1332 se situant à moins de 500 pb du locus CRISPR2).

Par conséquent, la présente invention a également pour objet un ensemble d'amorces, tel que défini ci-dessus, apte à amplifier un fragment de la séquence génomique d'une bactérie du genre *Salmonella.*

Dans un mode de mise en oeuvre particulier selon l'invention, l'amorce sens A1 est constituée d'une séquence oligonucléotidique présentant au moins 70%, de préférence, 80%, 85%, 95%, de préférence encore 99% d'identité avec, ou identique à un fragment de la séquence génomique de *S. enterica* sérotype Typhimurium LT2 ou de *S. enterica* sérotype Typhi CT18 situé dans une région de 1000 pb en position 5' du locus CRISPR1 (par exemple pour la souche LT2, la région correspondant aux nucléotides 183 à 1183 de la séquence génomique disponible sous le numéro AE008834 (GI : 16421485)), ledit fragment de la séquence génomique situé en position 5' du locus CRISPR1 étant de même taille que ladite amorce A1.

Dans un mode de mise en oeuvre particulier selon l'invention, l'amorce antisens A2 est constituée d'une séquence oligonucléotidique présentant 70%, de préférence, 80%, 85%, 95%, de préférence encore 99% d'identité avec, ou identique à un fragment de la séquence génomique complémentaire de la séquence génomique de *S. enterica* sérotype Typhimurium LT2 ou de *S. enterica* sérotype Typhi CT18 qui est situé en position 3' du locus CRISPR1 et en position 5' du locus CRISPR2 (par exemple pour la souche LT2, la région correspondant aux nucléotides 2727 à 18836, de préférence 2727 à 3727, de la séquence génomique disponible sous le numéro AE008834 (GI : 16421485)), ledit fragment étant de la même taille que ladite amorce A2.

Dans un mode de mise en oeuvre particulier selon l'invention l'amorce sens B1 est constitué d'une séquence oligonucléotidique présentant au moins 70%, de préférence, 80%, 85%, 95%, de préférence encore 99% d'identité avec, ou identique à un fragment de la séquence génomique de *S. enterica* sérotype Typhimurium LT2 ou de *S. enterica* sérotype Typhi CT18 qui est situé en position 3' du locus CRISPR1 et en position 5' du locus CRISPR2 (par exemple pour la souche LT2, la région correspondant aux nucléotides 2727 à 18836, de préférence 17836 à 18836, de la séquence génomique disponible sous le numéro AE008834 (GI : 16421485)), ledit fragment de la séquence génomique situé en position 3' du locus CRISPR1 et en position 5' du locus CRISPR2 étant de même taille que ladite amorce.

Dans un mode de mise en oeuvre particulier selon l'invention, l'amorce antisens B2 est constituée d'une séquence oligonucléotidique présentant au moins 70%, de préférence 80%, 85%, 95%, 95%, de préférence encore 99% d'identité avec, ou identique à un fragment de la séquence génomique complémentaire de la séquence génomique de *S. enterica* sérotype Typhimurium LT2 ou de *S. enterica* sérotype Typhi CT18 qui est situé dans une région de 1000 pb en position 3' du locus CRISPR2 (par exemple pour la souche LT2, la région correspondant aux nucléotides 862 à 1862 de la séquence génomique disponible sous le numéro AE008835 (GI : 16421501)), ledit fragment de la séquence génomique complémentaire étant de même taille que ladite amorce

Selon un mode de mise en oeuvre avantageux de la méthode conforme à l'invention, l'étape (b) décrite ci-dessus est réalisée à l'aide d'une méthode de séquençage de l'ADN. A titre d'exemple, le séquençage peut s'effectuer par la méthode Sanger ou de Maxam et Gilbert, qui sont des méthodes bien connues de l'Homme du métier.

Selon un mode de mise en oeuvre avantageux de la méthode conforme à l'invention, l'étape (b) décrite ci-dessus est réalisée par :
(i) hybridation des produits d'amplification avec un, plusieurs ou tous les ensemble(s) de sondes choisi parmi :
   - l'ensemble S1-1, comprenant ou constitué d'au moins une sonde comprenant au moins ou constituée d'au moins 8 nucléotides consécutifs de l'une quelconque des séquences d'acide nucléique SEQ ID NO : 1106 à 1119, 1123 à 1140, 1142, 1143, 1914 et/ou leurs séquences d'acide nucléique complémentaires (séquences nucléotidiques correspondant aux différentes séquences variables du locus CRISPR1 du sérotype Typhimurium) ;
   - l'ensemble S1-2, comprenant ou constitué d'au moins une sonde comprenant au moins ou constituée d'au moins 8 nucléotides consécutifs de l'une quelconque des séquences d'acide nucléique SEQ ID NO: 455, 1144 à 1153, 1155 à 1181 et/ou leurs séquences d'acide nucléique complémentaires (séquences nucléotidiques correspondant aux différentes séquences variables du locus CRISPR2 du sérotype Typhimurium) ;
   - l'ensemble S2-1, comprenant ou constitué d'au moins une sonde comprenant au moins ou constituée d'au moins 8 nucléotides consécutifs de l'une quelconque des séquences d'acide nucléique SEQ ID NO : 256, 378 à 381, 383 à 387, 389 à 391, 1106, 1514, 1528, 2148 à 2153 et/ou leurs séquences d'acide nucléique complémentaires (séquences nucléotidiques correspondant aux différentes séquences variables du locus CRISPR1 du sérotype Enteritidis) ;
   - l'ensemble S2-2, comprenant ou constitué d'au moins une sonde comprenant au moins ou constituée d'au moins 8 nucléotides consécutifs de l'une quelconque des séquences d'acide nucléique SEQ ID NO : 392 à 413, 519, 815, 972, 983, 989, 1529 à 1546, 1952 et/ou leurs séquences d'acide nucléique complémentaires (séquences nucléotidiques correspondant aux différentes séquences variables du locus CRISPR2 du sérotype Enteritidis) ;
   - l'ensemble S3-1, comprenant ou constitué d'au moins une sonde comprenant au moins ou constituée d'au moins 8 nucléotides consécutifs de l'une quelconque des séquences d'acide nucléique SEQ ID NO : 421 à 442, 444, 445, 447 à 450 et/ou leurs séquences d'acide nucléique complémentaires (séquences nucléotidiques correspondant aux différentes séquences variables du locus CRISPR1 du sérotype Hadar) ;
   - l'ensemble S3-2, comprenant ou constitué d'au moins une sonde comprenant au moins ou constituée d'au moins 8 nucléotides consécutifs de l'une quelconque des séquences d'acide nucléique SEQ ID NO : 451 à 465, 1144 à 1147, 1150 à 1152, 1154, 1155, 1158, 1169, 1172, 1175 à 1177, 1179, 1181 et/ou leurs séquences d'acide nucléique complémentaires (séquences nucléotidiques correspondant aux différentes séquences variables du locus CRISPR2 du sérotype Hadar) ;
   - l'ensemble S4-1, comprenant ou constitué d'au moins une sonde comprenant au moins ou constituée d'au moins 8 nucléotides consécutifs de l'une quelconque des séquences d'acide nucléique SEQ ID NO : 65, 382, 1120, 1263 à 1302, 2024 à 2038 et/ou leurs séquences d'acide nucléique complémentaires (séquences nucléotidiques correspondant aux différentes séquences variables du locus CRISPR1 du sérotype Virchow) ;
   - l'ensemble S4-2, comprenant ou constitué d'au moins une sonde comprenant au moins ou constituée d'au moins 8 nucléotides consécutifs de l'une quelconque des séquences d'acide nucléique SEQ ID NO : 83, 93, 94, 1304 à 1324 et/ou leurs séquences d'acide nucléique complémentaires (séquences nucléotidiques correspondant aux différentes séquences variables du locus CRISPR2 du sérotype Virchow) ;
   - l'ensemble S5-1, comprenant ou constitué d'au moins une sonde comprenant au moins ou constituée d'au moins 8 nucléotides consécutifs de l'une quelconque des séquences d'acide nucléique SEQ ID NO : 531 à 562, 1106 et/ou leurs séquences d'acide nucléique complémentaires (séquences nucléotidiques correspondant aux différentes séquences variables du locus CRISPR1 du sérotype Infantis) ;
   - l'ensemble S5-2, comprenant ou constitué d'au moins une sonde comprenant au moins ou constituée d'au moins 8 nucléotides consécutifs de l'une quelconque des séquences d'acide nucléique SEQ ID NO: 563 à 588 et/ou leurs séquences d'acide nucléique complémentaires (séquences nucléotidiques correspondant aux différentes séquences variables du locus CRISPR2 du sérotype Infantis) ;
   - l'ensemble S6-1, comprenant ou constitué d'au moins une sonde comprenant au moins ou constituée d'au moins 8 nucléotides consécutifs de l'une quelconque des séquences d'acide nucléique SEQ ID NO : 1254 à 1259 et/ou leurs séquences d'acide nucléique complémentaires (séquences nucléotidiques correspondant aux différentes séquences variables du locus CRISPR1 du sérotype Typhi) ;
   - l'ensemble S6-2, comprenant ou constitué d'au moins une sonde comprenant au moins ou constituée d'au moins 8 nucléotides consécutifs de la séquence d'acide nucléique SEQ ID NO : 1260 et/ou sa séquence d'acide nucléique complémentaire (séquences nucléotidiques correspondant à la séquence variable du locus CRISPR2 du sérotype Typhi) ;
   - l'ensemble S7-1, comprenant ou constitué d'au moins une sonde comprenant au moins ou constituée d'au moins 8 nucléotides consécutifs de l'une quelconque des séquences d'acide nucléique SEQ ID NO : 448, 449, 860 à 884, 1719 à 1724, 1727 à 1743, 1745 et/ou leurs séquences d'acide nucléique complémentaires (séquences nucléotidiques correspondant aux différentes séquences variables du locus CRISPR1 du sérotype Newport) ;
   - l'ensemble-S7-2, comprenant ou constitué d'au moins une sonde comprenant au moins ou constituée d'au moins 8 nucléotides consécutifs de l'une quelconque des séquences d'acide nucléique SEQ ID NO : 453 à 456, 563, 621, 622, 625, 626, 657, 885 à 896, 906 à 915, 933, 1144, 1145, 1155, 1156, 1172, 1174, 1748 à 1756, 1758, 1759, 1916 et/ou leurs séquences d'acide nucléique complémentaires (séquences nucléotidiques correspondant aux différentes séquences variables du locus CRISPR2 du sérotype Newport) ;
   - l'ensemble S8-1, comprenant ou constitué d'au moins une sonde comprenant au moins ou constituée d'au moins 8 nucléotides consécutifs de l'une quelconque des séquences d'acide nucléique SEQ ID NO : 295 à 299, 301 à 323, 738, 1106 à 1109, 1120, 1123, 1128 à 1131, 1133, 1137 à 1140, 1550, 1159 à 1561 et/ou leurs séquences d'acide nucléique complémentaires (séquences nucléotidiques correspondant aux différentes séquences variables du locus CRISPR1 du sérotype Derby) ;
   - l'ensemble S8-2, comprenant ou constitué d'au moins une sonde comprenant au moins ou constituée d'au moins 8 nucléotides consécutifs de l'une quelconque des séquences d'acide nucléique SEQ ID NO : 324 à 337, 455, 1144, 1145, 1149 à 1153, 1155 à 1160, 1163, 1172 à 1174 et/ou leurs séquences d'acide nucléique complémentaires (séquences nucléotidiques correspondant aux différentes séquences variables du locus CRISPR2 du sérotype Derby) ;
   - l'ensemble S9-1, comprenant ou constitué d'au moins une sonde comprenant au moins ou constituée d'au moins 8 nucléotides consécutifs de l'une quelconque des séquences d'acide nucléique SEQ ID NO : 295, 307, 602 à 614, 616 à 619, 1106, 1120, et/ou leurs séquences d'acide nucléique complémentaires (séquences nucléotidiques correspondant aux différentes séquences variables du locus CRISPR1 du sérotype Kottbus) ;
   - l'ensemble S9-2, comprenant ou constitué d'au moins une sonde comprenant au moins ou constituée d'au moins 8 nucléotides consécutifs de l'une quelconque des séquences d'acide nucléique SEQ ID NO : 563, 620 à 629, 885, 896, 907, 913, 914, 933, et/ou leurs séquences d'acide nucléique complémentaires (séquences nucléotidiques correspondant aux différentes séquences variables du locus CRISPR2 du sérotype Kottbus) ;
   - l'ensemble S10-1, comprenant ou constitué d'au moins une sonde comprenant au moins ou constituée d'au moins 8 nucléotides consécutifs de l'une quelconque des séquences d'acide nucléique SEQ ID NO : 422 à 425, 448 à 450, 504 à 518 et/ou leurs séquences d'acide nucléique complémentaires (séquences nucléotidiques correspondant aux différentes séquences variables du locus CRISPR1 du sérotype Indiana) ;
   - l'ensemble S10-2, comprenant ou constitué d'au moins une sonde comprenant au moins ou constituée d'au moins 8 nucléotides consécutifs de l'une quelconque des séquences d'acide nucléique SEQ ID NO : 246, 258, 269, 392, 415, 519 à 530, et/ou leurs séquences d'acide nucléique complémentaires (séquences nucléotidiques correspondant aux différentes séquences variables du locus CRISPR2 du sérotype Indiana) ;
   - l'ensemble S11-1, comprenant ou constitué d'au moins une sonde comprenant au moins ou constituée d'au moins 8 nucléotides consécutifs de l'une quelconque des séquences d'acide nucléique SEQ ID NO : 11 à 23, 238, 948, 1120, 1123, 1133 et/ou leurs séquences d'acide nucléique complémentaires (séquences nucléotidiques correspondant aux différentes séquences variables du locus CRISPR1 du sérotype Agona) ;
   - l'ensemble S11-2, comprenant ou constitué d'au moins une sonde comprenant au moins ou constituée d'au moins 8 nucléotides consécutifs de l'une quelconque des séquences d'acide nucléique SEQ ID NO : 24 à 31 et/ou leurs séquences d'acide nucléique complémentaires (séquences nucléotidiques correspondant aux différentes séquences variables du locus CRISPR2 du sérotype Agona) ;
   - l'ensemble S12-1, comprenant ou constitué d'au moins une sonde comprenant au moins ou constituée d'au moins 8 nucléotides consécutifs de l'une quelconque des séquences d'acide nucléique SEQ ID NO : 830 à 841, 1120 et/ou leurs séquences d'acide nucléique complémentaires (séquences nucléotidiques correspondant aux différentes séquences variables du locus CRISPR1 du sérotype Napoli) ;
   - l'ensemble S12-2, comprenant ou constitué d'au moins une sonde comprenant au moins ou constituée d'au moins 8 nucléotides consécutifs de l'une quelconque des séquences d'acide nucléique SEQ ID NO: 842 à 859 et/ou leurs séquences d'acide nucléique complémentaires (séquences nucléotidiques correspondant aux différentes séquences variables du locus CRISPR2 du sérotype Napoli) ;
   - l'ensemble S13-1, comprenant ou constitué d'au moins une sonde comprenant au moins ou constituée d'au moins 8 nucléotides consécutifs de l'une quelconque des séquences d'acide nucléique SEQ ID NO : 926 à 932 et/ou leurs séquences d'acide nucléique complémentaires (séquences nucléotidiques correspondant aux différentes séquences variables du locus CRISPR1 du sérotype Paratyphi A) ;
   - l'ensemble S13-2, comprenant ou constitué d'au moins une sonde comprenant au moins ou constituée d'au moins 8 nucléotides consécutifs de l'une quelconque des séquences d'acide nucléique SEQ ID NO : 933, 935, 936 et/ou leurs séquences d'acide nucléique complémentaires (séquences nucléotidiques correspondant aux différentes séquences variables du locus CRISPR2 du sérotype Paratyphi A) ;
   - l'ensemble S14-1, comprenant ou constitué d'au moins une sonde comprenant au moins ou constituée d'au moins 8 nucléotides consécutifs de l'une quelconque des séquences d'acide nucléique SEQ ID NO : 707, 937 à 971, 1106, 1128 à 1130, 1133, 1138, 1140, 1802 à 1808, 1915 et/ou leurs séquences d'acide nucléique complémentaires (séquences nucléotidiques correspondant aux différentes séquences variables du locus CRISPR1 des sérotypes Paratyphi B et Paratyphi B Java) ;
   - l'ensemble S14-2, comprenant ou constitué d'au moins une sonde comprenant au moins ou constituée d'au moins 8 nucléotides consécutifs de l'une quelconque des séquences d'acide nucléique SEQ ID NO : 861, 972 à 987, 989 à 992 à 996, 1172 1262, 1462, 1497, 1810 à 1825 et/ou leurs séquences d'acide nucléique complémentaires (séquences nucléotidiques correspondant aux différentes séquences variables du locus CRISPR2 des sérotypes Paratyphi B et Paratyphi B Java) ;
   - l'ensemble S15-1, comprenant ou constitué d'au moins une sonde comprenant au moins ou constituée d'au moins 8 nucléotides consécutifs de l'une quelconque des séquences d'acide nucléique SEQ ID NO : 232, 238,239, 997 à 1002, 1106 et/ou leurs séquences d'acide nucléique complémentaires (séquences nucléotidiques correspondant aux différentes séquences variables du locus CRISPR1 du sérotype Paratyphi C) ;
   - l'ensemble S15-2, comprenant ou constitué d'au moins une sonde comprenant au moins ou constituée d'au moins 8 nucléotides consécutifs de l'une quelconque des séquences d'acide nucléique SEQ ID NO : 280, 290, 291, 392, 393, 415, 1003 à 1006, 1262 et/ou leurs séquences d'acide nucléique complémentaires (séquences nucléotidiques correspondant aux différentes séquences variables du locus CRISPR2 du sérotype Paratyphi C) ;
   - l'ensemble S16-1, comprenant ou constitué d'au moins une sonde comprenant au moins ou constituée d'au moins 8 nucléotides consécutifs de l'une quelconque des séquences d'acide nucléique SEQ ID NO : 211 à 224, 421, 427, 428, 432; 443 à 445, 447 à 449, 504 et/ou leurs séquences d'acide nucléique complémentaires (séquences nucléotidiques correspondant aux différentes séquences variables du locus CRISPR1 du sérotype Bovismorbificans) ;
   - l'ensemble S16-2, comprenant ou constitué d'au moins une sonde comprenant au moins ou constituée d'au moins 8 nucléotides consécutifs de l'une quelconque des séquences d'acide nucléique SEQ ID NO : 196 à 209, 994, 1144, 1145, 1172 et/où leurs séquences d'acide nucléique complémentaires (séquences nucléotidiques correspondant aux différentes séquences variables du locus CRISPR2 du sérotype Bovismorbificans) ;
   - l'ensemble S17-1, comprenant ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Panama) ;
   - l'ensemble S17-2, comprenant ou constitué d'au moins une sonde comprenant au moins ou constituée d'au moins 8 nucléotides consécutifs de l'une quelconque des séquences d'acide nucléique SEQ ID NO : 918 à 925, 934 et/ou leurs séquences d'acide nucléique complémentaires (séquences nucléotidiques correspondant aux différentes séquences variables du locus CRISPR2 du sérotype Panama) ;
   - l'ensemble S18-1, comprenant ou constitué d'au moins une sonde comprenant au moins ou constituée d'au moins 8 nucléotides consécutifs de l'une quelconque des séquences d'acide nucléique SEQ ID NO : 11, 16, 67, 74, 442, 444, 446 à 448, 602, 937, 948, 1120, 1128 à 1130, 1182 à 1200, 1937 à 1950, et/ou leurs séquences d'acide nucléique complémentaires (séquences nucléotidiques correspondant aux différentes séquences variables du locus CRISPR1 du sérotype Saintpaul) ;
   - l'ensemble S18-2, comprenant ou constitué d'au moins une sonde comprenant au moins ou constituée d'au moins 8 nucléotides consécutifs de l'une quelconque des séquences d'acide nucléique SEQ ID NO : 972, 983, 989, 1201 à 1214, 1216 à 1227, 1952 à 1960 et/ou leurs séquences d'acide nucléique complémentaires (séquences nucléotidiques correspondant aux différentes séquences variables du locus CRISPR2 du sérotype Saintpaul) ;
   - l'ensemble S19-1, comprenant ou constitué d'au moins une sonde comprenant au moins ou constituée d'au moins 8 nucléotides consécutifs de l'une quelconque des séquences d'acide nucléique SEQ ID NO : 514, 1062 à 1089, 1100, 1284, 1886 à 1911 et/ou leurs séquences d'acide nucléique complémentaires (séquences nucléotidiques correspondant aux différentes séquences variables du locus CRISPR1 du sérotype Senftenberg) ;
   - l'ensemble S19-2, comprenant ou constitué d'au moins une sonde comprenant au moins ou constituée d'au moins 8 nucléotides consécutifs de l'une quelconque des séquences d'acide nucléique SEQ ID NO : 300, 1090 à 1097, 1101 à 1103, 1304, 1315, 1318 à 1320 et/ou leurs séquences d'acide nucléique complémentaires (séquences nucléotidiques correspondant aux différentes séquences variables du locus CRISPR2 du sérotype Senftenberg) ;
   - l'ensemble S20-1, comprenant ou constitué d'au moins une sonde comprenant au moins ou constituée d'au moins 8 nucléotides consécutifs de l'une quelconque des séquences d'acide nucléique SEQ ID NO : 67, 74 à 80 et/ou leurs séquences d'acide nucléique complémentaires (séquences nucléotidiques correspondant aux différentes séquences variables du locus CRISPR1 du sérotype Anatum) ;
   - l'ensemble S20-2, comprenant ou constitué d'au moins une sonde comprenant au moins ou constituée d'au moins 8 nucléotides consécutifs de l'une quelconque des séquences d'acide nucléique SEQ ID NO : 83 à 94, 1304, 1315, 1318 à 1322, 1324, 1390 à 1397 et/ou leurs séquences d'acide nucléique complémentaires (séquences nucléotidiques correspondant aux différentes séquences variables du locus CRISPR2 du sérotype Anatum) ;
   - l'ensemble S21-1, comprenant ou constitué d'au moins une sonde comprenant au moins ou constituée d'au moins 8 nucléotides consécutifs de l'une quelconque des séquences d'acide nucléique SEQ ID NO : 379, 383 à 385, 388 et/ou leurs séquences d'acide nucléique complémentaires (séquences nucléotidiques correspondant aux différentes séquences variables du locus CRISPR1 des sérotypes Gallinarum) ;
   - l'ensemble S21-2, comprenant ou constitué d'au moins une sonde comprenant au moins ou constituée d'au moins 8 nucléotides consécutifs de l'une quelconque des séquences d'acide nucléique SEQ ID NO : 392, 393, 404, 407 à 415 et/ou leurs séquences d'acide nucléique complémentaires (séquences nucléotidiques correspondant aux différentes séquences variables du locus CRISPR2 des sérotypes Gallinarum) ;
   - l'ensemble S22-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Brandenburg) ;
   - l'ensemble S22-2, comprenant ou constitué d'au moins une sonde comprenant au moins ou constituée d'au moins 8 nucléotides consécutifs de l'une quelconque des séquences d'acide nucléique SEQ ID NO : 225 à 231 et/ou leurs séquences d'acide nucléique complémentaires (séquences nucléotidiques correspondant aux différentes séquences variables du locus CRISPR2 du sérotype Brandenburg) ;
   - l'ensemble S23-1, comprenant ou constitué d'au moins une sonde comprenant au moins ou constituée d'au moins 8 nucléotides consécutifs de l'une quelconque des séquences d'acide nucléique SEQ ID NO: 378, 379 et/ou leurs séquences d'acide nucléique complémentaires (séquences nucléotidiques correspondant aux différentes séquences variables du locus CRISPR1 du sérotype Dublin) ;
   - l'ensemble S23-2, comprenant ou constitué d'au moins une sonde comprenant au moins ou constituée d'au moins 8 nucléotides consécutifs de l'une quelconque des séquences d'acide nucléique SEQ ID NO : 392, 393, 411, 412 et/ou leurs séquences d'acide nucléique complémentaires (séquences nucléotidiques correspondant aux différentes séquences variables du locus CRISPR2 du sérotype Dublin) ;
   - l'ensemble S24-1, comprenant ou constitué d'au moins une sonde comprenant au moins ou constituée d'au moins 8 nucléotides consécutifs de l'une quelconque des séquences d'acide nucléique SEQ ID NO: 1, 1106 et/ou leurs séquences d'acide nucléique complémentaires (séquences nucléotidiques correspondant aux différentes séquences variables du locus CRISPR1 du sérotype Abortusequi) ;
   - l'ensemble S24-2, comprenant ou constitué d'au moins une sonde comprenant au moins ou constituée d'au moins 8 nucléotides consécutifs de l'une quelconque des séquences d'acide nucléique SEQ ID NO : 2 à 10, 130 et/ou leurs séquences d'acide nucléique complémentaires (séquences nucléotidiques correspondant aux différentes séquences variables du locus CRISPR2 du sérotype Abortusequi) ;
   - l'ensemble S25-1, comprenant ou constitué d'au moins une sonde comprenant au moins ou constituée d'au moins 8 nucléotides consécutifs de l'une quelconque des séquences d'acide nucléique SEQ ID NO: 378, 379 et/ou leurs séquences d'acide nucléique complémentaires (séquences nucléotidiques correspondant aux différentes séquences variables du locus CRISPR1 du sérotype Kiel) ;
   - l'ensemble S25-2, comprenant ou constitué d'au moins une sonde comprenant au moins ou constituée d'au moins 8 nucléotides consécutifs de l'une quelconque des séquences d'acide nucléique SEQ ID NO : 392, 393, 411, 412 et/ou leurs séquences d'acide nucléique complémentaires (séquences nucléotidiques correspondant aux différentes séquences variables du locus CRISPR2 du sérotype Kiel) ;
   - l'ensemble S26-1, comprenant ou constitué d'au moins une sonde comprenant au moins ou constituée d'au moins 8 nucléotides consécutifs de l'une quelconque des séquences d'acide nucléique SEQ ID NO : 509, 510, 512, 513, 637 à 660, 1086, 1107 et/ou leurs séquences d'acide nucléique complémentaires (séquences nucléotidiques correspondant aux différentes séquences variables du locus CRISPR1 du sérotype Mbandaka) ;
   - l'ensemble S26-2, comprenant ou constitué d'au moins une sonde comprenant au moins ou constituée d'au moins 8 nucléotides consécutifs de l'une quelconque des séquences d'acide nucléique SEQ ID NO : 661 à 666, 668, 669, 672, 683, 690 à 695, 731, 918 et/ou leurs séquences d'acide nucléique complémentaires (séquences nucléotidiques correspondant aux différentes séquences variables du locus CRISPR2 du sérotype Mbandaka) ;
   - l'ensemble S27-1, comprenant ou constitué d'au moins une sonde comprenant au moins ou constituée d'au moins 8 nucléotides consécutifs de l'une quelconque des séquences d'acide nucléique SEQ ID NO : 916 et/ou sa séquence d'acide nucléique complémentaire (séquences nucléotidiques correspondant à la séquence variable du locus CRISPR1 du sérotype Miami) ;
   - l'ensemble S27-2, comprenant ou constitué d'au moins une sonde comprenant au moins ou constituée d'au moins 8 nucléotides consécutifs de l'une quelconque des séquences d'acide nucléique SEQ ID NO : 25, 696 à 700 et/ou leurs séquences d'acide nucléique complémentaires (séquences nucléotidiques correspondant aux différentes séquences variables du locus CRISPR2 du sérotype Miami) ;
   - l'ensemble S28-1, comprenant ou constitué d'au moins une sonde comprenant au moins ou constituée d'au moins 8 nucléotides consécutifs de l'une quelconque des séquences d'acide nucléique SEQ ID NO : 55, 701 à 730, 732 à 768, 1115, 1228, 1232 à 1237, 1601, 1671 à 1680, 1682 à 1694, 1747 et/ou leurs séquences d'acide nucléique complémentaires (séquences nucléotidiques correspondant aux différentes séquences variables du locus CRISPR1 du sérotype Montevideo) ;
   - l'ensemble S28-2, comprenant ou constitué d'au moins une sonde comprenant au moins ou constituée d'au moins 8 nucléotides consécutifs de l'une quelconque des séquences d'acide nucléique SEQ ID NO : 589, 769 à 829, 933, 1245, 1697, 1698 et/ou leurs séquences d'acide nucléique complémentaires (séquences nucléotidiques correspondant aux différentes séquences variables du locus CRISPR2 du sérotype Montevideo) ;
   - l'ensemble S29-1, comprenant ou constitué d'au moins une sonde comprenant au moins ou constituée d'au moins 8 nucléotides consécutifs de l'une quelconque des séquences d'acide nucléique SEQ ID NO : 379, 380, 383 à 385, 387, 389 à 391 et/ou leurs séquences d'acide nucléique complémentaires (séquences nucléotidiques correspondant aux différentes séquences variables du locus CRISPR1 du sérotype Nitra) ;
   - l'ensemble S29-2, comprenant ou constitué d'au moins une sonde comprenant au moins ou constituée d'au moins 8 nucléotides consécutifs de l'une quelconque des séquences d'acide nucléique SEQ ID NO : 392 à 394, 404, 407 à 413, 519 et/ou leurs séquences d'acide nucléique complémentaires (séquences nucléotidiques correspondant aux différentes séquences variables du locus CRISPR2 du sérotype Nitra) ;
   - l'ensemble S30-1, comprenant ou constitué d'au moins une sonde comprenant au moins ou constituée d'au moins 8 nucléotides consécutifs de l'une quelconque des séquences d'acide nucléique SEQ ID NO : 931, 932, 1104, 1105 et/ou leurs séquences d'acide nucléique complémentaires (séquences nucléotidiques correspondant aux différentes séquences variables du locus CRISPR1 du sérotype Sendai) ;
   - l'ensemble S30-2, comprenant ou constitué d'au moins une sonde comprenant au moins ou constituée d'au moins 8 nucléotides consécutifs de la séquence d'acide nucléique SEQ ID NO : 936 et/ou sa séquence d'acide nucléique complémentaire (séquences nucléotidiques correspondant à la séquence variable du locus CRISPR2 du sérotype Sendai) ;
   - l'ensemble S31-1, comprenant ou constitué d'au moins une sonde comprenant au moins ou constituée d'au moins 8 nucléotides consécutifs de l'une quelconque des séquences d'acide nucléique SEQ ID NO : 714 à 716, 1228 à 1239 et/ou leurs séquences d'acide nucléique complémentaires (séquences nucléotidiques correspondant aux différentes séquences variables du locus CRISPR1 du sérotype Schwarzengrund) ;
   - l'ensemble S31-2, comprenant ou constitué d'au moins une sonde comprenant au moins ou constituée d'au moins 8 nucléotides consécutifs de l'une quelconque des séquences d'acide nucléique SEQ ID NO : 196, 992, 995, 1172, 1240 à 1244, 1246 à 1253 et/ou leurs séquences d'acide nucléique complémentaires (séquences nucléotidiques correspondant aux différentes séquences variables du locus CRISPR2 du sérotype Schwarzengrund) ;
   - l'ensemble S32-1, comprenant ou constitué d'au moins une sonde comprenant au moins ou constituée d'au moins 8 nucléotides consécutifs de l'une quelconque des séquences d'acide nucléique SEQ ID NO : 232, 238, 239, 998, 999, 1002, 1106, 1261 et/ou leurs séquences d'acide nucléique complémentaires (séquences nucléotidiques correspondant aux différentes séquences variables du locus CRISPR1 du sérotype Typhisuis) ;
   - l'ensemble S32-2, comprenant ou constitué d'au moins une sonde comprenant au moins ou constituée d'au moins 8 nucléotides consécutifs de la séquence d'acide nucléique SEQ ID NO : 1262 et/ou sa séquence d'acide nucléique complémentaire (séquences nucléotidiques correspondant à la séquence variable du locus CRISPR2 du sérotype Typhisuis) ;
   - l'ensemble S33-1, comprenant ou constitué d'au moins une sonde comprenant au moins ou constituée d'au moins 8 nucléotides consécutifs de l'une quelconque des séquences d'acide nucléique SEQ ID NO : 466 à 486 et/ou leurs séquences d'acide nucléique complémentaires (séquences nucléotidiques correspondant aux différentes séquences variables du locus CRISPR1 des différents sérotypes de la sous-espèce *houtenae*) ;
   - l'ensemble S33-2, comprenant ou constitué d'au moins une sonde comprenant au moins ou constituée d'au moins 8 nucléotides consécutifs de l'une quelconque des séquences d'acide nucléique SEQ ID NO : 487 à 490 et/ou leurs séquences d'acide nucléique complémentaires (séquences nucléotidiques correspondant aux différentes séquences variables du locus CRISPR2 des différents sérotypes de la sous-espèce *houtenae*) ;
   - l'ensemble S34-1, comprenant ou constitué d'au moins une sonde comprenant au moins ou constituée d'au moins 8 nucléotides consécutifs de l'une quelconque des séquences d'acide nucléique SEQ ID NO : 338 à 376 et/ou leurs séquences d'acide nucléique complémentaires (séquences nucléotidiques correspondant aux différentes séquences variables du locus CRISPR1 des différents sérotypes de la sous-espèce *diarizonae*) ;
   - l'ensemble S34-2, comprenant ou constitué d'au moins une sonde comprenant au moins ou constituée d'au moins 8 nucléotides consécutifs de la séquence d'acide nucléique SEQ ID NO : 377 et/ou sa séquence d'acide nucléique complémentaire (séquences nucléotidiques correspondant à la séquence variable du locus CRISPR2 des différents sérotypes de la sous-espèce *diarizonae*) ;
   - l'ensemble S35-1, comprenant ou constitué d'au moins une sonde comprenant au moins ou constituée d'au moins 8 nucléotides consécutifs de l'une quelconque des séquences d'acide nucléique SEQ ID NO : 1007 à 1037, 1848 à 1884 et/ou leurs séquences d'acide nucléique complémentaires (séquences nucléotidiques correspondant aux différentes séquences variables du locus CRISPR1 de la sous-espèce *salamae*) ;
   - l'ensemble S35-2, comprenant ou constitué d'au moins une sonde comprenant au moins ou constituée d'au moins 8 nucléotides consécutifs de l'une quelconque des séquences d'acide nucléique SEQ ID NO : 1038 à 1061, 1885 et/ou leurs séquences d'acide nucléique complémentaires (séquences nucléotidiques correspondant aux différentes séquences variables du locus CRISPR2 de la sous-espèce *salamae*) ;
   - l'ensemble S36, comprenant ou constitué d'au moins une sonde comprenant au moins ou constituée d'au moins 8 nucléotides consécutifs de la séquence d'acide nucléique SEQ ID NO : 95 et/ou sa séquence d'acide nucléique complémentaire (séquences nucléotidiques correspondant à la séquence variable du locus CRISPR1 des différents sérotypes de la sous-espèce *arizonae*) ;
   - l'ensemble S37-1, comprenant ou constitué d'au moins une sonde comprenant au moins ou constituée d'au moins 8 nucléotides consécutifs de l'une quelconque des séquences d'acide nucléique SEQ ID NO : 491 à 499 et/ou leurs séquences d'acide nucléique complémentaires (séquences nucléotidiques correspondant aux différentes séquences variables du locus CRISPR1 des différents séryotpes de la sous-espèce *indica*) ;
   - l'ensemble S37-2, comprenant ou constitué d'au moins une sonde comprenant au moins ou constituée d'au moins 8 nucléotides consécutifs de l'une quelconque des séquences d'acide nucléique SEQ ID NO : 500 à 503, 1574 à 1589 et/ou leurs séquences d'acide nucléique complémentaires (séquences nucléotidiques correspondant aux différentes séquences variables du locus CRISPR2 des différents sérotypes de la sous-espèce *indica*) ;
   - l'ensemble S38-1, comprenant ou constitué d'au moins une sonde comprenant au moins ou constituée d'au moins 8 nucléotides consécutifs de l'une quelconque des séquences d'acide nucléique SEQ ID NO : 32 à 66, 382, 416, 667, 2036 et/ou leurs séquences d'acide nucléique complémentaires (séquences nucléotidiques correspondant aux différentes séquences variables du locus CRISPR1 du sérotype Altona) ;
   - l'ensemble S38-2, comprenant ou constitué d'au moins une sonde comprenant au moins ou constituée d'au moins 8 nucléotides consécutifs de l'une quelconque des séquences d'acide nucléique SEQ ID NO : 854, 933, 1095, 1306, 1354 à 1389, 1696 et/ou leurs séquences d'acide nucléique complémentaires (séquences nucléotidiques correspondant aux différentes séquences variables du locus CRISPR2 du sérotype Altona) ;
   - l'ensemble S39-1, comprenant ou constitué d'au moins une sonde comprenant au moins ou constituée d'au moins 8 nucléotides consécutifs de l'une quelconque des séquences d'acide nucléique SEQ ID NO : 240, 241, 1120, 1121 et/ou leurs séquences d'acide nucléique complémentaires (séquences nucléotidiques correspondant aux différentes séquences variables du locus CRISPR1 du sérotype Choleraesuis variété Decatur) ;
   - l'ensemble S39-2, comprenant ou constitué d'au moins une sonde comprenant au moins ou constituée d'au moins 8 nucléotides consécutifs de l'une quelconque des séquences d'acide nucléique SEQ ID NO : 225, 247 à 251, 253 à 256, 259 à 268, 270 à 279, 281 à 289, 292 à 294 et/ou leurs séquences d'acide nucléique complémentaires (séquences nucléotidiques correspondant aux différentes séquences variables du locus CRISPR2 du sérotype Choleraesuis variété Decatur) ;
   - l'ensemble S40-1, comprenant ou constitué d'au moins une sonde comprenant au moins ou constituée d'au moins 8 nucléotides consécutifs de l'une quelconque des séquences d'acide nucléique SEQ ID NO : 11, 232, 238, 239, 240, 242 à 245, 233 à 237, 959, 1106, 1120 et/ou leurs séquences d'acide nucléique complémentaires (séquences nucléotidiques correspondant aux différentes séquences variables du locus CRISPR1 du sérotype Choleraesuis variété Kunzendorf) ;
   - l'ensemble S40-2, comprenant ou constitué d'au moins une sonde comprenant au moins ou constituée d'au moins 8 nucléotides consécutifs de l'une quelconque des séquences d'acide nucléique SEQ ID NO : 246, 252, 258, 269, 280, 290, 291, 392, 563, 933 et/ou leurs séquences d'acide nucléique complémentaires (séquences nucléotidiques correspondant aux différentes séquences variables du locus CRISPR2 du sérotype Choleraesuis variété Kunzendorf) ;
   - l'ensemble S41-1, comprenant ou constitué d'au moins une sonde comprenant au moins ou constituée d'au moins 8 nucléotides consécutifs de l'une quelconque des séquences d'acide nucléique SEQ ID NO : 11, 242 à 245, 232 à 239, 959, 1000, 1106, 1139, 1120 et/ou leurs séquences d'acide nucléique complémentaires (séquences nucléotidiques correspondant aux différentes séquences variables du locus CRISPR1 des sérotypes Choleraesuis et Choleraesuis sensu stricto) ;
   - l'ensemble S41-2, comprenant ou constitué d'au moins une sonde comprenant au moins ou constituée d'au moins 8 nucléotides consécutifs de l'une quelconque des séquences d'acide nucléique SEQ ID NO : 246, 252, 258, 269, 280, 290, 291, 392, 563, 574, 933, 1003, 1004 et/ou leurs séquences d'acide nucléique complémentaires (séquences nucléotidiques correspondant aux différentes séquences variables du locus CRISPR2 des sérotypes Choleraesuis et Choleraesuis sensu stricto) ;
   - l'ensemble S42-1, comprenant ou constitué d'au moins une sonde comprenant au moins ou constituée d'au moins 8 nucléotides consécutifs de l'une quelconque des séquences d'acide nucléique SEQ ID NO : 416 à 420, 1122 et/ou leurs séquences d'acide nucléique complémentaires (séquences nucléotidiques correspondant aux différentes séquences variables du locus CRISPR1 du sérotype Goettingen) ;
   - l'ensemble S42-2, comprenant ou constitué d'au moins une sonde comprenant au moins ou constituée d'au moins 8 nucléotides consécutifs de la séquence d'acide nucléique SEQ ID NO: 1548 et/ou sa séquence d'acide nucléique complémentaire (séquences nucléotidiques correspondant à la séquence variable du locus CRISPR2 du sérotype Goettingen) ;
   - l'ensemble S43-1, comprenant ou constitué d'au moins une sonde comprenant au moins ou constituée d'au moins 8 nucléotides consécutifs de l'une quelconque des séquences d'acide nucléique SEQ ID NO : 630 à 636, 937, 948, 1120, 1143 et/ou leurs séquences d'acide nucléique complémentaires (séquences nucléotidiques correspondant aux différentes séquences variables du locus CRISPR1 du sérotype Manhattan) ;
   - l'ensemble S43-2, comprenant ou constitué d'au moins une sonde comprenant au moins ou constituée d'au moins 8 nucléotides consécutifs de l'une quelconque des séquences d'acide nucléique SEQ ID NO : 392, 1653 et/ou leurs séquences d'acide nucléique complémentaires (séquences nucléotidiques correspondant aux différentes séquences variables du locus CRISPR2 du sérotype Manhattan) ;
   - l'ensemble S44-1, comprenant ou constitué d'au moins une sonde comprenant au moins ou constituée d'au moins 8 nucléotides consécutifs de l'une quelconque des séquences d'acide nucléique SEQ ID NO: 96 à 155 et/ou leurs séquences d'acide nucléique complémentaires (séquences nucléotidiques correspondant aux différentes séquences variables du locus CRISPR1 de certains sérotypes de l'espèce *S. bongori*) ;
   - l'ensemble S44-2, comprenant ou constitué d'au moins une sonde comprenant au moins ou constituée d'au moins 8 nucléotides consécutifs de l'une quelconque des séquences d'acide nucléique SEQ ID NO : 156 à 195, 1421 à 1430 et/ou leurs séquences d'acide nucléique complémentaires (séquences nucléotidiques correspondant aux différentes séquences variables du locus CRISPR2 de certains sérotypes de l'espèce *S. bongori*) ;
   - l'ensemble S45-1, comprenant ou constitué d'au moins une sonde comprenant au moins ou constituée d'au moins 8 nucléotides consécutifs de l'une quelconque des séquences d'acide nucléique SEQ ID NO : 962, 1106, 1133, 1804, 1808 et/ou leurs séquences d'acide nucléique complémentaires (séquences nucléotidiques correspondant aux différentes séquences variables du locus CRISPR1 du sérotype Abony) ;
   - l'ensemble S45-2, comprenant ou constitué d'au moins une sonde comprenant au moins ou constituée d'au moins 8 nucléotides consécutifs de l'une quelconque des séquences d'acide nucléique SEQ ID NO : 972, 989, 1334 à 1339, 1474, 1499 et/ou leurs séquences d'acide nucléique complémentaires (séquences nucléotidiques correspondant aux différentes séquences variables du locus CRISPR2 du sérotype Abony) ;
   - l'ensemble S46-1, comprenant ou constitué d'au moins une sonde comprenant au moins ou constituée d'au moins 8 nucléotides consécutifs de l'une quelconque des séquences d'acide nucléique SEQ ID NO : 448, 860 à 875, 882, 883, 1340 à 1345, 1721 à 1723, et/ou leurs séquences d'acide nucléique complémentaires (séquences nucléotidiques correspondant aux différentes séquences variables du locus CRISPR1 du sérotype Aesch) ;
   - l'ensemble S46-2, comprenant ou constitué d'au moins une sonde comprenant au moins ou constituée d'au moins 8 nucléotides consécutifs de l'une quelconque des séquences d'acide nucléique SEQ ID NO : 563, 625, 626, 885, 887 à 893, 896, 933, 1346 à 1348, 1749, 1759, et/ou leurs séquences d'acide nucléique complémentaires (séquences nucléotidiques correspondant aux différentes séquences variables du locus CRISPR2 du sérotype Aesch) ;
   - l'ensemble S47-1, comprenant ou constitué d'au moins une sonde comprenant au moins ou constituée d'au moins 8 nucléotides consécutifs de l'une quelconque des séquences d'acide nucléique SEQ ID NO: 834, 1120, 1349, 1350, 1351, 1713, 1718 et/ou leurs séquences d'acide nucléique complémentaires (séquences nucléotidiques correspondant aux différentes séquences variables du locus CRISPR1 du sérotype Albany) ;
   - l'ensemble S47-2, comprenant ou constitué d'au moins une sonde comprenant au moins ou constituée d'au moins 8 nucléotides consécutifs de l'une quelconque des séquences d'acide nucléique SEQ ID NO : 842, 852, 1352, 1353 et/ou leurs séquences d'acide nucléique complémentaires (séquences nucléotidiques correspondant aux différentes séquences variables du locus CRISPR2 du sérotype Albany);
   - l'ensemble S48-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Arechavalata) ;
   - l'ensemble S48-2, comprenant ou constitué d'au moins une sonde comprenant au moins ou constituée d'au moins 8 nucléotides consécutifs de l'une quelconque des séquences d'acide nucléique SEQ ID NO : 225, 227 à 231, 1398 et/ou leurs séquences d'acide nucléique complémentaires (séquences nucléotidiques correspondant aux différentes séquences variables du locus CRISPR2 du sérotype Arechavalata) ;
   - l'ensemble S49-1, comprenant ou constitué d'au moins une sonde comprenant au moins ou constituée d'au moins 8 nucléotides consécutifs de l'une quelconque des séquences d'acide nucléique SEQ ID NO : 448, 449, 860, 871 à 873, 878, 895, 903 à 905, 907, 1719 à 1721, 1745 et/ou leurs séquences d'acide nucléique complémentaires (séquences nucléotidiques correspondant aux différentes séquences variables du locus CRISPR1 du sérotype Bardo) ;
   - l'ensemble S49-2, comprenant ou constitué d'au moins une sonde comprenant au moins ou constituée d'au moins 8 nucléotides consécutifs de l'une quelconque des séquences d'acide nucléique SEQ ID NO : 563, 622, 626, 885, 886 à 890, 893, 896, 907, 910 à 915, 933, 1756 à 1758 et/ou leurs séquences d'acide nucléique complémentaires (séquences nucléotidiques correspondant aux différentes séquences variables du locus CRISPR2 du sérotype Bardo) ;
   - l'ensemble S50-1, comprenant ou constitué d'au moins une sonde comprenant au moins ou constituée d'au moins 8 nucléotides consécutifs de l'une quelconque des séquences d'acide nucléique SEQ ID NO: 82, 1401 et/ou leurs séquences d'acide nucléique complémentaires (séquences nucléotidiques correspondant aux différentes séquences variables du locus CRISPR1 du sérotype Berta) ;
   - l'ensemble S50-2, comprenant ou constitué d'au moins une sonde comprenant au moins ou constituée d'au moins 8 nucléotides consécutifs de l'une quelconque des séquences d'acide nucléique SEQ ID NO : 246, 918, 933, 1401, 1448 et/ou leurs séquences d'acide nucléique complémentaires (séquences nucléotidiques correspondant aux différentes séquences variables du locus CRISPR2 du sérotype Berta) ;
   - l'ensemble S51-1, comprenant ou constitué d'au moins une sonde comprenant au moins ou constituée d'au moins 8 nucléotides consécutifs de l'une quelconque des séquences d'acide nucléique SEQ ID NO : 1, 37, 1106, 1402 à 1416, 2055 et/ou leurs séquences d'acide nucléique complémentaires (séquences nucléotidiques correspondant aux différentes séquences variables du locus CRISPR1 du sérotype Bispebjerg) ;
   - l'ensemble S51-2, comprenant ou constitué d'au moins une sonde comprenant au moins ou constituée d'au moins 8 nucléotides consécutifs de l'une quelconque des séquences d'acide nucléique SEQ ID NO : 2 à 4, 9, 1417 à 1420, 1547, 1572 et/ou leurs séquences d'acide nucléique complémentaires (séquences nucléotidiques correspondant aux différentes séquences variables du locus CRISPR2 du sérotype Bispebjerg) ;
   - l'ensemble S52-1, comprenant ou constitué d'au moins une sonde comprenant au moins ou constituée d'au moins 8 nucléotides consécutifs de l'une quelconque des séquences d'acide nucléique SEQ ID NO : 379, 380, 383, 385, 387, 389 à 391 et/ou leurs séquences d'acide nucléique complémentaires (séquences nucléotidiques correspondant aux différentes séquences variables du locus CRISPR1 du sérotype Blegdam) ;
   - l'ensemble S52-2, comprenant ou constitué d'au moins une sonde comprenant au moins ou constituée d'au moins 8 nucléotides consécutifs de l'une quelconque des séquences d'acide nucléique SEQ ID NO : 392 à 394, 404, 407, 412, 413, 519 et/ou leurs séquences d'acide nucléique complémentaires (séquences nucléotidiques correspondant aux différentes séquences variables du locus CRISPR2 du sérotype Blegdam) ;
   - l'ensemble S53-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Chester) ;
   - l'ensemble S53-2, comprenant ou constitué d'au moins une sonde comprenant au moins ou constituée d'au moins 8 nucléotides consécutifs de l'une quelconque des séquences d'acide nucléique SEQ ID NO : 933, 1445 à 1447 et/ou leurs séquences d'acide nucléique complémentaires (séquences nucléotidiques correspondant aux différentes séquences variables du locus CRISPR2 du sérotype Chester) ;
   - l'ensemble S54-1, comprenant ou constitué d'au moins une sonde comprenant au moins ou constituée d'au moins 8 nucléotides consécutifs de l'une quelconque des séquences d'acide nucléique SEQ ID NO : 1449 à 1454, 1460, 1467 à 1473 et/ou leurs séquences d'acide nucléique complémentaires (séquences nucléotidiques correspondant aux différentes séquences variables du locus CRISPR1 du sérotype Concord) ;
   - l'ensemble S54-2, comprenant ou constitué d'au moins une sonde comprenant au moins ou constituée d'au moins 8 nucléotides consécutifs de l'une quelconque des séquences d'acide nucléique SEQ ID NO : 972, 1474 à 1493, 1495 à 1499 et/ou leurs séquences d'acide nucléique complémentaires (séquences nucléotidiques correspondant aux différentes séquences variables du locus CRISPR2 du sérotype Concord) ;
   - l'ensemble S55-1, comprenant ou constitué d'au moins une sonde comprenant au moins ou constituée d'au moins 8 nucléotides consécutifs de l'une quelconque des séquences d'acide nucléique SEQ ID NO : 1106, 1107 à 1109, 1123, 1128 à 1131, 1133, 1137 à 1140, 1550, 1559 à 1561 et/ou leurs séquences d'acide nucléique complémentaires (séquences nucléotidiques correspondant aux différentes séquences variables du locus CRISPR1 du sérotype Crossness) ;
   - l'ensemble S55-2, comprenant ou constitué d'au moins une sonde comprenant au moins ou constituée d'au moins 8 nucléotides consécutifs de l'une quelconque des séquences d'acide nucléique SEQ ID NO : 455, 1144, 1145, 1149 à 1153, 1155 à 1160, 1163, 1172 à 1174 et/ou leurs séquences d'acide nucléique complémentaires (séquences nucléotidiques correspondant aux différentes séquences variables du locus CRISPR2 du sérotype Crossness) ;
   - l'ensemble S56-1, comprenant ou constitué d'au moins une sonde comprenant au moins ou constituée d'au moins 8 nucléotides consécutifs de l'une quelconque des séquences d'acide nucléique SEQ ID NO : 834, 1120, 1349 à 1351, 1713, 1718 et/ou leurs séquences d'acide nucléique complémentaires (séquences nucléotidiques correspondant aux différentes séquences variables du locus CRISPR1 du sérotype Duesseldorf) ;
   - l'ensemble S56-2, comprenant ou constitué d'au moins une sonde comprenant au moins ou constituée d'au moins 8 nucléotides consécutifs de l'une quelconque des séquences d'acide nucléique SEQ ID NO : 842, 852, 1352, 1353, 1501 à 1503 et/ou leurs séquences d'acide nucléique complémentaires (séquences nucléotidiques correspondant aux différentes séquences variables du locus CRISPR2 du sérotype Duesseldorf ;
   - l'ensemble S57-1, comprenant ou constitué d'au moins une sonde comprenant au moins ou constituée d'au moins 8 nucléotides consécutifs de l'une quelconque des séquences d'acide nucléique SEQ ID NO : 941, 1504 à 1508 et/ou leurs séquences d'acide nucléique complémentaires (séquences nucléotidiques correspondant aux différentes séquences variables du locus CRISPR1 du sérotype Emek) ;
   - l'ensemble S57-2, comprenant ou constitué d'au moins une sonde comprenant au moins ou constituée d'au moins 8 nucléotides consécutifs de l'une quelconque des séquences d'acide nucléique SEQ ID NO : 1260, 1509 à 1524, 1526, 1527 et/ou leurs séquences d'acide nucléique complémentaires (séquences nucléotidiques correspondant aux différentes séquences variables du locus CRISPR2 du sérotype Emek) ;
   - l'ensemble S58-1, comprenant ou constitué d'au moins une sonde comprenant au moins ou constituée d'au moins 8 nucléotides consécutifs de la séquence d'acide nucléique SEQ ID NO : 558 et/ou sa séquence d'acide nucléique complémentaire (séquences nucléotidiques correspondant à la séquence variable du locus CRISPR1 du sérotype Fulica) ;
   - l'ensemble S58-2, comprenant ou constitué d'au moins une sonde comprenant au moins ou constituée d'au moins 8 nucléotides consécutifs de l'une quelconque des séquences d'acide nucléique SEQ ID NO : 2, 4, 5, 1333, 1547 et/ou leurs séquences d'acide nucléique complémentaires (séquences nucléotidiques correspondant aux différentes séquences variables du locus CRISPR2 du sérotype Fulica) ;
   - l'ensemble S59-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Gueuletapee) ;
   - l'ensemble S59-2, comprenant ou constitué d'au moins une sonde comprenant au moins ou constituée d'au moins 8 nucléotides consécutifs de l'une quelconque des séquences d'acide nucléique SEQ ID NO : 597, 918 à 925, 933, 1549 et/ou leurs séquences d'acide nucléique complémentaires (séquences nucléotidiques correspondant aux différentes séquences variables du locus CRISPR2 du sérotype Gueuletapee) ;
   - l'ensemble S60-1, comprenant ou constitué d'au moins une sonde comprenant au moins ou constituée d'au moins 8 nucléotides consécutifs de l'une quelconque des séquences d'acide nucléique SEQ ID NO : 1106 à 1109, 1111 à 1116, 1118, 1123, 1128 à 1131, 1133, 1137 à 1140, 1150, 1151, 1555 à 1562 et/ou leurs séquences d'acide nucléique complémentaires (séquences nucléotidiques correspondant aux différentes séquences variables du locus CRISPR1 du sérotype Heidelberg) ;
   - l'ensemble S60-2, comprenant ou constitué d'au moins une sonde comprenant au moins ou constituée d'au moins 8 nucléotides consécutifs de l'une quelconque des séquences d'acide nucléique SEQ ID NO : 455, 1144, 1145, 1149 à 1153, 1155 à 1163, 1172 à 1174, 1563 et/ou leurs séquences d'acide nucléique complémentaires (séquences nucléotidiques correspondant aux différentes séquences variables du locus CRISPR2 du sérotype Heidelberg) ;
   - l'ensemble S61-1, comprenant ou constitué d'au moins une sonde comprenant au moins ou constituée d'au moins 8 nucléotides consécutifs de l'une quelconque des séquences d'acide nucléique SEQ ID NO : 558, 1565 à 1571 et/ou leurs séquences d'acide nucléique complémentaires (séquences nucléotidiques correspondant aux différentes séquences variables du locus CRISPR1 du sérotype Hessarek) ;
   - l'ensemble S61-2, comprenant ou constitué d'au moins une sonde comprenant au moins ou constituée d'au moins 8 nucléotides consécutifs de l'une quelconque des séquences d'acide nucléique SEQ ID NO : 2 à 4, 1547, 1572, 1573 et/ou leurs séquences d'acide nucléique complémentaires (séquences nucléotidiques correspondant aux différentes séquences variables du locus CRISPR2 du sérotype Hessarek) ;
   - l'ensemble S62-1, comprenant ou constitué d'au moins une sonde comprenant au moins ou constituée d'au moins 8 nucléotides consécutifs de l'une quelconque des séquences d'acide nucléique SEQ ID NO : 1591, 1592, 1667 et/ou leurs séquences d'acide nucléique complémentaires (séquences nucléotidiques correspondant aux différentes séquences variables du locus CRISPR1 du sérotype Itami) ;
   - l'ensemble S62-2, comprenant ou constitué d'au moins une sonde comprenant au moins ou constituée d'au moins 8 nucléotides consécutifs de l'une quelconque des séquences d'acide nucléique SEQ ID NO : 918, 1593 à 1600, 1744 et/ou leurs séquences d'acide nucléique complémentaires (séquences nucléotidiques correspondant aux différentes séquences variables du locus CRISPR2 du sérotype Itami) ;
   - l'ensemble S63-1, comprenant ou constitué d'au moins une sonde comprenant au moins ou constituée d'au moins 8 nucléotides consécutifs de l'une quelconque des séquences d'acide nucléique SEQ ID NO : 1601 à 1606, 1687 et/ou leurs séquences d'acide nucléique complémentaires (séquences nucléotidiques correspondant aux différentes séquences variables du locus CRISPR1 du sérotype Javiana) ;
   - l'ensemble S63-2, comprenant ou constitué d'au moins une sonde comprenant au moins ou constituée d'au moins 8 nucléotides consécutifs de l'une quelconque des séquences d'acide nucléique SEQ ID NO : 589 à 601, 973, 1607, 1608 et/ou leurs séquences d'acide nucléique complémentaires (séquences nucléotidiques correspondant aux différentes séquences variables du locus CRISPR2 du sérotype Javiana) ;
   - l'ensemble S64-1, comprenant ou constitué d'au moins une sonde comprenant au moins ou constituée d'au moins 8 nucléotides consécutifs de l'une quelconque des séquences d'acide nucléique SEQ ID NO : 1609 à 1621, 1494, 1525 et/ou leurs séquences d'acide nucléique complémentaires (séquences nucléotidiques correspondant aux différentes séquences variables du locus CRISPR1 du sérotype Johannesburg) ;
   - l'ensemble S64-2, comprenant ou constitué d'au moins une sonde comprenant au moins ou constituée d'au moins 8 nucléotides consécutifs de l'une quelconque des séquences d'acide nucléique SEQ ID NO : 918, 1697, 1698 et/ou leurs séquences d'acide nucléique complémentaires (séquences nucléotidiques correspondant aux différentes séquences variables du locus CRISPR2 du sérotype Johannesburg) ;
   - l'ensemble S65-1, comprenant ou constitué d'au moins une sonde comprenant au moins ou constituée d'au moins 8 nucléotides consécutifs de l'une quelconque des séquences d'acide nucléique SEQ ID NO : 444, 448, 1184, 1622 à 1637, et/ou leurs séquences d'acide nucléique complémentaires (séquences nucléotidiques correspondant aux différentes séquences variables du locus CRISPR1 du sérotype Kentucky) ;
   - l'ensemble S65-2, comprenant ou constitué d'au moins une sonde comprenant au moins ou constituée d'au moins 8 nucléotides consécutifs de l'une quelconque des séquences d'acide nucléique SEQ ID NO : 385, 519, 1638 à 1652, 1746 et/ou leurs séquences d'acide nucléique complémentaires (séquences nucléotidiques correspondant aux différentes séquences variables du locus CRISPR2 du sérotype Kentucky) ;
   - l'ensemble S66-I, comprenant ou constitué d'au moins une sonde comprenant au moins ou constituée d'au moins 8 nucléotides consécutifs de la séquence d'acide nucléique SEQ ID NO : 67 et/ou sa séquence d'acide nucléique complémentaire (séquences nucléotidiques correspondant à la séquence variable du locus CRISPR1 du sérotype Kundunchi) ;
   - l'ensemble S66-2, comprenant ou constitué d'au moins une sonde comprenant au moins ou constituée d'au moins 8 nucléotides consécutifs de l'une quelconque des séquences d'acide nucléique SEQ ID NO : 972, 983, 989, 1201, 1204, 1205, 1210 à 1212 et/ou leurs séquences d'acide nucléique complémentaires (séquences nucléotidiques correspondant aux différentes séquences variables du locus CRISPR2 du sérotype Kundunchi) ;
   - l'ensemble S67-1, comprenant ou constitué d'au moins une sonde comprenant au moins ou constituée d'au moins 8 nucléotides consécutifs de l'une quelconque des séquences d'acide nucléique SEQ ID NO : 448, 449, 860, 871, 878 à 881, 1721, 1724 et/ou leurs séquences d'acide nucléique complémentaires (séquences nucléotidiques correspondant aux différentes séquences variables du locus CRISPR1 du sérotype Lindenburg) ;
   - l'ensemble S67-2, comprenant ou constitué d'au moins une sonde comprenant au moins ou constituée d'au moins 8 nucléotides consécutifs de l'une quelconque des séquences d'acide nucléique SEQ ID NO : 563, 622, 885, 886, 896, 907, 910 à 915, 933, 1748, 1757 et/ou leurs séquences d'acide nucléique complémentaires (séquences nucléotidiques correspondant aux différentes séquences variables du locus CRISPR2 du sérotype Lindenburg) ;
   - l'ensemble S68-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variables du locus CRISPR1 du sérotype Maracaibo) ;
   - l'ensemble S68-2, comprenant ou constitué d'au moins une sonde comprenant au moins ou constituée d'au moins 8 nucléotides consécutifs de l'une quelconque des séquences d'acide nucléique SEQ ID NO : 1240, 1247, 1248, 1654 à 1656 et/ou leurs séquences d'acide nucléique complémentaires (séquences nucléotidiques correspondant aux différentes séquences variables du locus CRISPR2 du sérotype Maracaibo) ;
   - l'ensemble S69-1, comprenant ou constitué d'au moins une sonde comprenant au moins ou constituée d'au moins 8 nucléotides consécutifs de l'une quelconque des séquences d'acide nucléique SEQ ID NO : 627, 834, 1120, 1660 à 1669 et/ou leurs séquences d'acide nucléique complémentaires (séquences nucléotidiques correspondant aux différentes séquences variables du locus CRISPR1 du sérotype Mississippi) ;
   - l'ensemble S69-2, comprenant ou constitué d'au moins une sonde comprenant au moins ou constituée d'au moins 8 nucléotides consécutifs de l'une quelconque des séquences d'acide nucléique SEQ ID NO: 842, 1670 et/ou leurs séquences d'acide nucléique complémentaires (séquences nucléotidiques correspondant aux différentes séquences variables du locus CRISPR2 du sérotype Mississippi) ;
   - l'ensemble S70-1, comprenant ou constituée d'au moins une sonde comprenant au moins ou constituée d'au moins 8 nucléotides consécutifs de l'une quelconque des séquences d'acide nucléique SEQ ID NO : 941, 942, 949, 1699 à 1712, 2109 et/ou leurs séquences d'acide nucléique complémentaires (séquences nucléotidiques correspondant aux différentes séquences variables du locus CRISPR1 du sérotype Muenchen) ;
   - l'ensemble S70-2, comprenant ou constitué d'au moins une sonde comprenant au moins ou constituée d'au moins 8 nucléotides consécutifs de l'une quelconque des séquences d'acide nucléique SEQ ID NO : 392, 1653 et/ou leurs séquences d'acide nucléique complémentaires (séquences nucléotidiques correspondant aux différentes séquences variables du locus CRISPR2 du sérotype Muenchen) ;
   - l'ensemble S71-1, comprenant ou constitué d'au moins une sonde comprenant au moins ou constituée d'au moins 8 nucléotides consécutifs de l'une quelconque des séquences d'acide nucléique SEQ ID NO: 11, 1120, 1497, 1760 à 1775, 1809 et/ou leurs séquences d'acide nucléique complémentaires (séquences nucléotidiques correspondant aux différentes séquences variables du locus CRISPR1 du sérotype Niarembe) ;
   - l'ensemble S71-2, comprenant ou constitué d'au moins une sonde comprenant au moins ou constituée d'au moins 8 nucléotides consécutifs de l'une quelconque des séquences d'acide nucléique SEQ ID NO : 90, 1776 à 1785 et/ou leurs séquences d'acide nucléique complémentaires (séquences nucléotidiques correspondant aux différentes séquences variables du locus CRISPR2 du sérotype Niarembe) ;
   - l'ensemble S72-1, comprenant ou constitué d'au moins une sonde comprenant au moins ou constituée d'au moins 8 nucléotides consécutifs de l'une quelconque des séquences d'acide nucléique SEQ ID NO : 666, 1786 à 1791 et/ou leurs séquences d'acide nucléique complémentaires (séquences nucléotidiques correspondant aux différentes séquences variables du locus CRISPR1 du sérotype Overvecht) ;
   - l'ensemble S72-2, comprenant ou constitué d'au moins une sonde comprenant au moins ou constituée d'au moins 8 nucléotides consécutifs de l'une quelconque des séquences d'acide nucléique SEQ ID NO : 783, 1146, 1178, 1179, 1181, 1792 à 1799, 1801 et/ou leurs séquences d'acide nucléique complémentaires (séquences nucléotidiques correspondant aux différentes séquences variables du locus CRISPR2 du sérotype Overvecht) ;
   - l'ensemble S73-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Poona) ;
   - l'ensemble S73-2, comprenant ou constitué d'au moins une sonde comprenant au moins ou constituée d'au moins 8 nucléotides consécutifs de l'une quelconque des séquences d'acide nucléique SEQ ID NO : 82, 87, 918, 933, 1826 et/ou leurs séquences d'acide nucléique complémentaires (séquences nucléotidiques correspondant aux différentes séquences variables du locus CRISPR2 du sérotype Poona) ;
   - l'ensemble S74-1, comprenant ou constitué d'au moins une sonde comprenant au moins ou constituée d'au moins 8 nucléotides consécutifs de l'une quelconque des séquences d'acide nucléique SEQ ID NO : 295 à 297, 307, 313, 317, 323, 1120, 1825, 1827 à 1837, 2023 et/ou leurs séquences d'acide nucléique complémentaires (séquences nucléotidiques correspondant aux différentes séquences variables du locus CRISPR1 du sérotype Postdam) ;
   - l'ensemble S74-2, comprenant ou constitué d'au moins une sonde comprenant au moins ou constituée d'au moins 8 nucléotides consécutifs de l'une quelconque des séquences d'acide nucléique SEQ ID NO : 663, 1304, 1838 à 1847 et/ou leurs séquences d'acide nucléique complémentaires (séquences nucléotidiques correspondant aux différentes séquences variables du locus CRISPR2 du sérotype Postdam) ;
   - l'ensemble S75-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Reading) ;
   - l'ensemble S75-2, comprenant ou constitué d'au moins une sonde comprenant au moins ou constituée d'au moins 8 nucléotides consécutifs de la séquences d'acide nucléique SEQ ID NO : 225 et/ou sa séquence d'acide nucléique complémentaire (séquences nucléotidiques correspondant à la séquence variable du locus CRISPR2 du sérotype Reading) ;
   - l'ensemble S76-1, comprenant ou constitué d'au moins une sonde comprenant au moins ou constituée d'au moins 8 nucléotides consécutifs de l'une quelconque des séquences d'acide nucléique SEQ ID NO : 379, 380, 383 à 385, 387, 389, 390 et/ou leurs séquences d'acide nucléique complémentaires (séquences nucléotidiques correspondant aux différentes séquences variables du locus CRISPR1 du sérotype Rosenberg) ;
   - l'ensemble S76-2, comprenant ou constitué d'au moins une sonde comprenant au moins ou constituée d'au moins 8 nucléotides consécutifs de l'une quelconque des séquences d'acide nucléique SEQ ID NO : 392, 393, 404, 407 à 413 et/ou leurs séquences d'acide nucléique complémentaires (séquences nucléotidiques correspondant aux différentes séquences variables du locus CRISPR2 du sérotype Rosenberg) ;
   - l'ensemble S77-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Rubislaw) ;
   - l'ensemble S77-2, comprenant ou constitué d'au moins une sonde comprenant au moins ou constituée d'au moins 8 nucléotides consécutifs de l'une quelconque des séquences d'acide nucléique SEQ ID NO : 225 et/ou sa séquence d'acide nucléique complémentaire (séquences nucléotidiques correspondant à la séquence variable du locus CRISPR1 du sérotype Rubislaw) ;
   - l'ensemble S78-1, comprenant ou constitué d'au moins une sonde comprenant au moins ou constituée d'au moins 8 nucléotides consécutifs de la séquence d'acide nucléique SEQ ID NO : 1525, 1609 à 1621 et/ou leurs séquences d'acide nucléique complémentaires (séquences nucléotidiques correspondant aux différentes séquences variables du locus CRISPR2 du sérotype Sandiego) ;
   - l'ensemble S78-2, comprenant ou constitué d'au moins une sonde comprenant au moins ou constituée d'au moins 8 nucléotides consécutifs de l'une quelconque des séquences d'acide nucléique SEQ ID NO : 225 à 227, 229 à 231 et/ou leurs séquences d'acide nucléique complémentaires (séquences nucléotidiques correspondant aux différentes séquences variables du locus CRISPR2 du sérotype Sandiego) ;
   - l'ensemble S79-1, comprenant ou constitué d'au moins une sonde comprenant au moins ou constituée d'au moins 8 nucléotides consécutifs de l'une quelconque des séquences d'acide nucléique SEQ ID NO : 1106, 1456, 1917 à 1924 et/ou leurs séquences d'acide nucléique complémentaires (séquences nucléotidiques correspondant aux différentes séquences variables du locus CRISPR1 du sérotype Stourbridge) ;
   - l'ensemble S79-2, comprenant ou constitué d'au moins une sonde comprenant au moins ou constituée d'au moins 8 nucléotides consécutifs de l'une quelconque des séquences d'acide nucléique SEQ ID NO : 918, 1093, 1925 à 1936 et/ou leurs séquences d'acide nucléique complémentaires (séquences nucléotidiques correspondant aux différentes séquences variables du locus CRISPR2 du sérotype Stourbridge) ;
   - l'ensemble S80-1, comprenant ou constitué d'au moins une sonde comprenant au moins ou constituée d'au moins 8 nucléotides consécutifs de l'une quelconque des séquences d'acide nucléique SEQ ID NO : 1961 à 1963 et/ou leurs séquences d'acide nucléique complémentaires (séquences nucléotidiques correspondant aux différentes séquences variables du locus CRISPR1 du sérotype Tallahassee) ;
   - l'ensemble S80-2, comprenant ou constitué d'au moins une sonde comprenant au moins ou constituée d'au moins 8 nucléotides consécutifs de l'une quelconque des séquences d'acide nucléique SEQ ID NO : 25, 603, 2127, 2133, 1964 à 1966 et/ou leurs séquences d'acide nucléique complémentaires (séquences nucléotidiques correspondant aux différentes séquences variables du locus CRISPR2 du sérotype Tallahassee) ;
   - l'ensemble S81-1, comprenant ou constitué d'au moins une sonde comprenant au moins ou constituée d'au moins 8 nucléotides consécutifs de l'une quelconque des séquences d'acide nucléique SEQ ID NO : 67, 929, 1967 à 2005 et/ou leurs séquences d'acide nucléique complémentaires (séquences nucléotidiques correspondant aux différentes séquences variables du locus CRISPR1 du sérotype Tennessee) ;
   - l'ensemble S81-2, comprenant ou constitué d'au moins une sonde comprenant au moins ou constituée d'au moins 8 nucléotides consécutifs de l'une quelconque des séquences d'acide nucléique SEQ ID NO : 24, 769, 1550, 1659, 2006 à 2022 et/ou leurs séquences d'acide nucléique complémentaires (séquences nucléotidiques correspondant aux différentes séquences variables du locus CRISPR2 du sérotype Tennessee) ;
   - l'ensemble S82-1, comprenant ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Urbana) ;
   - l'ensemble S82-2, comprenant ou constitué d'au moins une sonde comprenant au moins ou constituée d'au moins 8 nucléotides consécutifs de l'une quelconque des séquences d'acide nucléique SEQ ID NO : 1697, 1698, 1800 et/ou leurs séquences d'acide nucléique complémentaires (séquences nucléotidiques correspondant aux différentes séquences variables du locus CRISPR2 du sérotype Urbana) ;
   - l'ensemble S83-1, comprenant ou constitué d'au moins une sonde comprenant au moins ou constituée d'au moins 8 nucléotides consécutifs de l'une quelconque des séquences d'acide nucléique SEQ ID NO : 948, 1120, 1123, 1325, 2039 à 2078 et/ou leurs séquences d'acide nucléique, complémentaires (séquences nucléotidiques correspondant aux différentes séquences variables du locus CRISPR1 du sérotype Weltevreden) ;
   - l'ensemble S83-2, comprenant ou constitué d'au moins une sonde comprenant au moins ou constituée d'au moins 8 nucléotides consécutifs de l'une quelconque des séquences d'acide nucléique SEQ ID NO : 254 à 256, 2079 à 2108 et/ou leurs séquences d'acide nucléique complémentaires (séquences nucléotidiques correspondant aux différentes séquences variables du locus CRISPR2 du sérotype Weltevreden) ;
   - l'ensemble S84-1, comprenant ou constitué d'au moins une sonde comprenant au moins ou constituée d'au moins 8 nucléotides consécutifs de l'une quelconque des séquences d'acide nucléique SEQ ID NO : 1312, 1690, 2141, 2142 et/ou leurs séquences d'acide nucléique complémentaires (séquences nucléotidiques correspondant aux différentes séquences variables du locus CRISPR1 du sérotype Zaiman) ;
   - l'ensemble S84-2, comprenant ou constitué d'au moins une sonde comprenant au moins ou constituée d'au moins 8 nucléotides consécutifs de l'une quelconque des séquences d'acide nucléique SEQ ID NO : 1697, 1698, 1800, 1924, 2143, 2144 et/ou leurs séquences d'acide nucléique complémentaires (séquences nucléotidiques correspondant aux différentes séquences variables du locus CRISPR2 du sérotype Zaiman) ; et
(ii) détection des sondes hybridées.

Selon un mode de mise en oeuvre avantageux de cet aspect de l'invention, lorsque les deux premiers nucléotides (en position 5') desdites séquences d'acide nucléique sont le dinucléotide « AC » ou le dinucléotide « AT », alors lesdites sondes comprenent au moins ou sont constituées d'au moins 8 nucléotides consécutifs de ces séquences d'acide nucléique sans lesdits nucléotides « AC » ou « AT » (c'est-à-dire dans lequelles lesdits nucléotides « AC » ou « AT » sont absents) et/ou leurs séquences d'acide nucléique complémentaires.

Au sens de la présente invention, on entend par le terme « au moins une sonde », une, plusieurs ou toutes les sondes de l'un des ensembles de sondes définis ci-dessus.

Le terme « sonde » désigne un oligonucléotide, constitué d'au moins 8 nucléotides, présentant les mêmes caractéristiques techniques qu'une amorce (définie ci-dessus), à l'exception que son extrémité 3'OH peut ne pas être apte à fixer une ADN polymérase. Une sonde (oligonucléotidique) possède une spécificité d'hybridation dans des conditions appropriées (notamment indiquées ci-après) pour former un complexe d'hybridation avec un acide nucléique cible. Les sondes selon la présente invention peuvent être synthétisées ou être dérivées de préparations biologiques appropriées selon des méthodes bien connues de l'Homme du métier. De préférence, les sondes selon la présente invention sont de l'ADN et présentent une longueur inférieure ou égale à 150 nucléotides, c'est-à-dire inférieure ou égale à 140, 130, 120, 110, 100, 90, 80, 70, 60, 50, 40, 30, 20 ou 10 nucléotides. De préférence encore, les sondes selon la présente invention présentent une longueur comprise entre 8 et 76 nucléotides, de préférence encore entre 8 et 34 nucléotides.

Selon une disposition avantageuse du mode de mise en oeuvre ci-dessus, l'étape (b) est réalisée par hybridation avec au moins une sonde de chacun des ensembles S1-1 à S6-2 (ensembles de sondes permettant de typer et/ou de sous-typer les bactéries du genre *Salmonella* de sérotype Typhimurium, Enteritidis, Hadar, Virchow, Infantis et Typhi).

Selon une autre disposition avantageuse du mode de mise en oeuvre ci-dessus, l'étape (b) est réalisée par hybridation avec au moins une sonde de chacun des ensembles S1-1 à S10-2 (ensembles de sondes permettant de typer et/ou de sous-typer les bactéries du genre *Salmonella,* outre celles de sérotypes précédemment cités, celles de sérotypes Newport, Derby, Kottbus et Indiana).

Selon encore une autre disposition avantageuse du mode de mise en oeuvre ci-dessus, l'étape (b) est réalisée par hybridation avec au moins une sonde de chacun des ensembles S1-1 à S44-2 (ensembles de sondes permettant de typer et/ou de sous-typer l'ensemble des sérotypes des bactéries du genre *Salmonella* ci-dessus mentionnés).

La présente invention a également pour objet une sonde ou un ensemble de sondes apte(s) à identifier une bactérie du genre *Salmonella,* chaque sonde comprenant au moins ou étant constituée d'au moins 8 nucléotides consécutifs de l'une quelconque des séquences d'acide nucléique sélectionnées dans le groupe constitué par les séquences d'acide nucléique SEQ ID NO : 1 à 256, 258 à 614, 616 à 876, 878 à 1043, 1045 à 1147, 1149 à 1302, 1304 à 1325, 1333 à 2150 et leurs séquences d'acide nucléique complémentaires.

Les sondes et amorces selon la présente invention sont aptes à s'hybrider à un acide nucléique cible dans des conditions stringentes telles que décrites ci-dessous ou bien connues de l'Homme de l'art (voir par exemple Sambrook J. et al., Molecular Cloning: A Laboratory Manual. 2000. Third Edition ; Ausubel FM. et al., Current Protocols in Molecular Biology, Greene Publishing Assoc. and Wiley-Interscience 1987 & Suppl. 49, 2000). Typiquement, les paramètres définissant les conditions de stringence dépendent de l'emploi (i) d'une force ionique faible et d'une température élevée pour le lavage, par exemple 0,015 M de chlorure de sodium/0,0015 M de citrate de sodium/0,1% de sodium dodécylsulfate (SDS) à 50°C ; (ii) d'un agent dénaturant durant l'étape d'hybridation de l'amorce ou de la sonde avec un acide nucléique cible, tel que le formamide, par exemple, 50% (v/v) de formamide avec 0,1% de sérum albumine bovine (BSA)/0,1% de Ficoll/0,1% de Polyvinylpyrrolidone/50 mM de tampon phosphate de sodium à pH 6,5 avec 750 mM de chlorure de sodium, 75 mM de citrate de sodium à 42°C; ou (iii) de 50% de formamide, SSC 5x (0,75 M NaCl, 0,075 M de citrate de sodium), 50 mM de phosphate de sodium (pH 6,8), 0,1% de pyrophosphate de sodium, solution de Denhard 5x, d'ADN de sperme de saumon soniqué (50 µg/ml), 0,1% de SDS, et 10% de sulfate dextran à 42°C, avec des lavages à 42°C dans du SSC 0,2x (chlorure de sodium/citrate de sodium) et 50% de formamide à 55°C, suivi d'un lavage dans des conditions de forte stringence consistant en SSC 0,1x contenant de l'EDTA à 55°C.

Selon ce mode de mise en oeuvre avantageux de la méthode conforme à l'invention, le produit d'amplification comprenant les loci CRISPR1 et/ou CRISPR2, est marqué, hybridé à au moins une des sondes telles que définies ci-dessus et détecté. Par exemple, le produit d'amplification (PCR) peut être marqué par une cyanine de type Cy5. Le produit marqué (c'est-à-dire liées de manière opérationnelle à au moins un marqueur) est alors fragmenté, de façon mécanique ou chimique, puis est hybridé (par exemple à une température de 42°C) sur une puce de type microarray comprenant au moins une des sondes telles que définies ci-dessus synthétisées *in situ.* Après lavage, les signaux d'hybridation sont détectés (par exemple à l'aide d'un scanner de fluorescence).

Le terme « marqueur » désigne un atome ou une molécule capable de générer directement ou indirectement un signal détectable et/ou quantifiable, et qui peut être lié de manière opérationnelle à un acide nucléique. Le signal généré par un marqueur peut être détecté par exemple par fluorescence, luminescence, radioactivité, colorimétrie, gravimétrie, diffraction ou absorption des rayons X, magnétisme, réaction enzymatique, spectrométrie de masse, affinité de liaison, hybridation, radiofréquence ou nanocristaux. Le marquage des acides nucléiques s'effectue par des méthodes connues en elles-mêmes de l'Homme du métier. A titre d'exemple, un acide nucléique peut être marqué par un radioisotope (par exemple, ³²P), un flurochrome tel que les cyanines Cy3 et Cy5, une sonde froide telle que la la biotine (dUTP-biotine) (la biotine est un ligand de l'avidine ou de la streptavidine, qui elle-même peut être couplée à un fluorochrome, à une enzyme ou captée par un anticorps anti-avidine dont le signal est amplifié). D'autres alternatives comprennent l'utilisation d'un marquage par la digoxigénine (dUTP-digoxigénine) révélé par des anticorps anti-digoxigénine ou par un couplage en présence de glutaraldéhyde à la peroxydase qui sera révélé rapidement sur film radiographique par bioluminescence grâce à l'hydrazure d'acide 3-aminophtalique (luminol).

Les amorces et sondes selon la présente invention peuvent aussi être marquées de manière à ce que les produits d'amplification génique ou d'hybridation respectivement soient détectés à l'aide du signal généré par le marqueur auquel elles sont liées.

L'échantillon selon la présente invention peut être d'origine très variée. A titre d'exemple, l'échantillon peut être un prélèvement d'origine alimentaire, humaine, animale ou environnementale (terre, eau ou déchet par exemple).

De préférence, l'échantillon est une souche bactérienne du genre *Salmonella* isolée.

Au sens de la présente invention, le terme « isolé » signifie qu'une culture pure d'une souche bactérienne du genre *Salmonella* a été obtenue à partir d'un mélange de souches bactériennes ou d'un prélèvement d'origine alimentaire, humaine, animale ou environnementale. L'isolement d'une souche bactérienne s'effectue par des méthodes classiques (par exemple : technique des stries, technique de suspension-dilution), connues en elles-mêmes de l'Homme du métier.

Selon un mode de mise en oeuvre avantageux conforme à l'invention, la méthode selon l'invention est précédée d'une étape d'extraction d'acides nucléiques présents dans ledit échantillon. Les différentes techniques d'extraction d'ADN bactérien sont bien connues de l'Homme du métier.

La présente invention a également pour objet un réactif de détection ou d'identification du type et du sous-type moléculaire d'une bactérie du genre *Salmonella.* Ledit réactif est sélectionné dans le groupe constitué par au moins un ensemble de sondes et/ou un ensemble d'amorces tels que définis ci-dessus.

La présente invention a également pour objet une puce à ADN comprenant les sondes comprises dans au moins l'un ou tous les ensembles de sondes tels que définis ci-dessus (S1-1 à S44-2), ou les sondes comprises dans les ensembles de sondes S1-1 à S10-2, ou encore les sondes comprises dans les ensembles de sondes S1-1 à S6-2.

Le terme « puce à ADN » désigne un ensemble de taches ou points (« spots ») liés à un support solide (par exemple, lame de verre, silicium, métal, silicone, gel, céramique, membrane de nylon ou plastique) ; chaque tache contenant indépendamment un ou plusieurs oligonucléotides (par exemples les sondes de la présente invention) qui y ont été déposés ou synthétisés *in situ.* Les puces à ADN sont connues sous différentes dénomination, telles que par exemple « biochips », « arrays », « microarrays » ou « DNA-microarrays ». Les taches (« spots ») peuvent présenter une taille allant de quelques millimètres à quelques micromètres. Une seule puce à ADN peut contenir d'une dizaine à plusieurs milliers de taches, chaque tache contenant une molécule d'acide nucléique (par exemples une sonde) différente. En général, une composition contenant l'ADN à tester est mise en contact avec la puce à ADN ; l'hybridation entre un oligonucléotide (une sonde) et l'ADN à tester (par exemple un fragment d'acide nucléique comprenant le locus CRISPR1 et/ou le locus CRISPR2 de *Salmonella*) peut alors se produire dans des conditions connues de l'Homme du métier (D. Amaratunga et al., Exploration and Analysis of DNA Microarray and Protein Array Data, John Wiley & Sons, Inc., 2003). La détection de l'hybridation permet alors de déterminer la présence d'une séquence d'ADN recherchée (par exemple la séquence variable d'un locus CRISPR de *Salmonella*).

La présente invention a également pour objet un kit ou coffret de détection ou d'identification du type et/ou du sous-type moléculaire d'une bactérie du genre *Salmonella,* comprenant au moins un ensemble d'amorces et/ou un ensemble de sondes, une base de référence tels que définis ci-dessus ou une partie de cette base de référence et optionnellement une puce à ADN telle que définie ci-dessus.

La présente invention a également pour objet l'utilisation d'un ensemble d'amorces, d'un ensemble de sondes, ou d'une puce à ADN tels que définis ci-dessus, pour détecter ou identifier le type et/ou le sous-type moléculaire d'une bactérie du genre *Salmonella*.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la Figure 1 représente schématiquement l'amplification génique séparée (Figure 1A) ou au cours d'une même réaction (Figure 1B) des loci CRISPR1 et CRISPR2 de *Salmonella.*

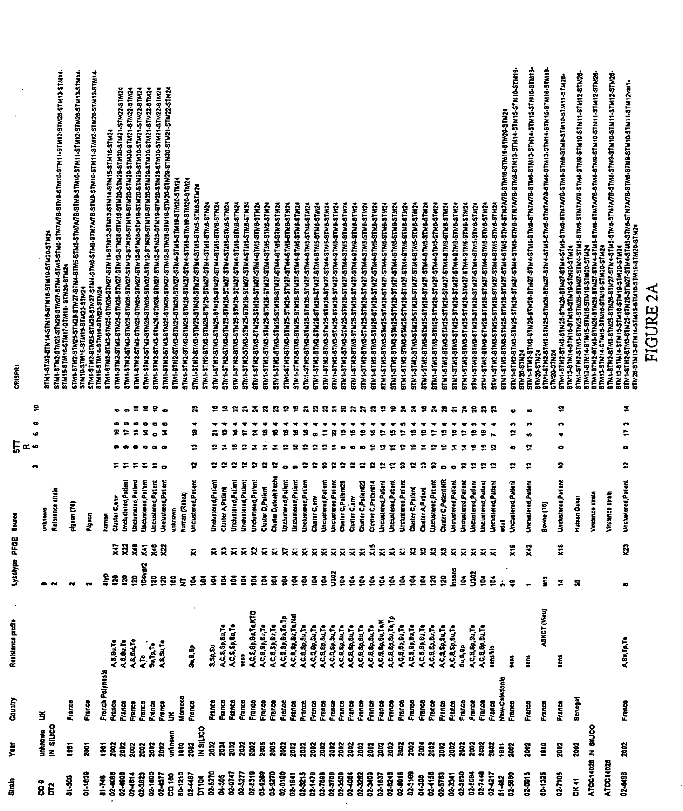

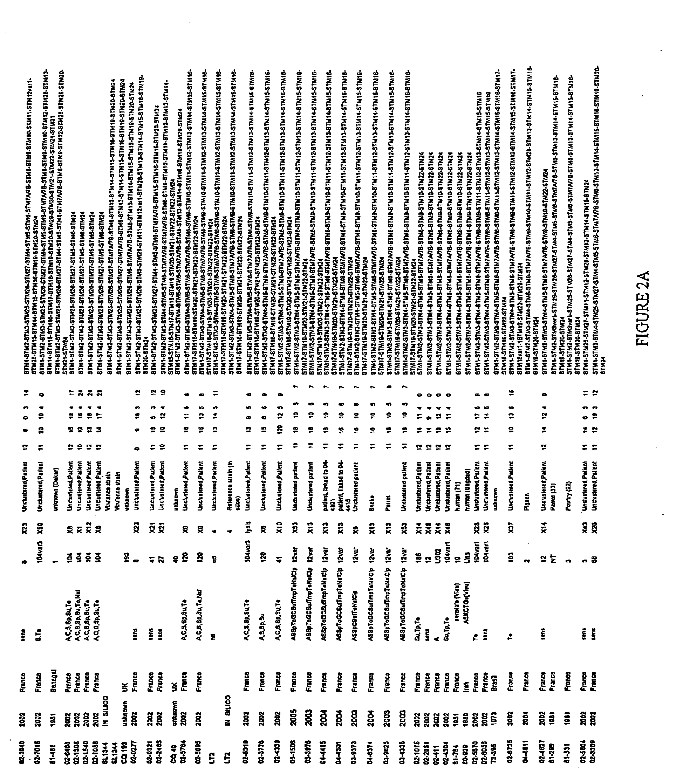

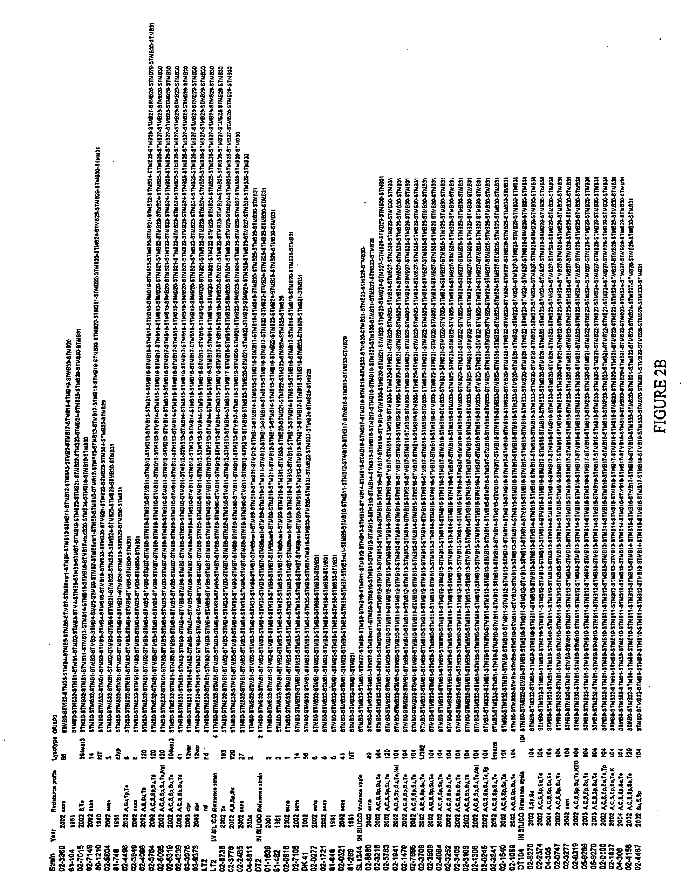

- la Figure 2 indique la composition en séquences variables (spacers) des loci CRISPR1 (Figure 2A) et CRISPR2 (Figure 2B) de 91 souches de *Salmonella* de sérotype Typhimurium. Sont aussi représentés les résultats du sous-typage classique : antibiotype (A = amoxicilline, C = chloramphénicol, Cip = ciprofloxacine, G = gentamicine, K = kanamycine, Nal = acide nalidixique, S = streptomycine, Sp = spectinomycine, Su ou Sul = sulfamides, T ou To = tobramycine, Te = tétracycline, Tp ou Tmp = triméthoprime, sens = sensible), lysotype (Anderson et al., 1977, J. Hyg. (Lond) 78, 297-300), profil d'électrophorèse en champ pulsé (PFGE, méthode décrite dans Weill et al., 2006, J. Clin. Microbiol. 44, 700-708; Weill et al., 2004, J. Clin. Microbiol. 42, 2432-2437), STTR (type selon la méthode « Multiple-Locus Variable-Number Tandem Repeat Analysis », méthode décrite dans Lindstedt et al., (2003, J. Clin. Microbiol. 41, 1469-1479) et des données épidémiologiques (origine humaine ou animale).

- la Figure 3 indique la composition en séquences variables (spacers) des loci CRISPR1 (Figure 3A) et CRISPR2 (Figure 3B) de 154 souches de *Salmonella* de sérotype Enteritidis. Sont aussi représentés les résultats du sous-typage classique et des données épidémiologiques (légende identique à celle de la Figure 2).
- la Figure 4 indique la composition en séquences variables (spacers) des loci CRISPR1 (Figure 4A) et CRISPR2 (Figure 4B), respectivement de 95 et 85 souches de *Salmonella* n'appartenant ni au sérotype Typhimurium ni au sérotype Enteritidis. Sont aussi représentés les résultats du sous-typage classique et des données épidémiologiques (cas sporadiques, épidémiques et origine des isolats).
- La Figure 5 montre la variation de la composition en séquences variables des CRISPR1 (Figure 5A) et CRISPR2 (Figure 5B) de quelques sérotypes de souches de *Salmonella.*
- Les Figures 6A et 6B représentent le résultat du séquençage (5'-3') des loci CRISPR1 et CRISPR2 respectivement d'une souche de *Salmonella* à identifier. Les séquences répétées ou Direct Repeats (DR) sont grisées (en caractère gras et italique sont représentés les nucléotides variables au sein des DR). Les séquences variables (spacers) sont encadrées.
- Les Figures 7A et 7B représentent le résultat du séquençage (5'-3') des loci CRISPR1 et CRISPR2 respectivement d'une souche de *Salmonella* à identifier (légende identique à celle de la Figure 6).
- La Figure 8 représente le résultat du séquençage (5'-3') du locus CRISPR1 d'une souche de *Salmonella* à identifier (légende identique à celle de la Figure 6).
- La Figure 9 indique pour chaque séquence variable des loci CRISPR de *Salmonella* le nom qui lui a été attribué.

La Figure 10 indique la composition en séquences variables (spacers) des loci CRISPR1 (Figure 10A) et CRISPR2 (Figure 10B) de 564 souches de *Salmonella.* Sont aussi représentés les résultats du sous-typage classique et des données épidémiologiques (légende identique à celle de la Figure 2). Cette figure comprend la composition en séquences variables des loci CRISPR1 et CRISPR2 de types et sous-types des bactéries du genre *Salmonella* listées aux Figures 2, 3 et 4.

Il doit être bien entendu, que ces exemples sont donnés uniquement à titre d'illustration de l'objet de l'invention, dont ils ne constituent en aucune manière une limitation.

### EXEMPLE 1: CONSTITUTION DE LA BASE DE REFERENCE REPERTORIANT LA COMPOSITION DES SEQUENCES VARIABLES DES DEUX LOCI CRISPR1 ET CRISPR2 DE SALMONELLA

### 1) Matériels et Méthodes

### 1-1) Conception des amorces oligonucléotidiques pour amplifier les loci CRISPR1 et/ou CRISPR2 chez Salmonella

Les amorces, aptes à amplifier les loci CRISPR1 et/ou CRISPR2 des bactéries du genre *Salmonella,* ont été conçues à partir des séquences génomiques de :
- *S*. *enterica* sérotype Typhimurium LT2 (génome disponible sous le numéro AE008834 dans la base de données GenBank en date du 9 août 2005),
- *S. enterica* subsp. *enterica* sérotype Choleraesuis souche SC-B67 (génome disponible sous le numéro AE017220 dans la base de données GenBank en date du 11 janvier 2006),
- *S. enterica* subsp. *enterica* sérotype Paratyphi A souche ATCC 9150 (génome disponible sous le numéro CP000026 dans la base de données GenBank en date du 8 avril 2005),
- *S. enterica* sérotype Typhi souche CT18 (génome disponible sous le numéro AL627276 dans la base de données GenBank en date du 14 novembre 2006),
- *S. enterica* sérotype Enteritidis PT4 NCTC 13349 (génome disponible sur le site internet de Sanger Institute à l'adresse suivante : http://www.sanger.ac.uk/Projects/Salmonella/),
- *S. bongori* 12419 ATCC 43975 (génome disponible sur le site internet de Sanger Institute à l'adresse suivante : http://www.sanger.ac.uk/Projects/Salmonella/, et au lien suivant : http://genome.wustl.edu/sub_genome_group.cgi?GROUP=3&SUB_GROUP=3),
- *S. enterica subsp.arizonae* de sérotype 62:z4,z23:- (génome disponible sous le numéro CP000880 dans la base de données GenBank en date du 28 novembre 2007) et *S. enterica* subsp. *diarizonae* de sérotype 61:1,v:1,5,7 (génomes disponibles sur internent au lien suivant : http://genome.wustl.edu/sub_genome_group.cgi?GROUP=3&SUB_GROUP=3)

### Amorces aptes à amplifier le locus CRISPR1 :

La position des nucléotides est donnée en référence au génome de *S*. *enterica* sérotype Typhimurium LT2, disponible sous le numéro AE008834 dans la base de données GenBank en date du 9 août 2005 ou de *S. enterica* sérotype Typhi CT18, disponible sous le numéro AL627276 dans la base de données GenBank en date du 14 novembre 2006.

Amorce sens (« forward ») dénommée SALCRISP1-FB (= A1) (position des nucléotides : AE008834, 1109-1128) : 5'-GT(A/G)GT(A/G)CGGATAATGCTGCC-3' (SEQ ID NO: 1326)

Amorce antisens (« reverse ») dénommée SALCRISP1-RB (= A2) (position des nucléotides : AE008834, 2878-2856) : 5'-CGTATTCCGGTAGAT(G/C/T)T(A/G/T)GATGG-3' (SEQ IDNO : 1327)

Amorce dénommée AriParaB-R (= A3) (« reverse ») (position des nucléotides : AE008834, 10289-10310) : 5'-CTATTTTGG(A/G)CT(A/G)CCGAC(A/G)ATG-3' (SEQ ID NO : 1328)
Cette amorce sert à amplifier les souches de *Salmonella* de la sous-espèce *arizonae,* les souches septicémiques de sérotype Paratyphi B, certaines souches de la sous-espèce *indica*, et la majorité des souches de sérotype Mbandaka. Ces souches ont une délétion en aval du locus CRISPR1.

Amorce dénommée BrPanCR1-R (= A4) (« reverse ») (position des nucléotides : AL627276, 232226-232206) :5'-GCAATCGGAGCGATTGATGGC-3' (SEQ ID NO : 1329)
Cette amorce sert à amplifier les souches des sérotypes Brandenburg et Panama. Ces souches ont une délétion en avant du locus CRISPR1.

Amorce dénommée 50K (= A5) (« reverse ») (position des nucléotides : AL627276, 233322-233341) : 5'-TCAACACTCTCTTCACCCAG-3' (SEQ ID NO: 2151).
Cette amorce sert à amplifier certains des sérotypes Newport and Bardo.

Amorce dénommée HoutWS24R (= A6) (« reverse ») (position des nucléotides: AL627276 (233298-233017) : 5'-TAACCAGCCCTCTTCTGCCTG-3' (SEQ ID NO: 2152)
Cette amorce sert à amplifier certaines souches de la sous-espèce *houtenae.*

### Amorces aptes à amplifier le locus CRISPR2 :

La position des nucléotides est donnée en référence au génome de *S*. *enterica* sérotype Typhimurium LT2, disponible sous le numéro AE008834 ou AE008835 dans la base de données GENBANK en date du 9 août 2005.

Amorce sens (« forward ») dénommée SALCRISP2-FB (= B1) (position des nucléotides: AE008834, 18727-18751) : 5'-GAGCAATAC(C/T)(C/T)T(A/G)ATCGTTAACGCC-3' (SEQ ID NO: 1330) (région manquante chez la sous-espèce *S. arizonae*)

Amorce antisens 1 (« reverse ») dénommée SALCRISP2-RA (= B2) (position des nucléotides : AE008835, 931-906) 5'-GTTGC(A/G/T)ATA(G/T)GT(C/T)G(A/G)T(A/G)G(A/G)ATGT(A/G)G-3' (SEQ ID NO : 1331) (région manquante chez la sous-espèce *diarizonae*)

Amorce antisens 2 dénommée SALCRISP2-RB (= B3) (position des nucléotides : AE008835, 1205-1185) 5'-CTGGCGGCTGTCTATGCAAAC-3' (SEQ IDNO : 1332)
Cette séquence présente cependant un mésappariement chez *S. enterica* sérotype Typhimurium LT2 en position 1201.

### 1-2) Amplification des loci CRISPR1 et CRISPR2

### 1-2-1) Souches de Salmonella utilisées

Les souches de *Salmonelle* analysées proviennent de la collection du Centre National de Référence des *Salmonella* et du Centre Collaborateur OMS des *Salmonella,* Unité Biodiversité des Bactéries Pathogènes Emergentes, 25 rue du Docteur Roux, 75724 Paris, Cedex 15. Les souches sont d'origine humaine ou animale ou alimentaire isolées de 1885 à 2005. Elles proviennent de cas sporadiques ou ont été collectées lors d'épidémies investiguées.

564 souches appartenant à 86 sérotypes des 2 espèces et 6 sous espèces de *Salmonella* ont été analysées (entre parenthèses, le nombre de souches analysées) :
i) l'espèce *S. enterica*
   - sous-espèce *enterica* :
      sérotypes Abony (1), Abortusequi (1), Aesch (1), Agona (12), Albany (1), Altona (2), Anatum (3), Arechavalata (1), Bardo (1), Berta (1), Bispebjerg (1), Blegdam (1), Bovismorbificans (3), Brandenburg (3), Chester (1), Choleraesuis variété Decatur (3), Choleraesuis (1), Choleraesuis variété Kunzendorf (9), Choleraesuis sensu stricto (2), Concord (1), Crossness (1), Derby (4), Dublin (6), Duesseldorf (1), Emek (2), Enteritidis (173), Fulica (1), Gallinarum variété Duisburg (1), Gallinarum variété Gallinarum (4), Gallinarum variété Pullorum (2), Goettingen (1), Gueuletapee (1), Hadar (5), Heidelberg (6), Hessarek (1), Indiana (3), Infantis (4), Itami (1), Javiana (4), Johannesburg (1), Kentucky (2), Kiel (3), Kottbus (2), Kundunchi (1), Lindenburg (1), Manhattan (8), Maracaibo (1), Mbandaka (2), Miami (1), Mississippi (1), Montevideo (12), Muenchen (1), Napoli (2), Newport (13), Niarembe (1), Nitra (3), Overvecht (1), Panama (3), Paratyphi A (14), Paratyphi B (35), Paratyphi C (4), Poona (1), Postdam (1), Reading (1), Rosenberg (1), Rubislaw (1), Saintpaul (5), Sandiego (1), Schwarzengrund (5), Sendai (1), Senftenberg (3), Stourbridge (6), Tallahassee (1), Tennessee (1), Typhi (20), Typhimurium (104), Typhisuis (1), Urbana (1), Virchow (4), Weltevreden (2), Zaiman (1)
   - sous-espèce *salamae* :
      sérotypes 11:1,z28:e,n,x (1), 57:z42:1,6:Rz53 (1), 6,7:1,w:1,5,7 (1)
   - sous-espèce *arizonae :*
      sérotypes 62:z4,z23:- (2), 53:g,z51:- (1), 56:z4,z23:- (1), 17:z29:-(1)
   - sous-espèce *diarizonae :*
      sérotypes 38:z10:z53 (1), 61:1,v:1,5,7 (1)
   - sous-espèce *houtenae :*
      sérotypes 6,7:z4,z24:- (1), 44:a:- (1), 1,40:z4,z24:- (1),
   - sous-espèce *indica :*
      sérotypes 11:b:1,7 (1), 6,7:z41:1,7 (1)
ii) l'espèce *bongori*
   sérotypes 60:z41:- (1), 66:z35:- (1), 48:z35:- (1), 66:z41:- (1)

### 1-2-2) Extraction de l'ADN de Salmonella

L'ADN a été extrait à l'aide du kit Instagene Matrix (BioRad) ou du kit Wizard (Proméga) en respectant les instructions du fabricant.

### 1-2-3) Réactions d'amplification génique

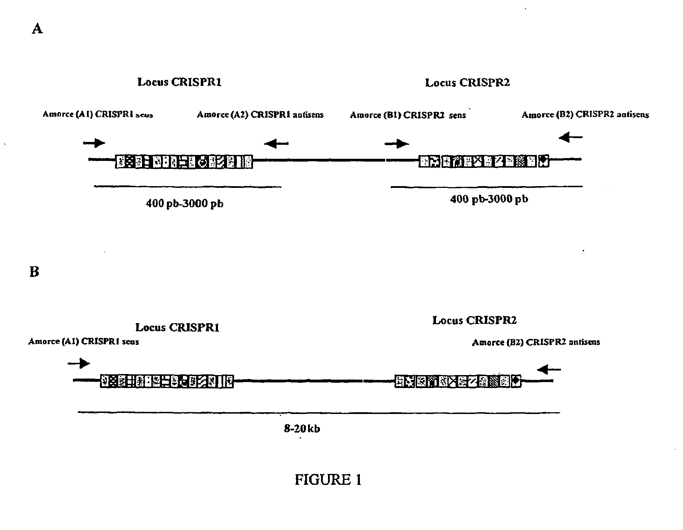

Les loci CRISPR1 et CRISPR2 de *Salmonella* ont été amplifiés soit séparément (Figure 1A) soit au cours d'une même réaction (Figure 1B), par réaction en chaîne par polymérase (PCR)

### 1-2-3-1) Amplification génique séparée des deux loci CRISPR1 et CRISPR2

### al Amorces utilisées :

Amorces utilisées pour amplifier le locus CRISPR1 par PCR multiplexe : SALCRISP1-FB (SEQ ID NO : 1326) et SALCRISP1-RB (SEQ ID NO : 1327), AriParaB-R (SEQ ID NO : 1328), BrPanCR1-R (SEQ ID NO: 1329), 50K (SEQ ID NO : 2151) et HoutWS24R (SEQ ID NO : 2152).

Amorces utilisées pour amplifier le locus CRISPR2 par PCR multiplexe : SALCRISP2-FB (SEQ ID NO : 1330), SALCRISP2-RA (SEQ ID NO : 1331) et SALCRISP2-RB (SEQ ID NO : 1332).

### b) Mélange réactionnel:

ADN, extrait selon la méthode ci-dessus, dilué au

| | |
|---|---|
| 1/10: | 2 µL |
| Amorces : | 10 pmoles pour chaque amorce |
| Désoxynucleosides triphosphate : | 100 µM |
| Taq DNA polymerase (Ampli Taq Gold, Roche) : | 0,85 U |
| Tampon 10 X (Roche) : | 10 % |
| MgCl₂ (Roche) : | 1,5 Mm |
| DMSO (Sigma) : | 5% |
| Eau pure qualité biologie moléculaire qsp : | 50 µL |

### c) Conditions d'amplification pour les deux loci:

94°C, 10 min (un cycle)
94°C, 1 min, 59°C, 1 min, 72°C, 1 min 30 s (35 cycles)
72°C 10 min (un cycle)

### d) Séparation électrophorétique des produits d'amplification

Dépôt des échantillons (par puits) : 5 µL de produit d'amplification PCR additionné d' 1 µL de tampon de dépôt 6 X (bleu de bromophenol 0,25% ; xylène cyanol 0,25% ; 30 % glycerol).

Marqueurs utilisés: 100 bp DNA ladder et 1 kb DNA ladder (Biolabs New England)

### Conditions de migration :

Dépôt sur gel à 1 % d'agarose en TBE 0,5 X [Tris HCl 45 mM (pH 8) ; acide borique 45 mM ; EDTA 10mM (pH 8)]

Electrophorèse horizontale en TBE 0,5 X à 100 V pendant 40 min.

Photo prise après 15 min de coloration au bromure d'éthidium.

### 1-2-3-2) Amplification génique des deux loci CRISPR1 et CRISPR2 au cours d'une même réaction d'amplification

L'amplification génique des deux loci CRISPR1 et CRISPR2 a été réalisée en une seule étape à l'aide du kit Expend Long Template PCR System (Roche).

### a) Amorces utilisées:

SALCRISP1-FB (SEQ ID NO : 1326) et SALCRISP2-RB (SEQ ID NO : 1332)

### b) Mélange réactionnel:

| | |
|---|---|
| ADN, extrait selon la méthode ci-dessus, pur : | 2,5 µL |
| Amorces : | 0,3 µM pour chaque amorce |
| Désoxynucleosides triphosphate : | 0,5 mM |
| Taq DNA polymerase (fournie avec le kit): | 3, 75 U |
| Tampon 2 10 X (fournie avec le kit): | 10% |
| Eau pure qualité biologie moléculaire qsp : | 50 µL |

### c) Conditions d'amplification pour les deux loci:

94°C, 2 min (un cycle)
94°C, 10 s, 58°C, 30 s, 68°C, 15 min (10 cycles)
94°C, 10 s, 58°C, 30 s, 68°C, 15 min avec une augmentation de 20 s lors de chaque cycle (20 cycles)
72°C, 7 min (un cycle)

### d) Séparation électrophorétigue des produits d'amplification

Dépôt des échantillons (par puits) : 5 µL de produit d'amplification PCR additionné d' 1 µL de tampon de dépôt 6 X (bleu de bromophenol 0,25% ; xylène cyanol 0,25% ; 30 % glycerol).

Marqueurs utilisés : Raoul^{™} (Qbiogene).

### Conditions de migration :

Dépôt sur gel à 0,75 % d'agarose en TBE 0,5 X [Tris HCl 45 mM (pH 8) ; acide borique 45 mM ; EDTA 10mM (pH 8)]

Electrophorèse horizontale en TBE 0,5 X à 100 V pendant 3 heures

Photo prise après 15 min de coloration au bromure d'éthidium.

### 2) Résultats des amplifications

### 2-1) Amplification du locus CRISPR1

Le locus CRISPR1 de toutes les souches listées à la figure 10 (à l'exception de deux souches *houtenae* car ne contenant pas de locus CRISPR1) a été amplifié à l'aide des amorces SALCRISP1-FB (SEQ ID NO : 1326), SALCRISP1-RB (SEQ ID NO : 1327), AriParaB-R (SEQ ID NO : 1328), BrPanCR1-R (SEQ ID NO : 1329), 50K (SEQ ID NO : 2151) et HoutWS24R (SEQ ID NO : 2152) ; la taille des amplicons est comprise entre 400 pb à 3000 pb.

### 2-2) Amplification du locus CRISPR2

Le locus CRISPR2 de toutes les souches listées à la figure 10, à l'exception des souches ci-dessous qui ne possèdent pas le locus CRISPR2 a été amplifié ; la taille des amplicons est comprise entre 400 pb à 3000 pb :
*S. enterica* subsp. *arizonae* sérotypes 62:z4,z23 :-, 53:g,z51:-, 56:z4,z23:-, 17:z29:-
*S. enterica* subsp. *diarizonae* sérotypes 61:1,v:1,5,7
*S. enterica* subsp. *houtenae* sérotypes 6,7:z4,z24 :-
*S. enterica* subsp. *indica* sérotypes 6,7:z41:1,7

Il est à remarquer que les sérotypes, pour lesquels il n'a pas été obtenu d'amplification du locus CRISPR2, ont pu être amplifiés par la méthode d'amplification génique en une seule étape (cf. ci-dessous).

### 2-3) Amplification des loci CRISPR1 et CRISPR2 au cours d'une même réaction

Les loci CRISPR1 et CRISPR2 des sérotypes suivants ont été amplifiés simultanément. La taille des amplicons est comprise entre 8 à 20 kb (par exemple, elle est de 20 kb pour la zone contenant les deux loci complets de *S. enterica* sérotype Typhimurium LT2) :
*S. enterica* subsp. *enterica* des sérotypes Brandenburg et Panama
*S. enterica* subsp. *arizonae* sérotypes 62:z4,z23 :-, 53:g,z51:-, 56:z4,z23:-, 17:z29:-
*S. enterica* subsp. *diarizonae* sérotypes 61:1,v:1,5,7
*S. enterica* subsp. *houtenae* sérotypes 6,7:z4,z24 :-, 44 :a :-

### 3) Séquençage des produits PCR

Tous les produits d'amplification PCR ont été séquencés selon une méthode connue de l'Homme de l'art, à l'aide du kit Big Dye version 3.1 chemistry (Applied Biosystems, Foster City, CA) et un appareil ABI 3700 (Applied Biosystems).

### 4) Analyse des séquences nucléotidique et extraction des séquences variables

Les séquences nucléotidiques ont été analysées manuellement Les séquences nucléotidiques des séquences variables (espaceurs) ont été extraites ainsi que leur ordre au sein des deux loci CRISPR. Un nom a été donné à chaque séquence variable en fonction du sérotype dans lequel elle a été identifiée pour la première fois. L'ensemble des séquences variables inventoriées est listé dans la liste des séquences (séquences SEQ ID NO : 1 à 256, 258 à 614, 616 à 876, 878 à 1043, 1045 à 1147, 1149 à 1302, 1304 à 1325, 1333 à 2150) et à la Figure 9.

### 5) Corrélation entre la composition en séquences variables et le sérotype et le sous-type

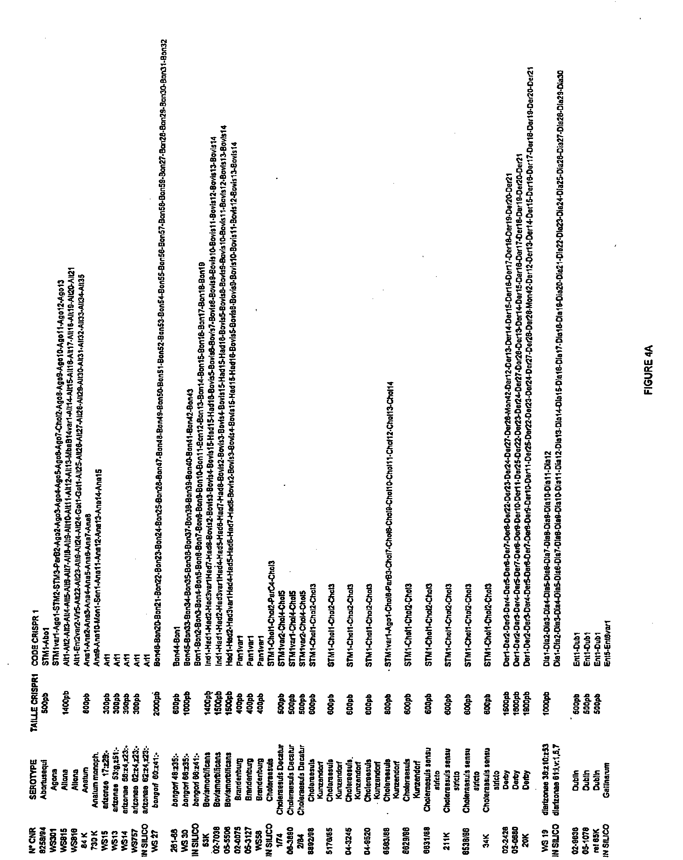

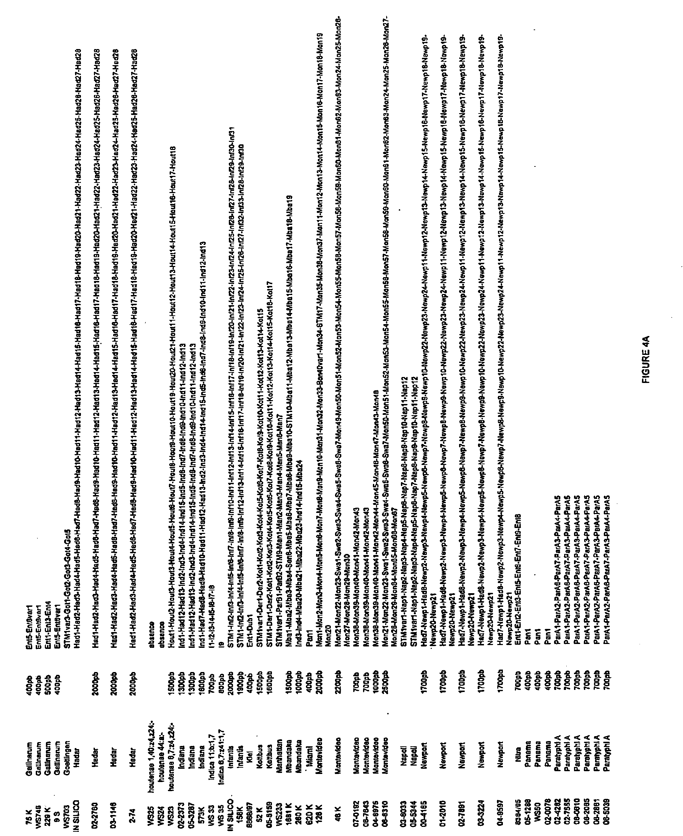

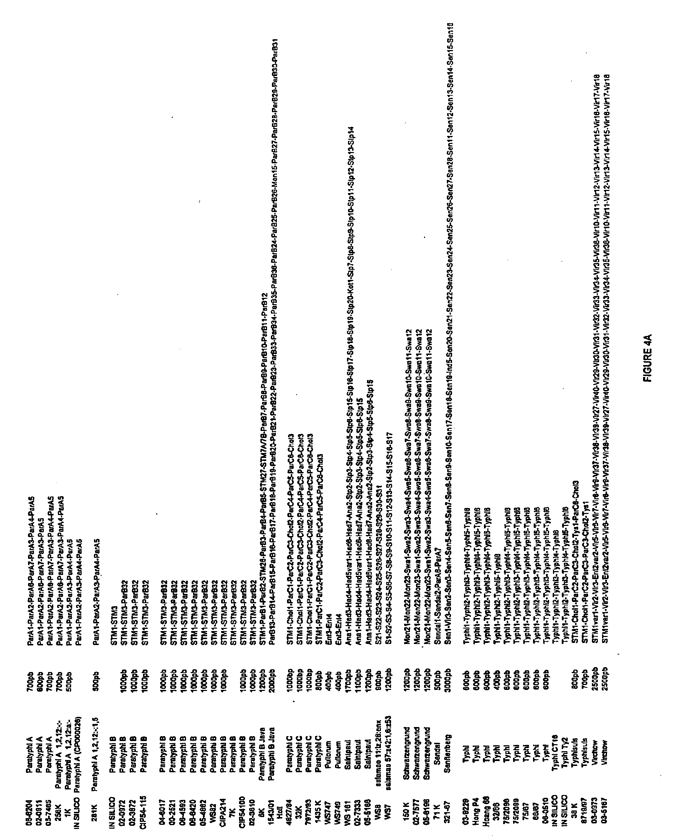

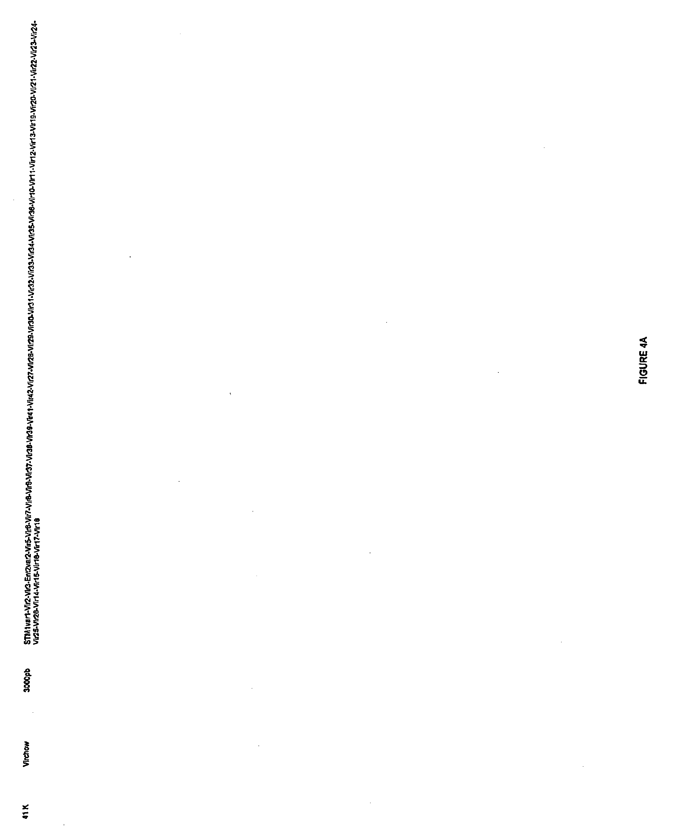

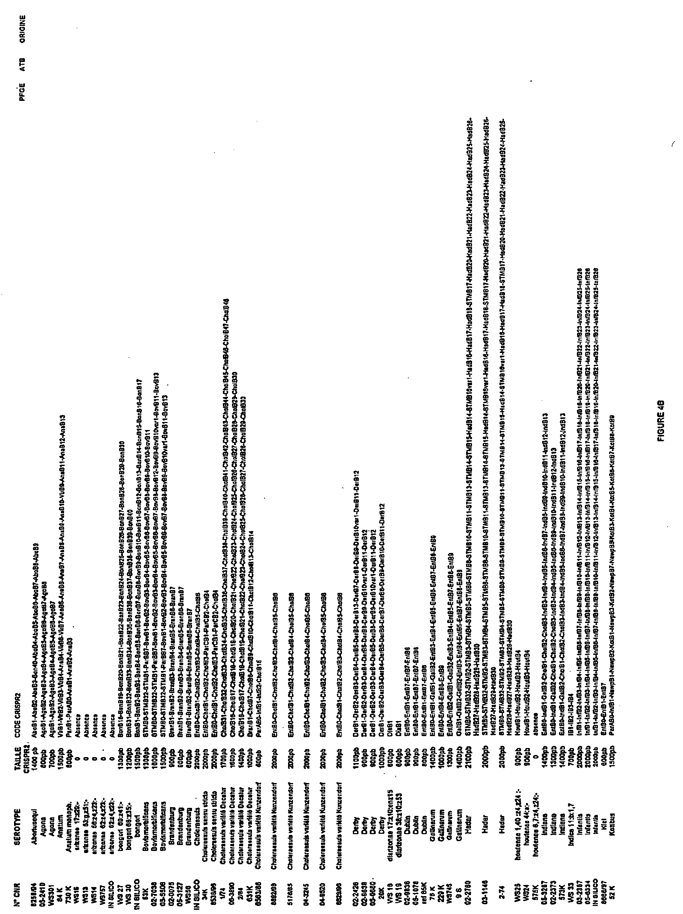

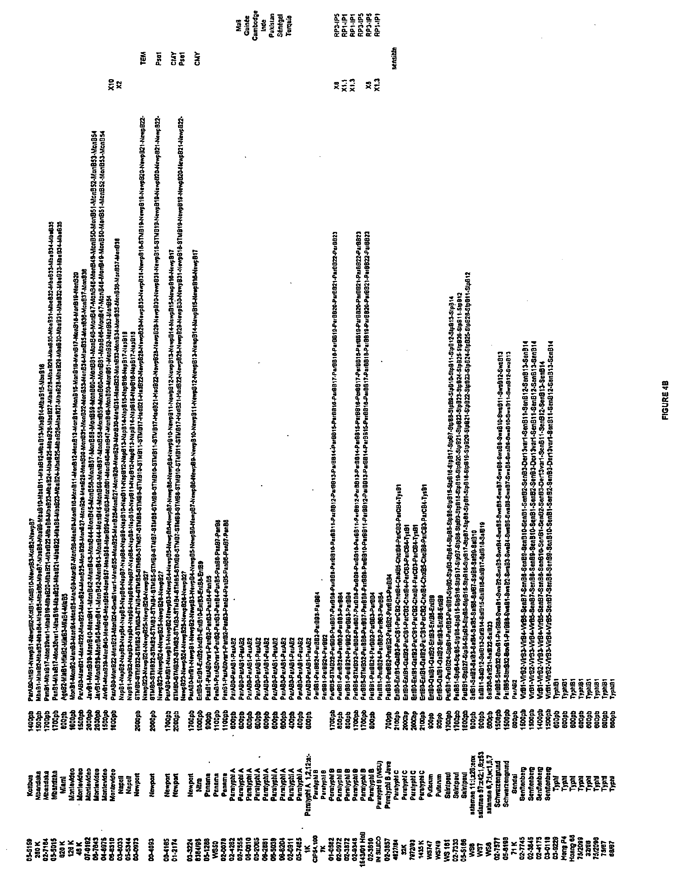

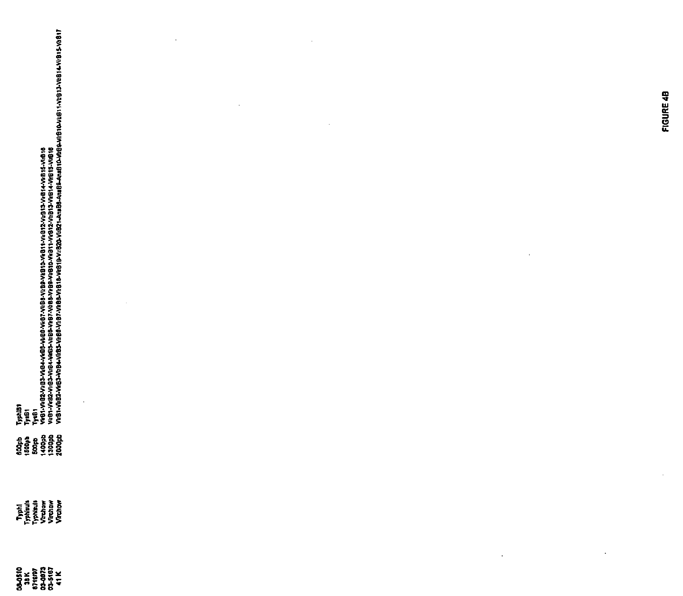

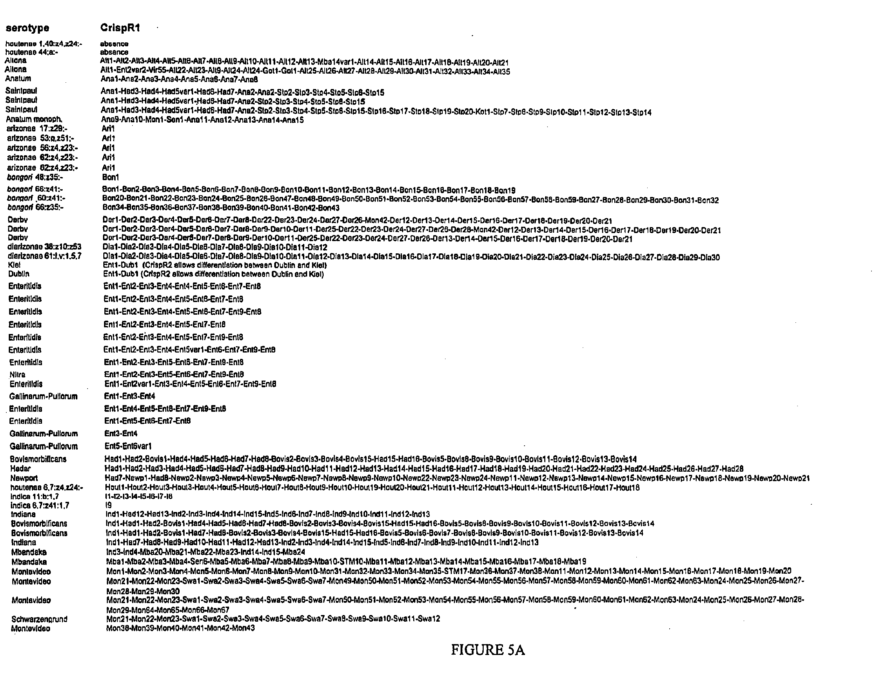

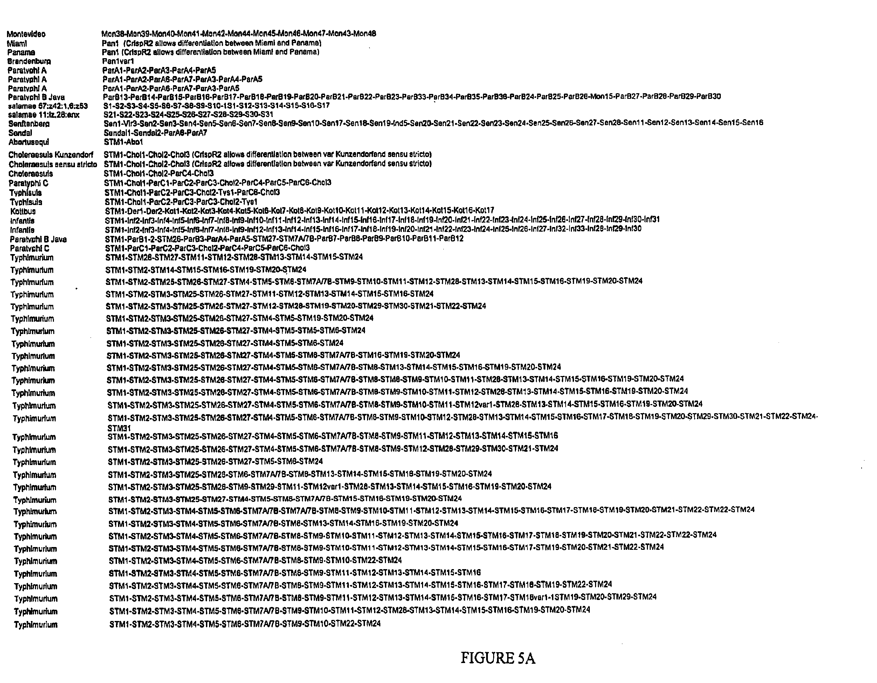

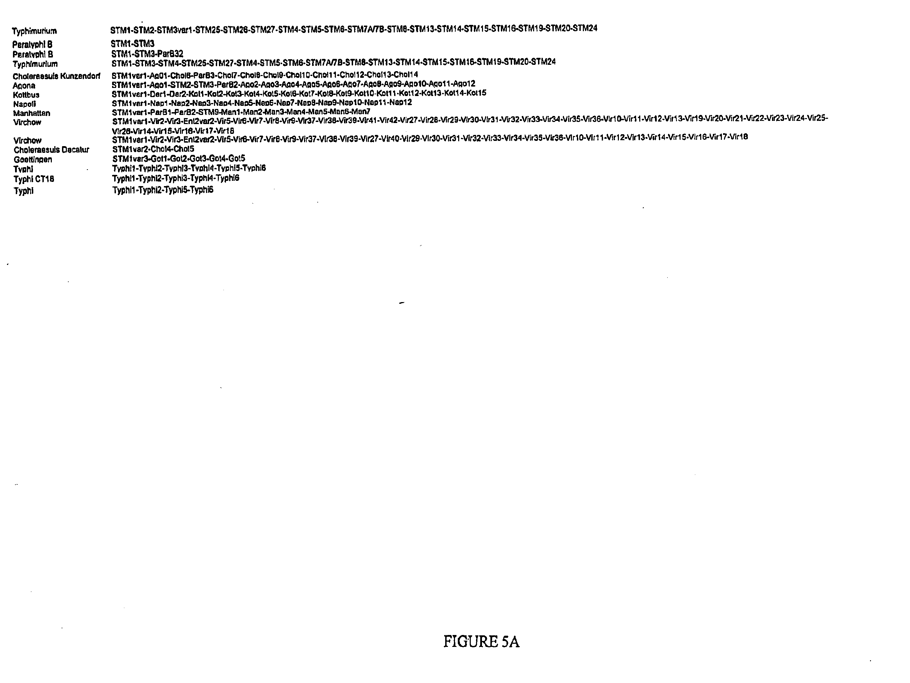

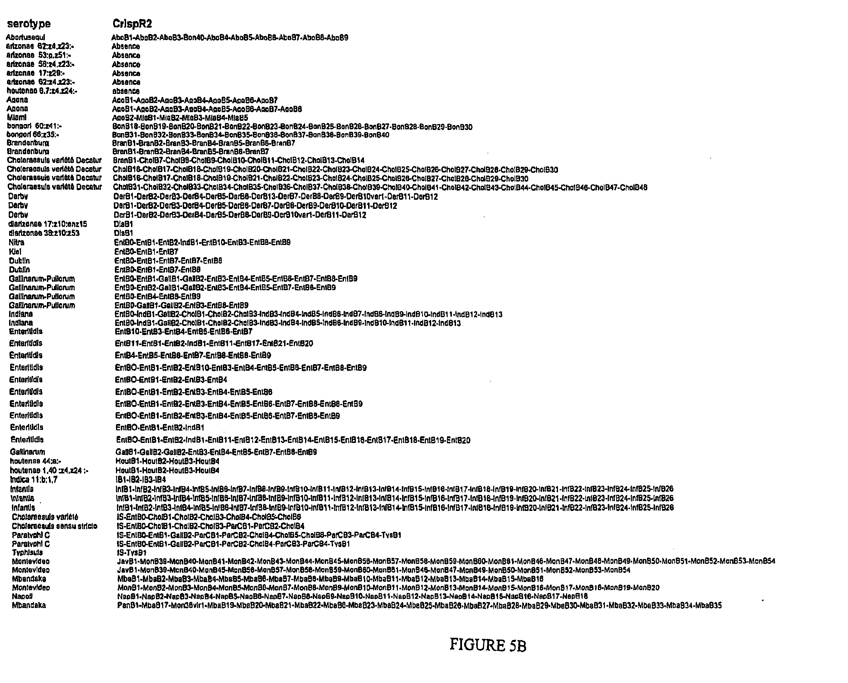

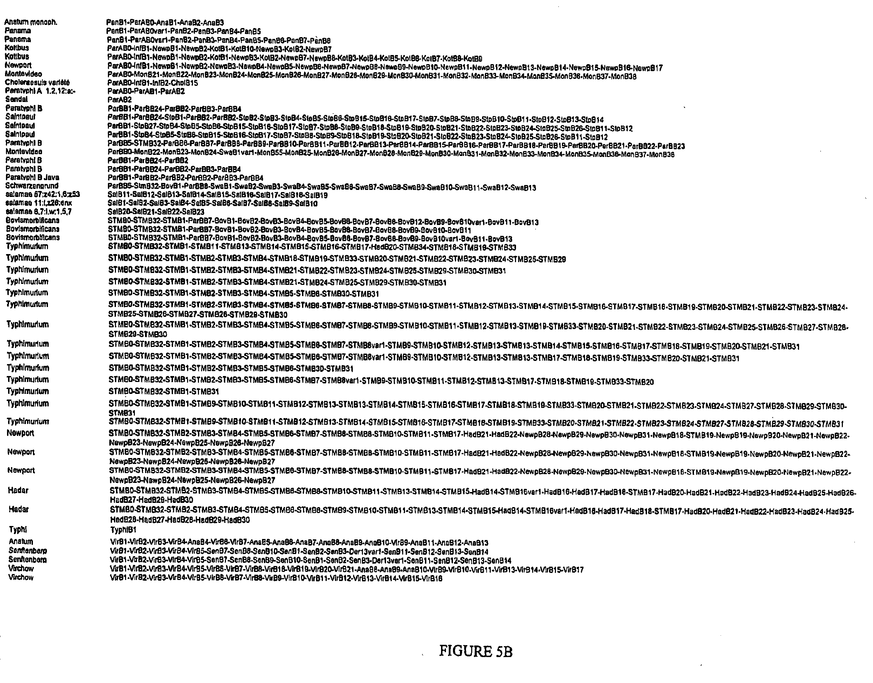

Les Figures 2, 3, 4, 5 et 10 (la Figure 10 comprend aussi les souches listées aux figures 2, 3, 4 et 5) donnent la composition en séquences variables (espaceurs) des souches de *Salmonella* analysées. Sont également mentionnés notamment pour les deux principaux sérotypes, Typhimurium et Enteritidis, les résultats du sous-typage classique (lysotype, antibiotype, profil d'électrophorèse en champ pulsé, type en MLVA) et des données épidémiologiques (cas sporadiques, cas épidémiques, etc.). La Figure 10 donne les différentes compositions en séquences variables (espaceurs) pour l'ensemble des souches de *Salmonelle* analysées.

### EXEMPLE 2: ANALYSE DE LA COMPOSITION EN SEQUENCES VARIABLES DES LOCI CRISPR DE 3 SOUCHES DE SALMONELLA DANS LE BUT DE DETERMINER LEUR TYPE ET SOUS-TYPE MOLECULAIRE

A partir d'un échantillon, trois souches de *Salmonella* ont été isolées. Pour chaque souche, l'ADN a été extrait selon la méthode décrite à l'Exemple 1 et le locus CRISPR1 et/ou le locus CRISPR2 ont été amplifiés au cours d'une amplification génique séparée telle que décrite au paragraphe 1-2-3-1) ci-dessus. La composition en séquences variables de chaque locus a été déterminée par séquençage de l'ADN.

Cette composition peut aussi être déterminée par hybridation des fragments d'ADN amplifiés (marqués lors de l'amplification ou après celle-ci avec un marqueur radioactif ou froid, par exemple cyanine 3 ou 5) avec les ensembles de sondes S1-1 à S44-2 à l'aide d'une puce à ADN.

Afin de déterminer le sérotype et le sous-type de chaque souche isolée, la composition des loci CRISPR1 et/ou CRISPR2 a été comparée à la base de référence (Figures 10 et 9).

### 1) Analyse de la première souche 02-1442

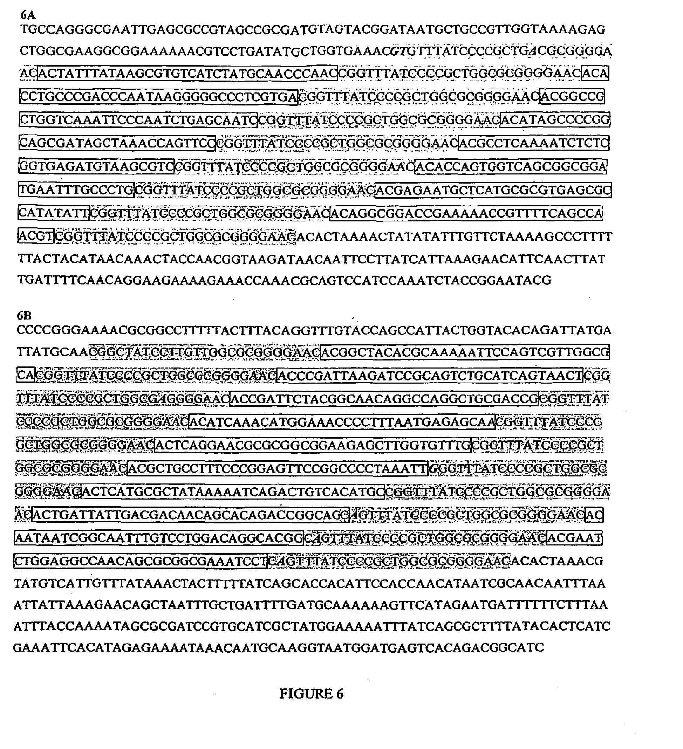

La séquence du locus CRISPR1 (5'vers 3') est représentée Figure 6A.

La composition en séquences variables (espaceurs) de cette séquence a été déterminée comme étant la suivante : Ent1-Ent2-Ent3-Ent4-Ent5-Ent6-Ent7-Ent8,
dans laquelle :
Ent1 (SEQ ID NO : 379) : ACTATTTATAAGCGTGTCATCTATGCAACCCAAC
Ent2 (SEQ ID NO : 380) : ACACCTGCCCGACCCAATAAGGGGGCCCTCGTGA
Ent3 (SEQ ID NO : 383) : ACGGCCGCTGGTCAAATTCCCAATCTGAGCAATC
Ent4 (SEQ ID NO : 384) : ACATAGCCCCGGCAGCGATAGCTAAACCAGTTCC
Ent5 (SEQ ID NO: 385) : ACGCCTCAAAATCTCTCGGTGAGATGTAAGCGTC
Ent6 (SEQ ID NO : 387) : ACACCAGTGGTCAGCGGCGGATGAATTTGCCCTG
Ent7 (SEQ ID NO : 389) : ACGAGAATGCTCATGCGCGTGAGCGCCATATATT
Ent8 (SEQ ID NO : 390) : ACAGGCGGACCGAAAAACCGTTTTCAGCCAACGT

La séquence du locus CRISPR2 (5'vers 3') est représentée Figure 6B.

La composition en séquences variables (espaceurs) de cette séquence a été déterminée comme étant la suivante : EntB0-EntB1-EntB2-EntB3-EntB4-EntB5-EntB6-EntB7-EntB8-EntB9,
dans laquelle :
EntB0 (SEQ ID NO : 392) : ACGGCTACACGCAAAAATTCCAGTCGTTGGCGCA
EntB1 (SEQ ID NO : 393) : ACCCGATTAAGATCCGCAGTCTGCATCAGTAACT
EntB2 (SEQ ID NO : 404) : ACCGATTCTACGGCAACAGGCCAGGCTGCGACCG
EntB3 (SEQ ID NO : 407) : ACATCAAACATGGAAACCCCTTTAATGAGAGCAA
EntB4 (SEQ ID NO : 408) : ACTCAGGAACGCGCGGCGGAAGAGCTTGGTGTTTG
EntB5 (SEQ ID NO : 409) : ACGCTGCCTTTCCCGGAGTTCCGGCCCCTAAATT
EntB6 (SEQ ID NO : 410) : ACTCATGCGCTATA.A.AAATCAGACTGTCACATGC
EntB7 (SEQ ID NO : 411) : ACTGATTATTGACGACAACAGCACAGACCGGCAG
EntB8 (SEQ ID NO : 412) : ACAATAATCGGCAATTTGTCCTGGACAGGCACGG
EntB9 (SEQ ID NO : 413) : ACGAATCTGGAGGCCAACAGCGCGGCGAAATCCT

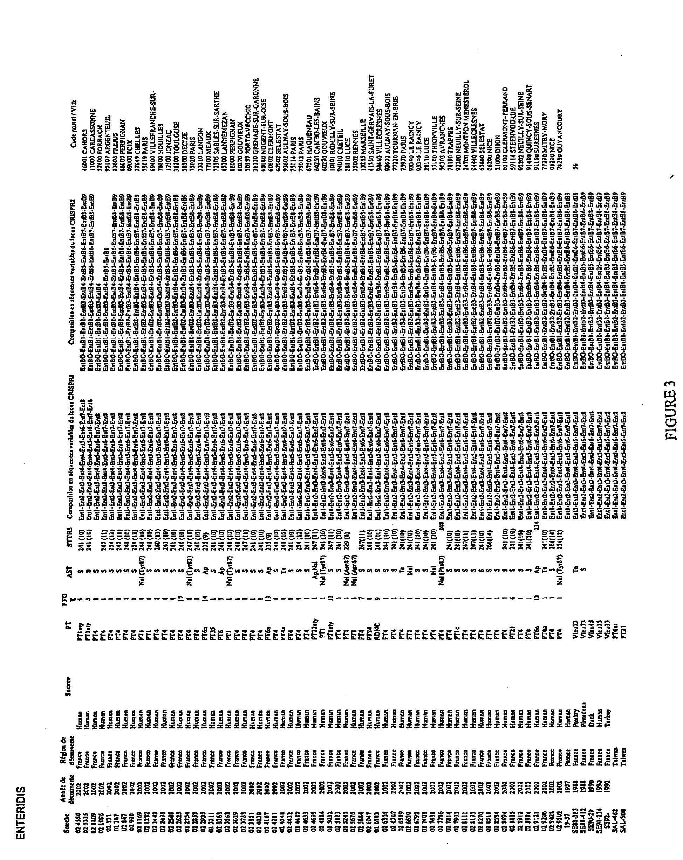

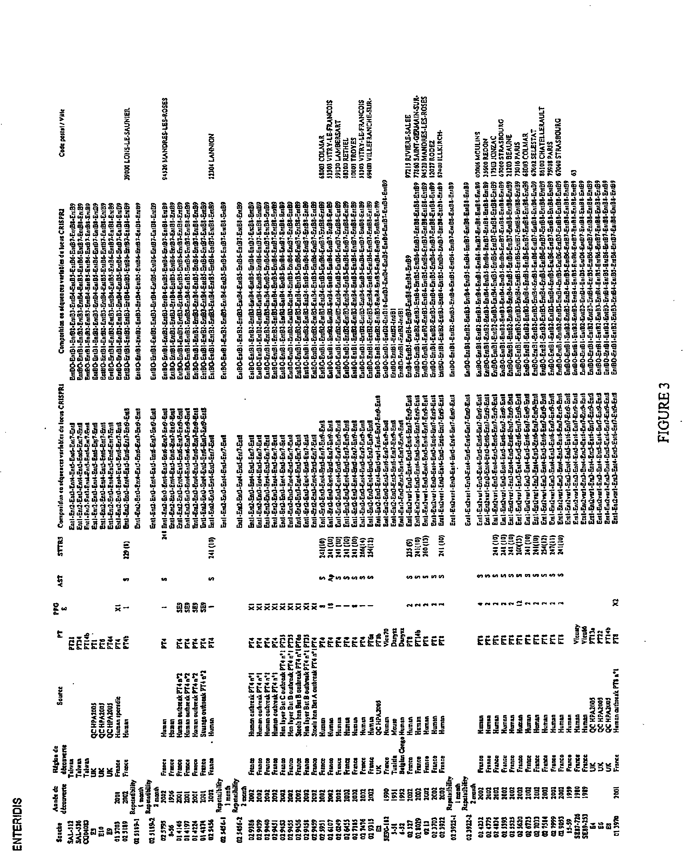

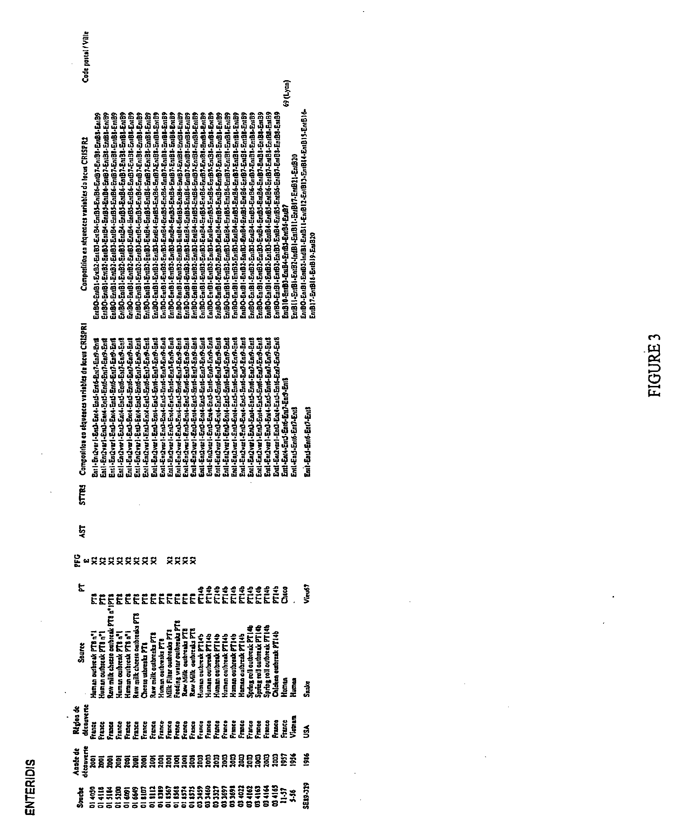

Après comparaison avec la base de référence (Figures 3 et 9) de la composition des deux loci CRISPR de la bactérie isolée, il a été déterminé que la souche 02-1142 avait la même composition en espaceurs que les souches de *Salmonella enterica* du sérotype Enteritidis. Ce résultat a été confirmé par sérotypage.

### 2) Analyse de la deuxième souche 02-4232

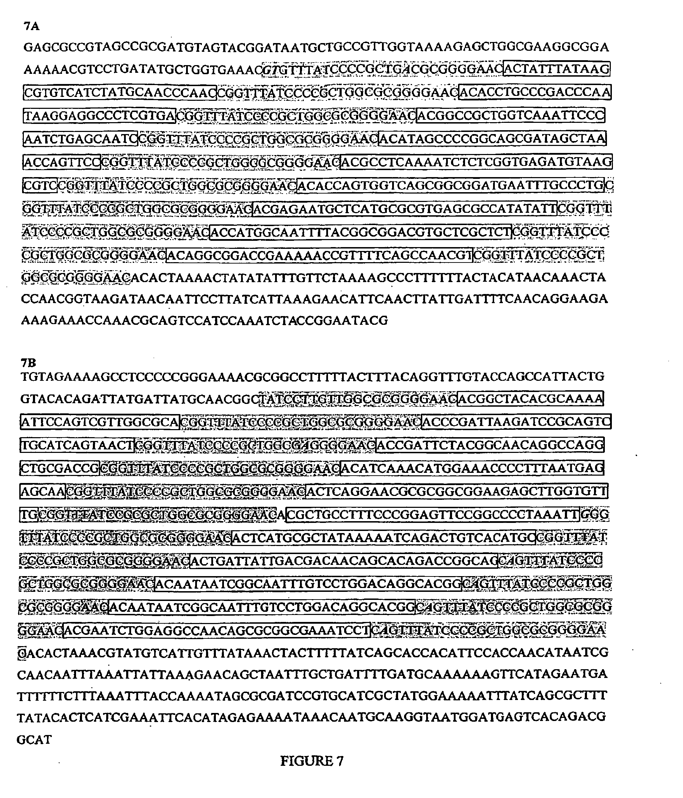

La séquence du locus CRISPR1 (5'vers 3') est représentée Figure 7A.

La composition en séquences variables (espaceurs) de cette séquence a été déterminée comme étant la suivante : Ent1-Ent2var1-Ent3-Ent4-Ent5-Ent6-Ent7-Ent9-Ent8,
dans laquelle :
Ent1 (SEQ ID NO : 379) : ACTATTTATAAGCGTGTCATCTATGCAACCCAAC
Ent2var1 (SEQ ID NO : 381) : ACACCTGCCCGACCCAATAAGGAGGCCCTCGTGA
Ent3 (SEQ ID NO:383) : ACGGCCGCTGGTCAAATTCCCAATCTGAGCAATC
Ent4 (SEQ ID NO : 233840) : ACATAGCCCCGGCAGCGATAGCTAAACCAGTTCC
Ent5 (SEQ ID NO : 385) : ACGCCTCAAAATCTCTCGGTGAGATGTAAGCGTC
Ent6 (SEQ ID NO : 387) : ACACCAGTGGTCAGCGGCGGATGAATTTGCCCTG
Ent7 (SEQ ID NO : 389) : ACGAGAATGCTCATGCGCGTGAGCGCCATATATT
Ent9 (SEQ ID NO : 391) : ACCATGGCAATTTTACGGCGGACGTGCTCGCTCT
Ent8 (SEQ ID NO : 390) : ACAGGCGGACCGAAAAACCGTTTTCAGCCAACGT

La séquence du locus CRISPR2 (5'vers 3') est représentée Figure 7B.

La composition en séquences variables (espaceurs) de cette séquence a été déterminée comme étant la suivante: EntB0-EntB1-EntB2-EntB3-EntB4-EntB5-EntB6-EntB7-EntB8-EntB8-EntB9,
dans laquelle :
EntB0 (SEQ ID NO : 392) : ACGGCTACACGCAAAAATTCCAGTCGTTGGCGCA
EntB1 (SEQ ID NO : 393) : ACCCGATTAAGATCCGCAGTCTGCATCAGTAACT
EntB2 (SEQ ID NO : 404) : ACCGATTCTACGGCAACAGGCCAGGCTGCGACCG
EntB3 (SEQ ID NO : 407) : ACATCAAACATGGAAACCCCTTTAATGAGAGCAA
EntB4 (SEQ ID NO : 408) : ACTCAGGAACGCGCGGCGGAAGAGCTTGGTGTTTG
EntB5 (SEQ ID NO : 409) : ACGCTGCCTTTCCCGGAGTTCCGGCCCCTAAATT
EntB6 (SEQ ID NO : 410) : ACTCATGCGCTATAAAAATCAGACTGTCACATGC
EntB7 (SEQ ID NO : 411) : ACTGATTATTGACGACAACAGCACAGACCGGCAG
EntB8 (SEQ ID NO : 412) : ACAATAATCGGCAATTTGTCCTGGACAGGCACGG
EntB9 (SEQ ID NO: 413) : ACGAATCTGGAGGCCAACAGCGCGGCGAA.ATCCT

Après comparaison avec la base de référence (Figures 3 et 9) de la composition des deux loci CRISPR de la bactérie isolée, il a été déterminé que la souche 02-4232 avait la même composition en espaceurs que les souches de *Salmonella enterica* du sérotype Enteritidis. Ce résultat a été confirmé par sérotypage. La composition en espaceurs était différente de la souche précédente 02-1142, ce qui concordait avec le résultat de sous-typage obtenu par lysotypie (la souche 02-4232 étant de lysotype PT4 et la souche 02-4232 du lysotype PT8).

### 3) Analyse de la troisième souche 02-1941

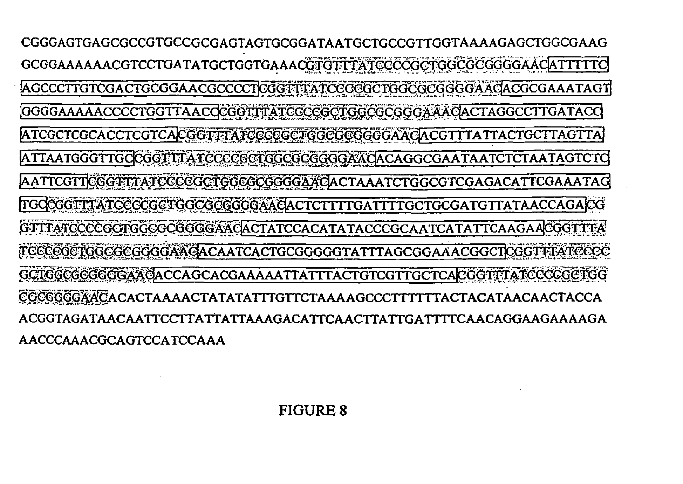

La séquence du locus CRISPR1 (5'vers 3') est représentée Figure 8A.

La composition en séquences variables (espaceurs) de cette séquence a été déterminée comme étant la suivante : STM1-STM2-STM3-STM25-STM26-STM27-STM4-STM5-STM6-STM24,
dans laquelle :
STM1 (SEQ ID NO : 1106) : ATTTTTCAGCCCTTGTCGACTGCGGAACGCCCCT
STM2 (SEQ ID NO : 1123) : ACGCGAAATAGTGGGGAAAAACCCCTGGTTAACC
STM3 (SEQ ID NO : 1133) : ACTAGGCCTTGATACCATCGCTCGCACCTCGTCA
STM25 (SEQ ID NO : 1128) ACGTTTATTACTGCTTAGTTAATTAATGGGTTGC
STM26 (SEQ ID NO : 1129) : ACAGGCGAATAATCTCTAATAGTCTCAATTCGTT
STM27 (SEQ ID NO : 1130) : ACTAAATCTGGCGTCGAGACATTCGAAATAGTGC
STM4 (SEQ ID NO : 1137) : ACTCTTTTGATTTTGCTGCGATGTTATAACCAGA
STM5 (SEQ ID NO : 1138) : ACTATCCACATATACCCGCAATCATATTCAAGAA
STM6 (SEQ ID NO : 1139) : ACAATCACTGCGGGGGTATTTAGCGGAAACGGCT
STM24 (SEQ ID NO : 1127) : ACCAGCACGAAAAATTATTTACTGTCGTTGCTCA

Après comparaison avec la base de référence (Figures 2 et 9) de la composition du locus CRISPR1 de la bactérie isolée, il a été déterminé que la souche 02-1941 avait la même composition en espaceurs que les souches de *Salmonella enterica* du sérotype Typhimurium. Ce résultat a été confirmé par sérotypage. De plus, sa composition en espaceurs a été retrouvée dans la base de données quasi-exclusivement chez les souches de *S. enterica,* sérotype Typhimurium.

L'analysé d'un seul locus CRISPR (ici le locus CRISPR1) a permis d'identifier la souche bactérienne isolée.

## Revendications

1. Méthode *in vitro* de typage et/ou de sous-typage moléculaire d'une bactérie du genre *Salmonella,* à partir d'un échantillon, laquelle méthode est **caractérisée en ce qu'**elle comprend au moins les étapes suivantes :
(a) l'amplification d'un fragment d'acide nucléique d'une bactérie du genre *Salmonella,* ledit fragment comprenant le locus CRISPR1 et/ou le locus CRISPR2, à l'aide d'au moins un ensemble d'amorces, lesquelles amorces présentent une taille inférieure ou égale à 50 nucléotides, lesdits ensembles d'amorces étant choisis dans le groupe constitué par :
- un ensemble d'amorces « A » apte à amplifier un fragment d'acide nucléique comprenant le locus CRISPR1, comprenant au moins une amorce sens A1 constituée d'une séquence oligonucléotidique présentant au moins 70% d'identité avec, ou identique à un fragment de la séquence génomique d'une bactérie du genre *Salmonella* situé dans une région de 1000 pb en position 5' du locus CRISPR1, ledit fragment de la séquence génomique situé en position 5' du locus CRISPR1 étant de même taille que ladite amorce A1, et au moins une amorce antisens A2 constituée d'une séquence oligonucléotidique présentant 70% d'identité, de préférence, 80%, 85%, 95%, de préférence encore 99% d'identité avec, ou identique à un fragment de la séquence génomique complémentaire de la séquence génomique d'une bactérie du genre *Salmonella* situé en position 3' du locus CRISPR1 et en position 5' du locus CRISPR2, ledit fragment de la séquence génomique complémentaire étant de même taille que ladite amorce A2 ;
- un ensemble d'amorces « B » apte à amplifier un fragment d'acide nucléique comprenant le locus CRISPR2, comprenant au moins une amorce sens B1 constitué d'une séquence oligonucléotidique présentant au moins 70% d'identité avec, ou identique à un fragment de la séquence génomique d'une bactérie du genre *Salmonella* situé en position 3' du locus CRISPR1 et en position 5' du locus CRISPR2, ledit fragment de la séquence génomique situé en position 3' du locus CRISPR1 et en position 5' du locus CRISPR2 étant de même taille que ladite amorce B1, et au moins une amorce antisens B2 constituée d'une séquence oligonucléotidique présentant au moins 70% d'identité avec, ou identique à un fragment de la séquence génomique complémentaire de la séquence génomique d'une bactérie du genre *Salmonella* situé dans une région de 1000 pb en position 3' du locus CRISPR2, ledit fragment de la séquence génomique complémentaire étant de même taille que ladite amorce B2 ;
- un ensemble d'amorces « C » apte à amplifier un fragment d'acide nucléique comprenant le locus CRISPR1 et le locus CRISPR2, comprenant au moins une amorce sens A1 telle que définie ci-dessus et au moins un amorce antisens B2 telle que définie ci-dessus ;
(b) la détermination du nombre et de la séquence nucléotidique des séquences variables du locus CRISPR1 et/ou du locus CRISPR2 amplifié(s) à l'étape (a) ; et
(c) la comparaison de ladite composition en séquences variables des loci CRISPR1 et/ou CRISPR2 avec une base de référence fournissant la composition en séquences variables des loci CRISPR1 et CRISPR2 de types et sous-types de bactéries du genre *Salmonella* listée aux figures 10A, 10B et 9 ou une partie de cette base.

2. Méthode selon la revendication 1, **caractérisée en ce que** l'identité de la composition en séquences variables déterminée à l'étape (b) avec une composition figurant dans la base de référence définie à la revendication 1 ou une partie de cette base est indicative du type et/ou du sous-type de la bactérie du genre *Salmonella* présente dans l'échantillon

3. Méthode selon la revendication 1 ou la revendication 2, **caractérisée en ce que** lesdites amorces présentent une taille comprise entre 15 et 30 nucléotides.

4. Méthode selon l'une quelconque des revendications 1 à 3, **caractérisée en ce que** ledit fragment d'acide nucléique comprenant le locus CRISPR1 et/ou le locus CRISPR2 présente une taille comprise entre 400 à 20 000 pb.

5. Méthode selon l'une quelconque des revendications 1 à 4, **caractérisée en ce que** ledit fragment de la séquence génomique d'une bactérie du genre *Salmonella* situé en position 5' du locus CRISPR1, et/ou ledit fragment de la séquence génomique complémentaire de la séquence génomique d'une bactérie du genre *Salmonella* situé en position 3' du locus CRISPR2, se situent à une distance du locus CRISPR1 ou CRISPR2 inférieure à 500 pb, de préférence inférieure à 100 pb.

6. Méthode selon l'une quelconque des revendications 1 à 5, **caractérisée en ce que** ladite séquence génomique d'une bactérie du genre *Salmonella* est choisie parmi la séquence génomique de *S. enterica* sérotype Typhimurium LT2 (séquence génomique disponible sous les numéros GI : 16421485 et GI : 16421501 dans la base de données GenBank) ou de *S. enterica* sérotype Typhi CT18 (séquence génomique disponible sous le numéro GI: 16503805 dans la base de données GenBank).

7. Méthode selon la revendication 5, **caractérisée en ce que** l'ensemble d'amorces est sélectionné dans le groupe constitué par :
- l'ensemble d'amorces, apte à amplifier le locus CRISPR1, constitué de l'amorce de séquence SEQ ID NO : 1326 associée à au moins une des amorces de séquence SEQ ID NOs : 1327, 1328, 1329, 2151 et 2152,
- l'ensemble d'amorces, apte à amplifier le locus CRISPR2, constitué de l'amorce de séquence SEQ ID NO : 1330, associée à au moins une des amorces de séquence SEQ ID NOs : 1331 et 1332, et
- l'ensemble d'amorces, apte à amplifier simultanément les deux loci CRISPR1 et CRISPR2, constitué de l'amorce de séquence SEQ ID NO: 1326, associée à au moins une des deux amorces de séquence SEQ ID NOs : 1331 et 1332.

8. Méthode selon l'une quelconque des revendications 1 à 7, **caractérisée en ce que** l'étape (a) d'amplification est réalisée par une méthode sélectionnée dans le groupe constitué par : la réaction en chaîne par polymérase (PCR), réaction en chaîne par ligase (LCR), « nucleic acid sequence-based amplification » (NASBA), « cycling probe technology » (CPT), PCR nichée et PCR multiplexe.

9. Méthode selon l'une quelconque des revendications 1 à 8, **caractérisée en ce que** l'étape (b) est réalisée à l'aide d'une méthode de séquençage de l'ADN.

10. Méthode selon l'une quelconque des revendications 1 à 8, **caractérisée en ce que** l'étape (b) est réalisée par :
(i) hybridation avec un, plusieurs ou tous les ensemble(s) de sondes choisi parmi :
- l'ensemble S1-1, comprenant ou constitué d'au moins une sonde comprenant au moins ou constituée d'au moins 8 nucléotides consécutifs de l'une quelconque des séquences d'acide nucléique SEQ ID NO : 1106 à 1119, 1123 à 1140, 1142, 1143, 1914 et/ou leurs séquences d'acide nucléique complémentaires (séquences nucléotidiques correspondant aux différentes séquences variables du locus CRISPR1 du sérotype Typhimurium) ;
- l'ensemble S1-2, comprenant ou constitué d'au moins une sonde comprenant au moins ou constituée d'au moins 8 nucléotides consécutifs de l'une quelconque des séquences d'acide nucléique SEQ ID NO : 455, 1144 à 1147, 1149 à 1153, 1155 à 1181 et/ou leurs séquences d'acide nucléique complémentaires (séquences nucléotidiques correspondant aux différentes séquences variables du locus CRISPR2 du sérotype Typhimurium) ;
- l'ensemble S2-1, comprenant ou constitué d'au moins une sonde comprenant au moins ou constituée d'au moins 8 nucléotides consécutifs de l'une quelconque des séquences d'acide nucléique SEQ ID NO : 256, 378 à 381, 383 à 387, 389 à 391, 1106, 1514, 1528, 2148 à 2153 et/ou leurs séquences d'acide nucléique complémentaires (séquences nucléotidiques correspondant aux différentes séquences variables du locus CRISPR1 du sérotype Enteritidis) ;
- l'ensemble S2-2, comprenant ou constitué d'au moins une sonde comprenant au moins ou constituée d'au moins 8 nucléotides consécutifs de l'une quelconque des séquences d'acide nucléique SEQ ID NO : 392 à 413, 519, 815, 972, 983, 989, 1529 à 1546, 1952 et/ou leurs séquences d'acide nucléique complémentaires (séquences nucléotidiques correspondant aux différentes séquences variables du locus CRISPR2 du sérotype Enteritidis) ;
- l'ensemble S3-1, comprenant ou constitué d'au moins une sonde comprenant au moins ou constituée d'au moins 8 nucléotides consécutifs de l'une quelconque des séquences d'acide nucléique SEQ ID NO : 421 à 442, 444, 445, 447 à 450 et/ou leurs séquences d'acide nucléique complémentaires (séquences nucléotidiques correspondant aux différentes séquences variables du locus CRISPR1 du sérotype Hadar) ;
- l'ensemble S3-2, comprenant ou constitué d'au moins une sonde comprenant au moins ou constituée d'au moins 8 nucléotides consécutifs de l'une quelconque des séquences d'acide nucléique SEQ ID NO : 451 à 465, 1144 à 1147, 1150 à 1152, 1154, 1155, 1158, 1169, 1172, 1175 à 1177, 1179, 1181 et/ou leurs séquences d'acide nucléique complémentaires (séquences nucléotidiques correspondant aux différentes séquences variables du locus CRISPR2 du sérotype Hadar) ;
- l'ensemble S4-1, comprenant ou constitué d'au moins une sonde comprenant au moins ou constituée d'au moins 8 nucléotides consécutifs de l'une quelconque des séquences d'acide nucléique SEQ ID NO: 65, 382, 1120, 1263 à 1302, 2024 à 2038 et/ou leurs séquences d'acide nucléique complémentaires (séquences nucléotidiques correspondant aux différentes séquences variables du locus CRISPR1 du sérotype Virchow) ;
- l'ensemble S4-2, comprenant ou constitué d'au moins une sonde comprenant au moins ou constituée d'au moins 8 nucléotides consécutifs de l'une quelconque des séquences d'acide nucléique SEQ ID NO : 83, 93, 94, 1304 à 1324 et/ou leurs séquences d'acide nucléique complémentaires (séquences nucléotidiques correspondant aux différentes séquences variables du locus CRISPR2 du sérotype Virchow) ;
- l'ensemble S5-1, comprenant ou constitué d'au moins une sonde comprenant au moins ou constituée d'au moins 8 nucléotides consécutifs de l'une quelconque des séquences d'acide nucléique SEQ ID NO : 531 à 562, 1106 et/ou leurs séquences d'acide nucléique complémentaires (séquences nucléotidiques correspondant aux différentes séquences variables du locus CRISPR1 du sérotype Infantis) ;
- l'ensemble S5-2, comprenant ou constitué d'au moins une sonde comprenant au moins ou constituée d'au moins 8 nucléotides consécutifs de l'une quelconque des séquences d'acide nucléique SEQ ID NO : 563 à 588 et/ou leurs séquences d'acide nucléique complémentaires (séquences nucléotidiques correspondant aux différentes séquences variables du locus CRISPR2 du sérotype Infantis) ;
- l'ensemble S6-1, comprenant ou constitué d'au moins une sonde comprenant au moins ou constituée d'au moins 8 nucléotides consécutifs de l'une quelconque des séquences d'acide nucléique SEQ ID NO : 1254 à 1259 et/ou leurs séquences d'acide nucléique complémentaires (séquences nucléotidiques correspondant aux différentes séquences variables du locus CRISPR1 du sérotype Typhi) ;
- l'ensemble S6-2, comprenant ou constitué d'au moins une sonde comprenant au moins ou constituée d'au moins 8 nucléotides consécutifs de la séquence d'acide nucléique SEQ ID NO : 1260 et/ou sa séquence d'acide nucléique complémentaire (séquences nucléotidiques correspondant à la séquence variable du locus CRISPR2 du sérotype Typhi) ;
- l'ensemble S7-1, comprenant ou constitué d'au moins une sonde comprenant au moins ou constituée d'au moins 8 nucléotides consécutifs de l'une quelconque des séquences d'acide nucléique SEQ ID NO : 448, 449, 860 à 876, 878 à 884, 1719 à 1724, 1727 à 1743, 1745 et/ou leurs séquences d'acide nucléique complémentaires (séquences nucléotidiques correspondant aux différentes séquences variables du locus CRISPR1 du sérotype Newport) ;
- l'ensemble S7-2, comprenant ou constitué d'au moins une sonde comprenant au moins ou constituée d'au moins 8 nucléotides consécutifs de l'une quelconque des séquences d'acide nucléique SEQ ID NO : 453 à 456, 563, 621, 622, 625, 626, 657, 885 à 896, 906 à 915, 933, 1144, 1145, 1155, 1156, 1172, 1174, 1748 à 1756, 1758, 1759, 1916 et/ou leurs séquences d'acide nucléique complémentaires (séquences nucléotidiques correspondant aux différentes séquences variables du locus CRISPR2 du sérotype Newport) ;
- l'ensemble S8-1, comprenant ou constitué d'au moins une sonde comprenant au moins ou constituée d'au moins 8 nucléotides consécutifs de l'une quelconque des séquences d'acide nucléique SEQ ID NO : 295 à 299, 301 à 323, 738, 1106 à 1109, 1120, 1123, 1128 à 1131, 1133, 1137 à 1140, 1550, 1159 à 1561 et/ou leurs séquences d'acide nucléique complémentaires (séquences nucléotidiques correspondant aux différentes séquences variables du locus CRISPR1 du sérotype Derby) ;
- l'ensemble S8-2, comprenant ou constitué d'au moins une sonde comprenant au moins ou constituée d'au moins 8 nucléotides consécutifs de l'une quelconque des séquences d'acide nucléique SEQ ID NO : 324 à 337, 455, 1144, 1145, 1149 à 1153, 1155 à 1160, 1163, 1172 à 1174 et/ou leurs séquences d'acide nucléique complémentaires (séquences nucléotidiques correspondant aux différentes séquences variables du locus CRISPR2 du sérotype Derby) ;
- l'ensemble S9-1, comprenant ou constitué d'au moins une sonde comprenant au moins ou constituée d'au moins 8 nucléotides consécutifs de l'une quelconque des séquences d'acide nucléique SEQ ID NO : 295, 307, 602 à 614, 616 à 619, 1106, 1120, et/ou leurs séquences d'acide nucléique complémentaires (séquences nucléotidiques correspondant aux différentes séquences variables du locus CRISPR1 du sérotype Kottbus) ;
- l'ensemble S9-2, comprenant ou constitué d'au moins une sonde comprenant au moins ou constituée d'au moins 8 nucléotides consécutifs de l'une quelconque des séquences d'acide nucléique SEQ ID NO : 563, 620 à 629, 885, 896, 907, 913, 914, 933, et/ou leurs séquences d'acide nucléique complémentaires (séquences nucléotidiques correspondant aux différentes séquences variables du locus CRISPR2 du sérotype Kottbus) ;
- l'ensemble S10-1, comprenant ou constitué d'au moins une sonde comprenant au moins ou constituée d'au moins 8 nucléotides consécutifs de l'une quelconque des séquences d'acide nucléique SEQ ID NO : 422 à 425, 448 à 450, 504 à 518 et/ou leurs séquences d'acide nucléique complémentaires (séquences nucléotidiques correspondant aux différentes séquences variables du locus CRISPR1 du sérotype Indiana) ;
- l'ensemble S10-2, comprenant ou constitué d'au moins une sonde comprenant au moins ou constituée d'au moins 8 nucléotides consécutifs de l'une quelconque des séquences d'acide nucléique SEQ ID NO : 246, 258, 269, 392, 415, 519 à 530, et/ou leurs séquences d'acide nucléique complémentaires (séquences nucléotidiques correspondant aux différentes séquences variables du locus CRISPR2 du sérotype Indiana) ;
- l'ensemble S11-1, comprenant ou constitué d'au moins une sonde comprenant au moins ou constituée d'au moins 8 nucléotides consécutifs de l'une quelconque des séquences d'acide nucléique SEQ ID NO : 11 à 23, 238, 948, 1120, 1123, 1133 et/ou leurs séquences d'acide nucléique complémentaires (séquences nucléotidiques correspondant aux différentes séquences variables du locus CRISPR1 du sérotype Agona) ;
- l'ensemble S11-2, comprenant ou constitué d'au moins une sonde comprenant au moins ou constituée d'au moins 8 nucléotides consécutifs de l'une quelconque des séquences d'acide nucléique SEQ ID NO: 24 à 31 et/ou leurs séquences d'acide nucléique complémentaires (séquences nucléotidiques correspondant aux différentes séquences variables du locus CRISPR2 du sérotype Agona) ;
- l'ensemble S12-1, comprenant ou constitué d'au moins une sonde comprenant au moins ou constituée d'au moins 8 nucléotides consécutifs de l'une quelconque des séquences d'acide nucléique SEQ ID NO : 830 à 841, 1120 et/ou leurs séquences d'acide nucléique complémentaires (séquences nucléotidiques correspondant aux différentes séquences variables du locus CRISPR1 du sérotype Napoli) ;
- l'ensemble S12-2, comprenant ou constitué d'au moins une sonde comprenant au moins ou constituée d'au moins 8 nucléotides consécutifs de l'une quelconque des séquences d'acide nucléique SEQ ID NO : 842 à 859 et/ou leurs séquences d'acide nucléique complémentaires (séquences nucléotidiques correspondant aux différentes séquences variables du locus CRISPR2 du sérotype Napoli) ;
- l'ensemble S13-1, comprenant ou constitué d'au moins une sonde comprenant au moins ou constituée d'au moins 8 nucléotides consécutifs de l'une quelconque des séquences d'acide nucléique SEQ ID NO : 926 à 932 et/ou leurs séquences d'acide nucléique complémentaires (séquences nucléotidiques correspondant aux différentes séquences variables du locus CRISPR1 du sérotype Paratyphi A) ;
- l'ensemble S13-2, comprenant ou constitué d'au moins une sonde comprenant au moins ou constituée d'au moins 8 nucléotides consécutifs de l'une quelconque des séquences d'acide nucléique SEQ ID NO : 933, 935, 936 et/ou leurs séquences d'acide nucléique complémentaires (séquences nucléotidiques correspondant aux différentes séquences variables du locus CRISPR2 du sérotype Paratyphi A) ;
- l'ensemble S14-1, comprenant ou constitué d'au moins une sonde comprenant au moins ou constituée d'au moins 8 nucléotides consécutifs de l'une quelconque des séquences d'acide nucléique SEQ ID NO : 707, 937 à 971, 1106, 1128 à 1130, 1133, 1138, 1140, 1802 à 1808, 1915 et/ou leurs séquences d'acide nucléique complémentaires (séquences nucléotidiques correspondant aux différentes séquences variables du locus CRISPR1 des sérotypes Paratyphi B et Paratyphi B Java) ;
- l'ensemble S14-2, comprenant ou constitué d'au moins une sonde comprenant au moins ou constituée d'au moins 8 nucléotides consécutifs de l'une quelconque des séquences d'acide nucléique SEQ ID NO : 861, 972 à 987, 989 à 992 à 996, 1172 1262, 1462, 1497, 1810 à 1825 et/ou leurs séquences d'acide nucléique complémentaires (séquences nucléotidiques correspondant aux différentes séquences variables du locus CRISPR2 des sérotypes Paratyphi B et Paratyphi B Java) ;
- l'ensemble S15-1, comprenant ou constitué d'au moins une sonde comprenant au moins ou constituée d'au moins 8 nucléotides consécutifs de l'une quelconque des séquences d'acide nucléique SEQ ID NO : 232, 238, 239, 997 à 1002, 1106 et/ou leurs séquences d'acide nucléique complémentaires (séquences nucléotidiques correspondant aux différentes séquences variables du locus CRISPR1 du sérotype Paratyphi C) ;
- l'ensemble S15-2, comprenant ou constitué d'au moins une sonde comprenant au moins ou constituée d'au moins 8 nucléotides consécutifs de l'une quelconque des séquences d'acide nucléique SEQ ID NO : 280, 290, 291, 392, 393, 415, 1003 à 1006, 1262 et/ou leurs séquences d'acide nucléique complémentaires (séquences nucléotidiques correspondant aux différentes séquences variables du locus CRISPR2 du sérotype Paratyphi C) ;
- l'ensemble S16-1, comprenant ou constitué d'au moins une sonde comprenant au moins ou constituée d'au moins 8 nucléotides consécutifs de l'une quelconque des séquences d'acide nucléique SEQ ID NO : 211 à 224, 421, 427, 428, 432, 443 à 445, 447 à 449, 504 et/ou leurs séquences d'acide nucléique complémentaires (séquences nucléotidiques correspondant aux différentes séquences variables du locus CRISPR1 du sérotype Bovismorbificans) ;
- l'ensemble S16-2, comprenant ou constitué d'au moins une sonde comprenant au moins ou constituée d'au moins 8 nucléotides consécutifs de l'une quelconque des séquences d'acide nucléique SEQ ID NO : 196 à 209, 994, 1144, 1145, 1172 et/ou leurs séquences d'acide nucléique complémentaires (séquences nucléotidiques correspondant aux différentes séquences variables du locus CRISPR2 du sérotype Bovismorbificans) ;
- l'ensemble S17-1, comprenant ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Panama) ;
- l'ensemble S17-2, comprenant ou constitué d'au moins une sonde comprenant au moins ou constituée d'au moins 8 nucléotides consécutifs de l'une quelconque des séquences d'acide nucléique SEQ ID NO : 918 à 925, 934 et/ou leurs séquences d'acide nucléique complémentaires (séquences nucléotidiques correspondant aux différentes séquences variables du locus CRISPR2 du sérotype Panama) ;
- l'ensemble S18-1, comprenant ou constitué d'au moins une sonde comprenant au moins ou constituée d'au moins 8 nucléotides consécutifs de l'une quelconque des séquences d'acide nucléique SEQ ID NO : 11, 16, 67, 74, 442, 444, 446 à 448, 602, 937, 948, 1120, 1128 à 1130, 1182 à 1200, 1937 à 1950, et/ou leurs séquences d'acide nucléique complémentaires (séquences nucléotidiques correspondant aux différentes séquences variables du locus CRISPR1 du sérotype Saintpaul) ;
- l'ensemble S18-2, comprenant ou constitué d'au moins une sonde comprenant au moins ou constituée d'au moins 8 nucléotides consécutifs de l'une quelconque des séquences d'acide nucléique SEQ ID NO : 972, 983, 989, 1201 à 1214, 1216 à 1227, 1952 à 1960 et/ou leurs séquences d'acide nucléique complémentaires (séquences nucléotidiques correspondant aux différentes séquences variables du locus CRISPR2 du sérotype Saintpaul) ;
- l'ensemble S19-1, comprenant ou constitué d'au moins une sonde comprenant au moins ou constituée d'au moins 8 nucléotides consécutifs de l'une quelconque des séquences d'acide nucléique SEQ ID NO : 514, 1062 à 1089, 1100, 1284, 1886 à 1911 et/ou leurs séquences d'acide nucléique complémentaires (séquences nucléotidiques correspondant aux différentes séquences variables du locus CRISPR1 du sérotype Senftenberg) ;
- l'ensemble S19-2, comprenant ou constitué d'au moins une sonde comprenant au moins ou constituée d'au moins 8 nucléotides consécutifs de l'une quelconque des séquences d'acide nucléique SEQ ID NO : 300, 1090 à 1097, 1101 à 1103, 1304, 1315, 1318 à 1320 et/ou leurs séquences d'acide nucléique complémentaires (séquences nucléotidiques correspondant aux différentes séquences variables du locus CRISPR2 du sérotype Senftenberg) ;
- l'ensemble S20-1, comprenant ou constitué d'au moins une sonde comprenant au moins ou constituée d'au moins 8 nucléotides consécutifs de l'une quelconque des séquences d'acide nucléique SEQ ID NO : 67, 74 à 80 et/ou leurs séquences d'acide nucléique complémentaires (séquences nucléotidiques correspondant aux différentes séquences variables du locus CRISPR1 du sérotype Anatum) ;
- l'ensemble S20-2, comprenant ou constitué d'au moins une sonde comprenant au moins ou constituée d'au moins 8 nucléotides consécutifs de l'une quelconque des séquences d'acide nucléique SEQ ID NO : 83 à 94, 1304, 1315, 1318 à 1322, 1324, 1390 à 1397 et/ou leurs séquences d'acide nucléique complémentaires (séquences nucléotidiques correspondant aux différentes séquences variables du locus CRISPR2 du sérotype Anatum) ;
- l'ensemble S21-1, comprenant ou constitué d'au moins une sonde comprenant au moins ou constituée d'au moins 8 nucléotides consécutifs de l'une quelconque des séquences d'acide nucléique SEQ ID NO : 379, 383 à 385, 388 et/ou leurs séquences d'acide nucléique complémentaires (séquences nucléotidiques correspondant aux différentes séquences variables du locus CRISPR1 des sérotypes Gallinarum) ;
- l'ensemble S21-2, comprenant ou constitué d'au moins une sonde comprenant au moins ou constituée d'au moins 8 nucléotides consécutifs de l'une quelconque des séquences d'acide nucléique SEQ ID NO : 392, 393, 404, 407 à 415 et/ou leurs séquences d'acide nucléique complémentaires (séquences nucléotidiques correspondant aux différentes séquences variables du locus CRISPR2 des sérotypes Gallinarum) ;
- l'ensemble S22-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Brandenburg) ;
- l'ensemble S22-2, comprenant ou constitué d'au moins une sonde comprenant au moins ou constituée d'au moins 8 nucléotides consécutifs de l'une quelconque des séquences d'acide nucléique SEQ ID NO : 225 à 231 et/ou leurs séquences d'acide nucléique complémentaires (séquences nucléotidiques correspondant aux différentes séquences variables du locus CRISPR2 du sérotype Brandenburg) ;
- l'ensemble S23-1, comprenant ou constitué d'au moins une sonde comprenant au moins ou constituée d'au moins 8 nucléotides consécutifs de l'une quelconque des séquences d'acide nucléique SEQ ID NO: 378, 379 et/ou leurs séquences d'acide nucléique complémentaires (séquences nucléotidiques correspondant aux différentes séquences variables du locus CRISPR1 du sérotype Dublin) ;
- l'ensemble S23-2, comprenant ou constitué d'au moins une sonde comprenant au moins ou constituée d'au moins 8 nucléotides consécutifs de l'une quelconque des séquences d'acide nucléique SEQ ID NO : 392, 393, 411, 412 et/ou leurs séquences d'acide nucléique complémentaires (séquences nucléotidiques correspondant aux différentes séquences variables du locus CRISPR2 du sérotype Dublin) ;
- l'ensemble S24-1, comprenant ou constitué d'au moins une sonde comprenant au moins ou constituée d'au moins 8 nucléotides consécutifs de l'une quelconque des séquences d'acide nucléique SEQ ID NO: 1, 1106 et/ou leurs séquences d'acide nucléique complémentaires (séquences nucléotidiques correspondant aux différentes séquences variables du locus CRISPR1 du sérotype Abortusequi) ;
- l'ensemble S24-2, comprenant ou constitué d'au moins une sonde comprenant au moins ou constituée d'au moins 8 nucléotides consécutifs de l'une quelconque des séquences d'acide nucléique SEQ ID NO : 2 à 10, 130 et/ou leurs séquences d'acide nucléique complémentaires (séquences nucléotidiques correspondant aux différentes séquences variables du locus CRISPR2 du sérotype Abortusequi) ;
- l'ensemble S25-1, comprenant ou constitué d'au moins une sonde comprenant au moins ou constituée d'au moins 8 nucléotides consécutifs de l'une quelconque des séquences d'acide nucléique SEQ ID NO: 378, 379 et/ou leurs séquences d'acide nucléique complémentaires (séquences nucléotidiques correspondant aux différentes séquences variables du locus CRISPR1 du sérotype Kiel) ;
- l'ensemble S25-2, comprenant ou constitué d'au moins une sonde comprenant au moins ou constituée d'au moins 8 nucléotides consécutifs de l'une quelconque des séquences d'acide nucléique SEQ ID NO : 392, 393, 411, 412 et/ou leurs séquences d'acide nucléique complémentaires (séquences nucléotidiques correspondant aux différentes séquences variables du locus CRISPR2 du sérotype Kiel) ;
- l'ensemble S26-1, comprenant ou constitué d'au moins une sonde comprenant au moins ou constituée d'au moins 8 nucléotides consécutifs de l'une quelconque des séquences d'acide nucléique SEQ ID NO : 509, 510, 512, 513, 637 à 660, 1086, 1107 et/ou leurs séquences d'acide nucléique complémentaires (séquences nucléotidiques correspondant aux différentes séquences variables du locus CRISPR1 du sérotype Mbandaka) ;
- l'ensemble S26-2, comprenant ou constitué d'au moins une sonde comprenant au moins ou constituée d'au moins 8 nucléotides consécutifs de l'une quelconque des séquences d'acide nucléique SEQ ID NO : 661 à 666, 668, 669, 672, 683, 690 à 695, 731, 918 et/ou leurs séquences d'acide nucléique complémentaires (séquences nucléotidiques correspondant aux différentes séquences variables du locus CRISPR2 du sérotype Mbandaka) ;
- l'ensemble S27-1, comprenant ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Miami) ;
- l'ensemble S27-2, comprenant ou constitué d'au moins une sonde comprenant au moins ou constituée d'au moins 8 nucléotides consécutifs de l'une quelconque des séquences d'acide nucléique SEQ ID NO : 25, 696 à 700 et/ou leurs séquences d'acide nucléique complémentaires (séquences nucléotidiques correspondant aux différentes séquences variables du locus CRISPR2 du sérotype Miami) ;
- l'ensemble S28-1, comprenant ou constitué d'au moins une sonde comprenant au moins ou constituée d'au moins 8 nucléotides consécutifs de l'une quelconque des séquences d'acide nucléique SEQ ID NO : 55, 701 à 730, 732 à 768, 1115, 1228, 1232 à 1237, 1601, 1671 à 1680, 1682 à 1694, 1747 et/ou leurs séquences d'acide nucléique complémentaires (séquences nucléotidiques correspondant aux différentes séquences variables du locus CRISPR1 du sérotype Montevideo) ;
- l'ensemble S28-2, comprenant ou constitué d'au moins une sonde comprenant au moins ou constituée d'au moins 8 nucléotides consécutifs de l'une quelconque des séquences d'acide nucléique SEQ ID NO : 589, 769 à 829, 933, 1245, 1697, 1698 et/ou leurs séquences d'acide nucléique complémentaires (séquences nucléotidiques correspondant aux différentes séquences variables du locus CRISPR2 du sérotype Montevideo) ;
- l'ensemble S29-1, comprenant ou constitué d'au moins une sonde comprenant au moins ou constituée d'au moins 8 nucléotides consécutifs de l'une quelconque des séquences d'acide nucléique SEQ ID NO : 379, 380, 383 à 385, 387, 389 à 391 et/ou leurs séquences d'acide nucléique complémentaires (séquences nucléotidiques correspondant aux différentes séquences variables du locus CRISPR1 du sérotype Nitra) ;
- l'ensemble S29-2, comprenant ou constitué d'au moins une sonde comprenant au moins ou constituée d'au moins 8 nucléotides consécutifs de l'une quelconque des séquences d'acide nucléique SEQ ID NO : 392 à 394, 404, 407 à 413, 519 et/ou leurs séquences d'acide nucléique complémentaires (séquences nucléotidiques correspondant aux différentes séquences variables du locus CRISPR2 du sérotype Nitra) ;
- l'ensemble S30-1, comprenant ou constitué d'au moins une sonde comprenant au moins ou constituée d'au moins 8 nucléotides consécutifs de l'une quelconque des séquences d'acide nucléique SEQ ID NO : 931, 932, 1104, 1105 et/ou leurs séquences d'acide nucléique complémentaires (séquences nucléotidiques correspondant aux différentes séquences variables du locus CRISPR1 du sérotype Sendai) ;
- l'ensemble S30-2, comprenant ou constitué d'au moins une sonde comprenant au moins ou constituée d'au moins 8 nucléotides consécutifs de la séquence d'acide nucléique SEQ ID NO : 936 et/ou sa séquence d'acide nucléique complémentaire (séquences nucléotidiques correspondant à la séquence variable du locus CRISPR2 du sérotype Sendai) ;
- l'ensemble S31-1, comprenant ou constitué d'au moins une sonde comprenant au moins ou constituée d'au moins 8 nucléotides consécutifs de l'une quelconque des séquences d'acide nucléique SEQ ID NO : 714 à 716, 1228 à 1239 et/ou leurs séquences d'acide nucléique complémentaires (séquences nucléotidiques correspondant aux différentes séquences variables du locus CRISPR1 du sérotype Schwarzengrund) ;
- l'ensemble S31-2, comprenant ou constitué d'au moins une sonde comprenant au moins ou constituée d'au moins 8 nucléotides consécutifs de l'une quelconque des séquences d'acide nucléique SEQ ID NO : 196, 992, 995, 1172, 1240 à 1244, 1246 à 1253 et/ou leurs séquences d'acide nucléique complémentaires (séquences nucléotidiques correspondant aux différentes séquences variables du locus CRISPR2 du sérotype Schwarzengrund) ;
- l'ensemble S32-1, comprenant ou constitué d'au moins une sonde comprenant au moins ou constituée d'au moins 8 nucléotides consécutifs de l'une quelconque des séquences d'acide nucléique SEQ ID NO : 232, 238, 239, 998, 999, 1002, 1106, 1261 et/ou leurs séquences d'acide nucléique complémentaires (séquences nucléotidiques correspondant aux différentes séquences variables du locus CRISPR1 du sérotype Typhisuis) ;
- l'ensemble S32-2, comprenant ou constitué d'au moins une sonde comprenant au moins ou constituée d'au moins 8 nucléotides consécutifs de la séquence d'acide nucléique SEQ ID NO : 1262 et/ou sa séquence d'acide nucléique complémentaire (séquences nucléotidiques correspondant à la séquence variable du locus CRISPR2 du sérotype Typhisuis) ;
- l'ensemble S33-1, comprenant ou constitué d'au moins une sonde comprenant au moins ou constituée d'au moins 8 nucléotides consécutifs de l'une quelconque des séquences d'acide nucléique SEQ ID NO: 466 à 486 et/ou leurs séquences d'acide nucléique complémentaires (séquences nucléotidiques correspondant aux différentes séquences variables du locus CRISPR1 des différents sérotypes de la sous-espèce *houtenae*) ;
- l'ensemble S33-2, comprenant ou constitué d'au moins une sonde comprenant au moins ou constituée d'au moins 8 nucléotides consécutifs de l'une quelconque des séquences d'acide nucléique SEQ ID NO : 487 à 490 et/ou leurs séquences d'acide nucléique complémentaires (séquences nucléotidiques correspondant aux différentes séquences variables du locus CRISPR2 des différents sérotypes de la sous-espèce *houtenae*) ;
- l'ensemble S34-1, comprenant ou constitué d'au moins une sonde comprenant au moins ou constituée d'au moins 8 nucléotides consécutifs de l'une quelconque des séquences d'acide nucléique SEQ ID NO : 338 à 376 et/ou leurs séquences d'acide nucléique complémentaires (séquences nucléotidiques correspondant aux différentes séquences variables du locus CRISPR1 des différents sérotypes de la sous-espèce *diarizonae*) ;
- l'ensemble S34-2, comprenant ou constitué d'au moins une sonde comprenant au moins ou constituée d'au moins 8 nucléotides consécutifs de la séquence d'acide nucléique SEQ ID NO : 377 et/ou sa séquence d'acide nucléique complémentaire (séquences nucléotidiques correspondant à la séquence variable du locus CRISPR2 des différents sérotypes de la sous-espèce *diarizonae*) ;
- l'ensemble S35-1, comprenant ou constitué d'au moins une sonde comprenant au moins ou constituée d'au moins 8 nucléotides consécutifs de l'une quelconque des séquences d'acide nucléique SEQ ID NO : 1007 à 1037, 1848 à 1884 et/ou leurs séquences d'acide nucléique complémentaires (séquences nucléotidiques correspondant aux différentes séquences variables du locus CRISPR1 de la sous-espèce *salamae*) ;
- l'ensemble S35-2, comprenant ou constitué d'au moins une sonde comprenant au moins ou constituée d'au moins 8 nucléotides consécutifs de l'une quelconque des séquences d'acide nucléique SEQ ID NO : 1038 à 1043, 1045 à 1061, 1885 et/ou leurs séquences d'acide nucléique complémentaires (séquences nucléotidiques correspondant aux différentes séquences variables du locus CRISPR2 de la sous-espèce *salamae*) ;
- l'ensemble S36, comprenant ou constitué d'au moins une sonde comprenant au moins ou constituée d'au moins 8 nucléotides consécutifs de la séquence d'acide nucléique SEQ ID NO : 95 et/ou sa séquence d'acide nucléique complémentaire (séquences nucléotidiques correspondant à la séquence variable du locus CRISPR1 des différents sérotypes de la sous-espèce *arizonae*) ;
- l'ensemble S37-1, comprenant ou constitué d'au moins une sonde comprenant au moins ou constituée d'au moins 8 nucléotides consécutifs de l'une quelconque des séquences d'acide nucléique SEQ ID NO : 491 à 499 et/ou leurs séquences d'acide nucléique complémentaires (séquences nucléotidiques correspondant aux différentes séquences variables du locus CRISPR1 des différents séryotpes de la sous-espèce *indica*) ;
- l'ensemble S37-2, comprenant ou constitué d'au moins une sonde comprenant au moins ou constituée d'au moins 8 nucléotides consécutifs de l'une quelconque des séquences d'acide nucléique SEQ ID NO : 500 à 503, 1574 à 1589 et/ou leurs séquences d'acide nucléique complémentaires (séquences nucléotidiques correspondant aux différentes séquences variables du locus CRISPR2 des différents sérotypes de la sous-espèce *indica*) ;
- l'ensemble S38-1, comprenant ou constitué d'au moins une sonde comprenant au moins ou constituée d'au moins 8 nucléotides consécutifs de l'une quelconque des séquences d'acide nucléique SEQ ID NO : 32 à 66, 382, 416, 667, 2036 et/ou leurs séquences d'acide nucléique complémentaires (séquences nucléotidiques correspondant aux différentes séquences variables du locus CRISPR1 du sérotype Altona) ;
- l'ensemble S38-2, comprenant ou constitué d'au moins une sonde comprenant au moins ou constituée d'au moins 8 nucléotides consécutifs de l'une quelconque des séquences d'acide nucléique SEQ ID NO : 854, 933, 1095, 1306, 1354 à 1389, 1696 et/ou leurs séquences d'acide nucléique complémentaires (séquences nucléotidiques correspondant aux différentes séquences variables du locus CRISPR2 du sérotype Altona) ;
- l'ensemble S39-1, comprenant ou constitué d'au moins une sonde comprenant au moins ou constituée d'au moins 8 nucléotides consécutifs de l'une quelconque des séquences d'acide nucléique SEQ ID NO : 240, 241, 1120, 1121 et/ou leurs séquences d'acide nucléique complémentaires (séquences nucléotidiques correspondant aux différentes séquences variables du locus CRISPR1 du sérotype Choleraesuis variété Decatur) ;
- l'ensemble S39-2, comprenant ou constitué d'au moins une sonde comprenant au moins ou constituée d'au moins 8 nucléotides consécutifs de l'une quelconque des séquences d'acide nucléique SEQ ID NO : 225, 247 à 251, 253 à 256, 259 à 268, 270 à 279, 281 à 289, 292 à 294 et/ou leurs séquences d'acide nucléique complémentaires (séquences nucléotidiques correspondant aux différentes séquences variables du locus CRISPR2 du sérotype Choleraesuis variété Decatur) ;
- l'ensemble S40-1, comprenant ou constitué d'au moins une sonde comprenant au moins ou constituée d'au moins 8 nucléotides consécutifs de l'une quelconque des séquences d'acide nucléique SEQ ID NO : 11, 232, 238, 239, 240, 242 à 245, 233 à 237, 959, 1106, 1120 et/ou leurs séquences d'acide nucléique complémentaires (séquences nucléotidiques correspondant aux différentes séquences variables du locus CRISPR1 du sérotype Choleraesuis variété Kunzendorf) ;
- l'ensemble S40-2, comprenant ou constitué d'au moins une sonde comprenant au moins ou constituée d'au moins 8 nucléotides consécutifs de l'une quelconque des séquences d'acide nucléique SEQ ID NO : 246, 252, 258, 269, 280, 290, 291, 392, 563, 933 et/ou leurs séquences d'acide nucléique complémentaires (séquences nucléotidiques correspondant aux différentes séquences variables du locus CRISPR2 du sérotype Choleraesuis variété Kunzendorf) ;
- l'ensemble S41-1, comprenant ou constitué d'au moins une sonde comprenant au moins ou constituée d'au moins 8 nucléotides consécutifs de l'une quelconque des séquences d'acide nucléique SEQ ID NO : 11, 242 à 245, 232 à 239, 959, 1000, 1106, 1139, 1120 et/ou leurs séquences d'acide nucléique complémentaires (séquences nucléotidiques correspondant aux différentes séquences variables du locus CRISPR1 des sérotypes Choleraesuis et Choleraesuis sensu stricto) ;
- l'ensemble S41-2, comprenant ou constitué d'au moins une sonde comprenant au moins ou constituée d'au moins 8 nucléotides consécutifs de l'une quelconque des séquences d'acide nucléique SEQ ID NO : 246, 252, 258, 269, 280, 290, 291, 392, 563, 574, 933, 1003, 1004 et/ou leurs séquences d'acide nucléique complémentaires (séquences nucléotidiques correspondant aux différentes séquences variables du locus CRISPR2 des sérotypes Choleraesuis et Choleraesuis sensu stricto) ;
- l'ensemble S42-1, comprenant ou constitué d'au moins une sonde comprenant au moins ou constituée d'au moins 8 nucléotides consécutifs de l'une quelconque des séquences d'acide nucléique SEQ ID NO : 416 à 420, 1122 et/ou leurs séquences d'acide nucléique complémentaires (séquences nucléotidiques correspondant aux différentes séquences variables du locus CRISPR1 du sérotype Goettingen) ;
- l'ensemble S42-2, comprenant ou constitué d'au moins une sonde comprenant au moins ou constituée d'au moins 8 nucléotides consécutifs de la séquence d'acide nucléique SEQ ID NO : 1548 et/ou sa séquence d'acide nucléique complémentaire (séquences nucléotidiques correspondant à la séquence variable du locus CRISPR2 du sérotype Goettingen) ;
- l'ensemble S43-1, comprenant ou constitué d'au moins une sonde comprenant au moins ou constituée d'au moins 8 nucléotides consécutifs de l'une quelconque des séquences d'acide nucléique SEQ ID NO : 630 à 636, 937, 948, 1120, 1143 et/ou leurs séquences d'acide nucléique complémentaires (séquences nucléotidiques correspondant aux différentes séquences variables du locus CRISPR1 du sérotype Manhattan) ;
- l'ensemble S43-2, comprenant ou constitué d'au moins une sonde comprenant au moins ou constituée d'au moins 8 nucléotides consécutifs de l'une quelconque des séquences d'acide nucléique SEQ ID NO : 392, 1653 et/ou leurs séquences d'acide nucléique complémentaires (séquences nucléotidiques correspondant aux différentes séquences variables du locus CRISPR2 du sérotype Manhattan) ;
- l'ensemble S44-1, comprenant ou constitué d'au moins une sonde comprenant au moins ou constituée d'au moins 8 nucléotides consécutifs de l'une quelconque des séquences d'acide nucléique SEQ ID NO : 96 à 155 et/ou leurs séquences d'acide nucléique complémentaires (séquences nucléotidiques correspondant aux différentes séquences variables du locus CRISPR1 de certains sérotypes de l'espèce *S. bongori*) ;
- l'ensemble S44-2, comprenant ou constitué d'au moins une sonde comprenant au moins ou constituée d'au moins 8 nucléotides consécutifs de l'une quelconque des séquences d'acide nucléique SEQ ID NO : 156 à 195, 1421 à 1430 et/ou leurs séquences d'acide nucléique complémentaires (séquences nucléotidiques correspondant aux différentes séquences variables du locus CRISPR2 de certains sérotypes de l'espèce *S. bongori*) ;
- l'ensemble S45-1, comprenant ou constitué d'au moins une sonde comprenant au moins ou constituée d'au moins 8 nucléotides consécutifs de l'une quelconque des séquences d'acide nucléique SEQ ID NO : 962, 1106, 1133, 1804, 1808 et/ou leurs séquences d'acide nucléique complémentaires (séquences nucléotidiques correspondant aux différentes séquences variables du locus CRISPR1 du sérotype Abony) ;
- l'ensemble S45-2, comprenant ou constitué d'au moins une sonde comprenant au moins ou constituée d'au moins 8 nucléotides consécutifs de l'une quelconque des séquences d'acide nucléique SEQ ID NO : 972, 989, 1334 à 1339, 1474, 1499 et/ou leurs séquences d'acide nucléique complémentaires (séquences nucléotidiques correspondant aux différentes séquences variables du locus CRISPR2 du sérotype Abony) ;
- l'ensemble S46-1, comprenant ou constitué d'au moins une sonde comprenant au moins ou constituée d'au moins 8 nucléotides consécutifs de l'une quelconque des séquences d'acide nucléique SEQ ID NO : 448, 860 à 875, 882, 883, 1340 à 1345, 1721 à 1723, et/ou leurs séquences d'acide nucléique complémentaires (séquences nucléotidiques correspondant aux différentes séquences variables du locus CRISPR1 du sérotype Aesch) ;
- l'ensemble S46-2, comprenant ou constitué d'au moins une sonde comprenant au moins ou constituée d'au moins 8 nucléotides consécutifs de l'une quelconque des séquences d'acide nucléique SEQ ID NO : 563, 625, 626, 885, 887 à 893, 896, 933, 1346 à 1348, 1749, 1759, et/ou leurs séquences d'acide nucléique complémentaires (séquences nucléotidiques correspondant aux différentes séquences variables du locus CRISPR2 du sérotype Aesch) ;
- l'ensemble S47-1, comprenant ou constitué d'au moins une sonde comprenant au moins ou constituée d'au moins 8 nucléotides consécutifs de l'une quelconque des séquences d'acide nucléique SEQ ID NO : 834, 1120, 1349, 1350, 1351, 1713, 1718 et/ou leurs séquences d'acide nucléique complémentaires (séquences nucléotidiques correspondant aux différentes séquences variables du locus CRISPR1 du sérotype Albany) ;
- l'ensemble S47-2, comprenant ou constitué d'au moins une sonde comprenant au moins ou constituée d'au moins 8 nucléotides consécutifs de l'une quelconque des séquences d'acide nucléique SEQ ID NO : 842, 852, 1352, 1353 et/ou leurs séquences d'acide nucléique complémentaires (séquences nucléotidiques correspondant aux différentes séquences variables du locus CRISPR2 du sérotype Albany) ;
- l'ensemble S48-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Arechavalata) ;
- l'ensemble S48-2, comprenant ou constitué d'au moins une sonde comprenant au moins ou constituée d'au moins 8 nucléotides consécutifs de l'une quelconque des séquences d'acide nucléique SEQ ID NO : 225, 227 à 231, 1398 et/ou leurs séquences d'acide nucléique complémentaires (séquences nucléotidiques correspondant aux différentes séquences variables du locus CRISPR2 du sérotype Arechavalata) ;
- l'ensemble S49-1, comprenant ou constitué d'au moins une sonde comprenant au moins ou constituée d'au moins 8 nucléotides consécutifs de l'une quelconque des séquences d'acide nucléique SEQ ID NO : 448, 449, 860, 871 à 873, 878, 895, 903 à 905, 907, 1719 à 1721, 1745 et/ou leurs séquences d'acide nucléique complémentaires (séquences nucléotidiques correspondant aux différentes séquences variables du locus CRISPR1 du sérotype Bardo) ;
- l'ensemble S49-2, comprenant ou constitué d'au moins une sonde comprenant au moins ou constituée d'au moins 8 nucléotides consécutifs de l'une quelconque des séquences d'acide nucléique SEQ ID NO : 563, 622, 626, 885, 886 à 890, 893, 896, 907, 910 à 915, 933, 1756 à 1758 et/ou leurs séquences d'acide nucléique complémentaires (séquences nucléotidiques correspondant aux différentes séquences variables du locus CRISPR2 du sérotype Bardo) ;
- l'ensemble S50-1, comprenant ou constitué d'au moins une sonde comprenant au moins ou constituée d'au moins 8 nucléotides consécutifs de l'une quelconque des séquences d'acide nucléique SEQ ID NO: 82, 1401 et/ou leurs séquences d'acide nucléique complémentaires (séquences nucléotidiques correspondant aux différentes séquences variables du locus CRISPR1 du sérotype Berta) ;
- l'ensemble S50-2, comprenant ou constitué d'au moins une sonde comprenant au moins ou constituée d'au moins 8 nucléotides consécutifs de l'une quelconque des séquences d'acide nucléique SEQ ID NO : 246, 918, 933, 1401, 1448 et/ou leurs séquences d'acide nucléique complémentaires (séquences nucléotidiques correspondant aux différentes séquences variables du locus CRISPR2 du sérotype Berta) ;
- l'ensemble S51-1, comprenant ou constitué d'au moins une sonde comprenant au moins ou constituée d'au moins 8 nucléotides consécutifs de l'une quelconque des séquences d'acide nucléique SEQ ID NO : 1, 37, 1106, 1402 à 1416, 2055 et/ou leurs séquences d'acide nucléique complémentaires (séquences nucléotidiques correspondant aux différentes séquences variables du locus CRISPR1 du sérotype Bispebjerg) ;
- l'ensemble S51-2, comprenant ou constitué d'au moins une sonde comprenant au moins ou constituée d'au moins 8 nucléotides consécutifs de l'une quelconque des séquences d'acide nucléique SEQ ID NO : 2 à 4, 9, 1417 à 1420, 1547, 1572 et/ou leurs séquences d'acide nucléique complémentaires (séquences nucléotidiques correspondant aux différentes séquences variables du locus CRISPR2 du sérotype Bispebjerg) ;
- l'ensemble S52-1, comprenant ou constitué d'au moins une sonde comprenant au moins ou constituée d'au moins 8 nucléotides consécutifs de l'une quelconque des séquences d'acide nucléique SEQ ID NO : 379, 380, 383, 385, 387, 389 à 391 et/ou leurs séquences d'acide nucléique complémentaires (séquences nucléotidiques correspondant aux différentes séquences variables du locus CRISPR1 du sérotype Blegdam) ;
- l'ensemble S52-2, comprenant ou constitué d'au moins une sonde comprenant au moins ou constituée d'au moins 8 nucléotides consécutifs de l'une quelconque des séquences d'acide nucléique SEQ ID NO : 392 à 394, 404, 407, 412, 413, 519 et/ou leurs séquences d'acide nucléique complémentaires (séquences nucléotidiques correspondant aux différentes séquences variables du locus CRISPR2 du sérotype Blegdam) ;
- l'ensemble S53-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Chester) ;
- l'ensemble S53-2, comprenant ou constitué d'au moins une sonde comprenant au moins ou constituée d'au moins 8 nucléotides consécutifs de l'une quelconque des séquences d'acide nucléique SEQ ID NO : 933, 1445 à 1447 et/ou leurs séquences d'acide nucléique complémentaires (séquences nucléotidiques correspondant aux différentes séquences variables du locus CRISPR2 du sérotype Chester) ;
- l'ensemble S54-1, comprenant ou constitué d'au moins une sonde comprenant au moins ou constituée d'au moins 8 nucléotides consécutifs de l'une quelconque des séquences d'acide nucléique SEQ ID NO : 1449 à 1454, 1460, 1467 à 1473 et/ou leurs séquences d'acide nucléique complémentaires (séquences nucléotidiques correspondant aux différentes séquences variables du locus CRISPR1 du sérotype Concord) ;
- l'ensemble S54-2, comprenant ou constitué d'au moins une sonde comprenant au moins ou constituée d'au moins 8 nucléotides consécutifs de l'une quelconque des séquences d'acide nucléique SEQ ID NO : 972, 1474 à 1493, 1495 à 1499 et/ou leurs séquences d'acide nucléique complémentaires (séquences nucléotidiques correspondant aux différentes séquences variables du locus CRISPR2 du sérotype Concord) ;
- l'ensemble S55-1, comprenant ou constitué d'au moins une sonde comprenant au moins ou constituée d'au moins 8 nucléotides consécutifs de l'une quelconque des séquences d'acide nucléique SEQ ID NO : 1106, 1107 à 1109, 1123, 1128 à 1131, 1133, 1137 à 1140, 1550, 1559 à 1561 et/ou leurs séquences d'acide nucléique complémentaires (séquences nucléotidiques correspondant aux différentes séquences variables du locus CRISPR1 du sérotype Crossness) ;
- l'ensemble S55-2, comprenant ou constitué d'au moins une sonde comprenant au moins ou constituée d'au moins 8 nucléotides consécutifs de l'une quelconque des séquences d'acide nucléique SEQ ID NO : 455, 1144, 1145, 1149 à 1153, 1155 à 1160, 1163, 1172 à 1174 et/ou leurs séquences d'acide nucléique complémentaires (séquences nucléotidiques correspondant aux différentes séquences variables du locus CRISPR2 du sérotype Crossness) ;
- l'ensemble S56-1, comprenant ou constitué d'au moins une sonde comprenant au moins ou constituée d'au moins 8 nucléotides consécutifs de l'une quelconque des séquences d'acide nucléique SEQ ID NO : 834, 1120, 1349 à 1351, 1713, 1718 et/ou leurs séquences d'acide nucléique complémentaires (séquences nucléotidiques correspondant aux différentes séquences variables du locus CRISPR1 du sérotype Duesseldorf) ;
- l'ensemble S56-2, comprenant ou constitué d'au moins une sonde comprenant au moins ou constituée d'au moins 8 nucléotides consécutifs de l'une quelconque des séquences d'acide nucléique SEQ ID NO : 842, 852, 1352, 1353, 1501 à 1503 et/ou leurs séquences d'acide nucléique complémentaires (séquences nucléotidiques correspondant aux différentes séquences variables du locus CRISPR2 du sérotype Duesseldorf) ;
- l'ensemble S57-1, comprenant ou constitué d'au moins une sonde comprenant au moins ou constituée d'au moins 8 nucléotides consécutifs de l'une quelconque des séquences d'acide nucléique SEQ ID NO : 941, 1504 à 1508 et/ou leurs séquences d'acide nucléique complémentaires (séquences nucléotidiques correspondant aux différentes séquences variables du locus CRISPR1 du sérotype Emek) ;
- l'ensemble S57-2, comprenant ou constitué d'au moins une sonde comprenant au moins ou constituée d'au moins 8 nucléotides consécutifs de l'une quelconque des séquences d'acide nucléique SEQ ID NO : 1260, 1509 à 1524, 1526, 1527 et/ou leurs séquences d'acide nucléique complémentaires (séquences nucléotidiques correspondant aux différentes séquences variables du locus CRISPR2 du sérotype Emek) ;
- l'ensemble S58-1, comprenant ou constitué d'au moins une sonde comprenant au moins ou constituée d'au moins 8 nucléotides consécutifs de la séquence d'acide nucléique SEQ ID NO : 558 et/ou sa séquence d'acide nucléique complémentaire (séquences nucléotidiques correspondant à la séquence variable du locus CRISPR1 du sérotype Fulica) ;
- l'ensemble S58-2, comprenant ou constitué d'au moins une sonde comprenant au moins ou constituée d'au moins 8 nucléotides consécutifs de l'une quelconque des séquences d'acide nucléique SEQ ID NO : 2, 4, 5, 1333, 1547 et/ou leurs séquences d'acide nucléique complémentaires (séquences nucléotidiques correspondant aux différentes séquences variables du locus CRISPR2 du sérotype Fulica) ;
- l'ensemble S59-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Gueuletapee) ;
- l'ensemble S59-2, comprenant ou constitué d'au moins une sonde comprenant au moins ou constituée d'au moins 8 nucléotides consécutifs de l'une quelconque des séquences d'acide nucléique SEQ ID NO : 597, 918 à 925, 933, 1549 et/ou leurs séquences d'acide nucléique complémentaires (séquences nucléotidiques correspondant aux différentes séquences variables du locus CRISPR2 du sérotype Gueuletapee) ;
- l'ensemble S60-1, comprenant ou constitué d'au moins une sonde comprenant au moins ou constituée d'au moins 8 nucléotides consécutifs de l'une quelconque des séquences d'acide nucléique SEQ ID NO : 1106 à 1109, 1111 à 1116, 1118, 1123, 1128 à 1131, 1133, 1137 à 1140, 1150, 1151, 1555 à 1562 et/ou leurs séquences d'acide nucléique complémentaires (séquences nucléotidiques correspondant aux différentes séquences variables du locus CRISPR1 du sérotype Heidelberg) ;
- l'ensemble S60-2, comprenant ou constitué d'au moins une sonde comprenant au moins ou constituée d'au moins 8 nucléotides consécutifs de l'une quelconque des séquences d'acide nucléique SEQ ID NO : 455, 1144, 1145, 1149 à 1153, 1155 à 1163, 1172 à 1174, 1563 et/ou leurs séquences d'acide nucléique complémentaires (séquences nucléotidiques correspondant aux différentes séquences variables du locus CRISPR2 du sérotype Heidelberg) ;
- l'ensemble S61-1, comprenant ou constitué d'au moins une sonde comprenant au moins ou constituée d'au moins 8 nucléotides consécutifs de l'une quelconque des séquences d'acide nucléique SEQ ID NO : 558, 1565 à 1571 et/ou leurs séquences d'acide nucléique complémentaires (séquences nucléotidiques correspondant aux différentes séquences variables du locus CRISPR1 du sérotype Hessarek) ;
- l'ensemble S61-2, comprenant ou constitué d'au moins une sonde comprenant au moins ou constituée d'au moins 8 nucléotides consécutifs de l'une quelconque des séquences d'acide nucléique SEQ ID NO : 2 à 4, 1547, 1572, 1573 et/ou leurs séquences d'acide nucléique complémentaires (séquences nucléotidiques correspondant aux différentes séquences variables du locus CRISPR2 du sérotype Hessarek) ;
- l'ensemble S62-1, comprenant ou constitué d'au moins une sonde comprenant au moins ou constituée d'au moins 8 nucléotides consécutifs de l'une quelconque des séquences d'acide nucléique SEQ ID NO : 1591, 1592, 1667 et/ou leurs séquences d'acide nucléique complémentaires (séquences nucléotidiques correspondant aux différentes séquences variables du locus CRISPR1 du sérotype Itami) ;
- l'ensemble S62-2, comprenant ou constitué d'au moins une sonde comprenant au moins ou constituée d'au moins 8 nucléotides consécutifs de l'une quelconque des séquences d'acide nucléique SEQ ID NO : 918, 1593 à 1600, 1744 et/ou leurs séquences d'acide nucléique complémentaires (séquences nucléotidiques correspondant aux différentes séquences variables du locus CRISPR2 du sérotype Itami) ;
- l'ensemble S63-1, comprenant ou constitué d'au moins une sonde comprenant au moins ou constituée d'au moins 8 nucléotides consécutifs de l'une quelconque des séquences d'acide nucléique SEQ ID NO : 1601 à 1606, 1687 et/ou leurs séquences d'acide nucléique complémentaires (séquences nucléotidiques correspondant aux différentes séquences variables du locus CRISPR1 du sérotype Javiana) ;
- l'ensemble S63-2, comprenant ou constitué d'au moins une sonde comprenant au moins ou constituée d'au moins 8 nucléotides consécutifs de l'une quelconque des séquences d'acide nucléique SEQ ID NO : 589 à 601, 973, 1607, 1608 et/ou leurs séquences d'acide nucléique complémentaires (séquences nucléotidiques correspondant aux différentes séquences variables du locus CRISPR2 du sérotype Javiana) ;
- l'ensemble S64-1, comprenant ou constitué d'au moins une sonde comprenant au moins ou constituée d'au moins 8 nucléotides consécutifs de l'une quelconque des séquences d'acide nucléique SEQ ID NO : 1609 à 1621, 1494, 1525 et/ou leurs séquences d'acide nucléique complémentaires (séquences nucléotidiques correspondant aux différentes séquences variables du locus CRISPR1 du sérotype Johannesburg) ;
- l'ensemble S64-2, comprenant ou constitué d'au moins une sonde comprenant au moins ou constituée d'au moins 8 nucléotides consécutifs de l'une quelconque des séquences d'acide nucléique SEQ ID NO : 918, 1697, 1698 et/ou leurs séquences d'acide nucléique complémentaires (séquences nucléotidiques correspondant aux différentes séquences variables du locus CRISPR2 du sérotype Johannesburg) ;
- l'ensemble S65-1, comprenant ou constitué d'au moins une sonde comprenant au moins ou constituée d'au moins 8 nucléotides consécutifs de l'une quelconque des séquences d'acide nucléique SEQ ID NO : 444, 448, 1184, 1622 à 1637, et/ou leurs séquences d'acide nucléique complémentaires (séquences nucléotidiques correspondant aux différentes séquences variables du locus CRISPR1 du sérotype Kentucky) ;
- l'ensemble S65-2, comprenant ou constitué d'au moins une sonde comprenant au moins ou constituée d'au moins 8 nucléotides consécutifs de l'une quelconque des séquences d'acide nucléique SEQ ID NO : 385, 519, 1638 à 1652, 1746 et/ou leurs séquences d'acide nucléique complémentaires (séquences nucléotidiques correspondant aux différentes séquences variables du locus CRISPR2 du sérotype Kentucky) ;
- l'ensemble S66-1, comprenant ou constitué d'au moins une sonde comprenant au moins ou constituée d'au moins 8 nucléotides consécutifs de la séquence d'acide nucléique SEQ ID NO : 67 et/ou sa séquence d'acide nucléique complémentaire (séquences nucléotidiques correspondant à la séquence variable du locus CRISPR1 du sérotype Kundunchi) ;
- l'ensemble S66-2, comprenant ou constitué d'au moins une sonde comprenant au moins ou constituée d'au moins 8 nucléotides consécutifs de l'une quelconque des séquences d'acide nucléique SEQ ID NO : 972, 983, 989, 1201, 1204, 1205, 1210 à 1212 et/ou leurs séquences d'acide nucléique complémentaires (séquences nucléotidiques correspondant aux différentes séquences variables du locus CRISPR2 du sérotype Kundunchi) ;
- l'ensemble S67-1, comprenant ou constitué d'au moins une sonde comprenant au moins ou constituée d'au moins 8 nucléotides consécutifs de l'une quelconque des séquences d'acide nucléique SEQ ID NO : 448, 449, 860, 871, 878 à 881, 1721, 1724 et/ou leurs séquences d'acide nucléique complémentaires (séquences nucléotidiques correspondant aux différentes séquences variables du locus CRISPR1 1 du sérotype Lindenburg) ;
- l'ensemble S67-2, comprenant ou constitué d'au moins une sonde comprenant au moins ou constituée d'au moins 8 nucléotides consécutifs de l'une quelconque des séquences d'acide nucléique SEQ ID NO : 563, 622, 885, 886, 896, 907, 910 à 915, 933, 1748, 1757 et/ou leurs séquences d'acide nucléique complémentaires (séquences nucléotidiques correspondant aux différentes séquences variables du locus CRISPR2 du sérotype Lindenburg) ;
- l'ensemble S68-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variables du locus CRISPR1 du sérotype Maracaibo) ;
- l'ensemble S68-2, comprenant ou constitué d'au moins une sonde comprenant au moins ou constituée d'au moins 8 nucléotides consécutifs de l'une quelconque des séquences d'acide nucléique SEQ ID NO : 1240, 1247, 1248, 1654 à 1656 et/ou leurs séquences d'acide nucléique complémentaires (séquences nucléotidiques correspondant aux différentes séquences variables du locus CRISPR2 du sérotype Maracaibo) ;
- l'ensemble S69-1, comprenant ou constitué d'au moins une sonde comprenant au moins ou constituée d'au moins 8 nucléotides consécutifs de l'une quelconque des séquences d'acide nucléique SEQ ID NO : 627, 834, 1120, 1660 à 1669 et/ou leurs séquences d'acide nucléique complémentaires (séquences nucléotidiques correspondant aux différentes séquences variables du locus CRISPR1 du sérotype Mississippi) ;
- l'ensemble S69-2, comprenant ou constitué d'au moins une sonde comprenant au moins ou constituée d'au moins 8 nucléotides consécutifs de l'une quelconque des séquences d'acide nucléique SEQ ID NO : 842, 1670 et/ou leurs séquences d'acide nucléique complémentaires (séquences nucléotidiques correspondant aux différentes séquences variables du locus CRISPR2 du sérotype Mississippi) ;
- l'ensemble S70-1, comprenant ou constitué d'au moins une sonde comprenant au moins ou constituée d'au moins 8 nucléotides consécutifs de l'une quelconque des séquences d'acide nucléique SEQ ID NO : 941, 942, 949, 1699 à 1712, 2109 et/ou leurs séquences d'acide nucléique complémentaires (séquences nucléotidiques correspondant aux différentes séquences variables du locus CRISPR1 du sérotype Muenchen) ;
- l'ensemble S70-2, comprenant ou constitué d'au moins une sonde comprenant au moins ou constituée d'au moins 8 nucléotides consécutifs de l'une quelconque des séquences d'acide nucléique SEQ ID NO : 392, 1653 et/ou leurs séquences d'acide nucléique complémentaires (séquences nucléotidiques correspondant aux différentes séquences variables du locus CRISPR2 du sérotype Muenchen);
- l'ensemble S71-1, comprenant ou constitué d'au moins une sonde comprenant au moins ou constituée d'au moins 8 nucléotides consécutifs de l'une quelconque des séquences d'acide nucléique SEQ ID NO : 11, 1120, 1497, 1760 à 1775, 1809 et/ou leurs séquences d'acide nucléique complémentaires (séquences nucléotidiques correspondant aux différentes séquences variables du locus CRISPR1 du sérotype Niarembe) ;
- l'ensemble S71-2, comprenant ou constitué d'au moins une sonde comprenant au moins ou constituée d'au moins 8 nucléotides consécutifs de l'une quelconque des séquences d'acide nucléique SEQ ID NO : 90, 1776 à 1785 et/ou leurs séquences d'acide nucléique complémentaires (séquences nucléotidiques correspondant aux différentes séquences variables du locus CRISPR2 du sérotype Niarembe) ;
- l'ensemble S72-1, comprenant ou constitué d'au moins une sonde comprenant au moins ou constituée d'au moins 8 nucléotides consécutifs de l'une quelconque des séquences d'acide nucléique SEQ ID NO : 666, 1786 à 1791 et/ou leurs séquences d'acide nucléique complémentaires (séquences nucléotidiques correspondant aux différentes séquences variables du locus CRISPR1 du sérotype Overvecht) ;
- l'ensemble S72-2, comprenant ou constitué d'au moins une sonde comprenant au moins ou constituée d'au moins 8 nucléotides consécutifs de l'une quelconque des séquences d'acide nucléique SEQ ID NO : 783, 1146, 1178, 1179, 1181, 1792 à 1799, 1801 et/ou leurs séquences d'acide nucléique complémentaires (séquences nucléotidiques correspondant aux différentes séquences variables du locus CRISPR2 du sérotype Overvecht) ;
- l'ensemble S73-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Poona) ;
- l'ensemble S73-2, comprenant ou constitué d'au moins une sonde comprenant au moins ou constituée d'au moins 8 nucléotides consécutifs de l'une quelconque des séquences d'acide nucléique SEQ ID NO : 82, 87, 918, 933, 1826 et/ou leurs séquences d'acide nucléique complémentaires (séquences nucléotidiques correspondant aux différentes séquences variables du locus CRISPR2 du sérotype Poona) ;
- l'ensemble S74-1, comprenant ou constitué d'au moins une sonde comprenant au moins ou constituée d'au moins 8 nucléotides consécutifs de l'une quelconque des séquences d'acide nucléique SEQ ID NO : 295 à 297, 307, 313, 317, 323, 1120, 1825, 1827 à 1837, 2023 et/ou leurs séquences d'acide nucléique complémentaires (séquences nucléotidiques correspondant aux différentes séquences variables du locus CRISPR1 du sérotype Postdam) ;
- l'ensemble S74-2, comprenant ou constitué d'au moins une sonde comprenant au moins ou constituée d'au moins 8 nucléotides consécutifs de l'une quelconque des séquences d'acide nucléique SEQ ID NO : 663, 1304, 1838 à 1847 et/ou leurs séquences d'acide nucléique complémentaires (séquences nucléotidiques correspondant aux différentes séquences variables du locus CRISPR2 du sérotype Postdam) ;
- l'ensemble S75-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Reading) ;
- l'ensemble S75-2, comprenant ou constitué d'au moins une sonde comprenant au moins ou constituée d'au moins 8 nucléotides consécutifs de la séquence d'acide nucléique SEQ ID NO : 225 et/ou sa séquence d'acide nucléique complémentaire (séquences nucléotidiques correspondant à la séquence variable du locus CRISPR2 du sérotype Reading) ;
- l'ensemble S76-1, comprenant ou constitué d'au moins une sonde comprenant au moins ou constituée d'au moins 8 nucléotides consécutifs de l'une quelconque des séquences d'acide nucléique SEQ ID NO : 379, 380, 383 à 385, 387, 389, 390 et/ou leurs séquences d'acide nucléique complémentaires (séquences nucléotidiques correspondant aux différentes séquences variables du locus CRISPR1 du sérotype Rosenberg) ;
- l'ensemble S76-2, comprenant ou constitué d'au moins une sonde comprenant au moins ou constituée d'au moins 8 nucléotides consécutifs de l'une quelconque des séquences d'acide nucléique SEQ ID NO : 392, 393, 404, 407 à 413 et/ou leurs séquences d'acide nucléique complémentaires (séquences nucléotidiques correspondant aux différentes séquences variables du locus CRISPR2 du sérotype Rosenberg) ;
- l'ensemble S77-1, comprenant ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Rubislaw) ;
- l'ensemble S77-2, comprenant ou constitué d'au moins une sonde comprenant au moins ou constituée d'au moins 8 nucléotides consécutifs de la séquence d'acide nucléique SEQ ID NO : 225 et/ou sa séquence d'acide nucléique complémentaire ;
- l'ensemble S78-1, comprenant ou constitué d'au moins une sonde comprenant au moins ou constituée d'au moins 8 nucléotides consécutifs de l'une quelconque des séquences d'acide nucléique SEQ ID NO : 1525, 1609 à 1621 et/ou leurs séquences d'acide nucléique complémentaires ;
- l'ensemble S78-2, comprenant ou constitué d'au moins une sonde comprenant au moins ou constituée d'au moins 8 nucléotides consécutifs de l'une quelconque des séquences d'acide nucléique SEQ ID NO : 225 à 227, 229 à 231 et/ou leurs séquences d'acide nucléique complémentaires (séquences nucléotidiques correspondant aux différentes séquences variables du locus CRISPR2 du sérotype Sandiego) ;
- l'ensemble S79-1, comprenant ou constitué d'au moins une sonde comprenant au moins ou constituée d'au moins 8 nucléotides consécutifs de l'une quelconque des séquences d'acide nucléique SEQ ID NO : 1106, 1456, 1917 à 1924 et/ou leurs séquences d'acide nucléique complémentaires (séquences nucléotidiques correspondant aux différentes séquences variables du locus CRISPR1 du sérotype Stourbridge) ;
- l'ensemble S79-2, comprenant ou constitué d'au moins une sonde comprenant au moins ou constituée d'au moins 8 nucléotides consécutifs de l'une quelconque des séquences d'acide nucléique SEQ ID NO : 918, 1093, 1925 à 1936 et/ou leurs séquences d'acide nucléique complémentaires (séquences nucléotidiques correspondant aux différentes séquences variables du locus CRISPR2 du sérotype Stourbridge) ;
- l'ensemble S80-1, comprenant ou constitué d'au moins une sonde comprenant au moins ou constituée d'au moins 8 nucléotides consécutifs de l'une quelconque des séquences d'acide nucléique SEQ ID NO : 1961 à 1963 et/ou leurs séquences d'acide nucléique complémentaires (séquences nucléotidiques correspondant aux différentes séquences variables du locus CRISPR1 du sérotype Tallahassee) ;
- l'ensemble S80-2, comprenant ou constitué d'au moins une sonde comprenant au moins ou constituée d'au moins 8 nucléotides consécutifs de l'une quelconque des séquences d'acide nucléique SEQ ID NO : 25, 603, 2127, 2133, 1964 à 1966 et/ou leurs séquences d'acide nucléique complémentaires (séquences nucléotidiques correspondant aux différentes séquences variables du locus CRISPR2 du sérotype Tallahassee) ;
- l'ensemble S81-1, comprenant ou constitué d'au moins une sonde comprenant au moins ou constituée d'au moins 8 nucléotides consécutifs de l'une quelconque des séquences d'acide nucléique SEQ ID NO : 67, 929, 1967 à 2005 et/ou leurs séquences d'acide nucléique complémentaires ;
- l'ensemble S81-2, comprenant ou constitué d'au moins une sonde comprenant au moins ou constituée d'au moins 8 nucléotides consécutifs de l'une quelconque des séquences d'acide nucléique SEQ ID NO : 24, 769, 1550, 1659, 2006 à 2022 et/ou leurs séquences d'acide nucléique complémentaires (séquences nucléotidiques correspondant aux différentes séquences variables du locus CRISPR2 du sérotype Tennessee) ;
- l'ensemble S82-1, comprenant ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Urbana) ;
- l'ensemble S82-2, comprenant ou constitué d'au moins une sonde comprenant au moins ou constituée d'au moins 8 nucléotides consécutifs de l'une quelconque des séquences d'acide nucléique SEQ ID NO : 1697, 1698, 1800 et/ou leurs séquences d'acide nucléique complémentaires (séquences nucléotidiques correspondant aux différentes séquences variables du locus CRISPR2 du sérotype Urbana) ;
- l'ensemble S83-1, comprenant ou constitué d'au moins une sonde comprenant au moins ou constituée d'au moins 8 nucléotides consécutifs de l'une quelconque des séquences d'acide nucléique SEQ ID NO : 948, 1120, 1123, 1325, 2039 à 2078 et/ou leurs séquences d'acide nucléique complémentaires (séquences nucléotidiques correspondant aux différentes séquences variables du locus CRISPR1 du sérotype Weltevreden) ;
- l'ensemble S83-2, comprenant ou constitué d'au moins une sonde comprenant au moins ou constituée d'au moins 8 nucléotides consécutifs de l'une quelconque des séquences d'acide nucléique SEQ ID NO : 254 à 256, 2079 à 2108 et/ou leurs séquences d'acide nucléique complémentaires (séquences nucléotidiques correspondant aux différentes séquences variables du locus CRISPR2 du sérotype Weltevreden) ;
- l'ensemble S84-1, comprenant ou constitué d'au moins une sonde comprenant au moins ou constituée d'au moins 8 nucléotides consécutifs de l'une quelconque des séquences d'acide nucléique SEQ ID NO : 1312, 1690, 2141, 2142 et/ou leurs séquences d'acide nucléique complémentaires (séquences nucléotidiques correspondant aux différentes séquences variables du locus CRISPR1 du sérotype Zaiman) ;
- l'ensemble S84-2, comprenant ou constitué d'au moins une sonde comprenant au moins ou constituée d'au moins 8 nucléotides consécutifs de l'une quelconque des séquences d'acide nucléique SEQ ID NO : 1697, 1698, 1800, 1924, 2143, 2144 et/ou leurs séquences d'acide nucléique complémentaires (séquences nucléotidiques correspondant aux différentes séquences variables du locus CRISPR2 du sérotype Zaiman) ; et
(ii) détection des sondes hybridées.

11. Méthode selon la revendication 10, **caractérisée en ce que** les sondes présentent une longueur comprise entre 8 et 76 nucléotides, de préférence entre 8 et 34 nucléotides.

12. Méthode selon la revendication 10 ou la revendication 11, **caractérisée en ce que** ladite étape (b) est réalisée par hybridation d'au moins une sonde de chacun des ensembles S1-1 à S6-2.

13. Méthode selon la revendication 12, **caractérisée en ce que** ladite étape (b) est réalisée par hybridation en outre d'au moins une sonde de chacun des ensembles S7-1 à S10-2.

14. Méthode selon la revendication 13, **caractérisée en ce que** ladite étape (b) est réalisée par hybridation d'au moins une sonde de chacun des ensembles S1-1 à S44-2.

15. Méthode selon l'une quelconque des revendications 1 à 14, **caractérisée en ce que** les fragments d'acide nucléique amplifiés sont marqués.

16. Méthode selon l'une quelconque des revendications 1 à 15, **caractérisée en ce que** l'échantillon est une souche bactérienne du genre *Salmonella* isolée.

17. Méthode selon l'une quelconque des revendications 1 à 16, **caractérisée en ce que** l'étape (a) est précédée d'une étape d'extraction d'acides nucléiques présents dans ledit échantillon.

18. Ensemble de sondes apte à détecter une bactérie du genre *Salmonella,* **caractérisé en ce qu'**il est choisi dans le groupe constitué par :
- l'ensemble S1-1, comprenant les ou constitué des sondes de séquences d'acide nucléique SEQ ID NO : 1106 à 1119, 1123 à 1140, 1142, 1143, 1914 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Typhimurium) ;
- l'ensemble S1-2, comprenant les ou constitué des sondes de séquences d'acide nucléique SEQ ID NO : 455, 1144 à 1147, 1149 à 1153, 1155 à 1181 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Typhimurium) ;
- l'ensemble S2-1, comprenant les ou constitué des sondes de séquences d'acide nucléique SEQ ID NO : 256, 378 à 381, 383 à 387, 389 à 391, 1106, 1514, 1528, 2148 à 2153 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Enteritidis) ;
- l'ensemble S2-2, comprenant les ou constitué des sondes de séquences d'acide nucléique SEQ ID NO : 392 à 413, 519, 815, 972, 983, 989, 1529 à 1546, 1952 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Enteritidis) ;
- l'ensemble S3-1, comprenant les ou constitué des sondes de séquences d'acide nucléique SEQ ID NO : 421 à 442, 444, 445, 447 à 450 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Hadar) ;
- l'ensemble S3-2, comprenant les ou constitué des sondes de séquences d'acide nucléique SEQ ID NO : 451 à 465, 1144 à 1147, 1150 à 1152, 1154, 1155, 1158, 1169, 1172, 1175 à 1177, 1179, 1181 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Hadar) ;
- l'ensemble S4-1, comprenant les ou constitué des sondes de séquences d'acide nucléique SEQ ID NO : 65, 382, 1120, 1263 à 1302, 2024 à 2038 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Virchow) ;
- l'ensemble S4-2, comprenant les ou constitué des sondes de séquences d'acide nucléique SEQ ID NO : 83, 93, 94, 1304 à 1324 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Virchow) ;
- l'ensemble S5-1, comprenant les ou constitué des sondes de séquences d'acide nucléique SEQ ID NO: 531 à 562, 1106 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Infantis) ;
- l'ensemble S5-2, comprenant les ou constitué des sondes de séquences d'acide nucléique SEQ ID NO : 563 à 588 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Infantis) ;
- l'ensemble S6-1, comprenant les ou constitué des sondes de séquences d'acide nucléique SEQ ID NO : 1254 à 1259 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Typhi) ;
- l'ensemble S6-2, comprenant les ou constitué d'au moins une sonde comprenant au moins ou constituée d'au moins 8 nucléotides consécutifs de la séquence d'acide nucléique SEQ ID NO : 1260 et/ou sa séquence d'acide nucléique complémentaire (séquences nucléotidiques correspondant à la séquence variable du locus CRISPR2 du sérotype Typhi) ;
- l'ensemble S7-1, comprenant les ou constitué des sondes de séquences d'acide nucléique SEQ ID NO : 448, 449, 860 à 876, 878 à 884, 1719 à 1724, 1727 à 1743, 1745 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Newport) ;
- l'ensemble S7-2, comprenant les ou constitué des sondes de séquences d'acide nucléique SEQ ID NO : 453 à 456, 563, 621, 622, 625, 626, 657, 885 à 896, 906 à 915, 933, 1144, 1145, 1155, 1156, 1172, 1174, 1748 à 1756, 1758, 1759, 1916 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Newport) ;
- l'ensemble S8-1, comprenant les ou constitué des sondes de séquences d'acide nucléique SEQ ID NO : 295 à 299, 301 à 323, 738, 1106 à 1109, 1120, 1123, 1128 à 1131, 1133, 1137 à 1140, 1550, 1159 à 1561 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Derby) ;
- l'ensemble S8-2, comprenant les ou constitué des sondes de séquences d'acide nucléique SEQ ID NO : 324 à 337, 455, 1144, 1145, 1149 à 1153, 1155 à 1160, 1163, 1172 à 1174 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Derby) ;
- l'ensemble S9-1, comprenant les ou constitué des sondes de séquences d'acide nucléique SEQ ID NO : 295, 307, 602 à 614, 616 à 619, 1106, 1120, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Kottbus) ;
- l'ensemble S9-2, comprenant les ou constitué des sondes de séquences d'acide nucléique SEQ ID NO : 563, 620 à 629, 885, 896, 907, 913, 914, 933, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Kottbus) ;
- l'ensemble S10-1, comprenant les ou constitué des sondes de séquences d'acide nucléique SEQ ID NO : 422 à 425, 448 à 450, 504 à 518 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Indiana) ;
- l'ensemble S10-2, comprenant les ou constitué des sondes de séquences d'acide nucléique SEQ ID NO : 246, 258, 269, 392, 415, 519 à 530, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Indiana) ;
- l'ensemble S11-1, comprenant les ou constitué des sondes de séquences d'acide nucléique SEQ ID NO : 11 à 23, 238, 948, 1120, 1123, 1133 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Agona) ;
- l'ensemble S11-2, comprenant les ou constitué des sondes de séquences d'acide nucléique SEQ ID NO : 24 à 31 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Agona) ;
- l'ensemble S12-1, comprenant les ou constitué des sondes de séquences d'acide nucléique SEQ ID NO: 830 à 841, 1120 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Napoli) ;
- l'ensemble S12-2, comprenant les ou constitué des sondes de séquences d'acide nucléique SEQ ID NO : 842 à 859 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Napoli) ;
- l'ensemble S13-1, comprenant les ou constitué des sondes de séquences d'acide nucléique SEQ ID NO : 926 à 932 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Paratyphi A) ;
- l'ensemble S13-2, comprenant les ou constitué des sondes de séquences d'acide nucléique SEQ ID NO : 933, 935, 936 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Paratyphi A) ;
- l'ensemble S14-1, comprenant les ou constitué des sondes de séquences d'acide nucléique SEQ ID NO : 707, 937 à 971, 1106, 1128 à 1130, 1133, 1138, 1140, 1802 à 1808, 1915 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des sérotypes Paratyphi B et Paratyphi B Java) ;
- l'ensemble S14-2, comprenant les ou constitué des sondes de séquences d'acide nucléique SEQ ID NO : 861, 972 à 987, 989 à 992 à 996, 1172 1262, 1462, 1497, 1810 à 1825 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 des sérotypes Paratyphi B et Paratyphi B Java) ;
- l'ensemble S15-1, comprenant les ou constitué des sondes de séquences d'acide nucléique SEQ ID NO : 232, 238, 239, 997 à 1002, 1106 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Paratyphi C) ;
- l'ensemble S15-2, comprenant les ou constitué des sondes de séquences d'acide nucléique SEQ ID NO : 280, 290, 291, 392, 393, 415, 1003 à 1006, 1262 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Paratyphi C) ;
- l'ensemble S16-1, comprenant les ou constitué d'au moins une sonde comprenant au moins ou constituée d'au moins 8 nucléotides consécutifs de l'une quelconque des séquences d'acide nucléique SEQ ID NO : 211 à 224, 421, 427, 428, 432, 443 à 445, 447 à 449, 504 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Bovismorbificans) ;
- l'ensemble S16-2, comprenant les ou constitué des sondes de séquences d'acide nucléique SEQ ID NO : 196 à 209, 994, 1144, 1145, 1172 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Bovismorbificans) ;
- l'ensemble S17-1, comprenant les ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Panama) ;
- l'ensemble S17-2, comprenant les ou constitué des sondes de séquences d'acide nucléique SEQ ID NO: 918 à 925, 934 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Panama) ;
- l'ensemble S18-1, comprenant les ou constitué des sondes de séquences d'acide nucléique SEQ ID NO : 11, 16, 67, 74, 442, 444, 446 à 448, 602, 937, 948, 1120, 1128 à 1130, 1182 à 1200, 1937 à 1950, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Saintpaul) ;
- l'ensemble S18-2, comprenant les ou constitué des sondes de séquences d'acide nucléique SEQ ID NO : 972, 983, 989, 1201 à 1214, 1216 à 1227, 1952 à 1960 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Saintpaul) ;
- l'ensemble S19-1, comprenant les ou constitué des sondes de séquences d'acide nucléique SEQ ID NO : 514, 1062 à 1089, 1100, 1284, 1886 à 1911 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Senftenberg) ;
- l'ensemble S19-2, comprenant les ou constitué des sondes de séquences d'acide nucléique SEQ ID NO : 300, 1090 à 1097, 1101 à 1103, 1304, 1315, 1318 à 1320 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Senftenberg) ;
- l'ensemble S20-1, comprenant les ou constitué des sondes de séquences d'acide nucléique SEQ ID NO : 67, 74 à 80 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Anatum) ;
- l'ensemble S20-2, comprenant les ou constitué des sondes de séquences d'acide nucléique SEQ ID NO : 83 à 94, 1304, 1315, 1318 à 1322, 1324, 1390 à 1397 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Anatum) ;
- l'ensemble S21-1, comprenant les ou constitué des sondes de séquences d'acide nucléique SEQ ID NO : 379, 383 à 385, 388 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des sérotypes Gallinarum) ;
- l'ensemble S21-2, comprenant les ou constitué des sondes de séquences d'acide nucléique SEQ ID NO : 392, 393, 404, 407 à 415 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 des sérotypes Gallinarum) ;
- l'ensemble S22-1, comprenant les ou constitué d'au moins une sonde comprenant au moins ou constituée d'au moins 8 nucléotides consécutifs de la séquence d'acide nucléique SEQ ID NO: 917 et/ou sa séquence d'acide nucléique complémentaire (séquences nucléotidiques correspondant à la séquence variable du locus CRISPR1 du sérotype Brandenburg) ;
- l'ensemble S22-2, comprenant les ou constitué des sondes de séquences d'acide nucléique SEQ ID NO : 225 à 231 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Brandenburg) ;
- l'ensemble S23-1, comprenant les ou constitué des sondes de séquences d'acide nucléique SEQ ID NO: 378, 379 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Dublin) ;
- l'ensemble S23-2, comprenant les ou constitué des sondes de séquences d'acide nucléique SEQ ID NO: 392, 393, 411, 412 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Dublin) ;
- l'ensemble S24-1, comprenant les ou constitué des sondes de séquences d'acide nucléique SEQ ID NO: 1, 1106 ou d'au moins 8 nucléotides consécutifs de ces séquences d'acide nucléiques et/ou leurs séquences d'acide nucléique complémentaires (séquences nucléotidiques correspondant aux différentes séquences variables du locus CRISPR1 du sérotype Abortusequi) ;
- l'ensemble S24-2, comprenant les ou constitué des sondes de séquences d'acide nucléique SEQ ID NO : 2 à 10, 130 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Abortusequi) ;
- l'ensemble S25-1, comprenant les ou constitué des sondes de séquences d'acide nucléique SEQ ID NO : 378, 379 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Kiel) ;
- l'ensemble S25-2, comprenant les ou constitué des sondes de séquences d'acide nucléique SEQ ID NO: 392, 393, 411, 412 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Kiel) ;
- l'ensemble S26-1, comprenant les ou constitué des sondes de séquences d'acide nucléique SEQ ID NO : 509, 510, 512, 513, 637 à 660, 1086, 1107 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Mbandaka) ;
- l'ensemble S26-2, comprenant les ou constitué des sondes de séquences d'acide nucléique SEQ ID NO : 661 à 666, 668, 669, 672, 683, 690 à 695, 731, 918 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Mbandaka) ;
- l'ensemble S27-1, comprenant les ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Miami) ;
- l'ensemble S27-2, comprenant les ou constitué des sondes de séquences d'acide nucléique SEQ ID NO : 25, 696 à 700 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Miami) ;
- l'ensemble S28-1, comprenant les ou constitué des sondes de séquences d'acide nucléique SEQ ID NO : 55, 701 à 730, 732 à 768, 1115, 1228, 1232 à 1237, 1601, 1671 à 1680, 1682 à 1694, 1747 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Montevideo) ;
- l'ensemble S28-2, comprenant les ou constitué des sondes de séquences d'acide nucléique SEQ ID NO : 589, 769 à 829, 933, 1245, 1697, 1698 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Montevideo) ;
- l'ensemble S29-1, comprenant les ou constitué des sondes de séquences d'acide nucléique SEQ ID NO : 379, 380, 383 à 385, 387, 389 à 391 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Nitra) ;
- l'ensemble S29-2, comprenant les ou constitué des sondes de séquences d'acide nucléique SEQ ID NO : 392 à 394, 404, 407 à 413, 519 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Nitra) ;
- l'ensemble S30-1, comprenant les ou constitué des sondes de séquences d'acide nucléique SEQ ID NO : 931, 932, 1104, 1105 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Sendai) ;
- l'ensemble S30-2, comprenant les ou constitué d'au moins une sonde comprenant au moins ou constituée d'au moins 8 nucléotides consécutifs de la séquence d'acide nucléique SEQ ID NO: 936 et/ou sa séquence d'acide nucléique complémentaire (séquences nucléotidiques correspondant à la séquence variable du locus CRISPR2 du sérotype Sendai) ;
- l'ensemble S31-1, comprenant les ou constitué des sondes de séquences d'acide nucléique SEQ ID NO : 714 à 716, 1228 à 1239 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Schwarzengrund) ;
- l'ensemble S31-2, comprenant les ou constitué des sondes de séquences d'acide nucléique SEQ ID NO : 196, 992, 995, 1172, 1240 à 1244, 1246 à 1253 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Schwarzengrund) ;
- l'ensemble S32-1, comprenant les ou constitué des sondes de séquences d'acide nucléique SEQ ID NO : 232, 238, 239, 998, 999, 1002, 1106, 1261 ou d'au moins 8 nucléotides consécutifs de ces séquences d'acide nucléique et/ou leurs séquences d'acide nucléique complémentaires (séquences nucléotidique correspondant aux différentes séquences variables du locus CRISPR1 du sérotype Typhisuis) ;
- l'ensemble S32-2, comprenant les ou constitué d'au moins une sonde comprenant au moins ou constituée d'au moins 8 nucléotides consécutifs de la séquence d'acide nucléique SEQ ID NO : 1262 et/ou sa séquence d'acide nucléique complémentaire (séquences nucléotidiques correspondant à la séquence variable du locus CRISPR2 du sérotype Typhisuis) ;
- l'ensemble S33-1, comprenant les ou constitué des sondes de séquences d'acide nucléique SEQ ID NO : 466 à 486 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des différents sérotypes de la sous-espèce *houtenae*) ;
- l'ensemble S33-2, comprenant les ou constitué des sondes de séquences d'acide nucléique SEQ ID NO: 487 à 490 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 des différents sérotypes de la sous-espèce *houtenae*) ;
- l'ensemble S34-1, comprenant les ou constitué des sondes de séquences d'acide nucléique SEQ ID NO : 338 à 376 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des différents sérotypes de la sous-espèce *diarizonae*) ;
- l'ensemble S34-2, comprenant les ou constitué d'au moins une sonde comprenant au moins ou constituée d'au moins 8 nucléotides consécutifs de la séquence d'acide nucléique SEQ ID NO : 377 et/ou sa séquence d'acide nucléique complémentaire (séquences nucléotidiques correspondant à la séquence variable du locus CRISPR2 des différents sérotypes de la sous-espèce *diarizonae*) ;
- l'ensemble S35-1, comprenant les ou constitué des sondes de séquences d'acide nucléique SEQ ID NO : 1007 à 1037, 1848 à 1884 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 de la sous-espèce *salamae*) ;
- l'ensemble S35-2, comprenant les ou constitué des sondes de séquences d'acide nucléique SEQ ID NO : 1038 à 1043, 1045 à 1061, 1885 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 de la sous-espèce *salamae*) ;
- l'ensemble S36, comprenant les ou constitué d'au moins une sonde comprenant au moins ou constituée d'au moins 8 nucléotides consécutifs de la séquence d'acide nucléique SEQ ID NO : 95 et/ou sa séquence d'acide nucléique complémentaire (séquences nucléotidiques correspondant à la séquence variable du locus CRISPR1 des différents sérotypes de la sous-espèce *arizonae*) ;
- l'ensemble S37-1, comprenant les ou constitué des sondes de séquences d'acide nucléique SEQ ID NO : 491 à 499 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des différents séryotpes de la sous-espèce *indica*) ;
- l'ensemble S37-2, comprenant les ou constitué des sondes de séquences d'acide nucléique SEQ ID NO : 500 à 503, 1574 à 1589 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 des différents sérotypes de la sous-espèce *indica*) ;
- l'ensemble S38-1, comprenant les ou constitué des sondes de séquences d'acide nucléique SEQ ID NO : 32 à 66, 382, 416, 667, 2036 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Altona) ;
- l'ensemble S38-2, comprenant les ou constitué des sondes de séquences d'acide nucléique SEQ ID NO : 854, 933, 1095, 1306, 1354 à 1389, 1696 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Altona) ;
- l'ensemble S39-1, comprenant les ou constitué des sondes de séquences d'acide nucléique SEQ ID NO : 240, 241, 1120, 1121 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Choleraesuis variété Decatur) ;
- l'ensemble S39-2, comprenant les ou constitué des sondes de séquences d'acide nucléique SEQ ID NO : 225, 247 à 251, 253 à 256, 259 à 268, 270 à 279, 281 à 289, 292 à 294 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Choleraesuis variété Decatur) ;
- l'ensemble S40-1, comprenant les ou constitué des sondes de séquences d'acide nucléique SEQ ID NO : 11, 232, 238, 239, 240, 242 à 245, 233 à 237, 959, 1106, 1120 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Choleraesuis variété Kunzendorf) ;
- l'ensemble S40-2, comprenant les ou constitué des sondes de séquences d'acide nucléique SEQ ID NO : 246, 252, 258, 269, 280, 290, 291, 392, 563, 933 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Choleraesuis variété Kunzendorf) ;
- l'ensemble S41-1, comprenant les ou constitué des sondes de séquences d'acide nucléique SEQ ID NO : 11, 242 à 245, 232 à 239, 959, 1000, 1106, 1139, 1120 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 des sérotypes Choleraesuis et Choleraesuis sensu stricto) ;
- l'ensemble S41-2, comprenant les ou constitué des sondes de séquences d'acide nucléique SEQ ID NO : 246, 252, 258, 269, 280, 290, 291, 392, 563, 574, 933, 1003, 1004 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 des sérotypes Choleraesuis et Choleraesuis sensu stricto) ;
- l'ensemble S42-1, comprenant les ou constitué des sondes de séquences d'acide nucléique SEQ ID NO: 416 à 420, 1122 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Goettingen) ;
- l'ensemble S42-2, comprenant les ou constitué d'au moins une sonde comprenant au moins ou constituée d'au moins 8 nucléotides consécutifs de la séquence d'acide nucléique SEQ ID NO : 1548 et/ou sa séquence d'acide nucléique complémentaire (séquences nucléotidiques correspondant à la séquence variable du locus CRISPR2 du sérotype Goettingen) ;
- l'ensemble S43-1, comprenant les ou constitué des sondes de séquences d'acide nucléique SEQ ID NO : 630 à 636, 937, 948, 1120, 1143 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Manhattan) ;
- l'ensemble S43-2, comprenant les ou constitué des sondes de séquences d'acide nucléique SEQ ID NO : 392, 1653 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Manhattan) ;
- l'ensemble S44-1, comprenant les ou constitué des sondes de séquences d'acide nucléique SEQ ID NO : 96 à 155 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 de certains sérotypes de l'espèce *S. bongori*) ;
- l'ensemble S44-2, comprenant les ou constitué des sondes de séquences d'acide nucléique SEQ ID NO : 156 à 195, 1421 à 1430 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 de certains sérotypes de l'espèce *S. bongori*) ;
- l'ensemble S45-1, comprenant les ou constitué des sondes de séquences d'acide nucléique SEQ ID NO: 962, 1106, 1133, 1804, 1808 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Abony) ;
- l'ensemble S45-2, comprenant les ou constitué des sondes de séquences d'acide nucléique SEQ ID NO : 972, 989, 1334 à 1339, 1474, 1499 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Abony) ;
- l'ensemble S46-1, comprenant les ou constitué des sondes de séquences d'acide nucléique SEQ ID NO : 448, 860 à 875, 882, 883, 1340 à 1345, 1721 à 1723, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Aesch) ;
- l'ensemble S46-2, comprenant les ou constitué des sondes de séquences d'acide nucléique SEQ ID NO : 563, 625, 626, 885, 887 à 893, 896, 933, 1346 à 1348, 1749, 1759, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Aesch) ;
- l'ensemble S47-1, comprenant les ou constitué des sondes de séquences d'acide nucléique SEQ ID NO : 834, 1120, 1349, 1350, 1351, 1713, 1718 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Albany) ;
- l'ensemble S47-2, comprenant les ou constitué des sondes de séquences d'acide nucléique SEQ ID NO : 842, 852, 1352, 1353 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Albany) ;
- l'ensemble S48-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Arechavalata) ;
- l'ensemble S48-2, comprenant les ou constitué des sondes de séquences d'acide nucléique SEQ ID NO: 225, 227 à 231, 1398 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Arechavalata) ;
- l'ensemble S49-1, comprenant les ou constitué des sondes de séquences d'acide nucléique SEQ ID NO : 448, 449, 860, 871 à 873, 878, 895, 903 à 905, 907, 1719 à 1721, 1745 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Bardo) ;
- l'ensemble S49-2, comprenant les ou constitué des sondes de séquences d'acide nucléique SEQ ID NO : 563, 622, 626, 885, 886 à 890, 893, 896, 907, 910 à 915, 933, 1756 à 1758 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Bardo) ;
- l'ensemble S50-1, comprenant les ou constitué des sondes de séquences d'acide nucléique SEQ ID NO : 82, 1401 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Berta) ;
- l'ensemble S50-2, comprenant les ou constitué des sondes de séquences d'acide nucléique SEQ ID NO : 246, 918, 933, 1401, 1448 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Berta) ;
- l'ensemble S51-1, comprenant les ou constitué des sondes de séquences d'acide nucléique SEQ ID NO : 1, 37, 1106, 1402 à 1416, 2055 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Bispebjerg) ;
- l'ensemble S51-2, comprenant les ou constitué des sondes de séquences d'acide nucléique SEQ ID NO : 2 à 4, 9, 1417 à 1420, 1547, 1572 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Bispebjerg) ;
- l'ensemble S52-1, comprenant les ou constitué des sondes de séquences d'acide nucléique SEQ ID NO : 379, 380, 383, 385, 387, 389 à 391 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Blegdam) ;
- l'ensemble S52-2, comprenant les ou constitué des sondes de séquences d'acide nucléique SEQ ID NO : 392 à 394, 404, 407, 412, 413, 519 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Blegdam) ;
- l'ensemble S53-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Chester) ;
- l'ensemble S53-2, comprenant les ou constitué des sondes de séquences d'acide nucléique SEQ ID NO: 933, 1445 à 1447 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Chester) ;
- l'ensemble S54-1, comprenant les ou constitué des sondes de séquences d'acide nucléique SEQ ID NO : 1449 à 1454, 1460, 1467 à 1473 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Concord) ;
- l'ensemble S54-2, comprenant les ou constitué des sondes de séquences d'acide nucléique SEQ ID NO : 972, 1474 à 1493, 1495 à 1499 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Concord) ;
- l'ensemble S55-1, comprenant les ou constitué des sondes de séquences d'acide nucléique SEQ ID NO : 1106, 1107 à 1109, 1123, 1128 à 1131, 1133, 1137 à 1140, 1550, 1559 à 1561 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Crossness) ;
- l'ensemble S55-2, comprenant les ou constitué des sondes de séquences d'acide nucléique SEQ ID NO: 455, 1144, 1145, 1149 à 1153, 1155 à 1160, 1163, 1172 à 1174 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Crossness) ;
- l'ensemble S56-1, comprenant les ou constitué des sondes de séquences d'acide nucléique SEQ ID NO : 834, 1120, 1349 à 1351, 1713, 1718 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Duesseldorf) ;
- l'ensemble S56-2, comprenant les ou constitué des sondes de séquences d'acide nucléique SEQ ID NO : 842, 852, 1352, 1353, 1501 à 1503 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Duesseldorf) ;
- l'ensemble S57-1, comprenant les ou constitué des sondes de séquences d'acide nucléique SEQ ID NO: 941, 1504 à 1508 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Emek) ;
- l'ensemble S57-2, comprenant les ou constitué des sondes de séquences d'acide nucléique SEQ ID NO : 1260, 1509 à 1524, 1526, 1527 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Emek) ;
- l'ensemble S58-1, comprenant les ou constitué d'au moins une sonde comprenant au moins ou constituée d'au moins 8 nucléotides consécutifs de la séquence d'acide nucléique SEQ ID NO : 558 et/ou sa séquence d'acide nucléique complémentaire (séquences nucléotidiques correspondant à la séquence variable du locus CRISPR1 du sérotype Fulica) ;
- l'ensemble S58-2, comprenant les ou constitué des sondes de séquences d'acide nucléique SEQ ID NO : 2, 4, 5, 1333, 1547 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Fulica) ;
- l'ensemble S59-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Gueuletapee) ;
- l'ensemble S59-2, comprenant les ou constitué des sondes de séquences d'acide nucléique SEQ ID NO : 597, 918 à 925, 933, 1549 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Gueuletapee) ;
- l'ensemble S60-1, comprenant les ou constitué des sondes de séquences d'acide nucléique SEQ ID NO : 1106 à 1109, 1111 à 1116, 1118, 1123, 1128 à 1131, 1133, 1137 à 1140, 1150, 1151, 1555 à 1562 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Heidelberg) ;
- l'ensemble S60-2, comprenant les ou constitué des sondes de séquences d'acide nucléique SEQ ID NO : 455, 1144, 1145, 1149 à 1153, 1155 à 1163, 1172 à 1174, 1563 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Heidelberg) ;
- l'ensemble S61-1, comprenant les ou constitué des sondes de séquences d'acide nucléique SEQ ID NO: 558, 1565 à 1571 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Hessarek) ;
- l'ensemble S61-2, comprenant les ou constitué des sondes de séquences d'acide nucléique SEQ ID NO : 2 à 4, 1547, 1572, 1573 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Hessarek) ;
- l'ensemble S62-1, comprenant les ou constitué des sondes de séquences d'acide nucléique SEQ ID NO: 1591, 1592, 1667 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Itami) ;
- l'ensemble S62-2, comprenant les ou constitué des sondes de séquences d'acide nucléique SEQ ID NO : 918, 1593 à 1600, 1744 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Itami) ;
- l'ensemble S63-1, comprenant les ou constitué des sondes de séquences d'acide nucléique SEQ ID NO: 1601 à 1606, 1687 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Javiana) ;
- l'ensemble S63-2, comprenant les ou constitué des sondes de séquences d'acide nucléique SEQ ID NO : 589 à 601, 973, 1607, 1608 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Javiana) ;
- l'ensemble S64-1, comprenant les ou constitué des sondes de séquences d'acide nucléique SEQ ID NO : 1609 à 1621, 1494, 1525 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Johannesburg) ;
- l'ensemble S64-2, comprenant les ou constitué des sondes de séquences d'acide nucléique SEQ ID NO: 918, 1697, 1698 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Johannesburg) ;
- l'ensemble S65-1, comprenant les ou constitué des sondes de séquences d'acide nucléique SEQ ID NO : 444, 448, 1184, 1622 à 1637, ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Kentucky) ;
- l'ensemble S65-2, comprenant les ou constitué des sondes de séquences d'acide nucléique SEQ ID NO : 385, 519, 1638 à 1652, 1746 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Kentucky) ;
- l'ensemble S66-1, comprenant les ou constitué d'au moins une sonde comprenant au moins ou constituée d'au moins 8 nucléotides consécutifs de la séquence d'acide nucléique SEQ ID NO : 67 et/ou sa séquence d'acide nucléique complémentaire (séquences nucléotidiques correspondant à la séquence variable du locus CRISPR1 du sérotype Kundunchi) ;
- l'ensemble S66-2, comprenant les ou constitué des sondes de séquences d'acide nucléique SEQ ID NO : 972, 983, 989, 1201, 1204, 1205, 1210 à 1212 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Kundunchi) ;
- l'ensemble S67-1, comprenant les ou constitué des sondes de séquences d'acide nucléique SEQ ID NO : 448, 449, 860, 871, 878 à 881, 1721, 1724 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Lindenburg) ;
- l'ensemble S67-2, comprenant les ou constitué des sondes de séquences d'acide nucléique SEQ ID NO : 563, 622, 885, 886, 896, 907, 910 à 915, 933, 1748, 1757 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Lindenburg) ;
- l'ensemble S68-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Maracaibo) ;
- l'ensemble S68-2, comprenant les ou constitué des sondes de séquences d'acide nucléique SEQ ID NO : 1240, 1247, 1248, 1654 à 1656 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Maracaibo) ;
- l'ensemble S69-1, comprenant les ou constitué des sondes de séquences d'acide nucléique SEQ ID NO : 627, 834, 1120, 1660 à 1669 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Mississippi) ;
- l'ensemble S69-2, comprenant les ou constitué des sondes de séquences d'acide nucléique SEQ ID NO : 842, 1670 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Mississippi) ;
- l'ensemble S70-1, comprenant les ou constitué des sondes de séquences d'acide nucléique SEQ ID NO : 941, 942, 949, 1699 à 1712, 2109 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Muenchen) ;
- l'ensemble S70-2, comprenant les ou constitué des sondes de séquences d'acide nucléique SEQ ID NO : 392, 1653 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Muenchen) ;
- l'ensemble S71-1, comprenant les ou constitué des sondes de séquences d'acide nucléique SEQ ID NO : 11, 1120, 1497, 1760 à 1775, 1809 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Niarembe) ;
- l'ensemble S71-2, comprenant les ou constitué des sondes de séquences d'acide nucléique SEQ ID NO: 90, 1776 à 1785 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Niarembe) ;
- l'ensemble S72-1, comprenant les ou constitué des sondes de séquences d'acide nucléique SEQ ID NO: 666, 1786 à 1791 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Overvecht) ;
- l'ensemble S72-2, comprenant les ou constitué des sondes de séquences d'acide nucléique SEQ ID NO : 783, 1146, 1178, 1179, 1181, 1792 à 1799, 1801 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Overvecht) ;
- l'ensemble S73-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Poona) ;
- l'ensemble S73-2, comprenant les ou constitué des sondes de séquences d'acide nucléique SEQ ID NO : 82, 87, 918, 933, 1826 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Poona) ;
- l'ensemble S74-1, comprenant les ou constitué des sondes de séquences d'acide nucléique SEQ ID NO : 295 à 297, 307, 313, 317, 323, 1120, 1825, 1827 à 1837, 2023 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Postdam) ;
- l'ensemble S74-2, comprenant les ou constitué des sondes de séquences d'acide nucléique SEQ ID NO : 663, 1304, 1838 à 1847 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Postdam) ;
- l'ensemble S75-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Reading) ;
- l'ensemble S75-2, comprenant les ou constitué d'au moins une sonde comprenant au moins ou constituée d'au moins 8 nucléotides consécutifs de la séquence d'acide nucléique SEQ ID NO : 225 et/ou sa séquence d'acide nucléique complémentaire (séquences nucléotidiques correspondant à la séquence variable du locus CRISPR2 du sérotype Reading) ;
- l'ensemble S76-1, comprenant les ou constitué des sondes de séquences d'acide nucléique SEQ ID NO : 379, 380, 383 à 385, 387, 389, 390 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Rosenberg) ;
- l'ensemble S76-2, comprenant les ou constitué des sondes de séquences d'acide nucléique SEQ ID NO : 392, 393, 404, 407 à 413 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Rosenberg) ;
- l'ensemble S77-1, comprenant les ou constitué d'au moins une sonde comprenant au moins ou constituée d'au moins 8 nucléotides consécutifs de la séquence d'acide nucléique SEQ ID NO : 917 et/ou sa séquence d'acide nucléique complémentaire (séquences nucléotidiques correspondant à la séquence variable du locus CRISPR1 du sérotype Rubislaw) ;
- l'ensemble S77-2, comprenant les ou constitué d'au moins une sonde comprenant au moins ou constituée d'au moins 8 nucléotides consécutifs de la séquence d'acide nucléique SEQ ID NO : 225 et/ou sa séquence d'acide nucléique complémentaire ;
- l'ensemble S78-1, comprenant les ou constitué des sondes de séquences d'acide nucléique SEQ ID NO: 1525, 1609 à 1621 ou d'au moins 8 nucléotides consécutifs de ces séquences d'acide nucléique et/ou leurs séquences d'acide nucléique complémentaires ;
- l'ensemble S78-2, comprenant les ou constitué des sondes de séquences d'acide nucléique SEQ ID NO : 225 à 227, 229 à 231 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Sandiego) ;
- l'ensemble S79-1, comprenant les ou constitué des sondes de séquences d'acide nucléique SEQ ID NO : 1106, 1456, 1917 à 1924 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Stourbridge) ;
- l'ensemble S79-2, comprenant les ou constitué des sondes de séquences d'acide nucléique SEQ ID NO : 918, 1093, 1925 à 1936 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Stourbridge) ;
- l'ensemble S80-1, comprenant les ou constitué des sondes de séquences d'acide nucléique SEQ ID NO : 1961 à 1963 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Tallahassee) ;
- l'ensemble S80-2, comprenant les ou constitué des sondes de séquences d'acide nucléique SEQ ID NO : 25, 603, 2127, 2133, 1964 à 1966 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Tallahassee) ;
- l'ensemble S81-1, comprenant les ou constitué des sondes de séquences d'acide nucléique SEQ ID NO : 67, 929, 1967 à 2005 ou d'au moins 8 nucléotides consécutifs de ces séquences d'acide nucléique et/ou leurs séquences d'acide nucléique complémentaires ;
- l'ensemble S81-2, comprenant les ou constitué des sondes de séquences d'acide nucléique SEQ ID NO : 24, 769, 1550, 1659, 2006 à 2022 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Tennessee) ;
- l'ensemble S82-1, comprenant les ou constitué d'au moins une sonde comprenant au moins ou constituée d'au moins 8 nucléotides consécutifs de la séquence d'acide nucléique SEQ ID NO : 916 et/ou sa séquence d'acide nucléique complémentaire (séquences nucléotidiques correspondant à la séquence variable du locus CRISPR1 du sérotype Urbana) ;
- l'ensemble S82-2, comprenant les ou constitué des sondes de séquences d'acide nucléique SEQ ID NO: 1697, 1698, 1800 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Urbana) ;
- l'ensemble S83-1, comprenant les ou constitué des sondes de séquences d'acide nucléique SEQ ID NO : 948, 1120, 1123, 1325, 2039 à 2078 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Weltevreden) ;
- l'ensemble S83-2, comprenant les ou constitué des sondes de séquences d'acide nucléique SEQ ID NO : 254 à 256, 2079 à 2108 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Weltevreden) ;
- l'ensemble S84-1, comprenant les ou constitué des sondes de séquences d'acide nucléique SEQ ID NO : 1312, 1690, 2141, 2142 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR1 du sérotype Zaiman) ;
- l'ensemble S84-2, comprenant les ou constitué des sondes de séquences d'acide nucléique SEQ ID NO : 1697, 1698, 1800, 1924, 2143, 2144 ou d'au moins 8 nucléotides consécutifs de ces séquences d'acide nucléique et/ou leurs séquences d'acide nucléique complémentaires (séquences nucléotidiques correspondant aux différentes séquences variables du locus CRISPR2 du sérotype Zaiman).

19. Ensemble d'amorces apte à amplifier un fragment de la séquence génomique d'une bactérie du genre *Salmonella,* **caractérisé en ce qu'**il est sélectionné dans le groupe constitué par :
- l'ensemble d'amorces constitué de l'amorce de séquence SEQ ID NO : 1326 associée à au moins une des amorces de séquence SEQ ID NOs : 1327, 1328, 1329 2151 et 2152,
- l'ensemble d'amorces constitué de l'amorce de séquence SEQ ID NO : 1330, associée à au moins une des amorces de séquence SEQ ID NOs : 1331 et 1332, et
- l'ensemble d'amorces constitué de l'amorce de séquence SEQ ID NO : 1326, associée à au moins une des deux amorces de séquence 1331 et 1332.

20. Puce à ADN, **caractérisée en ce qu'**elle comprend les sondes comprises dans au moins un des ensembles de sondes tels que définis à la revendication 18.

21. Kit ou coffret de détection ou d'identification du type et du sous-type moléculaire d'une bactérie du genre *Salmonella,* **caractérisé en ce qu'**il comprend au moins un ensemble de sondes tel que défini à la revendication 18 et un ensemble d'amorces selon la revendication 19 et optionnellement une puce à ADN selon la revendication 20.

22. Utilisation d'un ensemble de sondes tel que défini à la revendication 18, d'un ensemble d'amorces selon la revendication 19 ou d'une puce à ADN selon la revendication 20, pour détecter ou identifier le type et/ou le sous-type moléculaire d'une bactérie du genre *Salmonella.*

## Patentansprüche

1. In-vitro-Verfahren zur molekularen Typisierung und/oder Subtypisierung eines Bakteriums der Gattung *Salmonella* ausgehend von einer Probe, wobei das Verfahren **dadurch gekennzeichnet ist, dass** es wenigstens die folgenden Stufen umfasst:
(a) Amplifikation eines Nucleinsäurefragments eines Bakteriums der Gattung *Salmonella,* wobei das Fragment den Locus CRISPR1 und/oder den Locus CRISPR2 umfasst, mit Hilfe eines Primersatzes, wobei die Primer eine Größe von unter oder gleich 15 Nucleotiden aufweisen, wobei die Primersätze ausgewählt sind aus der Gruppe, bestehend aus:
- einem Primersatz "A", der geeignet ist, ein Nucleinsäurefragment, das den Locus CRISPR1 umfasst, zu amplifizieren, umfassend wenigstens einen Sinn-Primer A1, der aus einer Oligonucleotidsequenz besteht, die wenigstens 70% Identität aufweist mit oder identisch ist mit einem Fragment der genomischen Sequenz eines Bakteriums der Gattung *Salmonella,* das sich in einer Region mit tausend bp in Position 5' des Locus CRISPR1 befindet, wobei das Fragment der genomischen Sequenz, dass sich in Position 5' des Locus CRISPR1 befindet, die selbe Größe wie der Primer A1 hat, und wenigstens einen AntiSinn-Primer A2, der aus einer Oligonucleotidsequenz besteht, die 70% Identität bevorzugt 80%, 85%, 95%, noch bevorzugter 99% identität aufweist mit oder identisch ist mit einem Fragment der genomischen Sequenz, komplementär zu der genomischen Sequenz eines Bakteriums der Gattung *Salmonella,* das sich in Position 3' des Locus CRISPR1 und in Position 5' des Locus CRISPR2 befindet, aufweist oder zu diesem identisch ist, wobei das Fragment der komplementären genomischen Sequenz die selbe Größe wie der Primer A2 hat;
- einem Primersatz "B", der geeignet ist, ein Nucleinsäurefragment, das den Locus CRISPR2 umfasst, zu amplifizieren, umfassend wenigstens einen Sinn-Primer B1, der aus einer Oligonucleotidsequenz besteht, die wenigstens 70% Identität aufweist mit oder identisch ist mit einem Fragment der genomischen Sequenz eines Bakteriums der Gattung *Salmonella,* das sich in Position 3' des Locus CRISPR1 und in Position 5' des Locus CRISPR2 befindet, wobei das Fragment der genomischen Sequenz, das sich in Position 3' des Locus CRISPR1 und in Position 5' des Locus CRISPR2 befindet, die selbe Größe wie der Primer B1 hat, und wenigstens einen AntiSinn-Primer B2, der aus einer Oligonucleotidsequenz besteht, die wenigstens 70% Identität aufweist mit oder identisch ist mit einem Fragment der komplementären genomischen Sequenz der genomischen Sequenz eines Bakteriums der Gattung *Salmonella,* das sich in einer Region mit 1000 bp in Position 3' des Locus CRISPR2 befindet, wobei das Fragment der komplementären genomischen Sequenz die selbe Größe wie der Primer B2 hat.
- einem Primersatz "C", der geeignet ist, ein Nucleinsäurefragment, das den Locus CRISPR1 und den Locus CRISPR2 umfasst, zu amplifizieren, umfassend wenigstens einen Sinn-Primer A1, wie er oben definiert ist, und wenigstens einen AntiSinn-Primer B2, wie er oben definiert ist;
(b) Bestimmung der Zahl und der Nucleotidsequenz der variablen Sequenzen des Locus CRISPR1 und/oder des Locus CRISPR2, der/die in Stufe (a) amplifiziert wurde(n), und
(c) Vergleich der Zusammensetzung bezüglich variabler Sequenzen der Loci CRISPR1 und/oder CRISPR2 mit einer Referenzbasis, die die Zusammensetzung bezüglich variabler Sequenzen der Loci CRISPR1 und CRISPR2 von Typen und Subtypen von Bakterien der Gattung *Salmonella,* gezeigt in Figuren 10a, 10b und 9, bereitstellt, oder einem Teil dieser Basis.

2. Verfahren gemäß Anspruch 1, dadurch gekennzeichn e t , dass die Identität der Zusammensetzung bezüglich variabler Sequenzen, die in Stufe (b) bestimmt wurde mit einer Zusammensetzung, die in der Referenzbasis, definiert in Anspruch 1, oder einem Teil dieser Basis angegeben ist, für den Typ und/oder den Subtyp des Bakteriums der Gattung *Salmonel*la, das in der Probe vorliegt, indikativ ist.

3. Verfahren gemäß Anspruch 1 oder Anspruch 2, **dadurch gekennzeichnet, dass** die Primer eine Größe von zwischen 15 und 30 Nucleotiden aufweisen.

4. Verfahren gemäß einem der Ansprüche 1 bis 3, **dadurch gekennzeichnet, dass** das Nucleinsäurefragment, das den Lokus CRISPR1 und/oder den Locus CRISPR2 umfasst, eine Größe von zwischen 400 und 20000 bp aufweist.

5. Verfahren gemäß einem der Ansprüche 1 bis 4, **dadurch gekennzeichnet, dass** das Fragment der genomischen Sequenz eines Bakteriums der Gattung *Salmonella,* das sich in Position 5' des Locus CRISPR1 befindet, und/oder das Fragment der komplementären genomischen Sequenz der genomischen Sequenz eines Bakteriums der Gattung *Salmonella,* das sich in Position 3' des Locus CRISPR2 befindet, in einem Abstand vom Locus CRISPR1 oder CRISPR2 von weniger als 500 bp, vorzugsweise weniger als 100 bp, angeordnet sind/ist.

6. Verfahren gemäß einem der Ansprüche 1 bis 5, **dadurch gekennzeichnet, dass** die genomische Sequenz eines Bakteriums der Gattung *Salmonella* ausgewählt ist aus der genomischen Sequenz von *S. enterica,* Serotyp Typhimurium LT2 (genomische Sequenz verfügbar unter den Nummern GI: 16421485 und GI: 16421501 in der Datenbank GenBank) oder *S. enterica,* Serotyp Typhi CT18 (genomische Sequenz verfügbar unter der Nummer GI: 16503805 in der Datenbank GenBank).

7. Verfahren gemäß Anspruch 5, dadurch gekennzeichn e t , dass der Primersatz ausgewählt ist aus der Gruppe, bestehend aus:
- dem Primersatz, der geeignet ist, den Locus CRISPR1 zu amplifizieren, bestehend aus dem Primer der Sequenz SEQ ID NO: 1326, assoziiert mit wenigstens einem der Primer der Sequenz SEQ ID NOs: 1327, 1328, 1329, 2151 und 2152,
- dem Primersatz, der geeignet ist, den Locus CRISPR2 zu amplifizieren, bestehend aus dem Primer der Sequenz SEQ ID NO: 1330, assoziiert mit wenigstens einem der Primer der Sequenz SEQ ID NOs: 1331 und 1332, und
- dem Primersatz, der geeignet ist, die zwei Loci CRISPR1 und CRISPR2 gleichzeitig zu amplifizieren, bestehend aus dem Primer der Sequenz SEQ ID NO: 1326, assoziiert mit wenigstens einem der zwei Primer der Sequenz 1331 und 1332.

8. Verfahren gemäß einem der Ansprüche 1 bis 7, **dadurch gekennzeichnet, dass** die Stufe (a) der Amplifikation durch ein Verfahren ausgeführt wird, das ausgewählt wird aus der Gruppe, bestehend aus: Polymerasekettenreaktion (PCR), Ligasekettenreaktion (LCR), "Nucleinsäuresequenz-basierter Amplifikation" (NASBA), "Cycling probe technology" (CPT), ineinander geschachtelter PCR und Multiplex-PCR.

9. Verfahren gemäß einem der Ansprüche 1 bis 8, **dadurch gekennzeichnet, dass** die Stufe (b) mittels eines DNA-Sequenzierungsverfahrens durchgeführt wird.

10. Verfahren gemäß einem der Ansprüche 1 bis 8, **dadurch gekennzeichnet, dass** die Stufe (b) durchgeführt wird durch:
(i) Hybridisierung mit einem, mehreren oder allen Sondensätzen, ausgewählt aus:
- dem Satz S1-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO: 1106 bis 1119, 1123 bis 1140, 1142, 1143, 1914 und/oder ihrer komplementären Nucleinsäuresequenzen (Nucleotidsequenzen, die den verschiedenen variablen Sequenzen des Locus CRISPR1 des Serotyps Typhimorium entsprechen);
- dem Satz S1-2, umfassend oder bestehend aus wenigstens eine(r) Sonde, umfassend wenigstens oder bestehend aus wenigstens 8 aufeinanderfolgende(r) Nucleotide(n) einer der Nucleinsäuresequenzen SEQ ID NO: 455, 1144 bis 1147, 1149 bis 1153, 1155 bis 1181 und/oder ihrer komplementären Nucleinsäuresequenzen (Nucleotidsequenzen, die den verschiedenen variablen Sequenzen des Locus CRISPR2 des Serotyps Typhimorium entsprechen) ;
- dem Satz S2-1, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 256, 378 bis 381, 383 bis 387, 389 bis 391, 1106, 1514, 1528, 2148 bis 2153 und/oder ihrer komplementären Nucleinsäuresequenzen (Nucleotidsequenzen, die den verschiedenen variablen Sequenzen des Locus CRISPR1 des Serotyps Enteritidis entsprechen);
- dem Satz S2-2, umfassend oder bestehend aus wenigstens eine(r) Sonde, umfassend oder bestehend aus wenigstens 8 aufeinanderfolgende(n) Nucleotide(n) einer der folgenden Nucleinsäuresequenzen SEQ ID NO: 392 bis 413, 519, 815, 972, 983, 989, 1529 bis 1546, 1952 und/oder ihrer komplementären Nucleinsäuresequenzen (Nucleotidsequenzen, die den verschiedenen variablen Sequenzen des Locus CRISPR2 des Serotyps Enteritidis entsprechen);
- dem Satz S3-1, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 421 bis 442, 444, 445, 447 bis 450 und/oder ihren komplementären Nucleinsäuresequenzen (Nucleotidsequenzen, die den verschiedenen variablen Sequenzen des Locus CRISPR1 des Serotyps Hadar entsprechen);
- dem Satz S3-2, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 451 bis 465, 1144 bis 1147, 1150 bis 1152, 1154, 1155, 1158, 1169, 1172, 1175 bis 1177, 1179, 1181 und/oder ihrer komplementären Nucleinsäuresequenzen (Nucleotidsequenzen, die den verschiedenen variablen Sequenzen des Locus CRISPR2 des Serotyps Hadar entsprechen);
- dem Satz S4-1, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 65, 382, 1120, 1263 bis 1302, 2024 bis 2038 und/oder ihren komplementären Nucleinsäuresequenzen (Nucleotidsequenzen, die den verschiedenen variablen Sequenzen des Locus CRISPR1 des Serotyps Virchow entsprechen);
- dem Satz S4-2, umfassend oder bestehend aus wenigstens eine(r) Sonde umfassend oder bestehend aus wenigstens 8 aufeinanderfolgende(n) Nucleotide(n) einer der folgenden Nucleinsäuresequenzen SEQ ID NO: 83, 93, 94, 1304 bis 1324 und/oder ihren komplementären Nucleinsäuresequenzen (Nucleotidsequenzen, die verschiedenen variablen Sequenzen des Locus CRISPR2 des Serotyps Virchow entsprechen);
- dem Satz S5-1, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 531 bis 562, 1106 und/oder ihrer komplementären Nucleinsäuresequenzen (Nucleotidsequenzen, die den verschiedenen variablen Sequenzen des Locus CRISPR1 des Serotyps Infantis entsprechen);
- dem Satz S5-2, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 563 bis 588 und/oder ihrer komplementären Nucleinsäuresequenzen (Nucleotidsequenzen, die den verschiedenen variablen Sequenzen des Locus CRISPR2 des Serotyps Infantis entsprechen);
- dem Satz S6-1 umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO: 1254 bis 1259 und/oder ihrer komplementären Nucleinsäuresequenzen (Nucleotidsequenzen, die den verschiedenen variablen Sequenzen des Locus CRISPR1 des Serotyps Typhi entsprechen);
- dem Satz S6-2, umfassend oder bestehend aus wenigstens einer Sonde, umfassend oder bestehend aus wenigstens 8 aufeinanderfolgende(n) Nucleotide(n) der Nucleinsäuresequenz SEQ ID NO: 1260 und/oder ihrer komplementären Nucleinsäuresequenz (Nucleotidsequenzen, die der variablen Sequenz des Locus CRISPR2 des Serotyps Typhi entsprechen);
- dem Satz S7-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 448, 449, 860 bis 876, 878 bis 884, 1719 bis 1724, 1727 bis 1743, 1745 und/oder ihrer komplementären Nucleinsäuresequenzen (Nucleotidsequenzen, die den verschiedenen variablen Sequenzen des Locus CRISPR1 des Serotyps Newport entsprechen) ;
- dem Satz S7-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 453 bis 456, 563, 621, 622, 625, 626, 657, 885 bis 896, 906 bis 915, 933, 1144, 1145, 1155, 1156, 1172, 1174, 1748 bis 1756, 1758, 1759, 1916 und/oder ihrer komplementären Nucleinsäuresequenzen (Nucleotidsequenzen, die den verschiedenen variablen Sequenzen des Locus CRISPR2 des Serotyps Newport entsprechen) ;
- dem Satz S8-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 295 bis 299, 301 bis 323, 738, 1106 bis 1109, 1120, 1123, 1128 bis 1131, 1133, 1137 bis 1140, 1550, 1159 bis 1561 und/oder ihrer komplementären Nucleinsäuresequenzen (Nucleotidsequenzen, die den verschiedenen variablen Sequenzen des Locus CRISPR1 des Serotyps Derby entsprechen) ;
- dem Satz S8-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 324 bis 337, 455, 1144, 1145, 1149 bis 1153, 1155 bis 1160, 1163, 1172 bis 1174 und/oder ihrer komplementären Nucleinsäuresequenzen (Nucleotidsequenzen, die den verschiedenen variablen Sequenzen des Locus CRISPR2 des Serotyps Derby entsprechen) ;
- dem Satz S9-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 295, 307, 602 bis 614, 616 bis 619, 1106, 1120, und/oder ihrer komplementären Nucleinsäuresequenzen (Nucleotidsequenzen, die den verschiedenen variablen Sequenzen des Locus CRISPR1 des Serotyps Kottbus entsprechen) ;
- dem Satz S9-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 563, 620 bis 629, 885, 896, 907, 913, 914, 933, und/oder ihrer komplementären Nucleinsäuresequenzen (Nucleotidsequenzen, die den verschiedenen variablen Sequenzen des Locus CRISPR2 des Serotyps Kottbus entsprechen) ;
- dem Satz S10-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 422 bis 425, 448 bis 450, 504 bis 518 und/oder ihrer komplementären Nucleinsäuresequenzen (Nucleotidsequenzen, die den verschiedenen variablen Sequenzen des Locus CRISPR1 des Serotyps Indiana entsprechen) ;
- dem Satz S10-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 246, 258, 269, 392, 415, 519 bis 530, und/oder ihrer komplementären Nucleinsäuresequenzen (Nucleotidsequenzen, die den verschiedenen variablen Sequenzen des Locus CRISPR2 des Serotyps Indiana entsprechen) ;
- dem Satz S11-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 11 bis 23, 238, 948, 1120, 1123, 1133 und/oder ihrer komplementären Nucleinsäuresequenzen (Nucleotidsequenzen, die den verschiedenen variablen Sequenzen des Locus CRISPR1 des Serotyps Agona entsprechen) ;
- dem Satz S11-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 24 bis 31 und/oder ihrer komplementären Nucleinsäuresequenzen (Nucleotidsequenzen, die den verschiedenen variablen Sequenzen des Locus CRISPR2 des Serotyps Agona entsprechen) ;
- dem Satz S12-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 830 bis 841, 1120 und/oder ihrer komplementären Nucleinsäuresequenzen (Nucleotidsequenzen, die den verschiedenen variablen Sequenzen des Locus CRISPR1 des Serotyps Napoli entsprechen) ;
- dem Satz S12-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 842 bis 859 und/oder ihrer komplementären Nucleinsäuresequenzen (Nucleotidsequenzen, die den verschiedenen variablen Sequenzen des Locus CRISPR2 des Serotyps Napoli entsprechen) ;
- dem Satz S13-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 926 bis 932 und/oder ihrer komplementären Nucleinsäuresequenzen (Nucleotidsequenzen, die den verschiedenen variablen Sequenzen des Locus CRISPR1 des Serotyps Paratyphi A entsprechen) ;
- dem Satz S13-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 933, 935, 936 und/oder ihrer komplementären Nucleinsäuresequenzen (Nucleotidsequenzen, die den verschiedenen variablen Sequenzen des Locus CRISPR2 des Serotyps Paratyphi A entsprechen) ;
- dem Satz S14-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 707, 937 bis 971, 1106, 1128 bis 1130, 1133, 1138, 1140, 1802 bis 1808, 1915 und/oder ihrer komplementären Nucleinsäuresequenzen (Nucleotidsequenzen, die den verschiedenen variablen Sequenzen des Locus CRISPR1 der Serotypen Paratyphi B und Paratyphi B Java entsprechen) ;
- dem Satz S14-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 861, 972 bis 987, 989 bis 992 bis 996, 1172 1262, 1462, 1497, 1810 bis 1825 und/oder ihrer komplementären Nucleinsäuresequenzen (Nucleotidsequenzen, die den verschiedenen variablen Sequenzen des Locus CRISPR2 der Serotypen Paratyphi B und Paratyphi B Java entsprechen) ;
- dem Satz S15-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 232, 238, 239, 997 bis 1002, 1106 und/oder ihrer komplementären Nucleinsäuresequenzen (Nucleotidsequenzen, die den verschiedenen variablen Sequenzen des Locus CRISPR1 des Serotyps Paratyphi C entsprechen) ;
- dem Satz S15-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 280, 290, 291, 392, 393, 415, 1003 bis 1006, 1262 und/oder ihrer komplementären Nucleinsäuresequenzen (Nucleotidsequenzen, die den verschiedenen variablen Sequenzen des Locus CRISPR2 des Serotyps Paratyphi C entsprechen) ;
- dem Satz S16-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 211 bis 224, 421, 427, 428, 432, 443 bis 445, 447 bis 449, 504 und/oder ihrer komplementären Nucleinsäuresequenzen (Nucleotidsequenzen, die den verschiedenen variablen Sequenzen des Locus CRISPR1 des Serotyps Bovismorbificans entsprechen) ;
- dem Satz S16-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 196 bis 209, 994, 1144, 1145, 1172 und/oder ihrer komplementären Nucleinsäuresequenzen (Nucleotidsequenzen, die den verschiedenen variablen Sequenzen des Locus CRISPR2 des Serotyps Bovismorbificans entsprechen) ;
- dem Satz S17-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Panama entsprechen) ;
- dem Satz S17-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 918 bis 925, 934 und/oder ihrer komplementären Nucleinsäuresequenzen (Nucleotidsequenzen, die den verschiedenen variablen Sequenzen des Locus CRISPR2 des Serotyps Panama entsprechen) ;
- dem Satz S18-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 11, 16, 67, 74, 442, 444, 446 bis 448, 602, 937, 948, 1120, 1128 bis 1130, 1182 bis 1200, 1937 bis 1950, und/oder ihrer komplementären Nucleinsäuresequenzen (Nucleotidsequenzen, die den verschiedenen variablen Sequenzen des Locus CRISPR1 des Serotyps Saintpaul entsprechen) ;
- dem Satz S18-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 972, 983, 989, 1201 bis 1214, 1216 bis 1227, 1952 bis 1960 und/oder ihrer komplementären Nucleinsäuresequenzen (Nucleotidsequenzen, die den verschiedenen variablen Sequenzen des Locus CRISPR2 des Serotyps Saintpaul entsprechen) ;
- dem Satz S19-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 514, 1062 bis 1089, 1100, 1284, 1886 bis 1911 und/oder ihrer komplementären Nucleinsäuresequenzen (Nucleotidsequenzen, die den verschiedenen variablen Sequenzen des Locus CRISPR1 des Serotyps Senftenberg entsprechen) ;
- dem Satz S19-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 300, 1090 bis 1097, 1101 bis 1103, 1304, 1315, 1318 bis 1320 und/oder ihrer komplementären Nucleinsäuresequenzen (Nucleotidsequenzen, die den verschiedenen variablen Sequenzen des Locus CRISPR2 des Serotyps Senftenberg entsprechen) ;
- dem Satz S20-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 67, 74 bis 80 und/oder ihrer komplementären Nucleinsäuresequenzen (Nucleotidsequenzen, die den verschiedenen variablen Sequenzen des Locus CRISPR1 des Serotyps Anatum entsprechen) ;
- dem Satz S20-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 83 bis 94, 1304, 1315, 1318 bis 1322, 1324, 1390 bis 1397 und/oder ihrer komplementären Nucleinsäuresequenzen (Nucleotidsequenzen, die den verschiedenen variablen Sequenzen des Locus CRISPR2 des Serotyps Anatum entsprechen) ;
- dem Satz S21-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 379, 383 bis 385, 388 und/oder ihrer komplementären Nucleinsäuresequenzen (Nucleotidsequenzen, die den verschiedenen variablen Sequenzen des Locus CRISPR1 der Serotypen Gallinarum entsprechen) ;
- dem Satz S21-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 393, 404, 407 bis 415 und/oder ihrer komplementären Nucleinsäuresequenzen (Nucleotidsequenzen, die den verschiedenen variablen Sequenzen des Locus CRISPR2 der Serotypen Gallinarum entsprechen) ;
- dem Satz S22-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Brandenburg entsprechen) ;
- dem Satz S22-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 225 bis 231 und/oder ihrer komplementären Nucleinsäuresequenzen (Nucleotidsequenzen, die den verschiedenen variablen Sequenzen des Locus CRISPR2 des Serotyps Brandenburg entsprechen) ;
- dem Satz S23-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 378, 379 und/oder ihrer komplementären Nucleinsäuresequenzen (Nucleotidsequenzen, die den verschiedenen variablen Sequenzen des Locus CRISPR1 des Serotyps Dublin entsprechen) ;
- dem Satz S23-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 393, 411, 412 und/oder ihrer komplementären Nucleinsäuresequenzen (Nucleotidsequenzen, die den verschiedenen variablen Sequenzen des Locus CRISPR2 des Serotyps Dublin entsprechen) ;
- dem Satz S24-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1, 1106 und/oder ihrer komplementären Nucleinsäuresequenzen (Nucleotidsequenzen, die den verschiedenen variablen Sequenzen des Locus CRISPR1 des Serotyps Abortusequi entsprechen) ;
- dem Satz S24-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 2 bis 10, 130 und/oder ihrer komplementären Nucleinsäuresequenzen (Nucleotidsequenzen, die den verschiedenen variablen Sequenzen des Locus CRISPR2 des Serotyps Abortusequi entsprechen) ;
- dem Satz S25-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 378, 379 und/oder ihrer komplementären Nucleinsäuresequenzen (Nucleotidsequenzen, die den verschiedenen variablen Sequenzen des Locus CRISPR1 des Serotyps Kiel entsprechen) ;
- dem Satz S25-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 393, 411, 412 und/oder ihrer komplementären Nucleinsäuresequenzen (Nucleotidsequenzen, die den verschiedenen variablen Sequenzen des Locus CRISPR2 des Serotyps Kiel entsprechen) ;
- dem Satz S26-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 509, 510, 512, 513, 637 bis 660, 1086, 1107 und/oder ihrer komplementären Nucleinsäuresequenzen (Nucleotidsequenzen, die den verschiedenen variablen Sequenzen des Locus CRISPR1 des Serotyps Mbandaka entsprechen) ;
- dem Satz S26-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 661 bis 666, 668, 669, 672, 683, 690 bis 695, 731, 918 und/oder ihrer komplementären Nucleinsäuresequenzen (Nucleotidsequenzen, die den verschiedenen variablen Sequenzen des Locus CRISPR2 des Serotyps Mbandaka entsprechen) ;
- dem Satz S27-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Miami entsprechen) ;
- dem Satz S27-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 25, 696 bis 700 und/oder ihrer komplementären Nucleinsäuresequenzen (Nucleotidsequenzen, die den verschiedenen variablen Sequenzen des Locus CRISPR2 des Serotyps Miami entsprechen) ;
- dem Satz S28-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 55, 701 bis 730, 732 bis 768, 1115, 1228, 1232 bis 1237, 1601, 1671 bis 1680, 1682 bis 1694, 1747 und/oder ihrer komplementären Nucleinsäuresequenzen (Nucleotidsequenzen, die den verschiedenen variablen Sequenzen des Locus CRISPR1 des Serotyps Montevideo entsprechen) ;
- dem Satz S28-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 589, 769 bis 829, 933, 1245, 1697, 1698 und/oder ihrer komplementären Nucleinsäuresequenzen (Nucleotidsequenzen, die den verschiedenen variablen Sequenzen des Locus CRISPR2 des Serotyps Montevideo entsprechen) ;
- dem Satz S29-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 379, 380, 383 bis 385, 387, 389 bis 391 und/oder ihrer komplementären Nucleinsäuresequenzen (Nucleotidsequenzen, die den verschiedenen variablen Sequenzen des Locus CRISPR1 des Serotyps Nitra entsprechen) ;
- dem Satz S29-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392 bis 394, 404, 407 bis 413, 519 und/oder ihrer komplementären Nucleinsäuresequenzen (Nucleotidsequenzen, die den verschiedenen variablen Sequenzen des Locus CRISPR2 des Serotyps Nitra entsprechen) ;
- dem Satz S30-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 931, 932, 1104, 1105 und/oder ihrer komplementären Nucleinsäuresequenzen (Nucleotidsequenzen, die den verschiedenen variablen Sequenzen des Locus CRISPR1 des Serotyps Sendai entsprechen) ;
- dem Satz S30-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 936 und/oder ihrer komplementären Nucleinsäuresequenz (Nucleotidsequenzen, die der variablen Sequenz des Locus CRISPR2 des Serotyps Sendai entsprechen) ;
- dem Satz S31-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 714 bis 716, 1228 bis 1239 und/oder ihrer komplementären Nucleinsäuresequenzen (Nucleotidsequenzen, die den verschiedenen variablen Sequenzen des Locus CRISPR1 des Serotyps Schwarzengrund entsprechen) ;
- dem Satz S31-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 196, 992, 995, 1172, 1240 bis 1244, 1246 bis 1253 und/oder ihrer komplementären Nucleinsäuresequenzen (Nucleotidsequenzen, die den verschiedenen variablen Sequenzen des Locus CRISPR2 des Serotyps Schwarzengrund entsprechen) ;
- dem Satz S32-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 232, 238, 239, 998, 999, 1002, 1106, 1261 und/oder ihrer komplementären Nucleinsäuresequenzen (Nucleotidsequenzen, die den verschiedenen variablen Sequenzen des Locus CRISPR1 des Serotyps Typhisuis entsprechen) ;
- dem Satz S32-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 1262 und/oder ihrer komplementären Nucleinsäuresequenz (Nucleotidsequenzen, die der variablen Sequenz des Locus CRISPR2 des Serotyps Typhisuis entsprechen) ;
- dem Satz S33-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 466 bis 486 und/oder ihrer komplementären Nucleinsäuresequenzen (Nucleotidsequenzen, die den verschiedenen variablen Sequenzen des Locus CRISPR1 der verschiedenen Serotypen der Unterart *houtenae* entsprechen) ;
- dem Satz S33-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 487 bis 490 und/oder ihrer komplementären Nucleinsäuresequenzen (Nucleotidsequenzen, die den verschiedenen variablen Sequenzen des Locus CRISPR2 der verschiedenen Serotypen der Unterart houtenae entsprechen) ;
- dem Satz S34-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 338 bis 376 und/oder ihrer komplementären Nucleinsäuresequenzen (Nucleotidsequenzen, die den verschiedenen variablen Sequenzen des Locus CRISPR1 der verschiedenen Serotypen der Unterart *diarizonae* entsprechen) ;
- dem Satz S34-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 377 und/oder ihrer komplementären Nucleinsäuresequenz (Nucleotidsequenzen, die der variablen Sequenz des Locus CRISPR2 der verschiedenen Serotypen der Unterart *diarizonae* entsprechen) ;
- dem Satz S35-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1007 bis 1037, 1848 bis 1884 und/oder ihrer komplementären Nucleinsäuresequenzen (Nucleotidsequenzen, die den verschiedenen variablen Sequenzen des Locus CRISPR1 der Unterart *salamae* entsprechen) ;
- dem Satz S35-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1038 bis 1043, 1045 bis 1061, 1885 und/oder ihrer komplementären Nucleinsäuresequenzen (Nucleotidsequenzen, die den verschiedenen variablen Sequenzen des Locus CRISPR2 der Unterart *salamae* entsprechen) ;
- dem Satz S36, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 95 und/oder ihrer komplementären Nucleinsäuresequenz (Nucleotidsequenzen, die der variablen Sequenz des Locus CRISPR1 der verschiedenen Serotypen der Unterart *arizonae* entsprechen) ;
- dem Satz S37-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 491 bis 499 und/oder ihrer komplementären Nucleinsäuresequenzen (Nucleotidsequenzen, die den variablen Sequenzen des Locus CRISPR1 der verschiedenen Serotypen der Unterart *indica* entsprechen) ;
- dem Satz S37-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 500 bis 503, 1574 bis 1589 und/oder ihrer komplementären Nucleinsäuresequenzen (Nucleotidsequenzen, die den variablen Sequenzen des Locus CRISPR2 der verschiedenen Serotypen der Unterart *indica* entsprechen) ;
- dem Satz S38-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 32 bis 66, 382, 416, 667, 2036 und/oder ihrer komplementären Nucleinsäuresequenzen (Nucleotidsequenzen, die den verschiedenen variablen Sequenzen des Locus CRISPR1 des Serotyps Altona entsprechen) ;
- dem Satz S38-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 854, 933, 1095, 1306, 1354 bis 1389, 1696 und/oder ihrer komplementären Nucleinsäuresequenzen (Nucleotidsequenzen, die den verschiedenen variablen Sequenzen des Locus CRISPR2 des Serotyps Altona entsprechen) ;
- dem Satz S39-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 240, 241, 1120, 1121 und/oder ihrer komplementären Nucleinsäuresequenzen (Nucleotidsequenzen, die den verschiedenen variablen Sequenzen des Locus CRISPR1 des Serotyps Choleraesuis var. Decatur entsprechen) ;
- dem Satz S39-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 225, 247 bis 251, 253 bis 256, 259 bis 268, 270 bis 279, 281 bis 289, 292 bis 294 und/oder ihrer komplementären Nucleinsäuresequenzen (Nucleotidsequenzen, die den verschiedenen variablen Sequenzen des Locus CRISPR2 des Serotyps Choleraesuis var. Decatur entsprechen) ;
- dem Satz S40-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 11, 232, 238, 239, 240, 242 bis 245, 233 bis 237, 959, 1106, 1120 und/oder ihrer komplementären Nucleinsäuresequenzen (Nucleotidsequenzen, die den verschiedenen variablen Sequenzen des Locus CRISPR1 des Serotyps Choleraesuis var. Kunzendorf entsprechen) ;
- dem Satz S40-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 246, 252, 258, 269, 280, 290, 291, 392, 563, 933 und/oder ihrer komplementären Nucleinsäuresequenzen (Nucleotidsequenzen, die den verschiedenen variablen Sequenzen des Locus CRISPR2 des Serotyps Choleraesuis var. Kunzendorf entsprechen) ;
- dem Satz S41-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 11, 242 bis 245, 232 bis 239, 959, 1000, 1106, 1139, 1120 und/oder ihrer komplementären Nucleinsäuresequenzen (Nucleotidsequenzen, die den verschiedenen variablen Sequenzen des Locus CRISPR1 der Serotypen Choleraesuis und Choleraesuis sensu stricto entsprechen) ;
- dem Satz S41-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 246, 252, 258, 269, 280, 290, 291, 392, 563, 574, 933, 1003, 1004 und/oder ihrer komplementären Nucleinsäuresequenzen (Nucleotidsequenzen, die den verschiedenen variablen Sequenzen des Locus CRISPR2 der Serotypen Choleraesuis und Choleraesuis sensu stricto entsprechen) ;
- dem Satz S42-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 416 bis 420, 1122 und/oder ihrer komplementären Nucleinsäuresequenzen (Nucleotidsequenzen, die den verschiedenen variablen Sequenzen des Locus CRISPR1 des Serotyps Goettingen entsprechen) ;
- dem Satz S42-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 1548 und/oder ihrer komplementären Nucleinsäuresequenz (Nucleotidsequenzen, die der variablen Sequenz des Locus CRISPR2 des Serotyps Goettingen entsprechen) ;
- dem Satz S43-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 630 bis 636, 937, 948, 1120, 1143 und/oder ihrer komplementären Nucleinsäuresequenzen (Nucleotidsequenzen, die den verschiedenen variablen Sequenzen des Locus CRISPR1 des Serotyps Manhattan entsprechen) ;
- dem Satz S43-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 1653 und/oder ihrer komplementären Nucleinsäuresequenzen (Nucleotidsequenzen, die den verschiedenen variablen Sequenzen des Locus CRISPR2 des Serotyps Manhattan entsprechen) ;
- dem Satz S44-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 96 bis 155 und/oder ihrer komplementären Nucleinsäuresequenzen (Nucleotidsequenzen, die den verschiedenen variablen Sequenzen des Locus CRISPR1 der bestimmter Serotypen der Art *S. bongori* entsprechen) ;
- dem Satz S44-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 156 bis 195, 1421 bis 1430 und/oder ihrer komplementären Nucleinsäuresequenzen (Nucleotidsequenzen, die den verschiedenen variablen Sequenzen des Locus CRISPR2 der bestimmter Serotypen der Art *S. bongori* entsprechen) ;
- dem Satz S45-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 962, 1106, 1133, 1804, 1808 und/oder ihrer komplementären Nucleinsäuresequenzen (Nucleotidsequenzen, die den verschiedenen variablen Sequenzen des Locus CRISPR1 des Serotyps Abony entsprechen) ;
- dem Satz S45-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 972, 989, 1334 bis 1339, 1474, 1499 und/oder ihrer komplementären Nucleinsäuresequenzen (Nucleotidsequenzen, die den verschiedenen variablen Sequenzen des Locus CRISPR2 des Serotyps Abony entsprechen) ;
- dem Satz S46-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 448, 860 bis 875, 882, 883, 1340 bis 1345, 1721 bis 1723, und/oder ihrer komplementären Nucleinsäuresequenzen (Nucleotidsequenzen, die den verschiedenen variablen Sequenzen des Locus CRISPR1 des Serotyps Aesch entsprechen) ;
- dem Satz S46-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 563, 625, 626, 885, 887 bis 893, 896, 933, 1346 bis 1348, 1749, 1759, und/oder ihrer komplementären Nucleinsäuresequenzen (Nucleotidsequenzen, die den verschiedenen variablen Sequenzen des Locus CRISPR2 des Serotyps Aesch entsprechen) ;
- dem Satz S47-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 834, 1120, 1349, 1350, 1351, 1713, 1718 und/oder ihrer komplementären Nucleinsäuresequenzen (Nucleotidsequenzen, die den verschiedenen variablen Sequenzen des Locus CRISPR1 des Serotyps Albany entsprechen) ;
- dem Satz S47-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 842, 852, 1352, 1353 und/oder ihrer komplementären Nucleinsäuresequenzen (Nucleotidsequenzen, die den verschiedenen variablen Sequenzen des Locus CRISPR2 des Serotyps Albany entsprechen) ;
- dem Satz S48-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Arechavalata entsprechen) ;
- dem Satz S48-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 225, 227 bis 231, 1398 und/oder ihrer komplementären Nucleinsäuresequenzen (Nucleotidsequenzen, die den verschiedenen variablen Sequenzen des Locus CRISPR2 des Serotyps Arechavalata entsprechen) ;
- dem Satz S49-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 448, 449, 860, 871 bis 873, 878, 895, 903 bis 905, 907, 1719 bis 1721, 1745 und/oder ihrer komplementären Nucleinsäuresequenzen (Nucleotidsequenzen, die den verschiedenen variablen Sequenzen des Locus CRISPR1 des Serotyps Bardo entsprechen) ;
- dem Satz S49-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 563, 622, 626, 885, 886 bis 890, 893, 896, 907, 910 bis 915, 933, 1756 bis 1758 und/oder ihrer komplementären Nucleinsäuresequenzen (Nucleotidsequenzen, die den verschiedenen variablen Sequenzen des Locus CRISPR2 des Serotyps Bardo entsprechen) ;
- dem Satz S50-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 82, 1401 und/oder ihrer komplementären Nucleinsäuresequenzen (Nucleotidsequenzen, die den verschiedenen variablen Sequenzen des Locus CRISPR1 des Serotyps Berta entsprechen) ;
- dem Satz S50-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 246, 918, 933, 1401, 1448 und/oder ihrer komplementären Nucleinsäuresequenzen (Nucleotidsequenzen, die den verschiedenen variablen Sequenzen des Locus CRISPR2 des Serotyps Berta entsprechen) ;
- dem Satz S51-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1, 37, 1106, 1402 bis 1416, 2055 und/oder ihrer komplementären Nucleinsäuresequenzen (Nucleotidsequenzen, die den verschiedenen variablen Sequenzen des Locus CRISPR1 des Serotyps Bispebjerg entsprechen) ;
- dem Satz S51-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 2 bis 4, 9, 1417 bis 1420, 1547, 1572 und/oder ihrer komplementären Nucleinsäuresequenzen (Nucleotidsequenzen, die den verschiedenen variablen Sequenzen des Locus CRISPR2 des Serotyps Bispebjerg entsprechen) ;
- dem Satz S52-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 379, 380, 383, 385, 387, 389 bis 391 und/oder ihrer komplementären Nucleinsäuresequenzen (Nucleotidsequenzen, die den verschiedenen variablen Sequenzen des Locus CRISPR1 des Serotyps Blegdam entsprechen) ;
- dem Satz S52-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392 bis 394, 404, 407, 412, 413, 519 und/oder ihrer komplementären Nucleinsäuresequenzen (Nucleotidsequenzen, die den verschiedenen variablen Sequenzen des Locus CRISPR2 des Serotyps Blegdam entsprechen) ;
- dem Satz S53-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Chester entsprechen) ;
- dem Satz S53-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 933, 1445 bis 1447 und/oder ihrer komplementären Nucleinsäuresequenzen (Nucleotidsequenzen, die den verschiedenen variablen Sequenzen des Locus CRISPR2 des Serotyps Chester entsprechen) ;
- dem Satz S54-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1449 bis 1454, 1460, 1467 bis 1473 und/oder ihrer komplementären Nucleinsäuresequenzen (Nucleotidsequenzen, die den verschiedenen variablen Sequenzen des Locus CRISPR1 des Serotyps Concord entsprechen) ;
- dem Satz S54-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 972, 1474 bis 1493, 1495 bis 1499 und/oder ihrer komplementären Nucleinsäuresequenzen (Nucleotidsequenzen, die den verschiedenen variablen Sequenzen des Locus CRISPR2 des Serotyps Concord entsprechen) ;
- dem Satz S55-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1106, 1107 bis 1109, 1123, 1128 bis 1131, 1133, 1137 bis 1140, 1550, 1559 bis 1561 und/oder ihrer komplementären Nucleinsäuresequenzen (Nucleotidsequenzen, die den verschiedenen variablen Sequenzen des Locus CRISPR1 des Serotyps Crossness entsprechen) ;
- dem Satz S55-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 455, 1144, 1145, 1149 bis 1153, 1155 bis 1160, 1163, 1172 bis 1174 und/oder ihrer komplementären Nucleinsäuresequenzen (Nucleotidsequenzen, die den verschiedenen variablen Sequenzen des Locus CRISPR2 des Serotyps Crossness entsprechen) ;
- dem Satz S56-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 834, 1120, 1349 bis 1351, 1713, 1718 und/oder ihrer komplementären Nucleinsäuresequenzen (Nucleotidsequenzen, die den verschiedenen variablen Sequenzen des Locus CRISPR1 des Serotyps Duesseldorf entsprechen) ;
- dem Satz S56-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 842, 852, 1352, 1353, 1501 bis 1503 und/oder ihrer komplementären Nucleinsäuresequenzen (Nucleotidsequenzen, die den verschiedenen variablen Sequenzen des Locus CRISPR2 des Serotyps Duesseldorf entsprechen) ;
- dem Satz S57-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 941, 1504 bis 1508 und/oder ihrer komplementären Nucleinsäuresequenzen (Nucleotidsequenzen, die den verschiedenen variablen Sequenzen des Locus CRISPR1 des Serotyps Emek entsprechen) ;
- dem Satz S57-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1260, 1509 bis 1524, 1526, 1527 und/oder ihrer komplementären Nucleinsäuresequenzen (Nucleotidsequenzen, die den verschiedenen variablen Sequenzen des Locus CRISPR2 des Serotyps Emek entsprechen) ;
- dem Satz S58-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 558 und/oder ihrer komplementären Nucleinsäuresequenz (Nucleotidsequenzen, die der variablen Sequenz des Locus CRISPR1 des Serotyps Fulica entsprechen) ;
- dem Satz S58-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 2, 4, 5, 1333, 1547 und/oder ihrer komplementären Nucleinsäuresequenzen (Nucleotidsequenzen, die den verschiedenen variablen Sequenzen des Locus CRISPR2 des Serotyps Fulica entsprechen) ;
- dem Satz S59-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Gueuletapee entsprechen) ;
- dem Satz S59-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 597, 918 bis 925, 933, 1549 und/oder ihrer komplementären Nucleinsäuresequenzen (Nucleotidsequenzen, die den verschiedenen variablen Sequenzen des Locus CRISPR2 des Serotyps Gueuletapee entsprechen) ;
- dem Satz S60-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1106 bis 1109, 1111 bis 1116, 1118, 1123, 1128 bis 1131, 1133, 1137 bis 1140, 1150, 1151, 1555 bis 1562 und/oder ihrer komplementären Nucleinsäuresequenzen (Nucleotidsequenzen, die den verschiedenen variablen Sequenzen des Locus CRISPR1 des Serotyps Heidelberg entsprechen) ;
- dem Satz S60-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 455, 1144, 1145, 1149 bis 1153, 1155 bis 1163, 1172 bis 1174, 1563 und/oder ihrer komplementären Nucleinsäuresequenzen (Nucleotidsequenzen, die den verschiedenen variablen Sequenzen des Locus CRISPR2 des Serotyps Heidelberg entsprechen) ;
- dem Satz S61-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 558, 1565 bis 1571 und/oder ihrer komplementären Nucleinsäuresequenzen (Nucleotidsequenzen, die den verschiedenen variablen Sequenzen des Locus CRISPR1 des Serotyps Hessarek entsprechen) ;
- dem Satz S61-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 2 bis 4, 1547, 1572, 1573 und/oder ihrer komplementären Nucleinsäuresequenzen (Nucleotidsequenzen, die den verschiedenen variablen Sequenzen des Locus CRISPR2 des Serotyps Hessarek entsprechen) ;
- dem Satz S62-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1591, 1592, 1667 und/oder ihrer komplementären Nucleinsäuresequenzen (Nucleotidsequenzen, die den verschiedenen variablen Sequenzen des Locus CRISPR1 des Serotyps Itami entsprechen) ;
- dem Satz S62-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 918, 1593 bis 1600, 1744 und/oder ihrer komplementären Nucleinsäuresequenzen (Nucleotidsequenzen, die den verschiedenen variablen Sequenzen des Locus CRISPR2 des Serotyps Itami entsprechen) ;
- dem Satz S63-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1601 bis 1606, 1687 und/oder ihrer komplementären Nucleinsäuresequenzen (Nucleotidsequenzen, die den verschiedenen variablen Sequenzen des Locus CRISPR1 des Serotyps Javiana entsprechen) ;
- dem Satz S63-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 589 bis 601, 973, 1607, 1608 und/oder ihrer komplementären Nucleinsäuresequenzen (Nucleotidsequenzen, die den verschiedenen variablen Sequenzen des Locus CRISPR2 des Serotyps Javiana entsprechen) ;
- dem Satz S64-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1609 bis 1621, 1494, 1525 und/oder ihrer komplementären Nucleinsäuresequenzen (Nucleotidsequenzen, die den verschiedenen variablen Sequenzen des Locus CRISPR1 des Serotyps Johannesburg entsprechen) ;
- dem Satz S64-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 918, 1697, 1698 und/oder ihrer komplementären Nucleinsäuresequenzen (Nucleotidsequenzen, die den verschiedenen variablen Sequenzen des Locus CRISPR2 des Serotyps Johannesburg entsprechen) ;
- dem Satz S65-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 444, 448, 1184, 1622 bis 1637, und/oder ihrer komplementären Nucleinsäuresequenzen (Nucleotidsequenzen, die den verschiedenen variablen Sequenzen des Locus CRISPR1 des Serotyps Kentucky entsprechen) ;
- dem Satz S65-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 385, 519, 1638 bis 1652, 1746 und/oder ihrer komplementären Nucleinsäuresequenzen (Nucleotidsequenzen, die den verschiedenen variablen Sequenzen des Locus CRISPR2 des Serotyps Kentucky entsprechen) ;
- dem Satz S66-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 67 und/oder ihrer komplementären Nucleinsäuresequenz (Nucleotidsequenzen, die der variablen Sequenz des Locus CRISPR1 des Serotyps Kundunchi entsprechen) ;
- dem Satz S66-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 972, 983, 989, 1201, 1204, 1205, 1210 bis 1212 und/oder ihrer komplementären Nucleinsäuresequenzen (Nucleotidsequenzen, die den verschiedenen variablen Sequenzen des Locus CRISPR2 des Serotyps Kundunchi entsprechen) ;
- dem Satz S67-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 448, 449, 860, 871, 878 bis 881, 1721, 1724 und/oder ihrer komplementären Nucleinsäuresequenzen (Nucleotidsequenzen, die den verschiedenen variablen Sequenzen des Locus CRISPR1 des Serotyps Lindenburg entsprechen) ;
- dem Satz S67-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 563, 622, 885, 886, 896, 907, 910 bis 915, 933, 1748, 1757 und/oder ihrer komplementären Nucleinsäuresequenzen (Nucleotidsequenzen, die den verschiedenen variablen Sequenzen des Locus CRISPR2 des Serotyps Lindenburg entsprechen) ;
- dem Satz S68-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Maracaibo entsprechen) ;
- dem Satz S68-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1240, 1247, 1248, 1654 bis 1656 und/oder ihrer komplementären Nucleinsäuresequenzen (Nucleotidsequenzen, die den verschiedenen variablen Sequenzen des Locus CRISPR2 des Serotyps Maracaibo entsprechen) ;
- dem Satz S69-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 627, 834, 1120, 1660 bis 1669 und/oder ihrer komplementären Nucleinsäuresequenzen (Nucleotidsequenzen, die den verschiedenen variablen Sequenzen des Locus CRISPR1 des Serotyps Mississippi entsprechen) ;
- dem Satz S69-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 842, 1670 und/oder ihrer komplementären Nucleinsäuresequenzen (Nucleotidsequenzen, die den verschiedenen variablen Sequenzen des Locus CRISPR2 des Serotyps Mississippi entsprechen) ;
- dem Satz S70-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 941, 942, 949, 1699 bis 1712, 2109 und/oder ihrer komplementären Nucleinsäuresequenzen (Nucleotidsequenzen, die den verschiedenen variablen Sequenzen des Locus CRISPR1 des Serotyps Muenchen entsprechen) ;
- dem Satz S70-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 1653 und/oder ihrer komplementären Nucleinsäuresequenzen (Nucleotidsequenzen, die den verschiedenen variablen Sequenzen des Locus CRISPR2 des Serotyps Muenchen entsprechen) ;
- dem Satz S71-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 11, 1120, 1497, 1760 bis 1775, 1809 und/oder ihrer komplementären Nucleinsäuresequenzen (Nucleotidsequenzen, die den verschiedenen variablen Sequenzen des Locus CRISPR1 des Serotyps Niarembe entsprechen) ;
- dem Satz S71-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 90, 1776 bis 1785 und/oder ihrer komplementären Nucleinsäuresequenzen (Nucleotidsequenzen, die den verschiedenen variablen Sequenzen des Locus CRISPR2 des Serotyps Niarembe entsprechen) ;
- dem Satz S72-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 666, 1786 bis 1791 und/oder ihrer komplementären Nucleinsäuresequenzen (Nucleotidsequenzen, die den verschiedenen variablen Sequenzen des Locus CRISPR1 des Serotyps Overvecht entsprechen) ;
- dem Satz S72-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 783, 1146, 1178, 1179, 1181, 1792 bis 1799, 1801 und/oder ihrer komplementären Nucleinsäuresequenzen (Nucleotidsequenzen, die den verschiedenen variablen Sequenzen des Locus CRISPR2 des Serotyps Overvecht entsprechen) ;
- dem Satz S73-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Poona entsprechen) ;
- dem Satz S73-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 82, 87, 918, 933, 1826 und/oder ihrer komplementären Nucleinsäuresequenzen (Nucleotidsequenzen, die den verschiedenen variablen Sequenzen des Locus CRISPR2 des Serotyps Poona entsprechen) ;
- dem Satz S74-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 295 bis 297, 307, 313, 317, 323, 1120, 1825, 1827 bis 1837, 2023 und/oder ihrer komplementären Nucleinsäuresequenzen (Nucleotidsequenzen, die den verschiedenen variablen Sequenzen des Locus CRISPR1 des Serotyps Postdam entsprechen) ;
- dem Satz S74-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 663, 1304, 1838 bis 1847 und/oder ihrer komplementären Nucleinsäuresequenzen (Nucleotidsequenzen, die den verschiedenen variablen Sequenzen des Locus CRISPR2 des Serotyps Postdam entsprechen) ;
- dem Satz S75-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Reading entsprechen) ;
- dem Satz S75-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 225 und/oder ihrer komplementären Nucleinsäuresequenz (Nucleotidsequenzen, die der variablen Sequenz des Locus CRISPR2 des Serotyps Reading entsprechen) ;
- dem Satz S76-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 379, 380, 383 bis 385, 387, 389, 390 und/oder ihrer komplementären Nucleinsäuresequenzen (Nucleotidsequenzen, die den verschiedenen variablen Sequenzen des Locus CRISPR1 des Serotyps Rosenberg entsprechen) ;
- dem Satz S76-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 392, 393, 404, 407 bis 413 und/oder ihrer komplementären Nucleinsäuresequenzen (Nucleotidsequenzen, die den verschiedenen variablen Sequenzen des Locus CRISPR2 des Serotyps Rosenberg entsprechen) ;
- dem Satz S77-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Rubislaw entsprechen) ;
- dem Satz S77-2, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 225 und/oder ihrer komplementären Nucleinsäuresequenz ;
- dem Satz S78-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1525, 1609 bis 1621 und/oder ihrer komplementären Nucleinsäuresequenzen ;
- dem Satz S78-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 225 bis 227, 229 bis 231 und/oder ihrer komplementären Nucleinsäuresequenzen (Nucleotidsequenzen, die den verschiedenen variablen Sequenzen des Locus CRISPR2 des Serotyps Sandiego entsprechen) ;
- dem Satz S79-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1106, 1456, 1917 bis 1924 und/oder ihrer komplementären Nucleinsäuresequenzen (Nucleotidsequenzen, die den verschiedenen variablen Sequenzen des Locus CRISPR1 des Serotyps Stourbridge entsprechen) ;
- dem Satz S79-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 918, 1093, 1925 bis 1936 und/oder ihrer komplementären Nucleinsäuresequenzen (Nucleotidsequenzen, die den verschiedenen variablen Sequenzen des Locus CRISPR2 des Serotyps Stourbridge entsprechen) ;
- dem Satz S80-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1961 bis 1963 und/oder ihrer komplementären Nucleinsäuresequenzen (Nucleotidsequenzen, die den verschiedenen variablen Sequenzen des Locus CRISPR1 des Serotyps Tallahassee entsprechen) ;
- dem Satz S80-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 25, 603, 2127, 2133, 1964 bis 1966 und/oder ihrer komplementären Nucleinsäuresequenzen (Nucleotidsequenzen, die den verschiedenen variablen Sequenzen des Locus CRISPR2 des Serotyps Tallahassee entsprechen) ;
- dem Satz S81-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 67, 929, 1967 bis 2005 und/oder ihrer komplementären Nucleinsäuresequenzen ;
- dem Satz S81-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 24, 769, 1550, 1659, 2006 bis 2022 und/oder ihrer komplementären Nucleinsäuresequenzen (Nucleotidsequenzen, die den verschiedenen variablen Sequenzen des Locus CRISPR2 des Serotyps Tennessee entsprechen) ;
- dem Satz S82-1, umfassend oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Urbana entsprechen) ;
- dem Satz S82-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1697, 1698, 1800 und/oder ihrer komplementären Nucleinsäuresequenzen (Nucleotidsequenzen, die den verschiedenen variablen Sequenzen des Locus CRISPR2 des Serotyps Urbana entsprechen) ;
- dem Satz S83-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 948, 1120, 1123, 1325, 2039 bis 2078 und/oder ihrer komplementären Nucleinsäuresequenzen (Nucleotidsequenzen, die den verschiedenen variablen Sequenzen des Locus CRISPR1 des Serotyps Weltevreden entsprechen) ;
- dem Satz S83-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 254 bis 256, 2079 bis 2108 und/oder ihrer komplementären Nucleinsäuresequenzen (Nucleotidsequenzen, die den verschiedenen variablen Sequenzen des Locus CRISPR2 des Serotyps Weltevreden entsprechen) ;
- dem Satz S84-1, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1312, 1690, 2141, 2142 und/oder ihrer komplementären Nucleinsäuresequenzen (Nucleotidsequenzen, die den verschiedenen variablen Sequenzen des Locus CRISPR1 des Serotyps Zaiman entsprechen) ;
- dem Satz S84-2, umfassend oder bestehend aus wenigstens eine(r) Sonde, umfassend wenigstens oder bestehend aus wenigstens 8 aufeinanderfolgende(n) Nucleotide(n) einer der Nucleinsäuresequenzen SEQ ID NO : 1697, 1698, 1800, 1924, 2143, 2144 und/oder ihrer komplementären Nucleinsäuresequenzen (Nucleotidsequenzen, die den verschiedenen variablen Sequenzen des Locus CRISPR2 des Serotyps Zaiman entsprechen) ; et
(ii) Detektion der hybridisierten Sonden.

11. Verfahren gemäß Anspruch 10, **dadurch gekennzeichnet, dass** die Sonden eine Länge von zwischen 8 und 76 Nucleotiden, vorzugsweise zwischen 8 und 34 Nucleotiden, aufweisen.

12. Verfahren gemäß Anspruch 10 oder Anspruch 11, **dadurch gekennzeichnet, dass** die Stufe (b) durch Hybridisierung wenigstens einer Sonde von jedem der Sätze S1-1 bis S6-2 durchgeführt wird.

13. Verfahren gemäß Anspruch 12, **dadurch gekennzeichnet, dass** die Stufe (b) durch Hybridisierung außerdem wenigstens einer Sonde von jedem der Sätze S7-1 bis S10-2 durchgeführt wird.

14. Verfahren gemäß Anspruch 13, **dadurch gekennzeichnet, dass** die Stufe (b) durch Hybridisierung wenigstens einer Sonde von jedem der Sätze S1-1 bis S44-2 durchgeführt wird.

15. Verfahren gemäß einem der Ansprüche 1 bis 14, **dadurch gekennzeichnet, dass** die amplifizierten Nucleinsäurefragmente markiert werden.

16. Verfahren gemäß einem der Ansprüche 1 bis 15, **dadurch gekennzeichnet, dass** die Probe ein isolierter Bakterienstamm der Gattung *Salmonella* ist.

17. Verfahren gemäß einem der Ansprüche 1 bis 16, **dadurch gekennzeichnet , dass** der Stufe (a) eine Stufe der Extraktion von Nucleinsäuren, die in der Probe vorliegen, vorgeschaltet wird.

18. Sondensatz, der geeignet ist, ein Bakterium der Gattung *Salmonella* zu detektieren, **dadurch gekennzeichnet, dass** er ausgewählt ist aus der Gruppe, bestehend aus:
- dem Satz S1-1, umfassend die oder bestehend aus den Sonden der Nucleinsäuresequenzen SEQ ID NO: 1106 bis 1119, 1123 bis 1140, 1142, 1143, 1914 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Typhimurium entsprechen);
- dem Satz S1-2, umfassend die oder bestehend aus den Sonden der Nucleinsäuresequenzen SEQ ID NO: 455, 1144 bis 1147, 1149 bis 1153, 1155 bis 1181 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Typhimurium entsprechen);
- dem Satz S2-1, umfassend die oder bestehend aus den Sonden der Nucleinsäuresequenzen SEQ ID NO : 256, 378 bis 381, 383 bis 387, 389 bis 391, 1106, 1514, 1528, 2148 bis 2153 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Enteritidis entsprechen) ;
- dem Satz S2-2, umfassend die oder bestehend aus den Sonden der Nucleinsäuresequenzen SEQ ID NO : 392 bis 413, 519, 815, 972, 983, 989, 1529 bis 1546, 1952 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Enteritidis entsprechen) ;
- dem Satz S3-1, umfassend die oder bestehend aus den Sonden der Nucleinsäuresequenzen SEQ ID NO : 421 bis 442, 444, 445, 447 bis 450 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Hadar entsprechen) ;
- dem Satz S3-2, umfassend die oder bestehend aus den Sonden der Nucleinsäuresequenzen SEQ ID NO : 451 bis 465, 1144 bis 1147, 1150 bis 1152, 1154, 1155, 1158, 1169, 1172, 1175 bis 1177, 1179, 1181 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Hadar entsprechen) ;
- dem Satz S4-1, umfassend die oder bestehend aus den Sonden der Nucleinsäuresequenzen SEQ ID NO : 65, 382, 1120, 1263 bis 1302, 2024 bis 2038 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Virchow entsprechen) ;
- dem Satz S4-2, umfassend die oder bestehend aus den Sonden der Nucleinsäuresequenzen SEQ ID NO : 83, 93, 94, 1304 bis 1324 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Virchow entsprechen) ;
- dem Satz S5-1, umfassend die oder bestehend aus den Sonden der Nucleinsäuresequenzen SEQ ID NO : 531 bis 562, 1106 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Infantis entsprechen) ;
- dem Satz S5-2, umfassend die oder bestehend aus den Sonden der Nucleinsäuresequenzen SEQ ID NO : 563 bis 588 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Infantis entsprechen) ;
- dem Satz S6-1, umfassend die oder bestehend aus den Sonden der Nucleinsäuresequenzen SEQ ID NO : 1254 bis 1259 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Typhi entsprechen) ;
- dem Satz S6-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO: 1260 und/oder ihrer komplementären Nucleinsäuresequenz (Nucleotidsequenzen, die der variablen Sequenz des Locus CRISPR2 des Serotyps Typhin entsprechen);
- dem Satz S7-1, umfassend die oder bestehend aus den Sonden der Nucleinsäuresequenzen SEQ ID NO : 448, 449, 860 bis 876, 878 bis 884, 1719 bis 1724, 1727 bis 1743, 1745 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Newport entsprechen) ;
- dem Satz S7-2, umfassend die oder bestehend aus den Sonden der Nucleinsäuresequenzen SEQ ID NO : 453 bis 456, 563, 621, 622, 625, 626, 657, 885 bis 896, 906 bis 915, 933, 1144, 1145, 1155, 1156, 1172, 1174, 1748 bis 1756, 1758, 1759, 1916 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Newport entsprechen) ;
- dem Satz S8-1, umfassend die oder bestehend aus den Sonden der Nucleinsäuresequenzen SEQ ID NO : 295 bis 299, 301 bis 323, 738, 1106 bis 1109, 1120, 1123, 1128 bis 1131, 1133, 1137 bis 1140, 1550, 1159 bis 1561 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Derby entsprechen) ;
- dem Satz S8-2, umfassend die oder bestehend aus den Sonden der Nucleinsäuresequenzen SEQ ID NO : 324 bis 337, 455, 1144, 1145, 1149 bis 1153, 1155 bis 1160, 1163, 1172 bis 1174 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Derby entsprechen) ;
- dem Satz S9-1, umfassend die oder bestehend aus den Sonden der Nucleinsäuresequenzen SEQ ID NO : 295, 307, 602 bis 614, 616 bis 619, 1106, 1120, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Kottbus entsprechen) ;
- dem Satz S9-2, umfassend die oder bestehend aus den Sonden der Nucleinsäuresequenzen SEQ ID NO : 563, 620 bis 629, 885, 896, 907, 913, 914, 933, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Kottbus entsprechen) ;
- dem Satz S10-1, umfassend die oder bestehend aus den Sonden der Nucleinsäuresequenzen SEQ ID NO : 422 bis 425, 448 bis 450, 504 bis 518 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Indiana entsprechen) ;
- dem Satz S10-2, umfassend die oder bestehend aus den Sonden der Nucleinsäuresequenzen SEQ ID NO : 246, 258, 269, 392, 415, 519 bis 530, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Indiana entsprechen) ;
- dem Satz S11-1, umfassend die oder bestehend aus den Sonden der Nucleinsäuresequenzen.SEQ ID NO : 11 bis 23, 238, 948, 1120, 1123, 1133 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Agona entsprechen) ;
- dem Satz S11-2, umfassend die oder bestehend aus den Sonden der Nucleinsäuresequenzen SEQ ID NO : 24 bis 31 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Agona entsprechen) ;
- dem Satz S12-1, umfassend die oder bestehend aus den Sonden der Nucleinsäuresequenzen SEQ ID NO : 830 bis 841, 1120 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Napoli entsprechen) ;
- dem Satz S12-2, umfassend die oder bestehend aus den Sonden der Nucleinsäuresequenzen SEQ ID NO : 842 bis 859 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Napoli entsprechen) ;
- dem Satz S13-1, umfassend die oder bestehend aus den Sonden der Nucleinsäuresequenzen SEQ ID NO : 926 bis 932 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Paratyphi A entsprechen) ;
- dem Satz S13-2, umfassend die oder bestehend aus den Sonden der Nucleinsäuresequenzen SEQ ID NO : 933, 935, 936 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Paratyphi A entsprechen) ;
- dem Satz S14-1, umfassend die oder bestehend aus den Sonden der Nucleinsäuresequenzen SEQ ID NO : 707, 937 bis 971, 1106, 1128 bis 1130, 1133, 1138, 1140, 1802 bis 1808, 1915 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der Serotypen Paratyphi B und Paratyphi B Java entsprechen) ;
- dem Satz S14-2, umfassend die oder bestehend aus den Sonden der Nucleinsäuresequenzen SEQ ID NO : 861, 972 bis 987, 989 bis 992 bis 996, 1172 1262, 1462, 1497, 1810 bis 1825 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der Serotypen Paratyphi B und Paratyphi B Java entsprechen) ;
- dem Satz S15-1, umfassend die oder bestehend aus den Sonden der Nucleinsäuresequenzen SEQ ID NO : 232, 238, 239, 997 bis 1002, 1106 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Paratyphi C entsprechen) ;
- dem Satz S15-2, umfassend die oder bestehend aus den Sonden der Nucleinsäuresequenzen SEQ ID NO : 280, 290, 291, 392, 393, 415, 1003 bis 1006, 1262 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Paratyphi C entsprechen) ;
- dem Satz S16-1, umfassend oder bestehend aus wenigstens eine(r) Sonde, umfassend oder bestehend aus wenigstens 8 aufeinanderfolgende(n) Nucleotide(n) einer der Nucleinsäuresequenzen SEQ ID NO : 211 bis 224, 421, 427, 428, 432, 443 bis 445, 447 bis 449, 504 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Bovismorbificans entsprechen) ;
- dem Satz S16-2, umfassend die oder bestehend aus den Sonden der Nucleinsäuresequenzen SEQ ID NO : 196 bis 209, 994, 1144, 1145, 1172 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Bovismorbificans entsprechen) ;
- dem Satz S17-1, umfassend die oder bestehend aus wenigstens einer Sonde, umfassend wenigstens oder bestehend aus wenigstens 8 aufeinanderfolgende(n) Nucleotide(n) der Nucleinsäuresequenz umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Panama entsprechen) ;
- dem Satz S17-2, umfassend die oder bestehend aus den Sonden der Nucleinsäuresequenzen SEQ ID NO : 918 bis 925, 934 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Panama entsprechen) ;
- dem Satz S18-1, umfassend die oder bestehend aus den Sonden der Nucleinsäuresequenzen SEQ ID NO : 11, 16, 67, 74, 442, 444, 446 bis 448, 602, 937, 948, 1120, 1128 bis 1130, 1182 bis 1200, 1937 bis 1950, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Saintpaul entsprechen) ;
- dem Satz S18-2, umfassend die oder bestehend aus den Sonden der Nucleinsäuresequenzen SEQ ID NO : 972, 983, 989, 1201 bis 1214, 1216 bis 1227, 1952 bis 1960 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Saintpaul entsprechen) ;
- dem Satz S19-1, umfassend die oder bestehend aus den Sonden der Nucleinsäuresequenzen SEQ ID NO : 514, 1062 bis 1089, 1100, 1284, 1886 bis 1911 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Senftenberg entsprechen) ;
- dem Satz S19-2, umfassend die oder bestehend aus den Sonden der Nucleinsäuresequenzen SEQ ID NO : 300, 1090 bis 1097, 1101 bis 1103, 1304, 1315, 1318 bis 1320 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Senftenberg entsprechen) ;
- dem Satz S20-1, umfassend die oder bestehend aus den Sonden der Nucleinsäuresequenzen SEQ ID NO : 67, 74 bis 80 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Anatum entsprechen) ;
- dem Satz S20-2, umfassend die oder bestehend aus den Sonden der Nucleinsäuresequenzen SEQ ID NO : 83 bis 94, 1304, 1315, 1318 bis 1322, 1324, 1390 bis 1397 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Anatum entsprechen) ;
- dem Satz S21-1, umfassend die oder bestehend aus den Sonden der Nucleinsäuresequenzen SEQ ID NO : 379, 383 bis 385, 388 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der Serotypen Gallinarum entsprechen) ;
- dem Satz S21-2, umfassend die oder bestehend aus den Sonden der Nucleinsäuresequenzen SEQ ID NO : 392, 393, 404, 407 bis 415 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der Serotypen Gallinarum entsprechen) ;
- dem Satz S22-1, umfassend die oder bestehend aus wenigstens einer Sonde, umfassend wenigstens oder bestehend aus wenigstens 8 aufeinanderfolgende(n) Nucleotide(n) der Nucleinsäuresequenzumfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Brandenburg entsprechen) ;
- dem Satz S22-2, umfassend die oder bestehend aus den Sonden der Nucleinsäuresequenzen SEQ ID NO : 225 bis 231 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Brandenburg entsprechen) ;
- dem Satz S23-1, umfassend die oder bestehend aus den Sonden der Nucleinsäuresequenzen SEQ ID NO : 378, 379 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Dublin entsprechen) ;
- dem Satz S23-2, umfassend die oder bestehend aus den Sonden der Nucleinsäuresequenzen SEQ ID NO : 392, 393, 411, 412 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Dublin entsprechen) ;
- dem Satz S24-1, umfassend die oder bestehend aus den Sonden der Nucleinsäuresequenzen SEQ ID NO : 1, 1106 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Abortusequi entsprechen) ;
- dem Satz S24-2, umfassend die oder bestehend aus den Sonden der Nucleinsäuresequenzen SEQ ID NO : 2 bis 10, 130 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Abortusequi entsprechen) ;
- dem Satz S25-1, umfassend die oder bestehend aus den Sonden der Nucleinsäuresequenzen SEQ ID NO : 378, 379 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Kiel entsprechen) ;
- dem Satz S25-2, umfassend die oder bestehend aus den Sonden der Nucleinsäuresequenzen SEQ ID NO : 392, 393, 411, 412 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Kiel entsprechen) ;
- dem Satz S26-1, umfassend die oder bestehend aus den Sonden der Nucleinsäuresequenzen SEQ ID NO : 509, 510, 512, 513, 637 bis 660, 1086, 1107 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Mbandaka entsprechen) ;
- dem Satz S26-2, umfassend die oder bestehend aus den Sonden der Nucleinsäuresequenzen SEQ ID NO : 661 bis 666, 668, 669, 672, 683, 690 bis 695, 731, 918 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Mbandaka entsprechen) ;
- dem Satz S27-1, umfassend die oder bestehend aus wenigstens einer Sonde, umfassend wenigstens oder bestehend aus wenigstens 8 aufeinanderfolgende(n) Nucleotide(n) der Nucleinsäuresequenzumfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Miami entsprechen) ;
- dem Satz S27-2, umfassend die oder bestehend aus den Sonden der Nucleinsäuresequenzen SEQ ID NO : 25, 696 bis 700 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Miami entsprechen) ;
- dem Satz S28-1, umfassend die oder bestehend aus den Sonden der Nucleinsäuresequenzen SEQ ID NO : 55, 701 bis 730, 732 bis 768, 1115, 1228, 1232 bis 1237, 1601, 1671 bis 1680, 1682 bis 1694, 1747 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Montevideo entsprechen) ;
- dem Satz S28-2, umfassend die oder bestehend aus den Sonden der Nucleinsäuresequenzen SEQ ID NO : 589, 769 bis 829, 933, 1245, 1697, 1698 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Montevideo entsprechen) ;
- dem Satz S29-1, umfassend die oder bestehend aus den Sonden der Nucleinsäuresequenzen SEQ ID NO : 379, 380, 383 bis 385, 387, 389 bis 391 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Nitra entsprechen) ;
- dem Satz S29-2, umfassend die oder bestehend aus den Sonden der Nucleinsäuresequenzen SEQ ID NO : 392 bis 394, 404, 407 bis 413, 519 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Nitra entsprechen) ;
- dem Satz S30-1, umfassend die oder bestehend aus den Sonden der Nucleinsäuresequenzen SEQ ID NO : 931, 932, 1104, 1105 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Sendai entsprechen) ;
- dem Satz S30-2, umfassend die oder bestehend aus wenigstens einer Sonde, umfassend wenigstens oder bestehend aus wenigstens 8 aufeinanderfolgende(n) Nucleotide(n) der Nucleinsäuresequenzumfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 936 und/oder ihrer komplementären Nucleinsäuresequenz (Nucleotidsequenzen, die der variablen Sequenz des Locus CRISPR2 des Serotyps Sendai entsprechen) ;
- dem Satz S31-1, umfassend die oder bestehend aus den Sonden der Nucleinsäuresequenzen SEQ ID NO : 714 bis 716, 1228 bis 1239 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Schwarzengrund entsprechen) ;
- dem Satz S31-2, umfassend die oder bestehend aus den Sonden der Nucleinsäuresequenzen SEQ ID NO : 196, 992, 995, 1172, 1240 bis 1244, 1246 bis 1253 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Schwarzengrund entsprechen) ;
- dem Satz S32-1, umfassend die oder bestehend aus den Sonden der Nucleinsäuresequenzen SEQ ID NO : 232, 238, 239, 998, 999, 1002, 1106, 1261 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Typhisuis entsprechen) ;
- dem Satz S32-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 1262 und/oder ihrer komplementären Nucleinsäuresequenzen (Nucleotidsequenzen, die den verschiedenen variablen Sequenzen des Locus CRISPR2 des Serotyps Typhisuis entsprechen) ;
- dem Satz S33-1, umfassend die oder bestehend aus den Sonden der Nucleinsäuresequenzen SEQ ID NO : 466 bis 486 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der verschiedenen Serotypen der Unterart houtenae entsprechen) ;
- dem Satz S33-2, umfassend die oder bestehend aus den Sonden der Nucleinsäuresequenzen SEQ ID NO : 487 bis 490 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der verschiedenen Serotypen der Unterart houtenae entsprechen) ;
- dem Satz S34-1, umfassend die oder bestehend aus den Sonden der Nucleinsäuresequenzen SEQ ID NO : 338 bis 376 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der verschiedenen Serotypen der Unterart *diarizonae* entsprechen) ;
- dem Satz S34-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 377 und/oder ihrer komplementären Nucleinsäuresequenz (Nucleotidsequenzen, die der variablen Sequenz des Locus CRISPR2 der verschiedenen Serotypen der Unterart *diarizonae* entsprechen) ;
- dem Satz S35-1, umfassend die oder bestehend aus den Sonden der Nucleinsäuresequenzen SEQ ID NO : 1007 bis 1037, 1848 bis 1884 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der Unterart *salamae* entsprechen) ;
- dem Satz S35-2, umfassend die oder bestehend aus den Sonden der Nucleinsäuresequenzen SEQ ID NO : 1038 bis 1043, 1045 bis 1061, 1885 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der Unterart *salamae* entsprechen) ;
- dem Satz S36, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 95 und/oder ihrer komplementären Nucleinsäuresequenz (Nucleotidsequenzen, die der variablen Sequenz des Locus CRISPR1 der verschiedenen Serotypen der Unterart *arizonae* entsprechen) ;
- dem Satz S37-1, umfassend die oder bestehend aus den Sonden der Nucleinsäuresequenzen SEQ ID NO : 491 bis 499 oder wenigstens 8 aufeinanderfolgende(n) Nucleotide(n) dieser Nucleinsäuresequenzen und/oder ihrer komplementären Nucleinsäuresequenzen (Nucleotidsequenzen, die den variablen Sequenzen des Locus CRISPR1 der verschiedenen Serotypen der Unterart *indica* entsprechen) ;
- dem Satz S37-2, umfassend die oder bestehend aus den Sonden der Nucleinsäuresequenzen SEQ ID NO : 500 bis 503, 1574 bis 1589 oder wenigstens 8 aufeinanderfolgende(n) Nucleotide(n) dieser Nucleinsäuresequenzen und/oder ihrer komplementären Nucleinsäuresequenzen (Nucleotidsequenzen, die den variablen Sequenzen des Locus CRISPR2 der verschiedenen Serotypen der Unterart *indica* entsprechen) ;
- dem Satz S38-1, umfassend die oder bestehend aus den Sonden der Nucleinsäuresequenzen SEQ ID NO : 32 bis 66, 382, 416, 667, 2036 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Altona entsprechen) ;
- dem Satz S38-2, umfassend die oder bestehend aus den Sonden der Nucleinsäuresequenzen SEQ ID NO : 854, 933, 1095, 1306, 1354 bis 1389, 1696 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Altona entsprechen) ;
- dem Satz S39-1, umfassend die oder bestehend aus den Sonden der Nucleinsäuresequenzen SEQ ID NO : 240, 241, 1120, 1121 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Choleraesuis var. Decatur entsprechen) ;
- dem Satz S39-2, umfassend die oder bestehend aus den Sonden der Nucleinsäuresequenzen SEQ ID NO : 225, 247 bis 251, 253 bis 256, 259 bis 268, 270 bis 279, 281 bis 289, 292 bis 294 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Choleraesuis var. Decatur entsprechen) ;
- dem Satz S40-1, umfassend die oder bestehend aus den Sonden der Nucleinsäuresequenzen SEQ ID NO : 11, 232, 238, 239, 240, 242 bis 245, 233 bis 237, 959, 1106, 1120 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Choleraesuis var. Kunzendorf entsprechen) ;
- dem Satz S40-2, umfassend die oder bestehend aus den Sonden der Nucleinsäuresequenzen SEQ ID NO : 246, 252, 258, 269, 280, 290, 291, 392, 563, 933 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Choleraesuis var. Kunzendorf entsprechen) ;
- dem Satz S41-1, umfassend die oder bestehend aus den Sonden der Nucleinsäuresequenzen SEQ ID NO : 11, 242 bis 245, 232 bis 239, 959, 1000, 1106, 1139, 1120 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der Serotypen Choleraesuis und Choleraesuis sensu stricto entsprechen) ;
- dem Satz S41-2, umfassend die oder bestehend aus den Sonden der Nucleinsäuresequenzen SEQ ID NO : 246, 252, 258, 269, 280, 290, 291, 392, 563, 574, 933, 1003, 1004 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der Serotypen Choleraesuis und Choleraesuis sensu stricto entsprechen) ;
- dem Satz S42-1, umfassend die oder bestehend aus den Sonden der Nucleinsäuresequenzen SEQ ID NO : 416 bis 420, 1122 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Goettingen entsprechen) ;
- dem Satz S42-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 1548 und/oder ihrer komplementären Nucleinsäuresequenz (Nucleotidsequenzen, die der variablen Sequenz des Locus CRISPR2 des Serotyps Goettingen entsprechen) ;
- dem Satz S43-1, umfassend die oder bestehend aus den Sonden der Nucleinsäuresequenzen SEQ ID NO : 630 bis 636, 937, 948, 1120, 1143 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Manhattan entsprechen) ;
- dem Satz S43-2, umfassend die oder bestehend aus den Sonden der Nucleinsäuresequenzen SEQ ID NO : 392, 1653 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Manhattan entsprechen) ;
- dem Satz S44-1, umfassend die oder bestehend aus den Sonden der Nucleinsäuresequenzen SEQ ID NO : 96 bis 155 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 der bestimmter Serotypen der Art *S. bongori* entsprechen) ;
- dem Satz S44-2, umfassend die oder bestehend aus den Sonden der Nucleinsäuresequenzen SEQ ID NO : 156 bis 195, 1421 bis 1430 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 der bestimmter Serotypen der Art *S. bongori* entsprechen) ;
- dem Satz S45-1, umfassend die oder bestehend aus den Sonden der Nucleinsäuresequenzen SEQ ID NO : 962, 1106, 1133, 1804, 1808 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Abony entsprechen) ;
- dem Satz S45-2, umfassend die oder bestehend aus den Sonden der Nucleinsäuresequenzen SEQ ID NO : 972, 989, 1334 bis 1339, 1474, 1499 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Abony entsprechen) ;
- dem Satz S46-1, umfassend die oder bestehend aus den Sonden der Nucleinsäuresequenzen SEQ ID NO : 448, 860 bis 875, 882, 883, 1340 bis 1345, 1721 bis 1723, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Aesch entsprechen) ;
- dem Satz S46-2, umfassend die oder bestehend aus den Sonden der Nucleinsäuresequenzen SEQ ID NO : 563, 625, 626, 885, 887 bis 893, 896, 933, 1346 bis 1348, 1749, 1759, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Aesch entsprechen) ;
- dem Satz S47-1, umfassend die oder bestehend aus den Sonden der Nucleinsäuresequenzen SEQ ID NO : 834, 1120, 1349, 1350, 1351, 1713, 1718 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Albany entsprechen) ;
- dem Satz S47-2, umfassend die oder bestehend aus den Sonden der Nucleinsäuresequenzen SEQ ID NO : 842, 852, 1352, 1353 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Albany entsprechen) ;
- dem Satz S48-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Arechavalata entsprechen) ;
- dem Satz S48-2, umfassend die oder bestehend aus den Sonden der Nucleinsäuresequenzen SEQ ID NO : 225, 227 bis 231, 1398 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Arechavalata entsprechen) ;
- dem Satz S49-1, umfassend die oder bestehend aus den Sonden der Nucleinsäuresequenzen SEQ ID NO : 448, 449, 860, 871 bis 873, 878, 895, 903 bis 905, 907, 1719 bis 1721, 1745 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Bardo entsprechen) ;
- dem Satz S49-2, umfassend die oder bestehend aus den Sonden der Nucleinsäuresequenzen SEQ ID NO : 563, 622, 626, 885, 886 bis 890, 893, 896, 907, 910 bis 915, 933, 1756 bis 1758 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Bardo entsprechen) ;
- dem Satz S50-1, umfassend die oder bestehend aus den Sonden der Nucleinsäuresequenzen SEQ ID NO : 82, 1401 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Berta entsprechen) ;
- dem Satz S50-2, umfassend die oder bestehend aus den Sonden der Nucleinsäuresequenzen SEQ ID NO : 246, 918, 933, 1401, 1448 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Berta entsprechen) ;
- dem Satz S51-1, umfassend die oder bestehend aus den Sonden der Nucleinsäuresequenzen SEQ ID NO : 1, 37, 1106, 1402 bis 1416, 2055 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Bispebjerg entsprechen) ;
- dem Satz S51-2, umfassend die oder bestehend aus den Sonden der Nucleinsäuresequenzen SEQ ID NO : 2 bis 4, 9, 1417 bis 1420, 1547, 1572 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Bispebjerg entsprechen) ;
- dem Satz S52-1, umfassend die oder bestehend aus den Sonden der Nucleinsäuresequenzen SEQ ID NO : 379, 380, 383, 385, 387, 389 bis 391 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Blegdam entsprechen) ;
- dem Satz S52-2, umfassend die oder bestehend aus den Sonden der Nucleinsäuresequenzen SEQ ID NO : 392 bis 394, 404, 407, 412, 413, 519 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Blegdam entsprechen) ;
- dem Satz S53-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Chester entsprechen) ;
- dem Satz S53-2, umfassend die oder bestehend aus den Sonden der Nucleinsäuresequenzen SEQ ID NO : 933, 1445 bis 1447 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Chester entsprechen) ;
- dem Satz S54-1, umfassend die oder bestehend aus den Sonden der Nucleinsäuresequenzen SEQ ID NO : 1449 bis 1454, 1460, 1467 bis 1473 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Concord entsprechen) ;
- dem Satz S54-2, umfassend die oder bestehend aus den Sonden der Nucleinsäuresequenzen SEQ ID NO : 972, 1474 bis 1493, 1495 bis 1499 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Concord entsprechen) ;
- dem Satz S55-1, umfassend die oder bestehend aus den Sonden der Nucleinsäuresequenzen SEQ ID NO : 1106, 1107 bis 1109, 1123, 1128 bis 1131, 1133, 1137 bis 1140, 1550, 1559 bis 1561 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Crossness entsprechen) ;
- dem Satz S55-2, umfassend die oder bestehend aus den Sonden der Nucleinsäuresequenzen SEQ ID NO : 455, 1144, 1145, 1149 bis 1153, 1155 bis 1160, 1163, 1172 bis 1174 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Crossness entsprechen) ;
- dem Satz S56-1, umfassend die oder bestehend aus den Sonden der Nucleinsäuresequenzen SEQ ID NO : 834, 1120, 1349 bis 1351, 1713, 1718 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Duesseldorf entsprechen) ;
- dem Satz S56-2, umfassend die oder bestehend aus den Sonden der Nucleinsäuresequenzen SEQ ID NO : 842, 852, 1352, 1353, 1501 bis 1503 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Duesseldorf entsprechen) ;
- dem Satz S57-1, umfassend die oder bestehend aus den Sonden der Nucleinsäuresequenzen SEQ ID NO : 941, 1504 bis 1508 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Emek entsprechen) ;
- dem Satz S57-2, umfassend die oder bestehend aus den Sonden der Nucleinsäuresequenzen SEQ ID NO : 1260, 1509 bis 1524, 1526, 1527 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Emek entsprechen) ;
- dem Satz S58-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 558 und/oder ihrer komplementären Nucleinsäuresequenz (Nucleotidsequenzen, die der variablen Sequenz des Locus CRISPR1 des Serotyps Fulica entsprechen) ;
- dem Satz S58-2, umfassend die oder bestehend aus den Sonden der Nucleinsäuresequenzen SEQ ID NO : 2, 4, 5, 1333, 1547 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Fulica entsprechen) ;
- dem Satz S59-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Gueuletapee entsprechen) ;
- dem Satz S59-2, umfassend die oder bestehend aus den Sonden der Nucleinsäuresequenzen SEQ ID NO : 597, 918 bis 925, 933, 1549 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Gueuletapee entsprechen) ;
- dem Satz S60-1, umfassend die oder bestehend aus den Sonden der Nucleinsäuresequenzen SEQ ID NO : 1106 bis 1109, 1111 bis 1116, 1118, 1123, 1128 bis 1131, 1133, 1137 bis 1140, 1150, 1151, 1555 bis 1562 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Heidelberg entsprechen) ;
- dem Satz S60-2, umfassend die oder bestehend aus den Sonden der Nucleinsäuresequenzen SEQ ID NO : 455, 1144, 1145, 1149 bis 1153, 1155 bis 1163, 1172 bis 1174, 1563 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Heidelberg entsprechen) ;
- dem Satz S61-1, umfassend die oder bestehend aus den Sonden der Nucleinsäuresequenzen SEQ ID NO : 558, 1565 bis 1571 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Hessarek entsprechen) ;
- dem Satz S61-2, umfassend die oder bestehend aus den Sonden der Nucleinsäuresequenzen SEQ ID NO : 2 bis 4, 1547, 1572, 1573 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Hessarek entsprechen) ;
- dem Satz S62-1, umfassend die oder bestehend aus den Sonden der Nucleinsäuresequenzen SEQ ID NO : 1591, 1592, 1667 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Itami entsprechen) ;
- dem Satz S62-2, umfassend die oder bestehend aus den Sonden der Nucleinsäuresequenzen SEQ ID NO : 918, 1593 bis 1600, 1744 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Itami entsprechen) ;
- dem Satz S63-1, umfassend die oder bestehend aus den Sonden der Nucleinsäuresequenzen SEQ ID NO : 1601 bis 1606, 1687 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Javiana entsprechen) ;
- dem Satz S63-2, umfassend die oder bestehend aus den Sonden der Nucleinsäuresequenzen SEQ ID NO : 589 bis 601, 973, 1607, 1608 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Javiana entsprechen) ;
- dem Satz S64-1, umfassend die oder bestehend aus den Sonden der Nucleinsäuresequenzen SEQ ID NO : 1609 bis 1621, 1494, 1525 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Johannesburg entsprechen) ;
- dem Satz S64-2, umfassend die oder bestehend aus den Sonden der Nucleinsäuresequenzen SEQ ID NO : 918, 1697, 1698 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Johannesburg entsprechen) ;
- dem Satz S65-1, umfassend die oder bestehend aus den Sonden der Nucleinsäuresequenzen SEQ ID NO : 444, 448, 1184, 1622 bis 1637, oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Kentucky entsprechen) ;
- dem Satz S65-2, umfassend die oder bestehend aus den Sonden der Nucleinsäuresequenzen SEQ ID NO : 385, 519, 1638 bis 1652, 1746 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Kentucky entsprechen) ;
- dem Satz S66-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 67 und/oder ihrer komplementären Nucleinsäuresequenz (Nucleotidsequenzen, die der variablen Sequenz des Locus CRISPR1 des Serotyps Kundunchi entsprechen) ;
- dem Satz S66-2, umfassend die oder bestehend aus den Sonden der Nucleinsäuresequenzen SEQ ID NO : 972, 983, 989, 1201, 1204, 1205, 1210 bis 1212 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Kundunchi entsprechen) ;
- dem Satz S67-1, umfassend die oder bestehend aus den Sonden der Nucleinsäuresequenzen SEQ ID NO : 448, 449, 860, 871, 878 bis 881, 1721, 1724 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Lindenburg entsprechen) ;
- dem Satz S67-2, umfassend die oder bestehend aus den Sonden der Nucleinsäuresequenzen SEQ ID NO : 563, 622, 885, 886, 896, 907, 910 bis 915, 933, 1748, 1757 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Lindenburg entsprechen) ;
- dem Satz S68-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenzen (Nucleotidsequenzen, die den verschiedenen variablen Sequenzen des Locus CRISPR1 des Serotyps Maracaibo entsprechen) ;
- dem Satz S68-2, umfassend die oder bestehend aus den Sonden der Nucleinsäuresequenzen SEQ ID NO : 1240, 1247, 1248, 1654 bis 1656 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Maracaibo entsprechen) ;
- dem Satz S69-1, umfassend die oder bestehend aus den Sonden der Nucleinsäuresequenzen SEQ ID NO : 627, 834, 1120, 1660 bis 1669 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Mississippi entsprechen) ;
- dem Satz S69-2, umfassend die oder bestehend aus den Sonden der Nucleinsäuresequenzen SEQ ID NO : 842, 1670 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Mississippi entsprechen) ;
- dem Satz S70-1, umfassend die oder bestehend aus den Sonden der Nucleinsäuresequenzen SEQ ID NO : 941, 942, 949, 1699 bis 1712, 2109 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Muenchen entsprechen) ;
- dem Satz S70-2, umfassend die oder bestehend aus den Sonden der Nucleinsäuresequenzen SEQ ID NO : 392, 1653 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Muenchen entsprechen) ;
- dem Satz S71-1, umfassend die oder bestehend aus den Sonden der Nucleinsäuresequenzen SEQ ID NO : 11, 1120, 1497, 1760 bis 1775, 1809 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Niarembe entsprechen) ;
- dem Satz S71-2, umfassend die oder bestehend aus den Sonden der Nucleinsäuresequenzen SEQ ID NO : 90, 1776 bis 1785 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Niarembe entsprechen) ;
- dem Satz S72-1, umfassend die oder bestehend aus den Sonden der Nucleinsäuresequenzen SEQ ID NO : 666, 1786 bis 1791 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Overvecht entsprechen) ;
- dem Satz S72-2, umfassend die oder bestehend aus den Sonden der Nucleinsäuresequenzen SEQ ID NO : 783, 1146, 1178, 1179, 1181, 1792 bis 1799, 1801 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Overvecht entsprechen) ;
- dem Satz S73-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Poona entsprechen) ;
- dem Satz S73-2, umfassend die oder bestehend aus den Sonden der Nucleinsäuresequenzen SEQ ID NO : 82, 87, 918, 933, 1826 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Poona entsprechen) ;
- dem Satz S74-1, umfassend die oder bestehend aus den Sonden der Nucleinsäuresequenzen SEQ ID NO : 295 bis 297, 307, 313, 317, 323, 1120, 1825, 1827 bis 1837, 2023 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Postdam entsprechen) ;
- dem Satz S74-2, umfassend die oder bestehend aus den Sonden der Nucleinsäuresequenzen SEQ ID NO : 663, 1304, 1838 bis 1847 oder wenigstens 8 aufeinanderfolgende (n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Postdam entsprechen) ;
- dem Satz S75-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Reading entsprechen) ;
- dem Satz S75-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 225 und/oder ihrer komplementären Nucleinsäuresequenz (Nucleotidsequenzen, die der variablen Sequenz des Locus CRISPR2 des Serotyps Reading entsprechen) ;
- dem Satz S76-1, umfassend die oder bestehend aus den Sonden der Nucleinsäuresequenzen SEQ ID NO : 379, 380, 383 bis 385, 387, 389, 390 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Rosenberg entsprechen) ;
- dem Satz S76-2, umfassend die oder bestehend aus den Sonden der Nucleinsäuresequenzen SEQ ID NO : 392, 393, 404, 407 bis 413 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Rosenberg entsprechen) ;
- dem Satz S77-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 917 und/oder ihrer komplementären Nucleinsäuresequenz (Nucleotidsequenzen, die der variablen Sequenz des Locus CRISPR1 des Serotyps Rubislaw entsprechen) ;
- dem Satz S77-2, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 225 und/oder ihrer komplementären Nucleinsäuresequenz ;
- dem Satz S78-1, umfassend die oder bestehend aus den Sonden der Nucleinsäuresequenzen SEQ ID NO : 1525, 1609 bis 1621 oder wenigstens 8 aufeinanderfolgende(n) Nucleotide(n) dieser Nucleinsäuresequenzen und/oder ihrer komplementären Nucleinsäuresequenzen ;
- dem Satz S78-2, umfassend die oder bestehend aus den Sonden der Nucleinsäuresequenzen SEQ ID NO : 225 bis 227, 229 bis 231 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Sandiego entsprechen) ;
- dem Satz S79-1, umfassend die oder bestehend aus den Sonden der Nucleinsäuresequenzen SEQ ID NO : 1106, 1456, 1917 bis 1924 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Stourbridge entsprechen) ;
- dem Satz S79-2, umfassend die oder bestehend aus den Sonden der Nucleinsäuresequenzen SEQ ID NO : 918, 1093, 1925 bis 1936 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Stourbridge entsprechen) ;
- dem Satz S80-1, umfassend die oder bestehend aus den Sonden der Nucleinsäuresequenzen SEQ ID NO : 1961 bis 1963 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Tallahassee entsprechen) ;
- dem Satz S80-2, umfassend die oder bestehend aus den Sonden der Nucleinsäuresequenzen SEQ ID NO : 25, 603, 2127, 2133, 1964 bis 1966 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Tallahassee entsprechen) ;
- dem Satz S81-1, umfassend die oder bestehend aus den Sonden der Nucleinsäuresequenzen SEQ ID NO : 67, 929, 1967 bis 2005 oder wenigstens 8 aufeinanderfolgende(n) Nucleotide(n) dieser Nucleinsäuresequenzen und/oder ihrer komplementären Nucleinsäuresequenzen ;
- dem Satz S81-2, umfassend die oder bestehend aus den Sonden der Nucleinsäuresequenzen SEQ ID NO : 24, 769, 1550, 1659, 2006 bis 2022 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Tennessee entsprechen) ;
- dem Satz S82-1, umfassend die oder bestehend aus wenigstens einer Sonde, umfassend wenigstens oder bestehend aus wenigstens 8 aufeinanderfolgende(n) Nucleotide(n) der Nucleinsäuresequenz SEQ ID NO : 916 und/oder ihrer komplementären Nucleinsäuresequenz (Nucleotidsequenzen, die der variablen Sequenz des Locus CRISPR1 des Serotyps Urbana entsprechen) ;
- dem Satz S82-2, umfassend die oder bestehend aus den Sonden der Nucleinsäuresequenzen SEQ ID NO : 1697, 1698, 1800 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Urbana entsprechen) ;
- dem Satz S83-1, umfassend die oder bestehend aus den Sonden der Nucleinsäuresequenzen SEQ ID NO : 948, 1120, 1123, 1325, 2039 bis 2078 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Weltevreden entsprechen) ;
- dem Satz S83-2, umfassend die oder bestehend aus den Sonden der Nucleinsäuresequenzen SEQ ID NO : 254 bis 256, 2079 bis 2108 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Weltevreden entsprechen) ;
- dem Satz S84-1, umfassend die oder bestehend aus den Sonden der Nucleinsäuresequenzen SEQ ID NO : 1312, 1690, 2141, 2142 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR1 des Serotyps Zaiman entsprechen) ;
- dem Satz S84-2, umfassend die oder bestehend aus den Sonden der Nucleinsäuresequenzen SEQ ID NO : 1697, 1698, 1800, 1924, 2143, 2144 oder wenigstens 8 aufeinanderfolgende(n) Nucleotide(n) dieser Nucleinsäuresequenzen und/oder ihrer komplementären Nucleinsäuresequenzen (Nucleotidsequenzen, die den verschiedenen variablen Sequenzen des Locus CRISPR2 des Serotyps Zaiman entsprechen).

19. Primersatz, der geeignet ist, ein Fragment der genomischen Sequenz eines Bakteriums der Gattung *Salmonella* zu amplifizieren, **dadurch gekennzeichnet, dass** er ausgewählt ist aus der Gruppe, bestehend aus:
- dem Primersatz, der aus dem Primer der Sequenz SEQ ID NO: 1326, assoziiert mit wenigstens einem der Primer der Sequenz SEQ ID NOs: 1327, 1328, 1329, 2151 und 2152, besteht,
- dem Primersatz, der aus dem Primer der Sequenz SEQ ID NO: 1330, assoziiert mit wenigstens einem der Primer der Sequenz SEQ ID NOs: 1331 und 1332, besteht, und
- dem Primersatz, der aus dem Primer der Sequenz SEQ ID NO: 1326, assoziiert mit wenigstens einem der zwei Primer der Sequenz 1331 und 1332, besteht.

20. DNA-Chip, **dadurch gekennzeichnet, dass** er die Sonden umfasst, die in wenigstens einem der Sondensätze, wie in Anspruch 18 definiert, umfasst sind.

21. Kit oder Paket zur Detektion- oder Identifizierungspaket des molekularen Typs oder Subtyps eines Bakteriums der Gattung *Salmonella,* **dadurch gekennzeichnet, dass** er/sie wenigstens einen Sondensatz, wie in Anspruch 18 definiert, und einen Primersatz gemäß Anspruch 19 und gegebenenfalls einen DNA-Chip gemäß Anspruch 20 umfasst.

22. Verwendung eines Sondensatzes, wie er in Anspruch 18 definiert ist, eines Primersatzes gemäß Anspruch 19 oder eines DNA-Chips gemäß Anspruch 20, um den molekularen Typ und/oder Subtyp eines Bakteriums der Gattung *Salmonella* zu detektieren oder zu identifizieren.

## Claims

1. An in vitro method for molecular typing and/or subtyping of a bacterium of the *Salmonella* genus, using a sample, said method being **characterised in that** it comprises at least the following steps:
(a) amplifying a nucleic acid fragment from a bacterium of the *Salmonella* genus, said fragment comprising the CRISPR1 locus and/or the CRISPR2 locus, using at least one set of primers, which primers have a size of less than or equal to 50 nucleotides, said sets of primers being chosen from the group constituted of:
- a set of primers "A", capable of amplifying a nucleic acid fragment comprising the CRISPR1 locus, comprising at least one forward primer A1 constituted of an oligonucleotide sequence which exhibits at least 70% identity with, or which is identical to, a fragment of the genomic sequence of a bacterium of the *Salmonella* genus located in a region of 1000 bp in a position 5' of the CRISPR1 locus, said fragment of the genomic sequence located in a position 5' of the CRISPR1 locus being of the same size as said primer A1, and at least one reverse primer A2 constituted of an oligonucleotide sequence which exhibits 70% identity with, preferably 80%, 85%, 95%, more preferably 99% identity or which is identical to, a fragment of the genomic sequence complementary to the genomic sequence of a bacterium of the *Salmonella* genus located in a position 3' of the CRISPR1 locus and in a position 5' of the CRISPR2 locus, said complementary genomic sequence fragment being of the same size as said primer A2;
- a set of primers "B", capable of amplifying a nucleic acid fragment comprising the CRISPR2 locus, comprising at least one forward primer B1 constituted of an oligonucleotide sequence which exhibits at least 70% identity with, or which is identical to, a fragment of the genomic sequence of a bacterium of the *Salmonella* genus located in a position 3' of the CRISPR1 locus and in a position 5' of the CRISPR2 locus, said fragment of the genomic sequence located in a position 3' of the CRISPR1 locus and in a position 5' of the CRISPR2 locus being of the same size as said primer B1, and at least one reverse primer B2 constituted of an oligonucleotide sequence which exhibits at least 70% identity with, or which is identical to, a fragment of the genomic sequence complementary to the genomic sequence of a bacterium of the *Salmonella* genus located in a region of 1000 bp in a position 3' of the CRISPR2 locus, said complementary genomic sequence fragment being of the same size as said primer B2;
- a set of primers "C", capable of amplifying a nucleic acid fragment comprising the CRISPR1 locus and the CRISPR2 locus, comprising at least one forward primer A1 as defined above and at least one reverse primer B2 as defined above;
(b) determining the number and the nucleotide sequence of the variable sequences of the CRISPR1 locus and/or the CRISPR2 locus which is (are) amplified in step (a); and
(c) comparing said variable-sequence composition of the loci CRISPR1 and/or CRISPR2 with a reference base which provides the variable-sequence composition of the loci CRISPR1 and CRISPR2 of types and subtypes of bacteria of the *Salmonella* genus, listed in Figs. 10A, 10B and 9, or a part of this base.

2. The method as claimed in claim 1, **characterised in that** the identity of the variable-sequence composition determined in step (b) with respect to a composition appearing in the reference base defined in claim 1, or a part of this base, is indicative of the type and/or of the subtype of the bacterium of the *Salmonella* genus present in the sample.

3. The method as claimed in claim 1 or claim 2, **characterised in that** said primers have a size of between 15 and 30 nucleotides.

4. The method as claimed in any one of claims 1 to 3, **characterised in that** said nucleic acid fragment comprising the CRISPR1 locus and/or the CRISPR2 locus has a size of between 400 and 20 000 bp.

5. The method as claimed in any one of claims 1 to 4, **characterised in that** said fragment of the genomic sequence of a bacterium of the *Salmonella* genus located in a position 5' of the CRISPR1 locus, and/or said fragment of the genomic sequence complementary to the genomic sequence of a bacterium of the *Salmonella* genus located in a position 3' of the CRISPR2 locus, is (are) at a distance from the CRISPR1 locus or the CRISPR2 locus of less than 500 bp, preferably less than 100 bp.

6. The method as claimed in any one of claims 1 to 5, **characterised in that** said genomic sequence of a bacterium of the *Salmonella* genus is chosen from the genomic sequence of *S. enterica* serotype Typhimurium LT2 (genomic sequence available under numbers GI: 16421485 and GI: 16421501 in the GenBank database) or of *S. enterica* serotype Typhi CT18 (genomic sequence available under number GI: 16503805 in the GenBank database).

7. The method as claimed in claim 5, **characterised in that** the set of primers is selected from the group constituted of:
- the set of primers, capable of amplifying the CRISPR1 locus, constituted of the primer of sequence SEQ ID No: 1326, combined with at least one of the primers of sequence SEQ ID Nos: 1327, 1328, 1329, 2151 and 2152,
- the set of primers, capable of amplifying the CRISPR2 locus, constituted of the primer of sequence SEQ ID No: 1330, combined with at least one of the primers of sequence SEQ ID Nos: 1331 and 1332, and
- the set of primers, capable of simultaneously amplifying the two loci CRISPR1 and CRISPR2, constituted of the primer of sequence SEQ ID No: 1326, combined with at least one of the two primers of sequence SEQ ID Nos: 1331 and 1332.

8. The method as claimed in any one of claims 1 to 7, **characterised in that** amplification step (a) is carried out by a method selected from the group constituted of: polymerase chain reaction (PCR), ligase chain reaction (LCR), nucleic acid sequence-based amplification (NASBA), cycling probe technology (CPT), nested PCR and multiplex PCR.

9. The method as claimed in any one of claims 1 to 8, **characterised in that** step (b) is carried out using a DNA sequencing method.

10. The method as claimed in any one of claims 1 to 8, **characterised in that** step (b) is carried out by:
(i) hybridisation with one, several or all the set(s) of probes chosen from:
- set S1-1, comprising or constituted of at least one probe comprising at least or constituted of at least 8 consecutive nucleotides of any one of the nucleic acid sequences SEQ ID Nos: 1106 to 1119, 1123 to 1140, 1142, 1143, 1914 and/or the nucleic acid sequences complementary thereto (nucleotide sequences corresponding to the various variable sequences of the CRISPR1 locus of the serotype Typhimurium);
- set S1-2, comprising or constituted of at least one probe comprising at least or constituted of at least 8 consecutive nucleotides of any one of the nucleic acid sequences SEQ ID Nos: 455, 1144 to 1147, 1149 to 1153, 1155 to 1181 and/or the nucleic acid sequences complementary thereto (nucleotide sequences corresponding to the various variable sequences of the CRISPR2 locus of the serotype Typhimurium);
- set S2-1, comprising or constituted of at least one probe comprising at least or constituted of at least 8 consecutive nucleotides of any one of the nucleic acid sequences SEQ ID Nos: 256, 378 to 381, 383 to 387, 389 to 391, 1106, 1514, 1528, 2148 to 2153 and/or the nucleic acid sequences complementary thereto (nucleotide sequences corresponding to the various variable sequences of the CRISPR1 locus of the serotype Enteritidis);
- set S2-2, comprising or constituted of at least one probe comprising at least or constituted of at least 8 consecutive nucleotides of any one of the nucleic acid sequences SEQ ID Nos: 392 to 413, 519, 815, 972, 983, 989, 1529 to 1546, 1952 and/or the nucleic acid sequences complementary thereto (nucleotide sequences corresponding to the various variable sequences of the CRISPR2 locus of the serotype Enteritidis);
- set S3-1, comprising or constituted of at least one probe comprising at least or constituted of at least 8 consecutive nucleotides of any one of the nucleic acid sequences SEQ ID Nos: 421 to 442, 444, 445, 447 to 450 and/or the nucleic acid sequences complementary thereto (nucleotide sequences corresponding to the various variable sequences of the CRISPR1 locus of the serotype Hadar);
- set S3-2, comprising or constituted of at least one probe comprising at least or constituted of at least 8 consecutive nucleotides of any one of the nucleic acid sequences SEQ ID Nos: 451 to 465, 1144 to 1147, 1150 to 1152, 1154, 1155, 1158, 1169, 1172, 1175 to 1177, 1179, 1181 and/or the nucleic acid sequences complementary thereto (nucleotide sequences corresponding to the various variable sequences of the CRISPR2 locus of the serotype Hadar);
- set S4-1, comprising or constituted of at least one probe comprising at least or constituted of at least 8 consecutive nucleotides of any one of the nucleic acid sequences SEQ ID Nos: 65, 382, 1120, 1263 to 1302, 2024 to 2038 and/or the nucleic acid sequences complementary thereto (nucleotide sequences corresponding to the various variable sequences of the CRISPR1 locus of the serotype Virchow);
- set S4-2, comprising or constituted of at least one probe comprising at least or constituted of at least 8 consecutive nucleotides of any one of the nucleic acid sequences SEQ ID Nos: 83, 93, 94, 1304 to 1324 and/or the nucleic acid sequences complementary thereto (nucleotide sequences corresponding to the various variable sequences of the CRISPR2 locus of the serotype Virchow);
- set S5-1, comprising or constituted of at least one probe comprising at least or constituted of at least 8 consecutive nucleotides of any one of the nucleic acid sequences SEQ ID Nos: 531 to 562, 1106 and/or the nucleic acid sequences complementary thereto (nucleotide sequences corresponding to the various variable sequences of the CRISPR1 locus of the serotype Infantis);
- set S5-2, comprising or constituted of at least one probe comprising at least or constituted of at least 8 consecutive nucleotides of any one of the nucleic acid sequences SEQ ID Nos: 563 to 588 and/or the nucleic acid sequences complementary thereto (nucleotide sequences corresponding to the various variable sequences of the CRISPR2 locus of the serotype Infantis);
- set S6-1, comprising or constituted of at least one probe comprising at least or constituted of at least 8 consecutive nucleotides of any one of the nucleic acid sequences SEQ ID Nos: 1254 to 1259 and/or the nucleic acid sequences complementary thereto (nucleotide sequences corresponding to the various variable sequences of the CRISPR1 locus of the serotype Typhi);
- set S6-2, comprising or constituted of at least one probe comprising at least or constituted of at least 8 consecutive nucleotides of the nucleic acid sequence SEQ ID No: 1260 and/or the complementary nucleic acid sequence thereto (nucleotide sequences corresponding to the variable sequence of the CRISPR2 locus of the serotype Typhi);
- set S7-1, comprising or constituted of at least one probe comprising at least or constituted of at least 8 consecutive nucleotides of any one of the nucleic acid sequences SEQ ID NO : 448, 449, 860 to 876, 878 to 884, 1719 to 1724, 1727 to 1743, 1745 and/or the nucleic acid sequences complementary thereto (nucleotide sequences corresponding to the various variable sequences of the CRISPR1 locus of the serotype Newport);
- set S7-2, comprising or constituted of at least one probe comprising at least or constituted of at least 8 consecutive nucleotides of any one of the nucleic acid sequences SEQ ID NO: 453 to 456, 563, 621, 622, 625, 626, 657, 885 to 896, 906 to 915, 933, 1144, 1145, 1155, 1156, 1172, 1174, 1748 to 1756, 1758, 1759, 1916 and/or the nucleic acid sequences complementary thereto (nucleotide sequences corresponding to the various variable sequences of the CRISPR2 locus of the serotype Newport);
- set S8-1, comprising or constituted of at least one probe comprising at least or constituted of at least 8 consecutive nucleotides of any one of the nucleic acid sequences SEQ ID NO : 295 to 299, 301 to 323, 738, 1106 to 1109, 1120, 1123, 1128 to 1131, 1133, 1137 to 1140, 1550, 1159 to 1561 and/or the nucleic acid sequences complementary thereto (nucleotide sequences corresponding to the various variable sequences of the CRISPR1 locus of the serotype Derby);
- set S8-2, comprising or constituted of at least one probe comprising at least or constituted of at least 8 consecutive nucleotides of any one of the nucleic acid sequences SEQ ID NO : 324 to 337, 455, 1144, 1145, 1149 to 1153, 1155 to 1160, 1163, 1172 to 1174 and/or the nucleic acid sequences complementary thereto (nucleotide sequences corresponding to the various variable sequences of the CRISPR2 locus of the serotype Derby);
- set S9-1, comprising or constituted of at least one probe comprising at least or constituted of at least 8 consecutive nucleotides of any one of the nucleic acid sequences SEQ ID NO : 295, 307, 602 to 614, 616 to 619, 1106, 1120 and/or the nucleic acid sequences complementary thereto (nucleotide sequences corresponding to the various variable sequences of the CRISPR1 locus of the serotype Kottbus);
- set S9-2, comprising or constituted of at least one probe comprising at least or constituted of at least 8 consecutive nucleotides of any one of the nucleic acid sequences SEQ ID NO : 563, 620 to 629, 885, 896, 907, 913, 914, 933 and/or the nucleic acid sequences complementary thereto (nucleotide sequences corresponding to the various variable sequences of the CRISPR2 locus of the serotype Kottbus);
- set S10-1, comprising or constituted of at least one probe comprising at least or constituted of at least 8 consecutive nucleotides of any one of the nucleic acid sequences SEQ ID NO : 422 to 425, 448 to 450, 504 to 518 and/or the nucleic acid sequences complementary thereto (nucleotide sequences corresponding to the various variable sequences of the CRISPR1 locus of the serotype Indiana);
- set S10-2, comprising or constituted of at least one probe comprising at least or constituted of at least 8 consecutive nucleotides of any one of the nucleic acid sequences SEQ ID NO : 246, 258, 269, 392, 415, 519 to 530 and/or the nucleic acid sequences complementary thereto (nucleotide sequences corresponding to the various variable sequences of the CRISPR2 locus of the serotype Indiana);
- set S11-1, comprising or constituted of at least one probe comprising at least or constituted of at least 8 consecutive nucleotides of any one of the nucleic acid sequences SEQ ID NO : 11 to 23, 238, 948, 1120, 1123, 1133 and/or the nucleic acid sequences complementary thereto (nucleotide sequences corresponding to the various variable sequences of the CRISPR1 locus of the serotype Agona);
- set S11-2, comprising or constituted of at least one probe comprising at least or constituted of at least 8 consecutive nucleotides of any one of the nucleic acid sequences SEQ ID NO : 24 to 31 and/or the nucleic acid sequences complementary thereto (nucleotide sequences corresponding to the various variable sequences of the CRISPR2 locus of the serotype Agona);
- set S12-1, comprising or constituted of at least one probe comprising at least or constituted of at least 8 consecutive nucleotides of any one of the nucleic acid sequences SEQ ID NO : 830 to 841, 1120 and/or the nucleic acid sequences complementary thereto (nucleotide sequences corresponding to the various variable sequences of the CRISPR1 locus of the serotype Napoli);
- set S12-2, comprising or constituted of at least one probe comprising at least or constituted of at least 8 consecutive nucleotides of any one of the nucleic acid sequences SEQ ID NO : 842 to 859 and/or the nucleic acid sequences complementary thereto (nucleotide sequences corresponding to the various variable sequences of the CRISPR2 locus of the serotype Napoli);
- set S13-1, comprising or constituted of at least one probe comprising at least or constituted of at least 8 consecutive nucleotides of any one of the nucleic acid sequences SEQ ID NO : 926 to 932 and/or the nucleic acid sequences complementary thereto (nucleotide sequences corresponding to the various variable sequences of the CRISPR1 locus of the serotype Paratyphi A);
- set S13-2, comprising or constituted of at least one probe comprising at least or constituted of at least 8 consecutive nucleotides of any one of the nucleic acid sequences SEQ ID NO : 933, 935, 936 and/or the nucleic acid sequences complementary thereto (nucleotide sequences corresponding to the various variable sequences of the CRISPR2 locus of the serotype Paratyphi A);
- set S14-1, comprising or constituted of at least one probe comprising at least or constituted of at least 8 consecutive nucleotides of any one of the nucleic acid sequences SEQ ID NO : 707, 937 to 971, 1106, 1128 to 1130, 1133, 1138, 1140, 1802 to 1808, 1915 and/or the nucleic acid sequences complementary thereto (nucleotide sequences corresponding to the various variable sequences of the CRISPR1 locus of the serotypes Paratyphi B and Paratyphi B Java);
- set S14-2, comprising or constituted of at least one probe comprising at least or constituted of at least 8 consecutive nucleotides of any one of the nucleic acid sequences SEQ ID NO: 861, 972 to 987, 989 to 992 to 996, 1172 1262, 1462, 1497, 1810 to 1825 and/or the nucleic acid sequences complementary thereto (nucleotide sequences corresponding to the various variable sequences of the CRISPR2 locus of the serotypes Paratyphi B and Paratyphi B Java);
- set S15-1, comprising or constituted of at least one probe comprising at least or constituted of at least 8 consecutive nucleotides of any one of the nucleic acid sequences SEQ ID NO : 232, 238, 239, 997 to 1002, 1106 and/or the nucleic acid sequences complementary thereto (nucleotide sequences corresponding to the various variable sequences of the CRISPR1 locus of the serotype Paratyphi C);
- set S15-2, comprising or constituted of at least one probe comprising at least or constituted of at least 8 consecutive nucleotides of any one of the nucleic acid sequences SEQ ID NO : 280, 290, 291, 392, 393, 415, 1003 to 1006, 1262 and/or the nucleic acid sequences complementary thereto (nucleotide sequences corresponding to the various variable sequences of the CRISPR2 locus of the serotype Paratyphi C);
- set S16-1, comprising or constituted of at least one probe comprising at least or constituted of at least 8 consecutive nucleotides of any one of the nucleic acid sequences SEQ ID NO : 211 to 224, 421, 427, 428, 432, 443 to 445, 447 to 449, 504 and/or the nucleic acid sequences complementary thereto (nucleotide sequences corresponding to the various variable sequences of the CRISPR1 locus of the serotype Bovismorbificans);
- set S16-2, comprising or constituted of at least one probe comprising at least or constituted of at least 8 consecutive nucleotides of any one of the nucleic acid sequences SEQ ID NO : 196 to 209, 994, 1144, 1145, 1172 and/or the nucleic acid sequences complementary thereto (nucleotide sequences corresponding to the various variable sequences of the CRISPR2 locus of the serotype Bovismorbificans);
- set S17-1, comprising or constituted of at least one probe comprising at least or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Panama);
- set S17-2, comprising or constituted of at least one probe comprising at least or constituted of at least 8 consecutive nucleotides of any one of the nucleic acid sequences SEQ ID NO : 918 to 925, 934 and/or the nucleic acid sequences complementary thereto (nucleotide sequences corresponding to the various variable sequences of the CRISPR2 locus of the serotype Panama);
- set S18-1, comprising or constituted of at least one probe comprising at least or constituted of at least 8 consecutive nucleotides of any one of the nucleic acid sequences SEQ ID NO : 11, 16, 67, 74, 442, 444, 446 to 448, 602, 937, 948, 1120, 1128 to 1130, 1182 to 1200, 1937 to 1950 and/or the nucleic acid sequences complementary thereto (nucleotide sequences corresponding to the various variable sequences of the CRISPR1 locus of the serotype Saintpaul);
- set S18-2, comprising or constituted of at least one probe comprising at least or constituted of at least 8 consecutive nucleotides of any one of the nucleic acid sequences SEQ ID NO : 972, 983, 989, 1201 to 1214, 1216 to 1227, 1952 to 1960 and/or the nucleic acid sequences complementary thereto (nucleotide sequences corresponding to the various variable sequences of the CRISPR2 locus of the serotype Saintpaul);
- set S19-1, comprising or constituted of at least one probe comprising at least or constituted of at least 8 consecutive nucleotides of any one of the nucleic acid sequences SEQ ID NO : 514, 1062 to 1089, 1100, 1284, 1886 to 1911 and/or the nucleic acid sequences complementary thereto (nucleotide sequences corresponding to the various variable sequences of the CRISPR1 locus of the serotype Senftenberg);
- set S19-2, comprising or constituted of at least one probe comprising at least or constituted of at least 8 consecutive nucleotides of any one of the nucleic acid sequences SEQ ID NO : 300, 1090 to 1097, 1101 to 1103, 1304, 1315, 1318 to 1320 and/or the nucleic acid sequences complementary thereto (nucleotide sequences corresponding to the various variable sequences of the CRISPR2 locus of the serotype Senftenberg);
- set S20-1, comprising or constituted of at least one probe comprising at least or constituted of at least 8 consecutive nucleotides of any one of the nucleic acid sequences SEQ ID NO : 67, 74 to 80 and/or the nucleic acid sequences complementary thereto (nucleotide sequences corresponding to the various variable sequences of the CRISPR1 locus of the serotype Anatum);
- set S20-2, comprising or constituted of at least one probe comprising at least or constituted of at least 8 consecutive nucleotides of any one of the nucleic acid sequences SEQ ID NO : 83 to 94, 1304, 1315, 1318 to 1322, 1324, 1390 to 1397 and/or the nucleic acid sequences complementary thereto (nucleotide sequences corresponding to the various variable sequences of the CRISPR2 locus of the serotype Anatum);
- set S21-1, comprising or constituted of at least one probe comprising at least or constituted of at least 8 consecutive nucleotides of any one of the nucleic acid sequences SEQ ID NO : 379, 383 to 385, 388 and/or the nucleic acid sequences complementary thereto (nucleotide sequences corresponding to the various variable sequences of the CRISPR1 locus of the serotypes Gallinarum);
- set S21-2, comprising or constituted of at least one probe comprising at least or constituted of at least 8 consecutive nucleotides of any one of the nucleic acid sequences SEQ ID NO : 392, 393, 404, 407 to 415 and/or the nucleic acid sequences complementary thereto (nucleotide sequences corresponding to the various variable sequences of the CRISPR2 locus of the serotypes Gallinarum);
- set S22-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Brandenburg);
- set S22-2, comprising or constituted of at least one probe comprising at least or constituted of at least 8 consecutive nucleotides of any one of the nucleic acid sequences SEQ ID NO : 225 to 231 and/or the nucleic acid sequences complementary thereto (nucleotide sequences corresponding to the various variable sequences of the CRISPR2 locus of the serotype Brandenburg);
- set S23-1, comprising or constituted of at least one probe comprising at least or constituted of at least 8 consecutive nucleotides of any one of the nucleic acid sequences SEQ ID NO : 378, 379 and/or the nucleic acid sequences complementary thereto (nucleotide sequences corresponding to the various variable sequences of the CRISPR1 locus of the serotype Dublin);
- set S23-2, comprising or constituted of at least one probe comprising at least or constituted of at least 8 consecutive nucleotides of any one of the nucleic acid sequences SEQ ID NO : 392, 393, 411, 412 and/or the nucleic acid sequences complementary thereto (nucleotide sequences corresponding to the various variable sequences of the CRISPR2 locus of the serotype Dublin);
- set S24-1, comprising or constituted of at least one probe comprising at least or constituted of at least 8 consecutive nucleotides of any one of the nucleic acid sequences SEQ ID NO : 1, 1106 and/or the nucleic acid sequences complementary thereto (nucleotide sequences corresponding to the various variable sequences of the CRISPR1 locus of the serotype Abortusequi);
- set S24-2, comprising or constituted of at least one probe comprising at least or constituted of at least 8 consecutive nucleotides of any one of the nucleic acid sequences SEQ ID NO: 2 to 10, 130 and/or the nucleic acid sequences complementary thereto (nucleotide sequences corresponding to the various variable sequences of the CRISPR2 locus of the serotype Abortusequi);
- set S25-1, comprising or constituted of at least one probe comprising at least or constituted of at least 8 consecutive nucleotides of any one of the nucleic acid sequences SEQ ID NO : 378, 379 and/or the nucleic acid sequences complementary thereto (nucleotide sequences corresponding to the various variable sequences of the CRISPR1 locus of the serotype Kiel);
- set S25-2, comprising or constituted of at least one probe comprising at least or constituted of at least 8 consecutive nucleotides of any one of the nucleic acid sequences SEQ ID NO : 392, 393, 411, 412 and/or the nucleic acid sequences complementary thereto (nucleotide sequences corresponding to the various variable sequences of the CRISPR2 locus of the serotype Kiel);
- set S26-1, comprising or constituted of at least one probe comprising at least or constituted of at least 8 consecutive nucleotides of any one of the nucleic acid sequences SEQ ID NO : 509, 510, 512, 513, 637 to 660, 1086, 1107 and/or the nucleic acid sequences complementary thereto (nucleotide sequences corresponding to the various variable sequences of the CRISPR1 locus of the serotype Mbandaka);
- set S26-2, comprising or constituted of at least one probe comprising at least or constituted of at least 8 consecutive nucleotides of any one of the nucleic acid sequences SEQ ID NO : 661 to 666, 668, 669, 672, 683, 690 to 695, 731, 918 and/or the nucleic acid sequences complementary thereto (nucleotide sequences corresponding to the various variable sequences of the CRISPR2 locus of the serotype Mbandaka);
- set S27-1, comprising or constituted of at least one probe comprising at least or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Miami);
- set S27-2, comprising or constituted of at least one probe comprising at least or constituted of at least 8 consecutive nucleotides of any one of the nucleic acid sequences SEQ ID NO : 25, 696 to 700 and/or the nucleic acid sequences complementary thereto (nucleotide sequences corresponding to the various variable sequences of the CRISPR2 locus of the serotype Miami);
- set S28-1, comprising or constituted of at least one probe comprising at least or constituted of at least 8 consecutive nucleotides of any one of the nucleic acid sequences SEQ ID NO : 55, 701 to 730, 732 to 768, 1115, 1228, 1232 to 1237, 1601, 1671 to 1680, 1682 to 1694, 1747 and/or the nucleic acid sequences complementary thereto (nucleotide sequences corresponding to the various variable sequences of the CRISPR1 locus of the serotype Montevideo);
- set S28-2, comprising or constituted of at least one probe comprising at least or constituted of at least 8 consecutive nucleotides of any one of the nucleic acid sequences SEQ ID NO : 589, 769 to 829, 933, 1245, 1697, 1698 and/or the nucleic acid sequences complementary thereto (nucleotide sequences corresponding to the various variable sequences of the CRISPR2 locus of the serotype Montevideo);
- set S29-1, comprising or constituted of at least one probe comprising at least or constituted of at least 8 consecutive nucleotides of any one of the nucleic acid sequences SEQ ID NO : 379, 380, 383 to 385, 387, 389 to 391 and/or the nucleic acid sequences complementary thereto (nucleotide sequences corresponding to the various variable sequences of the CRISPR1 locus of the serotype Nitra);
- set S29-2, comprising or constituted of at least one probe comprising at least or constituted of at least 8 consecutive nucleotides of any one of the nucleic acid sequences SEQ ID NO : 392 to 394, 404, 407 to 413, 519 and/or the nucleic acid sequences complementary thereto (nucleotide sequences corresponding to the various variable sequences of the CRISPR2 locus of the serotype Nitra);
- set S30-1, comprising or constituted of at least one probe comprising at least or constituted of at least 8 consecutive nucleotides of any one of the nucleic acid sequences SEQ ID NO : 931, 932, 1104, 1105 and/or the nucleic acid sequences complementary thereto (nucleotide sequences corresponding to the various variable sequences of the CRISPR1 locus of the serotype Sendai);
- set S30-2, comprising or constituted of at least one probe comprising at least or constituted of at least 8 consecutive nucleotides of the nucleic acid sequence SEQ ID NO : 936 and/or the nucleic acid sequence complementary thereto (nucleotide sequences corresponding to the variable sequence of the CRISPR2 locus of the serotype Sendai);
- set S31-1, comprising or constituted of at least one probe comprising at least or constituted of at least 8 consecutive nucleotides of any one of the nucleic acid sequences SEQ ID NO : 714 to 716, 1228 to 1239 and/or the nucleic acid sequences complementary thereto (nucleotide sequences corresponding to the various variable sequences of the CRISPR1 locus of the serotype Schwarzengrund);
- set S31-2, comprising or constituted of at least one probe comprising at least or constituted of at least 8 consecutive nucleotides of any one of the nucleic acid sequences SEQ ID NO : 196, 992, 995, 1172, 1240 to 1244, 1246 to 1253 and/or the nucleic acid sequences complementary thereto (nucleotide sequences corresponding to the various variable sequences of the CRISPR2 locus of the serotype Schwarzengrund);
- set S32-1, comprising or constituted of at least one probe comprising at least or constituted of at least 8 consecutive nucleotides of any one of the nucleic acid sequences SEQ ID NO : 232, 238, 239, 998, 999, 1002, 1106, 1261 and/or the nucleic acid sequences complementary thereto (nucleotide sequences corresponding to the various variable sequences of the CRISPR1 locus of the serotype Typhisuis);
- set S32-2, comprising or constituted of at least one probe comprising at least or constituted of at least 8 consecutive nucleotides of the nucleic acid sequence SEQ ID NO : 1262 and/or the nucleic acid sequence complementary thereto (nucleotide sequences corresponding to the variable sequence of the CRISPR2 locus of the serotype Typhisuis);
- set S33-1, comprising or constituted of at least one probe comprising at least or constituted of at least 8 consecutive nucleotides of any one of the nucleic acid sequences SEQ ID NO : 466 to 486 and/or the nucleic acid sequences complementary thereto (nucleotide sequences corresponding to the various variable sequences of the CRISPR1 locus of the different serotypes of the subspecies *houtenae);*
- set S33-2, comprising or constituted of at least one probe comprising at least or constituted of at least 8 consecutive nucleotides of any one of the nucleic acid sequences SEQ ID NO : 487 to 490 and/or the nucleic acid sequences complementary thereto (nucleotide sequences corresponding to the various variable sequences of the CRISPR2 locus of the different serotypes of the subspecies *houtenae);*
- set S34-1, comprising or constituted of at least one probe comprising at least or constituted of at least 8 consecutive nucleotides of any one of the nucleic acid sequences SEQ ID NO : 338 to 376 and/or the nucleic acid sequences complementary thereto (nucleotide sequences corresponding to the various variable sequences of the CRISPR1 locus of the different serotypes of the subspecies *diarizonae);*
- set S34-2, comprising or constituted of at least one probe comprising at least or constituted of at least 8 consecutive nucleotides of the nucleic acid sequence SEQ ID NO : 377 and/or the nucleic acid sequence complementary thereto (nucleotide sequences corresponding to the variable sequence of the CRISPR2 locus of the different serotypes of the subspecies *diarizonae);*
- set S35-1, comprising or constituted of at least one probe comprising at least or constituted of at least 8 consecutive nucleotides of any one of the nucleic acid sequences SEQ ID NO : 1007 to 1037, 1848 to 1884 and/or the nucleic acid sequences complementary thereto (nucleotide sequences corresponding to the various variable sequences of the CRISPR1 locus of the subspecies *salamae*);
- set S35-2, comprising or constituted of at least one probe comprising at least or constituted of at least 8 consecutive nucleotides of any one of the nucleic acid sequences SEQ ID NO : 1038 to 1043, 1045 to 1061, 1885 and/or the nucleic acid sequences complementary thereto (nucleotide sequences corresponding to the various variable sequences of the CRISPR2 locus of the subspecies *salamae*);
- set S36, comprising or constituted of at least one probe comprising at least or constituted of at least 8 consecutive nucleotides of the nucleic acid sequence SEQ ID NO : 95 and/or the nucleic acid sequence complementary thereto (nucleotide sequences corresponding to the variable sequence of the CRISPR1 locus of the different serotypes of the subspecies *arizonae*);
- set S37-1, comprising or constituted of at least one probe comprising at least or constituted of at least 8 consecutive nucleotides of any one of the nucleic acid sequences SEQ ID NO : 491 to 499 and/or the nucleic acid sequences complementary thereto (nucleotide sequences corresponding to the various variable sequences of the CRISPR1 locus of the different serotypes of the subspecies *indica);*
- set S37-2, comprising or constituted of at least one probe comprising at least or constituted of at least 8 consecutive nucleotides of any one of the nucleic acid sequences SEQ ID NO : 500 to 503, 1574 to 1589 and/or the nucleic acid sequences complementary thereto (nucleotide sequences corresponding to the various variable sequences of the CRISPR2 locus of the different serotypes of the subspecies *indica);*
- set S38-1, comprising or constituted of at least one probe comprising at least or constituted of at least 8 consecutive nucleotides of any one of the nucleic acid sequences SEQ ID NO : 32 to 66, 382, 416, 667, 2036 and/or the nucleic acid sequences complementary thereto (nucleotide sequences corresponding to the various variable sequences of the CRISPR1 locus of the serotype Altona);
- set S38-2, comprising or constituted of at least one probe comprising at least or constituted of at least 8 consecutive nucleotides of any one of the nucleic acid sequences SEQ ID NO : 854, 933, 1095, 1306, 1354 to 1389, 1696 and/or the nucleic acid sequences complementary thereto (nucleotide sequences corresponding to the various variable sequences of the CRISPR2 locus of the serotype Altona);
- set S39-1, comprising or constituted of at least one probe comprising at least or constituted of at least 8 consecutive nucleotides of any one of the nucleic acid sequences SEQ ID NO : 240, 241, 1120, 1121 and/or the nucleic acid sequences complementary thereto (nucleotide sequences corresponding to the various variable sequences of the CRISPR1 locus of the serotype Choleraesuis variety Decatur);
- set S39-2, comprising or constituted of at least one probe comprising at least or constituted of at least 8 consecutive nucleotides of any one of the nucleic acid sequences SEQ ID NO : 225, 247 to 251, 253 to 256, 259 to 268, 270 to 279, 281 to 289, 292 to 294 and/or the nucleic acid sequences complementary thereto (nucleotide sequences corresponding to the various variable sequences of the CRISPR2 locus of the serotype Choleraesuis variety Decatur);
- set S40-1, comprising or constituted of at least one probe comprising at least or constituted of at least 8 consecutive nucleotides of any one of the nucleic acid sequences SEQ ID NO : 11, 232, 238, 239, 240, 242 to 245, 233 to 237, 959, 1106, 1120 and/or the nucleic acid sequences complementary thereto (nucleotide sequences corresponding to the various variable sequences of the CRISPR1 locus of the serotype Choleraesuis variety Kunzendorf);
- set S40-2, comprising or constituted of at least one probe comprising at least or constituted of at least 8 consecutive nucleotides of any one of the nucleic acid sequences SEQ ID NO : 246, 252, 258, 269, 280, 290, 291, 392, 563, 933 and/or the nucleic acid sequences complementary thereto (nucleotide sequences corresponding to the various variable sequences of the CRISPR2 locus of the serotype Choleraesuis variety Kunzendorf);
- set S41-1, comprising or constituted of at least one probe comprising at least or constituted of at least 8 consecutive nucleotides of any one of the nucleic acid sequences SEQ ID NO : 11, 242 to 245, 232 to 239, 959, 1000, 1106, 1139, 1120 and/or the nucleic acid sequences complementary thereto (nucleotide sequences corresponding to the various variable sequences of the CRISPR1 locus of the serotypes Choleraesuis and Choleraesuis sensu stricto);
- set S41-2, comprising or constituted of at least one probe comprising at least or constituted of at least 8 consecutive nucleotides of any one of the nucleic acid sequences SEQ ID NO : 246, 252, 258, 269, 280, 290, 291, 392, 563, 574, 933, 1003, 1004 and/or the nucleic acid sequences complementary thereto (nucleotide sequences corresponding to the various variable sequences of the CRISPR2 locus of the serotypes Choleraesuis and Choleraesuis sensu stricto);
- set S42-1, comprising or constituted of at least one probe comprising at least or constituted of at least 8 consecutive nucleotides of any one of the nucleic acid sequences SEQ ID NO : 416 to 420, 1122 and/or the nucleic acid sequences complementary thereto (nucleotide sequences corresponding to the various variable sequences of the CRISPR1 locus of the serotype Goettingen);
- set S42-2, comprising or constituted of at least one probe comprising at least or constituted of at least 8 consecutive nucleotides of the nucleic acid sequence SEQ ID NO : 1548 and/or the nucleic acid sequence complementary thereto (nucleotide sequences corresponding to the variable sequence of the CRISPR2 locus of the serotype Goettingen);
- set S43-1, comprising or constituted of at least one probe comprising at least or constituted of at least 8 consecutive nucleotides of any one of the nucleic acid sequences SEQ ID NO : 630 to 636, 937, 948, 1120, 1143 and/or the nucleic acid sequences complementary thereto (nucleotide sequences corresponding to the various variable sequences of the CRISPR1 locus of the serotype Manhattan);
- set S43-2, comprising or constituted of at least one probe comprising at least or constituted of at least 8 consecutive nucleotides of any one of the nucleic acid sequences SEQ ID NO : 392, 1653 and/or the nucleic acid sequences complementary thereto (nucleotide sequences corresponding to the various variable sequences of the CRISPR2 locus of the serotype Manhattan);
- set S44-1, comprising or constituted of at least one probe comprising at least or constituted of at least 8 consecutive nucleotides of any one of the nucleic acid sequences SEQ ID NO : 96 to 155 and/or the nucleic acid sequences complementary thereto (nucleotide sequences corresponding to the various variable sequences of the CRISPR1 locus of certain serotypes of the species *S. bongori*);
- set S44-2, comprising or constituted of at least one probe comprising at least or constituted of at least 8 consecutive nucleotides of any one of the nucleic acid sequences SEQ ID NO : 156 to 195, 1421 to 1430 and/or the nucleic acid sequences complementary thereto (nucleotide sequences corresponding to the various variable sequences of the CRISPR2 locus of certain serotypes of the species *S. bongori);*
- set S45-1, comprising or constituted of at least one probe comprising at least or constituted of at least 8 consecutive nucleotides of any one of the nucleic acid sequences SEQ ID NO : 962, 1106, 1133, 1804, 1808 and/or the nucleic acid sequences complementary thereto (nucleotide sequences corresponding to the various variable sequences of the CRISPR1 locus of the serotype Abony);
- set S45-2, comprising or constituted of at least one probe comprising at least or constituted of at least 8 consecutive nucleotides of any one of the nucleic acid sequences SEQ ID NO : 972, 989, 1334 to 1339, 1474, 1499 and/or the nucleic acid sequences complementary thereto (nucleotide sequences corresponding to the various variable sequences of the CRISPR2 locus of the serotype Abony);
- set S46-1, comprising or constituted of at least one probe comprising at least or constituted of at least 8 consecutive nucleotides of any one of the nucleic acid sequences SEQ ID NO : 448, 860 to 875, 882, 883, 1340 to 1345, 1721 to 1723 and/or the nucleic acid sequences complementary thereto (nucleotide sequences corresponding to the various variable sequences of the CRISPR1 locus of the serotype Aesch);
- set S46-2, comprising or constituted of at least one probe comprising at least or constituted of at least 8 consecutive nucleotides of any one of the nucleic acid sequences SEQ ID NO : 563, 625, 626, 885, 887 to 893, 896, 933, 1346 to 1348, 1749, 1759 and/or the nucleic acid sequences complementary thereto (nucleotide sequences corresponding to the various variable sequences of the CRISPR2 locus of the serotype Aesch);
- set S47-1, comprising or constituted of at least one probe comprising at least or constituted of at least 8 consecutive nucleotides of any one of the nucleic acid sequences SEQ ID NO: 834, 1120, 1349, 1350, 1351, 1713, 1718 and/or the nucleic acid sequences complementary thereto (nucleotide sequences corresponding to the various variable sequences of the CRISPR1 locus of the serotype Albany);
- set S47-2, comprising or constituted of at least one probe comprising at least or constituted of at least 8 consecutive nucleotides of any one of the nucleic acid sequences SEQ ID NO : 842, 852, 1352, 1353 and/or the nucleic acid sequences complementary thereto (nucleotide sequences corresponding to the various variable sequences of the CRISPR2 locus of the serotype Albany);
- set S48-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Arechavalata);
- set S48-2, comprising or constituted of at least one probe comprising at least or constituted of at least 8 consecutive nucleotides of any one of the nucleic acid sequences SEQ ID NO : 225, 227 to 231, 1398 and/or the nucleic acid sequences complementary thereto (nucleotide sequences corresponding to the various variable sequences of the CRISPR2 locus of the serotype Arechavalata);
- set S49-1, comprising or constituted of at least one probe comprising at least or constituted of at least 8 consecutive nucleotides of any one of the nucleic acid sequences SEQ ID NO : 448, 449, 860, 871 to 873, 878, 895, 903 to 905, 907, 1719 to 1721, 1745 and/or the nucleic acid sequences complementary thereto (nucleotide sequences corresponding to the various variable sequences of the CRISPR1 locus of the serotype Bardo);
- set S49-2, comprising or constituted of at least one probe comprising at least or constituted of at least 8 consecutive nucleotides of any one of the nucleic acid sequences SEQ ID NO : 563, 622, 626, 885, 886 to 890, 893, 896, 907, 910 to 915, 933, 1756 to 1758 and/or the nucleic acid sequences complementary thereto (nucleotide sequences corresponding to the various variable sequences of the CRISPR2 locus of the serotype Bardo);
- set S50-1, comprising or constituted of at least one probe comprising at least or constituted of at least 8 consecutive nucleotides of any one of the nucleic acid sequences SEQ ID NO : 82, 1401 and/or the nucleic acid sequences complementary thereto (nucleotide sequences corresponding to the various variable sequences of the CRISPR1 locus of the serotype Berta);
- set S50-2, comprising or constituted of at least one probe comprising at least or constituted of at least 8 consecutive nucleotides of any one of the nucleic acid sequences SEQ ID NO : 246, 918, 933, 1401, 1448 and/or the nucleic acid sequences complementary thereto (nucleotide sequences corresponding to the various variable sequences of the CRISPR2 locus of the serotype Berta);
- set S51-1, comprising or constituted of at least one probe comprising at least or constituted of at least 8 consecutive nucleotides of any one of the nucleic acid sequences SEQ ID NO : 1, 37, 1106, 1402 to 1416, 2055 and/or the nucleic acid sequences complementary thereto (nucleotide sequences corresponding to the various variable sequences of the CRISPR1 locus of the serotype Bispebjerg);
- set S51-2, comprising or constituted of at least one probe comprising at least or constituted of at least 8 consecutive nucleotides of any one of the nucleic acid sequences SEQ ID NO : 2 to 4, 9, 1417 to 1420, 1547, 1572 and/or the nucleic acid sequences complementary thereto (nucleotide sequences corresponding to the various variable sequences of the CRISPR2 locus of the serotype Bispebjerg);
- set S52-1, comprising or constituted of at least one probe comprising at least or constituted of at least 8 consecutive nucleotides of any one of the nucleic acid sequences SEQ ID NO : 379, 380, 383, 385, 387, 389 to 391 and/or the nucleic acid sequences complementary thereto (nucleotide sequences corresponding to the various variable sequences of the CRISPR1 locus of the serotype Blegdam);
- set S52-2, comprising or constituted of at least one probe comprising at least or constituted of at least 8 consecutive nucleotides of any one of the nucleic acid sequences SEQ ID NO : 392 to 394, 404, 407, 412, 413, 519 and/or the nucleic acid sequences complementary thereto (nucleotide sequences corresponding to the various variable sequences of the CRISPR2 locus of the serotype Blegdam);
- set S53-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Chester);
- set S53-2, comprising or constituted of at least one probe comprising at least or constituted of at least 8 consecutive nucleotides of any one of the nucleic acid sequences SEQ ID NO : 933, 1445 to 1447 and/or the nucleic acid sequences complementary thereto (nucleotide sequences corresponding to the various variable sequences of the CRISPR2 locus of the serotype Chester);
- set S54-1, comprising or constituted of at least one probe comprising at least or constituted of at least 8 consecutive nucleotides of any one of the nucleic acid sequences SEQ ID NO : 1449 to 1454, 1460, 1467 to 1473 and/or the nucleic acid sequences complementary thereto (nucleotide sequences corresponding to the various variable sequences of the CRISPR1 locus of the serotype Concord);
- set S54-2, comprising or constituted of at least one probe comprising at least or constituted of at least 8 consecutive nucleotides of any one of the nucleic acid sequences SEQ ID NO : 972, 1474 to 1493, 1495 to 1499 and/or the nucleic acid sequences complementary thereto (nucleotide sequences corresponding to the various variable sequences of the CRISPR2 locus of the serotype Concord);
- set S55-1, comprising or constituted of at least one probe comprising at least or constituted of at least 8 consecutive nucleotides of any one of the nucleic acid sequences SEQ ID NO : 1106, 1107 to 1109, 1123, 1128 to 1131, 1133, 1137 to 1140, 1550, 1559 to 1561 and/or the nucleic acid sequences complementary thereto (nucleotide sequences corresponding to the various variable sequences of the CRISPR1 locus of the serotype Crossness);
- set S55-2, comprising or constituted of at least one probe comprising at least or constituted of at least 8 consecutive nucleotides of any one of the nucleic acid sequences SEQ ID NO : 455, 1144, 1145, 1149 to 1153, 1155 to 1160, 1163, 1172 to 1174 and/or the nucleic acid sequences complementary thereto (nucleotide sequences corresponding to the various variable sequences of the CRISPR2 locus of the serotype Crossness);
- set S56-1, comprising or constituted of at least one probe comprising at least or constituted of at least 8 consecutive nucleotides of any one of the nucleic acid sequences SEQ ID NO : 834, 1120, 1349 to 1351, 1713, 1718 and/or the nucleic acid sequences complementary thereto (nucleotide sequences corresponding to the various variable sequences of the CRISPR1 locus of the serotype Duesseldorf);
- set S56-2, comprising or constituted of at least one probe comprising at least or constituted of at least 8 consecutive nucleotides of any one of the nucleic acid sequences SEQ ID NO : 842, 852, 1352, 1353, 1501 to 1503 and/or the nucleic acid sequences complementary thereto (nucleotide sequences corresponding to the various variable sequences of the CRISPR2 locus of the serotype Duesseldorf);
- set S57-1, comprising or constituted of at least one probe comprising at least or constituted of at least 8 consecutive nucleotides of any one of the nucleic acid sequences SEQ ID NO : 941, 1504 to 1508 and/or the nucleic acid sequences complementary thereto (nucleotide sequences corresponding to the various variable sequences of the CRISPR1 locus of the serotype Emek);
- set S57-2, comprising or constituted of at least one probe comprising at least or constituted of at least 8 consecutive nucleotides of any one of the nucleic acid sequences SEQ ID NO : 1260, 1509 to 1524, 1526, 1527 and/or the nucleic acid sequences complementary thereto (nucleotide sequences corresponding to the various variable sequences of the CRISPR2 locus of the serotype Emek);
- set S58-1, comprising or constituted of at least one probe comprising at least or constituted of at least 8 consecutive nucleotides of the nucleic acid sequence SEQ ID NO : 558 and/or the nucleic acid sequence complementary thereto (nucleotide sequences corresponding to the variable sequence of the CRISPR1 locus of the serotype Fulica);
- set S58-2, comprising or constituted of at least one probe comprising at least or constituted of at least 8 consecutive nucleotides of any one of the nucleic acid sequences SEQ ID NO : 2, 4, 5, 1333, 1547 and/or the nucleic acid sequences complementary thereto (nucleotide sequences corresponding to the various variable sequences of the CRISPR2 locus of the serotype Fulica);
- set S59-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Gueuletapee);
- set S59-2, comprising or constituted of at least one probe comprising at least or constituted of at least 8 consecutive nucleotides of any one of the nucleic acid sequences SEQ ID NO : 597, 918 to 925, 933, 1549 and/or the nucleic acid sequences complementary thereto (nucleotide sequences corresponding to the various variable sequences of the CRISPR2 locus of the serotype Gueuletapee);
- set S60-1, comprising or constituted of at least one probe comprising at least or constituted of at least 8 consecutive nucleotides of any one of the nucleic acid sequences SEQ ID NO : 1106 to 1109, 1111 to 1116, 1118, 1123, 1128 to 1131, 1133, 1137 to 1140, 1150, 1151, 1555 to 1562 and/or the nucleic acid sequences complementary thereto (nucleotide sequences corresponding to the various variable sequences of the CRISPR1 locus of the serotype Heidelberg);
- set S60-2, comprising or constituted of at least one probe comprising at least or constituted of at least 8 consecutive nucleotides of any one of the nucleic acid sequences SEQ ID NO : 455, 1144, 1145, 1149 to 1153, 1155 to 1163, 1172 to 1174, 1563 and/or the nucleic acid sequences complementary thereto (nucleotide sequences corresponding to the various variable sequences of the CRISPR2 locus of the serotype Heidelberg);
- set S61-1, comprising or constituted of at least one probe comprising at least or constituted of at least 8 consecutive nucleotides of any one of the nucleic acid sequences SEQ ID NO : 558, 1565 to 1571 and/or the nucleic acid sequences complementary thereto (nucleotide sequences corresponding to the various variable sequences of the CRISPR1 locus of the serotype Hessarek);
- set S61-2, comprising or constituted of at least one probe comprising at least or constituted of at least 8 consecutive nucleotides of any one of the nucleic acid sequences SEQ ID NO : 2 to 4, 1547, 1572, 1573 and/or the nucleic acid sequences complementary thereto (nucleotide sequences corresponding to the various variable sequences of the CRISPR2 locus of the serotype Hessarek);
- set S62-1, comprising or constituted of at least one probe comprising at least or constituted of at least 8 consecutive nucleotides of any one of the nucleic acid sequences SEQ ID NO : 1591, 1592, 1667 and/or the nucleic acid sequences complementary thereto (nucleotide sequences corresponding to the various variable sequences of the CRISPR1 locus of the serotype Itami);
- set S62-2, comprising or constituted of at least one probe comprising at least or constituted of at least 8 consecutive nucleotides of any one of the nucleic acid sequences SEQ ID NO : 918, 1593 to 1600, 1744 and/or the nucleic acid sequences complementary thereto (nucleotide sequences corresponding to the various variable sequences of the CRISPR2 locus of the serotype Itami);
- set S63-1, comprising or constituted of at least one probe comprising at least or constituted of at least 8 consecutive nucleotides of any one of the nucleic acid sequences SEQ ID NO : 1601 to 1606, 1687 and/or the nucleic acid sequences complementary thereto (nucleotide sequences corresponding to the various variable sequences of the CRISPR1 locus of the serotype Javiana);
- set S63-2, comprising or constituted of at least one probe comprising at least or constituted of at least 8 consecutive nucleotides of any one of the nucleic acid sequences SEQ ID NO: 589 to 601, 973, 1607, 1608 and/or the nucleic acid sequences complementary thereto (nucleotide sequences corresponding to the various variable sequences of the CRISPR2 locus of the serotype Javiana);
- set S64-1, comprising or constituted of at least one probe comprising at least or constituted of at least 8 consecutive nucleotides of any one of the nucleic acid sequences SEQ ID NO : 1609 to 1621, 1494, 1525 and/or the nucleic acid sequences complementary thereto (nucleotide sequences corresponding to the various variable sequences of the CRISPR1 locus of the serotype Johannesburg);
- set S64-2, comprising or constituted of at least one probe comprising at least or constituted of at least 8 consecutive nucleotides of any one of the nucleic acid sequences SEQ ID NO : 918, 1697, 1698 and/or the nucleic acid sequences complementary thereto (nucleotide sequences corresponding to the various variable sequences of the CRISPR2 locus of the serotype Johannesburg);
- set S65-1, comprising or constituted of at least one probe comprising at least or constituted of at least 8 consecutive nucleotides of any one of the nucleic acid sequences SEQ ID NO: 444, 448, 1184, 1622 to 1637 and/or the nucleic acid sequences complementary thereto (nucleotide sequences corresponding to the various variable sequences of the CRISPR1 locus of the serotype Kentucky);
- set S65-2, comprising or constituted of at least one probe comprising at least or constituted of at least 8 consecutive nucleotides of any one of the nucleic acid sequences SEQ ID NO : 385, 519, 1638 to 1652, 1746 and/or the nucleic acid sequences complementary thereto (nucleotide sequences corresponding to the various variable sequences of the CRISPR2 locus of the serotype Kentucky);
- set S66-1, comprising or constituted of at least one probe comprising at least or constituted of at least 8 consecutive nucleotides of the nucleic acid sequence SEQ ID NO : 67 and/or the nucleic acid sequence complementary thereto (nucleotide sequences corresponding to the variable sequence of the CRISPR1 locus of the serotype Kundunchi);
- set S66-2, comprising or constituted of at least one probe comprising at least or constituted of at least 8 consecutive nucleotides of any one of the nucleic acid sequences SEQ ID NO : 972, 983, 989, 1201, 1204, 1205, 1210 to 1212 and/or the nucleic acid sequences complementary thereto (nucleotide sequences corresponding to the various variable sequences of the CRISPR2 locus of the serotype Kundunchi);
- set S67-1, comprising or constituted of at least one probe comprising at least or constituted of at least 8 consecutive nucleotides of any one of the nucleic acid sequences SEQ ID NO : 448, 449, 860, 871, 878 to 881, 1721, 1724 and/or the nucleic acid sequences complementary thereto (nucleotide sequences corresponding to the various variable sequences of the CRISPR1 locus of the serotype Lindenburg);
- set S67-2, comprising or constituted of at least one probe comprising at least or constituted of at least 8 consecutive nucleotides of any one of the nucleic acid sequences SEQ ID NO : 563, 622, 885, 886, 896, 907, 910 to 915, 933, 1748, 1757 and/or the nucleic acid sequences complementary thereto (nucleotide sequences corresponding to the various variable sequences of the CRISPR2 locus of the serotype Lindenburg);
- set S68-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Maracaibo);
- set S68-2, comprising or constituted of at least one probe comprising at least or constituted of at least 8 consecutive nucleotides of any one of the nucleic acid sequences SEQ ID NO : 1240, 1247, 1248, 1654 to 1656 and/or the nucleic acid sequences complementary thereto (nucleotide sequences corresponding to the various variable sequences of the CRISPR2 locus of the serotype Maracaibo);
- set S69-1, comprising or constituted of at least one probe comprising at least or constituted of at least 8 consecutive nucleotides of any one of the nucleic acid sequences SEQ ID NO : 627, 834, 1120, 1660 to 1669 and/or the nucleic acid sequences complementary thereto (nucleotide sequences corresponding to the various variable sequences of the CRISPR1 locus of the serotype Mississippi);
- set S69-2, comprising or constituted of at least one probe comprising at least or constituted of at least 8 consecutive nucleotides of any one of the nucleic acid sequences SEQ ID NO : 842, 1670 and/or the nucleic acid sequences complementary thereto (nucleotide sequences corresponding to the various variable sequences of the CRISPR2 locus of the serotype Mississippi);
- set S70-1, comprising or constituted of at least one probe comprising at least or constituted of at least 8 consecutive nucleotides of any one of the nucleic acid sequences SEQ ID NO : 941, 942, 949, 1699 to 1712, 2109 and/or the nucleic acid sequences complementary thereto (nucleotide sequences corresponding to the various variable sequences of the CRISPR1 locus of the serotype Muenchen);
- set S70-2, comprising or constituted of at least one probe comprising at least or constituted of at least 8 consecutive nucleotides of any one of the nucleic acid sequences SEQ ID NO : 392, 1653 and/or the nucleic acid sequences complementary thereto (nucleotide sequences corresponding to the various variable sequences of the CRISPR2 locus of the serotype Muenchen);
- set S71-1, comprising or constituted of at least one probe comprising at least or constituted of at least 8 consecutive nucleotides of any one of the nucleic acid sequences SEQ ID NO : 11, 1120, 1497, 1760 to 1775, 1809 and/or the nucleic acid sequences complementary thereto (nucleotide sequences corresponding to the various variable sequences of the CRISPR1 locus of the serotype Niarembe);
- set S71-2, comprising or constituted of at least one probe comprising at least or constituted of at least 8 consecutive nucleotides of any one of the nucleic acid sequences SEQ ID NO : 90, 1776 to 1785 and/or the nucleic acid sequences complementary thereto (nucleotide sequences corresponding to the various variable sequences of the CRISPR2 locus of the serotype Niarembe);
- set S72-1, comprising or constituted of at least one probe comprising at least or constituted of at least 8 consecutive nucleotides of any one of the nucleic acid sequences SEQ ID NO : 666, 1786 to 1791 and/or the nucleic acid sequences complementary thereto (nucleotide sequences corresponding to the various variable sequences of the CRISPR1 locus of the serotype Overvecht);
- set S72-2, comprising or constituted of at least one probe comprising at least or constituted of at least 8 consecutive nucleotides of any one of the nucleic acid sequences SEQ ID NO : 783, 1146, 1178, 1179, 1181, 1792 to 1799, 1801 and/or the nucleic acid sequences complementary thereto (nucleotide sequences corresponding to the various variable sequences of the CRISPR2 locus of the serotype Overvecht);
- set S73-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Poona);
- set S73-2, comprising or constituted of at least one probe comprising at least or constituted of at least 8 consecutive nucleotides of any one of the nucleic acid sequences SEQ ID NO : 82, 87, 918, 933, 1826 and/or the nucleic acid sequences complementary thereto (nucleotide sequences corresponding to the various variable sequences of the CRISPR2 locus of the serotype Poona);
- set S74-1, comprising or constituted of at least one probe comprising at least or constituted of at least 8 consecutive nucleotides of any one of the nucleic acid sequences SEQ ID NO : 295 to 297, 307, 313, 317, 323, 1120, 1825, 1827 to 1837, 2023 and/or the nucleic acid sequences complementary thereto (nucleotide sequences corresponding to the various variable sequences of the CRISPR1 locus of the serotype Postdam);
- set S74-2, comprising or constituted of at least one probe comprising at least or constituted of at least 8 consecutive nucleotides of any one of the nucleic acid sequences SEQ ID NO : 663, 1304, 1838 to 1847 and/or the nucleic acid sequences complementary thereto (nucleotide sequences corresponding to the various variable sequences of the CRISPR2 locus of the serotype Postdam);
- set S75-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Reading);
- set S75-2, comprising or constituted of at least one probe comprising at least or constituted of at least 8 consecutive nucleotides of the nucleic acid sequence SEQ ID NO : 225 and/or the nucleic acid sequence complementary thereto (nucleotide sequences corresponding to the variable sequence of the CRISPR2 locus of the serotype Reading);
- set S76-1, comprising or constituted of at least one probe comprising at least or constituted of at least 8 consecutive nucleotides of any one of the nucleic acid sequences SEQ ID NO : 379, 380, 383 to 385, 387, 389, 390 and/or the nucleic acid sequences complementary thereto (nucleotide sequences corresponding to the various variable sequences of the CRISPR1 locus of the serotype Rosenberg);
- set S76-2, comprising or constituted of at least one probe comprising at least or constituted of at least 8 consecutive nucleotides of any one of the nucleic acid sequences SEQ ID NO : 392, 393, 404, 407 to 413 and/or the nucleic acid sequences complementary thereto (nucleotide sequences corresponding to the various variable sequences of the CRISPR2 locus of the serotype Rosenberg);
- set S77-1, comprising or constituted of at least one probe comprising at least or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Rubislaw);
- set S77-2, comprising or constituted of at least one probe comprising at least or constituted of at least 8 consecutive nucleotides of the nucleic acid sequence SEQ ID NO : 225 and/or the nucleic acid sequence complementary thereto;
- set S78-1, comprising or constituted of at least one probe comprising at least or constituted of at least 8 consecutive nucleotides of any one of the nucleic acid sequences SEQ ID NO : 1525, 1609 to 1621 and/or the nucleic acid sequences complementary thereto;
- set S78-2, comprising or constituted of at least one probe comprising at least or constituted of at least 8 consecutive nucleotides of any one of the nucleic acid sequences SEQ ID NO : 225 to 227, 229 to 231 and/or the nucleic acid sequences complementary thereto (nucleotide sequences corresponding to the various variable sequences of the CRISPR2 locus of the serotype Sandiego);
- set S79-1, comprising or constituted of at least one probe comprising at least or constituted of at least 8 consecutive nucleotides of any one of the nucleic acid sequences SEQ ID NO : 1106, 1456, 1917 to 1924 and/or the nucleic acid sequences complementary thereto (nucleotide sequences corresponding to the various variable sequences of the CRISPR1 locus of the serotype Stourbridge);
- set S79-2, comprising or constituted of at least one probe comprising at least or constituted of at least 8 consecutive nucleotides of any one of the nucleic acid sequences SEQ ID NO : 918, 1093, 1925 to 1936 and/or the nucleic acid sequences complementary thereto (nucleotide sequences corresponding to the various variable sequences of the CRISPR2 locus of the serotype Stourbridge);
- set S80-1, comprising or constituted of at least one probe comprising at least or constituted of at least 8 consecutive nucleotides of any one of the nucleic acid sequences SEQ ID NO : 1961 to 1963 and/or the nucleic acid sequences complementary thereto (nucleotide sequences corresponding to the various variable sequences of the CRISPR1 locus of the serotype Tallahassee);
- set S80-2, comprising or constituted of at least one probe comprising at least or constituted of at least 8 consecutive nucleotides of any one of the nucleic acid sequences SEQ ID NO : 25, 603, 2127, 2133, 1964 to 1966 and/or the nucleic acid sequences complementary thereto (nucleotide sequences corresponding to the various variable sequences of the CRISPR2 locus of the serotype Tallahassee);
- set S81-1, comprising or constituted of at least one probe comprising at least or constituted of at least 8 consecutive nucleotides of any one of the nucleic acid sequences SEQ ID NO : 67, 929, 1967 to 2005 and/or the nucleic acid sequences complementary thereto;
- set S81-2, comprising or constituted of at least one probe comprising at least or constituted of at least 8 consecutive nucleotides of any one of the nucleic acid sequences SEQ ID NO : 24, 769, 1550, 1659, 2006 to 2022 and/or the nucleic acid sequences complementary thereto (nucleotide sequences corresponding to the various variable sequences of the CRISPR2 locus of the serotype Tennessee);
- set S82-1, comprising or constituted of at least one probe comprising at least or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Urbana);
- set S82-2, comprising or constituted of at least one probe comprising at least or constituted of at least 8 consecutive nucleotides of any one of the nucleic acid sequences SEQ ID NO : 1697, 1698, 1800 and/or the nucleic acid sequences complementary thereto (nucleotide sequences corresponding to the various variable sequences of the CRISPR2 locus of the serotype Urbana);
- set S83-1, comprising or constituted of at least one probe comprising at least or constituted of at least 8 consecutive nucleotides of any one of the nucleic acid sequences SEQ ID NO : 948, 1120, 1123, 1325, 2039 to 2078 and/or the nucleic acid sequences complementary thereto (nucleotide sequences corresponding to the various variable sequences of the CRISPR1 locus of the serotype Weltevreden);
- set S83-2, comprising or constituted of at least one probe comprising at least or constituted of at least 8 consecutive nucleotides of any one of the nucleic acid sequences SEQ ID NO : 254 to 256, 2079 to 2108 and/or the nucleic acid sequences complementary thereto (nucleotide sequences corresponding to the various variable sequences of the CRISPR2 locus of the serotype Weltevreden);
- set S84-1, comprising or constituted of at least one probe comprising at least or constituted of at least 8 consecutive nucleotides of any one of the nucleic acid sequences SEQ ID NO : 1312, 1690, 2141, 2142 and/or the nucleic acid sequences complementary thereto (nucleotide sequences corresponding to the various variable sequences of the CRISPR1 locus of the serotype Zaiman);
- set S84-2, comprising or constituted of at least one probe comprising at least or constituted of at least 8 consecutive nucleotides of any one of the nucleic acid sequences SEQ ID NO : 1697, 1698, 1800, 1924, 2143, 2144 and/or the nucleic acid sequences complementary thereto (nucleotide sequences corresponding to the various variable sequences of the CRISPR2 locus of the serotype Zaiman);
(ii) detection of the hybridized probes.

11. The method as claimed in claim 10, **characterised in that** the probes have a length of between 8 and 76 nucleotides, preferably between 8 and 34 nucleotides.

12. The method as claimed in claim 10 or claim 11, **characterised in that** said step (b) is carried out by hybridisation of at least one probe of each of sets S1-1 to S6-2.

13. The method as claimed in claim 12, **characterised in that** said step (b) is carried out by hybridisation, in addition, of at least one probe of each of sets S7-1 to S10-2.

14. The method as claimed in claim 13, **characterised in that** said step (b) is carried out by hybridisation of at least one probe of each of sets S1-1 to S44-2.

15. The method as claimed in any one of claims 1 to 14, **characterised in that** the amplified nucleic acid fragments are labelled.

16. The method as claimed in any one of claims 1 to 15, **characterised in that** the sample is an isolated bacterial strain of the *Salmonella* genus.

17. The method as claimed in any one of claims 1 à 16, **characterised in that** step (a) is preceded by a step of extraction of nucleic acids present in said sample.

18. A set of probes capable of detecting a bacterium of the *Salmonella* genus, **characterised in that** it is chosen from the group constituted of:
- set S1-1, comprising or constituted of the probes of nucleic acid sequences SEQ ID Nos : 1106 to 1119, 1123 to 1140, 1142, 1143, 1914 or of at least 8 consecutive nucleotides of these nucleic acid sequences and/or the nucleic acid sequences complementary thereto (nucleotide sequences corresponding to the various variable sequences of the CRISPR1 locus of the serotype Typhimurium);
- set S1-2, comprising or constituted of the probes of nucleic acid sequences SEQ ID Nos: 455, 1144 to 1147, 1149 to 1153, 1155 to 1181 or of at least 8 consecutive nucleotides of these nucleic acid sequences and/or the nucleic acid sequences complementary thereto (nucleotide sequences corresponding to the various variable sequences of the CRISPR2 locus du serotype Typhimurium);
- set S2-1, comprising or constituted of the probes of nucleic acid sequences SEQ ID Nos: 256, 378 to 381, 383 to 387, 389 to 391, 1106, 1514, 1528, 2148 to 2153 or of at least 8 consecutive nucleotides of these nucleic acid sequences and/or the nucleic acid sequences complementary thereto (nucleotide sequences corresponding to the various variable sequences of the CRISPR1 locus of the serotype Enteritidis);
- set S2-2, comprising or constituted of the probes of nucleic acid sequences SEQ ID NO : 392 to 413, 519, 815, 972, 983, 989, 1529 to 1546, 1952 or of at least 8 consecutive nucleotides of these nucleic acid sequences and/or the nucleic acid sequences complementary thereto (nucleotide sequences corresponding to the various variable sequences of the CRISPR2 locus of the serotype Enteritidis);
- set S3-1, comprising or constituted of the probes of nucleic acid sequences SEQ ID NO : 421 to 442, 444, 445, 447 to 450 or of at least 8 consecutive nucleotides of these nucleic acid sequences and/or the nucleic acid sequences complementary thereto (nucleotide sequences corresponding to the various variable sequences of the CRISPR1 locus of the serotype Hadar);
- set S3-2, comprising or constituted of the probes of nucleic acid sequences SEQ ID NO : 451 to 465, 1144 to 1147, 1150 to 1152, 1154, 1155, 1158, 1169, 1172, 1175 to 1177, 1179, 1181 or of at least 8 consecutive nucleotides of these nucleic acid sequences and/or the nucleic acid sequences complementary thereto (nucleotide sequences corresponding to the various variable sequences of the CRISPR2 locus of the serotype Hadar);
- set S4-1, comprising or constituted of the probes of nucleic acid sequences SEQ ID NO : 65, 382, 1120, 1263 to 1302, 2024 to 2038 or of at least 8 consecutive nucleotides of these nucleic acid sequences and/or the nucleic acid sequences complementary thereto (nucleotide sequences corresponding to the various variable sequences of the CRISPR1 locus of the serotype Virchow);
- set S4-2, comprising or constituted of the probes of nucleic acid sequences SEQ ID NO : 83, 93, 94, 1304 to 1324 or of at least 8 consecutive nucleotides of these nucleic acid sequences and/or the nucleic acid sequences complementary thereto (nucleotide sequences corresponding to the various variable sequences of the CRISPR2 locus of the serotype Virchow);
- set S5-1, comprising or constituted of the probes of nucleic acid sequences SEQ ID NO : 531 to 562, 1106 or of at least 8 consecutive nucleotides of these nucleic acid sequences and/or the nucleic acid sequences complementary thereto (nucleotide sequences corresponding to the various variable sequences of the CRISPR1 locus of the serotype Infantis);
- set S5-2, comprising or constituted of the probes of nucleic acid sequences SEQ ID NO : 563 to 588 or of at least 8 consecutive nucleotides of these nucleic acid sequences and/or the nucleic acid sequences complementary thereto (nucleotide sequences corresponding to the various variable sequences of the CRISPR2 locus of the serotype Infantis);
- set S6-1, comprising or constituted of the probes of nucleic acid sequences SEQ ID NO : 1254 to 1259 or of at least 8 consecutive nucleotides of these nucleic acid sequences and/or the nucleic acid sequences complementary thereto (nucleotide sequences corresponding to the various variable sequences of the CRISPR1 locus of the serotype Typhi);
- set S6-2, comprising or constituted by at least one probe comprising at least or constituted by at least 8 consecutive nucleotides of the nucleic acid sequence SEQ ID No: 1260 and/or the nucleic acid sequence complementary thereto (nucleotide sequences corresponding to the variable sequence of the CRISPR2 locus of the serotype Typhi);
- set S7-1, comprising or constituted of the probes of nucleic acid sequences SEQ ID NO : 448, 449, 860 to 876, 878 to 884, 1719 to 1724, 1727 to 1743, 1745 or of at least 8 consecutive nucleotides of these nucleic acid sequences and/or the nucleic acid sequences complementary thereto (nucleotide sequences corresponding to the various variable sequences of the CRISPR1 locus of the serotype Newport);
- set S7-2, comprising or constituted of the probes of nucleic acid sequences SEQ ID NO : 453 to 456, 563, 621, 622, 625, 626, 657, 885 to 896, 906 to 915, 933, 1144, 1145, 1155, 1156, 1172, 1174, 1748 to 1756, 1758, 1759, 1916 or of at least 8 consecutive nucleotides of these nucleic acid sequences and/or the nucleic acid sequences complementary thereto (nucleotide sequences corresponding to the various variable sequences of the CRISPR2 locus of the serotype Newport);
- set S8-1, comprising or constituted of the probes of nucleic acid sequences SEQ ID NO : 295 to 299, 301 to 323, 738, 1106 to 1109, 1120, 1123, 1128 to 1131, 1133, 1137 à 1140, 1550, 1159 to 1561 or of at least 8 consecutive nucleotides of these nucleic acid sequences and/or the nucleic acid sequences complementary thereto (nucleotide sequences corresponding to the various variable sequences of the CRISPR1 locus of the serotype Derby);
- set S8-2, comprising or constituted of the probes of nucleic acid sequences SEQ ID NO : 324 to 337, 455, 1144, 1145, 1149 to 1153, 1155 to 1160, 1163, 1172 to 1174 or of at least 8 consecutive nucleotides of these nucleic acid sequences and/or the nucleic acid sequences complementary thereto (nucleotide sequences corresponding to the various variable sequences of the CRISPR2 locus of the serotype Derby);
- set S9-1, comprising or constituted of the probes of nucleic acid sequences SEQ ID NO : 295, 307, 602 to 614, 616 to 619, 1106, 1120 or of at least 8 consecutive nucleotides of these nucleic acid sequences and/or the nucleic acid sequences complementary thereto (nucleotide sequences corresponding to the various variable sequences of the CRISPR1 locus of the serotype Kottbus);
- set S9-2, comprising or constituted of the probes of nucleic acid sequences SEQ ID NO : 563, 620 to 629, 885, 896, 907, 913, 914, 933 or of at least 8 consecutive nucleotides of these nucleic acid sequences and/or the nucleic acid sequences complementary thereto (nucleotide sequences corresponding to the various variable sequences of the CRISPR2 locus of the serotype Kottbus);
- set S10-1, comprising or constituted of the probes of nucleic acid sequences SEQ ID NO : 422 to 425, 448 to 450, 504 to 518 or of at least 8 consecutive nucleotides of these nucleic acid sequences and/or the nucleic acid sequences complementary thereto (nucleotide sequences corresponding to the various variable sequences of the CRISPR1 locus of the serotype Indiana);
- set S10-2, comprising or constituted of the probes of nucleic acid sequences SEQ ID NO : 246, 258, 269, 392, 415, 519 to 530 or of at least 8 consecutive nucleotides of these nucleic acid sequences and/or the nucleic acid sequences complementary thereto (nucleotide sequences corresponding to the various variable sequences of the CRISPR2 locus of the serotype Indiana);
- set S11-1, comprising or constituted of the probes of nucleic acid sequences SEQ ID NO: 11 to 23, 238, 948, 1120, 1123, 1133 or of at least 8 consecutive nucleotides of these nucleic acid sequences and/or the nucleic acid sequences complementary thereto (nucleotide sequences corresponding to the various variable sequences of the CRISPR1 locus of the serotype Agona);
- set S11-2, comprising or constituted of the probes of nucleic acid sequences SEQ ID NO : 24 to 31 or of at least 8 consecutive nucleotides of these nucleic acid sequences and/or the nucleic acid sequences complementary thereto (nucleotide sequences corresponding to the various variable sequences of the CRISPR2 locus of the serotype Agona);
- set S12-1, comprising or constituted of the probes of nucleic acid sequences SEQ ID NO : 830 to 841, 1120 or of at least 8 consecutive nucleotides of these nucleic acid sequences and/or the nucleic acid sequences complementary thereto (nucleotide sequences corresponding to the various variable sequences of the CRISPR1 locus of the serotype Napoli);
- set S12-2, comprising or constituted of the probes of nucleic acid sequences SEQ ID NO : 842 to 859 or of at least 8 consecutive nucleotides of these nucleic acid sequences and/or the nucleic acid sequences complementary thereto (nucleotide sequences corresponding to the various variable sequences of the CRISPR2 locus of the serotype Napoli);
- set S13-1, comprising or constituted of the probes of nucleic acid sequences SEQ ID NO : 926 to 932 or of at least 8 consecutive nucleotides of these nucleic acid sequences and/or the nucleic acid sequences complementary thereto (nucleotide sequences corresponding to the various variable sequences of the CRISPR1 locus of the serotype Paratyphi A);
- set S13-2, comprising or constituted of the probes of nucleic acid sequences SEQ ID NO : 933, 935, 936 or of at least 8 consecutive nucleotides of these nucleic acid sequences and/or the nucleic acid sequences complementary thereto (nucleotide sequences corresponding to the various variable sequences of the CRISPR2 locus of the serotype Paratyphi A);
- set S14-1, comprising or constituted of the probes of nucleic acid sequences SEQ ID NO: 707, 937 to 971, 1106, 1128 to 1130, 1133, 1138, 1140, 1802 to 1808, 1915 or of at least 8 consecutive nucleotides of these nucleic acid sequences and/or the nucleic acid sequences complementary thereto (nucleotide sequences corresponding to the various variable sequences of the CRISPR1 locus of the serotypes Paratyphi B and Paratyphi B Java);
- set S14-2, comprising or constituted of the probes of nucleic acid sequences SEQ ID NO : 861, 972 to 987, 989 to 992 to 996, 1172 1262, 1462, 1497, 1810 to 1825 or of at least 8 consecutive nucleotides of these nucleic acid sequences and/or the nucleic acid sequences complementary thereto (nucleotide sequences corresponding to the various variable sequences of the CRISPR2 locus of the serotypes Paratyphi B and Paratyphi B Java);
- set S15-1, comprising or constituted of the probes of nucleic acid sequences SEQ ID NO : 232, 238, 239, 997 to 1002, 1106 or of at least 8 consecutive nucleotides of these nucleic acid sequences and/or the nucleic acid sequences complementary thereto (nucleotide sequences corresponding to the various variable sequences of the CRISPR1 locus of the serotype Paratyphi C);
- set S15-2, comprising or constituted of the probes of nucleic acid sequences SEQ ID NO : 280, 290, 291, 392, 393, 415, 1003 to 1006, 1262 or of at least 8 consecutive nucleotides of these nucleic acid sequences and/or the nucleic acid sequences complementary thereto (nucleotide sequences corresponding to the various variable sequences of the CRISPR2 locus of the serotype Paratyphi C);
- set S16-1, comprising or constituted of at least one probe comprising or constituted of at least 8 consecutive nucleotides in any one of nucleic acid sequences SEQ ID NO : 211 to 224, 421, 427, 428, 432, 443 to 445, 447 to 449, 504 or of at least 8 consecutive nucleotides of these nucleic acid sequences and/or the nucleic acid sequences complementary thereto (nucleotide sequences corresponding to the various variable sequences of the CRISPR1 locus of the serotype Bovismorbificans);
- set S16-2, comprising or constituted of the probes of nucleic acid sequences SEQ ID NO : 196 to 209, 994, 1144, 1145, 1172 or of at least 8 consecutive nucleotides of these nucleic acid sequences and/or the nucleic acid sequences complementary thereto (nucleotide sequences corresponding to the various variable sequences of the CRISPR2 locus of the serotype Bovismorbificans);
- set S17-1, comprising or constituted of at least one probe comprising or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Panama);
- set S17-2, comprising or constituted of the probes of nucleic acid sequences SEQ ID NO : 918 to 925, 934 or of at least 8 consecutive nucleotides of these nucleic acid sequences and/or the nucleic acid sequences complementary thereto (nucleotide sequences corresponding to the various variable sequences of the CRISPR2 locus of the serotype Panama);
- set S18-1, comprising or constituted of the probes of nucleic acid sequences SEQ ID NO: 11, 16, 67, 74, 442, 444, 446 to 448, 602, 937, 948, 1120, 1128 to 1130, 1182 to 1200, 1937 to 1950 or of at least 8 consecutive nucleotides of these nucleic acid sequences and/or the nucleic acid sequences complementary thereto (nucleotide sequences corresponding to the various variable sequences of the CRISPR1 locus of the serotype Saintpaul);
- set S18-2, comprising or constituted of the probes of nucleic acid sequences SEQ ID NO : 972, 983, 989, 1201 to 1214, 1216 to 1227, 1952 to 1960 or of at least 8 consecutive nucleotides of these nucleic acid sequences and/or the nucleic acid sequences complementary thereto (nucleotide sequences corresponding to the various variable sequences of the CRISPR2 locus of the serotype Saintpaul);
- set S19-1, comprising or constituted of the probes of nucleic acid sequences SEQ ID NO : 514, 1062 to 1089, 1100, 1284, 1886 to 1911 or of at least 8 consecutive nucleotides of these nucleic acid sequences and/or the nucleic acid sequences complementary thereto (nucleotide sequences corresponding to the various variable sequences of the CRISPR1 locus of the serotype Senftenberg);
- set S19-2, comprising or constituted of the probes of nucleic acid sequences SEQ ID NO : 300, 1090 to 1097, 1101 to 1103, 1304, 1315, 1318 to 1320 or of at least 8 consecutive nucleotides of these nucleic acid sequences and/or the nucleic acid sequences complementary thereto (nucleotide sequences corresponding to the various variable sequences of the CRISPR2 locus of the serotype Senftenberg);
- set S20-1, comprising or constituted of the probes of nucleic acid sequences SEQ ID NO : 67, 74 to 80 or of at least 8 consecutive nucleotides of these nucleic acid sequences and/or the nucleic acid sequences complementary thereto (nucleotide sequences corresponding to the various variable sequences of the CRISPR1 locus of the serotype Anatum);
- set S20-2, comprising or constituted of the probes of nucleic acid sequences SEQ ID NO : 83 to 94, 1304, 1315, 1318 to 1322, 1324, 1390 to 1397 or of at least 8 consecutive nucleotides of these nucleic acid sequences and/or the nucleic acid sequences complementary thereto (nucleotide sequences corresponding to the various variable sequences of the CRISPR2 locus of the serotype Anatum);
- set S21-1, comprising or constituted of the probes of nucleic acid sequences SEQ ID NO : 379, 383 to 385, 388 or of at least 8 consecutive nucleotides of these nucleic acid sequences and/or the nucleic acid sequences complementary thereto (nucleotide sequences corresponding to the various variable sequences of the CRISPR1 locus of the serotypes Gallinarum);
- set S21-2, comprising or constituted of the probes of nucleic acid sequences SEQ ID NO : 392, 393, 404, 407 to 415 or of at least 8 consecutive nucleotides of these nucleic acid sequences and/or the nucleic acid sequences complementary thereto (nucleotide sequences corresponding to the various variable sequences of the CRISPR2 locus of the serotypes Gallinarum);
- set S22-1, comprising or constituted of at least one probe comprising or constituted of at least 8 consecutive nucleotides of the nucleic acid sequence SEQ ID NO: 917 and/or the nucleic acid sequence complementary thereto (nucleotide sequences corresponding to the variable sequence of the CRISPR1 locus of the serotype Brandenburg);
- set S22-2, comprising or constituted of the probes of nucleic acid sequences SEQ ID NO : 225 to 231 or of at least 8 consecutive nucleotides of these nucleic acid sequences and/or the nucleic acid sequences complementary thereto (nucleotide sequences corresponding to the various variable sequences of the CRISPR2 locus of the serotype Brandenburg);
- set S23-1, comprising or constituted of the probes of nucleic acid sequences SEQ ID NO : 378, 379 or of at least 8 consecutive nucleotides of these nucleic acid sequences and/or the nucleic acid sequences complementary thereto (nucleotide sequences corresponding to the various variable sequences of the CRISPR1 locus of the serotype Dublin);
- set S23-2, comprising or constituted of the probes of nucleic acid sequences SEQ ID NO : 392, 393, 411, 412 or of at least 8 consecutive nucleotides of these nucleic acid sequences and/or the nucleic acid sequences complementary thereto (nucleotide sequences corresponding to the various variable sequences of the CRISPR2 locus of the serotype Dublin);
- set S24-1, comprising or constituted of the probes of nucleic acid sequences SEQ ID NO : 1, 1106 or of at least 8 consecutive nucleotides of these nucleic acid sequences and/or the nucleic acid sequences complementary thereto (nucleotide sequences corresponding to the various variable sequences of the CRISPR1 locus of the serotype Abortusequi);
- set S24-2, comprising or constituted of the probes of nucleic acid sequences SEQ ID NO : 2 to 10, 130 or of at least 8 consecutive nucleotides of these nucleic acid sequences and/or the nucleic acid sequences complementary thereto (nucleotide sequences corresponding to the various variable sequences of the CRISPR2 locus of the serotype Abortusequi);
- set S25-1, comprising or constituted of the probes of nucleic acid sequences SEQ ID NO : 378, 379 or of at least 8 consecutive nucleotides of these nucleic acid sequences and/or the nucleic acid sequences complementary thereto (nucleotide sequences corresponding to the various variable sequences of the CRISPR1 locus of the serotype Kiel);
- set S25-2, comprising or constituted of the probes of nucleic acid sequences SEQ ID NO : 392, 393, 411, 412 or of at least 8 consecutive nucleotides of these nucleic acid sequences and/or the nucleic acid sequences complementary thereto (nucleotide sequences corresponding to the various variable sequences of the CRISPR2 locus of the serotype Kiel);
- set S26-1, comprising or constituted of the probes of nucleic acid sequences SEQ ID NO : 509, 510, 512, 513, 637 to 660, 1086, 1107 or of at least 8 consecutive nucleotides of these nucleic acid sequences and/or the nucleic acid sequences complementary thereto (nucleotide sequences corresponding to the various variable sequences of the CRISPR1 locus of the serotype Mbandaka);
- set S26-2, comprising or constituted of the probes of nucleic acid sequences SEQ ID NO : 661 to 666, 668, 669, 672, 683, 690 to 695, 731, 918 or of at least 8 consecutive nucleotides of these nucleic acid sequences and/or the nucleic acid sequences complementary thereto (nucleotide sequences corresponding to the various variable sequences of the CRISPR2 locus of the serotype Mbandaka);
- set S27-1, comprising or constituted of at least one probe comprising or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Miami);
- set S27-2, comprising or constituted of the probes of nucleic acid sequences SEQ ID NO : 25, 696 to 700 or of at least 8 consecutive nucleotides of these nucleic acid sequences and/or the nucleic acid sequences complementary thereto (nucleotide sequences corresponding to the various variable sequences of the CRISPR2 locus of the serotype Miami);
- set S28-1, comprising or constituted of the probes of nucleic acid sequences SEQ ID NO : 55, 701 to 730, 732 to 768, 1115, 1228, 1232 to 1237, 1601, 1671 to 1680, 1682 to 1694, 1747 or of at least 8 consecutive nucleotides of these nucleic acid sequences and/or the nucleic acid sequences complementary thereto (nucleotide sequences corresponding to the various variable sequences of the CRISPR1 locus of the serotype Montevideo);
- set S28-2, comprising or constituted of the probes of nucleic acid sequences SEQ ID NO : 589, 769 to 829, 933, 1245, 1697, 1698 or of at least 8 consecutive nucleotides of these nucleic acid sequences and/or the nucleic acid sequences complementary thereto (nucleotide sequences corresponding to the various variable sequences of the CRISPR2 locus of the serotype Montevideo);
- set S29-1, comprising or constituted of the probes of nucleic acid sequences SEQ ID NO : 379, 380, 383 to 385, 387, 389 to 391 or of at least 8 consecutive nucleotides of these nucleic acid sequences and/or the nucleic acid sequences complementary thereto (nucleotide sequences corresponding to the various variable sequences of the CRISPR1 locus of the serotype Nitra);
- set S29-2, comprising or constituted of the probes of nucleic acid sequences SEQ ID NO : 392 to 394, 404, 407 to 413, 519 or of at least 8 consecutive nucleotides of these nucleic acid sequences and/or the nucleic acid sequences complementary thereto (nucleotide sequences corresponding to the various variable sequences of the CRISPR2 locus of the serotype Nitra);
- set S30-1, comprising or constituted of the probes of nucleic acid sequences SEQ ID NO : 931, 932, 1104, 1105 or of at least 8 consecutive nucleotides of these nucleic acid sequences and/or the nucleic acid sequences complementary thereto (nucleotide sequences corresponding to the various variable sequences of the CRISPR1 locus of the serotype Sendai);
- set S30-2, comprising or constituted of at least one probe comprising or constituted of at least 8 consecutive nucleotides of the nucleic acid sequence SEQ ID NO: 936 and/or the nucleic acid sequence complementary thereto (nucleotide sequences corresponding to the variable sequence of the CRISPR2 locus of the serotype Sendai);
- set S31-1, comprising or constituted of the probes of nucleic acid sequences SEQ ID NO : 714 to 716, 1228 to 1239 or of at least 8 consecutive nucleotides of these nucleic acid sequences and/or the nucleic acid sequences complementary thereto (nucleotide sequences corresponding to the various variable sequences of the CRISPR1 locus of the serotype Schwarzengrund);
- set S31-2, comprising or constituted of the probes of nucleic acid sequences SEQ ID NO : 196, 992, 995, 1172, 1240 to 1244, 1246 to 1253 or of at least 8 consecutive nucleotides of these nucleic acid sequences and/or the nucleic acid sequences complementary thereto (nucleotide sequences corresponding to the various variable sequences of the CRISPR2 locus of the serotype Schwarzengrund);
- set S32-1, comprising or constituted of the probes of nucleic acid sequences SEQ ID NO : 232, 238, 239, 998, 999, 1002, 1106, 1261 or of at least 8 consecutive nucleotides of these nucleic acid sequences and/or the nucleic acid sequences complementary thereto (nucleotide sequences corresponding to the various variable sequences of the CRISPR1 locus of the serotype Typhisuis);
- set S32-2, comprising or constituted of at least one probe comprising or constituted of at least 8 consecutive nucleotides of the nucleic acid sequence SEQ ID NO: 1262 and/or the nucleic acid sequence complementary thereto (nucleotide sequences corresponding to the variable sequence of the CRISPR2 locus of the serotype Typhisuis);
- set S33-1, comprising or constituted of the probes of nucleic acid sequences SEQ ID NO : 466 to 486 or of at least 8 consecutive nucleotides of these nucleic acid sequences and/or the nucleic acid sequences complementary thereto (nucleotide sequences corresponding to the various variable sequences of the CRISPR1 locus of the different serotypes of the subspecies *houtenae);*
- set S33-2, comprising or constituted of the probes of nucleic acid sequences SEQ ID NO : 487 to 490 or of at least 8 consecutive nucleotides of these nucleic acid sequences and/or the nucleic acid sequences complementary thereto (nucleotide sequences corresponding to the various variable sequences of the CRISPR2 locus of the different serotypes of the subspecies *houtenae*);
- set S34-1, comprising or constituted of the probes of nucleic acid sequences SEQ ID NO : 338 to 376 or of at least 8 consecutive nucleotides of these nucleic acid sequences and/or the nucleic acid sequences complementary thereto (nucleotide sequences corresponding to the various variable sequences of the CRISPR1 locus of the different serotypes of the subspecies *diarizonae);*
- set S34-2, comprising or constituted of at least one probe comprising or constituted of at least 8 consecutive nucleotides of the nucleic acid sequence SEQ ID NO: 377 and/or the nucleic acid sequence complementary thereto (nucleotide sequences corresponding to the variable sequence of the CRISPR2 locus of the different serotypes of the subspecies *diarizonae);*
- set S35-1, comprising or constituted of the probes of nucleic acid sequences SEQ ID NO : 1007 to 1037, 1848 to 1884 or of at least 8 consecutive nucleotides of these nucleic acid sequences and/or the nucleic acid sequences complementary thereto (nucleotide sequences corresponding to the various variable sequences of the CRISPR1 locus of the subspecies *salamae);*
- set S35-2, comprising or constituted of the probes of nucleic acid sequences SEQ ID NO : 1038 to 1043, 1045 to 1061, 1885 or of at least 8 consecutive nucleotides of these nucleic acid sequences and/or the nucleic acid sequences complementary thereto (nucleotide sequences corresponding to the various variable sequences of the CRISPR2 locus of the subspecies *salamae);*
- set S36, comprising or constituted of at least one probe comprising or constituted of at least 8 consecutive nucleotides of the nucleic acid sequence SEQ ID NO : 95 and/or the nucleic acid sequence complementary thereto (nucleotide sequences corresponding to the variable sequence of the CRISPR1 locus of the different serotypes of the subspecies *arizonae*);
- set S37-1, comprising or constituted of the probes of nucleic acid sequences SEQ ID NO : 491 to 499 or of at least 8 consecutive nucleotides of these nucleic acid sequences and/or the nucleic acid sequences complementary thereto (nucleotide sequences corresponding to the various variable sequences of the CRISPR1 locus of the different serotypes of the subspecies *indica);*
- set S37-2, comprising or constituted of the probes of nucleic acid sequences SEQ ID NO : 500 to 503, 1574 to 1589 or of at least 8 consecutive nucleotides of these nucleic acid sequences and/or the nucleic acid sequences complementary thereto (nucleotide sequences corresponding to the various variable sequences of the CRISPR2 locus of the different serotypes of the subspecies *indica);*
- set S38-1, comprising or constituted of the probes of nucleic acid sequences SEQ ID NO : 32 to 66, 382, 416, 667, 2036 or of at least 8 consecutive nucleotides of these nucleic acid sequences and/or the nucleic acid sequences complementary thereto (nucleotide sequences corresponding to the various variable sequences of the CRISPR1 locus of the serotype Altona);
- set S38-2, comprising or constituted of the probes of nucleic acid sequences SEQ ID NO : 854, 933, 1095, 1306, 1354 to 1389, 1696 or of at least 8 consecutive nucleotides of these nucleic acid sequences and/or the nucleic acid sequences complementary thereto (nucleotide sequences corresponding to the various variable sequences of the CRISPR2 locus of the serotype Altona);
- set S39-1, comprising or constituted of the probes of nucleic acid sequences SEQ ID NO : 240, 241, 1120, 1121 or of at least 8 consecutive nucleotides of these nucleic acid sequences and/or the nucleic acid sequences complementary thereto (nucleotide sequences corresponding to the various variable sequences of the CRISPR1 locus of the serotype Choleraesuis variety Decatur);
- set S39-2, comprising or constituted of the probes of nucleic acid sequences SEQ ID NO : 225, 247 to 251, 253 to 256, 259 to 268, 270 to 279, 281 to 289, 292 to 294 or of at least 8 consecutive nucleotides of these nucleic acid sequences and/or the nucleic acid sequences complementary thereto (nucleotide sequences corresponding to the various variable sequences of the CRISPR2 locus of the serotype Choleraesuis variety Decatur);
- set S40-1, comprising or constituted of the probes of nucleic acid sequences SEQ ID NO : 11, 232, 238, 239, 240, 242 to 245, 233 to 237, 959, 1106, 1120 or of at least 8 consecutive nucleotides of these nucleic acid sequences and/or the nucleic acid sequences complementary thereto (nucleotide sequences corresponding to the various variable sequences of the CRISPR1 locus of the serotype Choleraesuis variety Kunzendorf);
- set S40-2, comprising or constituted of the probes of nucleic acid sequences SEQ ID NO : 246, 252, 258, 269, 280, 290, 291, 392, 563, 933 or of at least 8 consecutive nucleotides of these nucleic acid sequences and/or the nucleic acid sequences complementary thereto (nucleotide sequences corresponding to the various variable sequences of the CRISPR2 locus of the serotype Choleraesuis variety Kunzendorf);
- set S41-1, comprising or constituted of the probes of nucleic acid sequences SEQ ID NO : 11, 242 to 245, 232 to 239, 959, 1000, 1106, 1139, 1120 or of at least 8 consecutive nucleotides of these nucleic acid sequences and/or the nucleic acid sequences complementary thereto (nucleotide sequences corresponding to the various variable sequences of the CRISPR1 locus of the serotypes Choleraesuis and Choleraesuis sensu stricto);
- set S41-2, comprising or constituted of the probes of nucleic acid sequences SEQ ID NO : 246, 252, 258, 269, 280, 290, 291, 392, 563, 574, 933, 1003, 1004 or of at least 8 consecutive nucleotides of these nucleic acid sequences and/or the nucleic acid sequences complementary thereto (nucleotide sequences corresponding to the various variable sequences of the CRISPR2 locus of the serotypes Choleraesuis and Choleraesuis sensu stricto);
- set S42-1, comprising or constituted of the probes of nucleic acid sequences SEQ ID NO : 416 to 420, 1122 or of at least 8 consecutive nucleotides of these nucleic acid sequences and/or the nucleic acid sequences complementary thereto (nucleotide sequences corresponding to the various variable sequences of the CRISPR1 locus of the serotype Goettingen);
- set S42-2, comprising or constituted of at least one probe comprising or constituted of at least 8 consecutive nucleotides of the nucleic acid sequence SEQ ID NO: 1548 and/or the nucleic acid sequence complementary thereto (nucleotide sequences corresponding to the variable sequence of the CRISPR2 locus of the serotype Goettingen);
- set S43-1, comprising or constituted of the probes of nucleic acid sequences SEQ ID NO : 630 to 636, 937, 948, 1120, 1143 or of at least 8 consecutive nucleotides of these nucleic acid sequences and/or the nucleic acid sequences complementary thereto (nucleotide sequences corresponding to the various variable sequences of the CRISPR1 locus of the serotype Manhattan);
- set S43-2, comprising or constituted of the probes of nucleic acid sequences SEQ ID NO : 392, 1653 or of at least 8 consecutive nucleotides of these nucleic acid sequences and/or the nucleic acid sequences complementary thereto (nucleotide sequences corresponding to the various variable sequences of the CRISPR2 locus of the serotype Manhattan);
- set S44-1, comprising or constituted of the probes of nucleic acid sequences SEQ ID NO : 96 to 155 or of at least 8 consecutive nucleotides of these nucleic acid sequences and/or the nucleic acid sequences complementary thereto (nucleotide sequences corresponding to the various variable sequences of the CRISPR1 locus of certain serotypes of the species *S. bongori*);
- set S44-2, comprising or constituted of the probes of nucleic acid sequences SEQ ID NO : 156 to 195, 1421 to 1430 or of at least 8 consecutive nucleotides of these nucleic acid sequences and/or the nucleic acid sequences complementary thereto (nucleotide sequences corresponding to the various variable sequences of the CRISPR2 locus of certain serotypes of the species *S. bongori*);
- set S45-1, comprising or constituted of the probes of nucleic acid sequences SEQ ID NO : 962, 1106, 1133, 1804, 1808 or of at least 8 consecutive nucleotides of these nucleic acid sequences and/or the nucleic acid sequences complementary thereto (nucleotide sequences corresponding to the various variable sequences of the CRISPR1 locus of the serotype Abony);
- set S45-2, comprising or constituted of the probes of nucleic acid sequences SEQ ID NO : 972, 989, 1334 to 1339, 1474, 1499 or of at least 8 consecutive nucleotides of these nucleic acid sequences and/or the nucleic acid sequences complementary thereto (nucleotide sequences corresponding to the various variable sequences of the CRISPR2 locus of the serotype Abony);
- set S46-1, comprising or constituted of the probes of nucleic acid sequences SEQ ID NO : 448, 860 to 875, 882, 883, 1340 to 1345, 1721 to 1723 or of at least 8 consecutive nucleotides of these nucleic acid sequences and/or the nucleic acid sequences complementary thereto (nucleotide sequences corresponding to the various variable sequences of the CRISPR1 locus of the serotype Aesch);
- set S46-2, comprising or constituted of the probes of nucleic acid sequences SEQ ID NO : 563, 625, 626, 885, 887 to 893, 896, 933, 1346 to 1348, 1749, 1759 or of at least 8 consecutive nucleotides of these nucleic acid sequences and/or the nucleic acid sequences complementary thereto (nucleotide sequences corresponding to the various variable sequences of the CRISPR2 locus of the serotype Aesch);
- set S47-1, comprising or constituted of the probes of nucleic acid sequences SEQ ID NO: 834, 1120, 1349, 1350, 1351, 1713, 1718 or of at least 8 consecutive nucleotides of these nucleic acid sequences and/or the nucleic acid sequences complementary thereto (nucleotide sequences corresponding to the various variable sequences of the CRISPR1 locus of the serotype Albany);
- set S47-2, comprising or constituted of the probes of nucleic acid sequences SEQ ID NO : 842, 852, 1352, 1353 or of at least 8 consecutive nucleotides of these nucleic acid sequences and/or the nucleic acid sequences complementary thereto (nucleotide sequences corresponding to the various variable sequences of the CRISPR2 locus of the serotype Albany);
- set S48-1, comprising or constituted of at least one probe comprising or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Arechavalata);
- set S48-2, comprising or constituted of the probes of nucleic acid sequences SEQ ID NO : 225, 227 to 231, 1398 or of at least 8 consecutive nucleotides of these nucleic acid sequences and/or the nucleic acid sequences complementary thereto (nucleotide sequences corresponding to the various variable sequences of the CRISPR2 locus of the serotype Arechavalata);
- set S49-1, comprising or constituted of the probes of nucleic acid sequences SEQ ID NO : 448, 449, 860, 871 to 873, 878, 895, 903 to 905, 907, 1719 to 1721, 1745 or of at least 8 consecutive nucleotides of these nucleic acid sequences and/or the nucleic acid sequences complementary thereto (nucleotide sequences corresponding to the various variable sequences of the CRISPR1 locus of the serotype Bardo);
- set S49-2, comprising or constituted of the probes of nucleic acid sequences SEQ ID NO : 563, 622, 626, 885, 886 to 890, 893, 896, 907, 910 to 915, 933, 1756 to 1758 or of at least 8 consecutive nucleotides of these nucleic acid sequences and/or the nucleic acid sequences complementary thereto (nucleotide sequences corresponding to the various variable sequences of the CRISPR2 locus of the serotype Bardo);
- set S50-1, comprising or constituted of the probes of nucleic acid sequences SEQ ID NO : 82, 1401 or of at least 8 consecutive nucleotides of these nucleic acid sequences and/or the nucleic acid sequences complementary thereto (nucleotide sequences corresponding to the various variable sequences of the CRISPR1 locus of the serotype Berta);
- set S50-2, comprising or constituted of the probes of nucleic acid sequences SEQ ID NO : 246, 918, 933, 1401, 1448 or of at least 8 consecutive nucleotides of these nucleic acid sequences and/or the nucleic acid sequences complementary thereto (nucleotide sequences corresponding to the various variable sequences of the CRISPR2 locus of the serotype Berta);
- set S51-1, comprising or constituted of the probes of nucleic acid sequences SEQ ID NO : 1, 37, 1106, 1402 to 1416, 2055 or of at least 8 consecutive nucleotides of these nucleic acid sequences and/or the nucleic acid sequences complementary thereto (nucleotide sequences corresponding to the various variable sequences of the CRISPR1 locus of the serotype Bispebjerg);
- set S51-2, comprising or constituted of the probes of nucleic acid sequences SEQ ID NO : 2 to 4, 9, 1417 to 1420, 1547, 1572 or of at least 8 consecutive nucleotides of these nucleic acid sequences and/or the nucleic acid sequences complementary thereto (nucleotide sequences corresponding to the various variable sequences of the CRISPR2 locus of the serotype Bispebjerg);
- set S52-1, comprising or constituted of the probes of nucleic acid sequences SEQ ID NO : 379, 380, 383, 385, 387, 389 to 391 or of at least 8 consecutive nucleotides of these nucleic acid sequences and/or the nucleic acid sequences complementary thereto (nucleotide sequences corresponding to the various variable sequences of the CRISPR1 locus of the serotype Blegdam);
- set S52-2, comprising or constituted of the probes of nucleic acid sequences SEQ ID NO : 392 to 394, 404, 407, 412, 413, 519 or of at least 8 consecutive nucleotides of these nucleic acid sequences and/or the nucleic acid sequences complementary thereto (nucleotide sequences corresponding to the various variable sequences of the CRISPR2 locus of the serotype Blegdam);
- set S53-1, comprising or constituted of at least one probe comprising or constituted of at least 8 consecutive nucleotides of the nucleic acid sequence SEQ ID NO: 917 and/or the nucleic acid sequence complementary thereto (nucleotide sequences corresponding to the variable sequence of the CRISPR1 locus of the serotype Chester);
- set S53-2, comprising or constituted of the probes of nucleic acid sequences SEQ ID NO : 933, 1445 to 1447 or of at least 8 consecutive nucleotides of these nucleic acid sequences and/or the nucleic acid sequences complementary thereto (nucleotide sequences corresponding to the various variable sequences of the CRISPR2 locus of the serotype Chester);
- set S54-1, comprising or constituted of the probes of nucleic acid sequences SEQ ID NO : 1449 to 1454, 1460, 1467 to 1473 or of at least 8 consecutive nucleotides of these nucleic acid sequences and/or the nucleic acid sequences complementary thereto (nucleotide sequences corresponding to the various variable sequences of the CRISPR1 locus of the serotype Concord);
- set S54-2, comprising or constituted of the probes of nucleic acid sequences SEQ ID NO : 972, 1474 to 1493, 1495 to 1499 or of at least 8 consecutive nucleotides of these nucleic acid sequences and/or the nucleic acid sequences complementary thereto (nucleotide sequences corresponding to the various variable sequences of the CRISPR2 locus of the serotype Concord);
- set S55-1, comprising or constituted of the probes of nucleic acid sequences SEQ ID NO : 1106, 1107 to 1109, 1123, 1128 to 1131, 1133, 1137 to 1140, 1550, 1559 to 1561 or of at least 8 consecutive nucleotides of these nucleic acid sequences and/or the nucleic acid sequences complementary thereto (nucleotide sequences corresponding to the various variable sequences of the CRISPR1 locus of the serotype Crossness);
- set S55-2, comprising or constituted of the probes of nucleic acid sequences SEQ ID NO : 455, 1144, 1145, 1149 to 1153, 1155 to 1160, 1163, 1172 to 1174 or of at least 8 consecutive nucleotides of these nucleic acid sequences and/or the nucleic acid sequences complementary thereto (nucleotide sequences corresponding to the various variable sequences of the CRISPR2 locus of the serotype Crossness);
- set S56-1, comprising or constituted of the probes of nucleic acid sequences SEQ ID NO: 834, 1120, 1349 to 1351, 1713, 1718 or of at least 8 consecutive nucleotides of these nucleic acid sequences and/or the nucleic acid sequences complementary thereto (nucleotide sequences corresponding to the various variable sequences of the CRISPR1 locus of the serotype Duesseldorf);
- set S56-2, comprising or constituted of the probes of nucleic acid sequences SEQ ID NO : 842, 852, 1352, 1353, 1501 to 1503 or of at least 8 consecutive nucleotides of these nucleic acid sequences and/or the nucleic acid sequences complementary thereto (nucleotide sequences corresponding to the various variable sequences of the CRISPR2 locus of the serotype Duesseldorf);
- set S57-1, comprising or constituted of the probes of nucleic acid sequences SEQ ID NO : 941, 1504 to 1508 or of at least 8 consecutive nucleotides of these nucleic acid sequences and/or the nucleic acid sequences complementary thereto (nucleotide sequences corresponding to the various variable sequences of the CRISPR1 locus of the serotype Emek);
- set S57-2, comprising or constituted of the probes of nucleic acid sequences SEQ ID NO : 1260, 1509 to 1524, 1526, 1527 or of at least 8 consecutive nucleotides of these nucleic acid sequences and/or the nucleic acid sequences complementary thereto (nucleotide sequences corresponding to the various variable sequences of the CRISPR2 locus of the serotype Emek);
- set S58-1, comprising or constituted of at least one probe comprising or constituted of at least 8 consecutive nucleotides of the nucleic acid sequence SEQ ID NO: 558 and/or the nucleic acid sequence complementary thereto (nucleotide sequences corresponding to the variable sequence of the CRISPR1 locus of the serotype Fulica);
- set S58-2, comprising or constituted of the probes of nucleic acid sequences SEQ ID NO : 2, 4, 5, 1333, 1547 or of at least 8 consecutive nucleotides of these nucleic acid sequences and/or the nucleic acid sequences complementary thereto (nucleotide sequences corresponding to the various variable sequences of the CRISPR2 locus of the serotype Fulica);
- set S59-1, comprising or constituted of at least one probe comprising or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Gueuletapee);
- set S59-2, comprising or constituted of the probes of nucleic acid sequences SEQ ID NO : 597, 918 to 925, 933, 1549 or of at least 8 consecutive nucleotides of these nucleic acid sequences and/or the nucleic acid sequences complementary thereto (nucleotide sequences corresponding to the various variable sequences of the CRISPR2 locus of the serotype Gueuletapee);
- set S60-1, comprising or constituted of the probes of nucleic acid sequences SEQ ID NO: 1106 to 1109, 1111 to 1116, 1118, 1123, 1128 to 1131, 1133, 1137 to 1140, 1150, 1151, 1555 to 1562 or of at least 8 consecutive nucleotides of these nucleic acid sequences and/or the nucleic acid sequences complementary thereto (nucleotide sequences corresponding to the various variable sequences of the CRISPR1 locus of the serotype Heidelberg);
- set S60-2, comprising or constituted of the probes of nucleic acid sequences SEQ ID NO : 455, 1144, 1145, 1149 to 1153, 1155 to 1163, 1172 to 1174, 1563 or of at least 8 consecutive nucleotides of these nucleic acid sequences and/or the nucleic acid sequences complementary thereto (nucleotide sequences corresponding to the various variable sequences of the CRISPR2 locus of the serotype Heidelberg);
- set S61-1, comprising or constituted of the probes of nucleic acid sequences SEQ ID NO : 558, 1565 to 1571 or of at least 8 consecutive nucleotides of these nucleic acid sequences and/or the nucleic acid sequences complementary thereto (nucleotide sequences corresponding to the various variable sequences of the CRISPR1 locus of the serotype Hessarek);
- set S61-2, comprising or constituted of the probes of nucleic acid sequences SEQ ID NO : 2 to 4, 1547, 1572, 1573 or of at least 8 consecutive nucleotides of these nucleic acid sequences and/or the nucleic acid sequences complementary thereto (nucleotide sequences corresponding to the various variable sequences of the CRISPR2 locus of the serotype Hessarek);
- set S62-1, comprising or constituted of the probes of nucleic acid sequences SEQ ID NO : 1591, 1592, 1667 or of at least 8 consecutive nucleotides of these nucleic acid sequences and/or the nucleic acid sequences complementary thereto (nucleotide sequences corresponding to the various variable sequences of the CRISPR1 locus of the serotype Itami);
- set S62-2, comprising or constituted of the probes of nucleic acid sequences SEQ ID NO : 918, 1593 to 1600, 1744 or of at least 8 consecutive nucleotides of these nucleic acid sequences and/or the nucleic acid sequences complementary thereto (nucleotide sequences corresponding to the various variable sequences of the CRISPR2 locus of the serotype Itami);
- set S63-1, comprising or constituted of the probes of nucleic acid sequences SEQ ID NO : 1601 to 1606, 1687 or of at least 8 consecutive nucleotides of these nucleic acid sequences and/or the nucleic acid sequences complementary thereto (nucleotide sequences corresponding to the various variable sequences of the CRISPR1 locus of the serotype Javiana);
- set S63-2, comprising or constituted of the probes of nucleic acid sequences SEQ ID NO : 589 to 601, 973, 1607, 1608 or of at least 8 consecutive nucleotides of these nucleic acid sequences and/or the nucleic acid sequences complementary thereto (nucleotide sequences corresponding to the various variable sequences of the CRISPR2 locus of the serotype Javiana);
- set S64-1, comprising or constituted of the probes of nucleic acid sequences SEQ ID NO : 1609 to 1621, 1494, 1525 or of at least 8 consecutive nucleotides of these nucleic acid sequences and/or the nucleic acid sequences complementary thereto (nucleotide sequences corresponding to the various variable sequences of the CRISPR1 locus of the serotype Johannesburg);
- set S64-2, comprising or constituted of the probes of nucleic acid sequences SEQ ID NO : 918, 1697, 1698 or of at least 8 consecutive nucleotides of these nucleic acid sequences and/or the nucleic acid sequences complementary thereto (nucleotide sequences corresponding to the various variable sequences of the CRISPR2 locus of the serotype Johannesburg);
- set S65-1, comprising or constituted of the probes of nucleic acid sequences SEQ ID NO : 444, 448, 1184, 1622 to 1637 or of at least 8 consecutive nucleotides of these nucleic acid sequences and/or the nucleic acid sequences complementary thereto (nucleotide sequences corresponding to the various variable sequences of the CRISPR1 locus of the serotype Kentucky);
- set S65-2, comprising or constituted of the probes of nucleic acid sequences SEQ ID NO : 385, 519, 1638 to 1652, 1746 or of at least 8 consecutive nucleotides of these nucleic acid sequences and/or the nucleic acid sequences complementary thereto (nucleotide sequences corresponding to the various variable sequences of the CRISPR2 locus of the serotype Kentucky);
- set S66-1, comprising or constituted of at least one probe comprising or constituted of at least 8 consecutive nucleotides of the nucleic acid sequence SEQ ID NO: 67 and/or the nucleic acid sequence complementary thereto (nucleotide sequences corresponding to the variable sequence of the CRISPR1 locus of the serotype Kundunchi);
- set S66-2, comprising or constituted of the probes of nucleic acid sequences SEQ ID NO : 972, 983, 989, 1201, 1204, 1205, 1210 to 1212 or of at least 8 consecutive nucleotides of these nucleic acid sequences and/or the nucleic acid sequences complementary thereto (nucleotide sequences corresponding to the various variable sequences of the CRISPR2 locus of the serotype Kundunchi);
- set S67-1, comprising or constituted of the probes of nucleic acid sequences SEQ ID NO : 448, 449, 860, 871, 878 to 881, 1721, 1724 or of at least 8 consecutive nucleotides of these nucleic acid sequences and/or the nucleic acid sequences complementary thereto (nucleotide sequences corresponding to the various variable sequences of the CRISPR1 locus of the serotype Lindenburg);
- set S67-2, comprising or constituted of the probes of nucleic acid sequences SEQ ID NO : 563, 622, 885, 886, 896, 907, 910 to 915, 933, 1748, 1757 or of at least 8 consecutive nucleotides of these nucleic acid sequences and/or the nucleic acid sequences complementary thereto (nucleotide sequences corresponding to the various variable sequences of the CRISPR2 locus of the serotype Lindenburg);
- set S68-1, comprising or constituted of at least one probe comprising or constituted of at least 8 consecutive nucleotides of the nucleic acid sequence SEQ ID NO : 917 and/or the nucleic acid sequence complementary thereto (nucleotide sequences corresponding to the variable sequence of the CRISPR1 locus of the serotype Maracaibo);
- set S68-2, comprising or constituted of the probes of nucleic acid sequences SEQ ID NO : 1240, 1247, 1248, 1654 to 1656 or of at least 8 consecutive nucleotides of these nucleic acid sequences and/or the nucleic acid sequences complementary thereto (nucleotide sequences corresponding to the various variable sequences of the CRISPR2 locus of the serotype Maracaibo);
- set S69-1, comprising or constituted of the probes of nucleic acid sequences SEQ ID NO : 627, 834, 1120, 1660 to 1669 or of at least 8 consecutive nucleotides of these nucleic acid sequences and/or the nucleic acid sequences complementary thereto (nucleotide sequences corresponding to the various variable sequences of the CRISPR1 locus of the serotype Mississippi);
- set S69-2, comprising or constituted of the probes of nucleic acid sequences SEQ ID NO : 842, 1670 or of at least 8 consecutive nucleotides of these nucleic acid sequences and/or the nucleic acid sequences complementary thereto (nucleotide sequences corresponding to the various variable sequences of the CRISPR2 locus of the serotype Mississippi);
- set S70-1, comprising or constituted of the probes of nucleic acid sequences SEQ ID NO : 941, 942, 949, 1699 to 1712, 2109 or of at least 8 consecutive nucleotides of these nucleic acid sequences and/or the nucleic acid sequences complementary thereto (nucleotide sequences corresponding to the various variable sequences of the CRISPR1 locus of the serotype Muenchen);
- set S70-2, comprising or constituted of the probes of nucleic acid sequences SEQ ID NO : 392, 1653 or of at least 8 consecutive nucleotides of these nucleic acid sequences and/or the nucleic acid sequences complementary thereto (nucleotide sequences corresponding to the various variable sequences of the CRISPR2 locus of the serotype Muenchen);
- set S71-1, comprising or constituted of the probes of nucleic acid sequences SEQ ID NO: 11, 1120, 1497, 1760 to 1775, 1809 or of at least 8 consecutive nucleotides of these nucleic acid sequences and/or the nucleic acid sequences complementary thereto (nucleotide sequences corresponding to the various variable sequences of the CRISPR1 locus of the serotype Niarembe);
- set S71-2, comprising or constituted of the probes of nucleic acid sequences SEQ ID NO : 90, 1776 to 1785 or of at least 8 consecutive nucleotides of these nucleic acid sequences and/or the nucleic acid sequences complementary thereto (nucleotide sequences corresponding to the various variable sequences of the CRISPR2 locus of the serotype Niarembe);
- set S72-1, comprising or constituted of the probes of nucleic acid sequences SEQ ID NO : 666, 1786 to 1791 or of at least 8 consecutive nucleotides of these nucleic acid sequences and/or the nucleic acid sequences complementary thereto (nucleotide sequences corresponding to the various variable sequences of the CRISPRl locus of the serotype Overvecht);
- set S72-2, comprising or constituted of the probes of nucleic acid sequences SEQ ID NO : 783, 1146, 1178, 1179, 1181, 1792 to 1799, 1801 or of at least 8 consecutive nucleotides of these nucleic acid sequences and/or the nucleic acid sequences complementary thereto (nucleotide sequences corresponding to the various variable sequences of the CRISPR2 locus of the serotype Overvecht);
- set S73-1, comprising or constituted of at least one probe comprising or constituted of at least 8 consecutive nucleotides of the nucleic acid sequence SEQ ID NO: 917 and/or the nucleic acid sequence complementary thereto (nucleotide sequences corresponding to the variable sequence of the CRISPR1 locus of the serotype Poona);
- set S73-2, comprising or constituted of the probes of nucleic acid sequences SEQ ID NO : 82, 87, 918, 933, 1826 or of at least 8 consecutive nucleotides of these nucleic acid sequences and/or the nucleic acid sequences complementary thereto (nucleotide sequences corresponding to the various variable sequences of the CRISPR2 locus of the serotype Poona);
- set S74-1, comprising or constituted of the probes of nucleic acid sequences SEQ ID NO : 295 to 297, 307, 313, 317, 323, 1120, 1825, 1827 to 1837, 2023 or of at least 8 consecutive nucleotides of these nucleic acid sequences and/or the nucleic acid sequences complementary thereto (nucleotide sequences corresponding to the various variable sequences of the CRISPR1 locus of the serotype Postdam);
- set S74-2, comprising or constituted of the probes of nucleic acid sequences SEQ ID NO: 663, 1304, 1838 to 1847 or of at least 8 consecutive nucleotides of these nucleic acid sequences and/or the nucleic acid sequences complementary thereto (nucleotide sequences corresponding to the various variable sequences of the CRISPR2 locus of the serotype Postdam);
- set S75-1, comprising or constituted of at least one probe comprising or constituted of at least 8 consecutive nucleotides of the nucleic acid sequence SEQ ID NO: 917 and/or the nucleic acid sequence complementary thereto (nucleotide sequences corresponding to the variable sequence of the CRISPR1 locus of the serotype Reading);
- set S75-2, comprising or constituted of at least one probe comprising or constituted of at least 8 consecutive nucleotides of the nucleic acid sequence SEQ ID NO: 225 and/or the nucleic acid sequence complementary thereto (nucleotide sequences corresponding to the variable sequence of the CRISPR2 locus of the serotype Reading);
- set S76-1, comprising or constituted of the probes of nucleic acid sequences SEQ ID NO : 379, 380, 383 to 385, 387, 389, 390 or of at least 8 consecutive nucleotides of these nucleic acid sequences and/or the nucleic acid sequences complementary thereto (nucleotide sequences corresponding to the various variable sequences of the CRISPR1 locus of the serotype Rosenberg);
- set S76-2, comprising or constituted of the probes of nucleic acid sequences SEQ ID NO: 392, 393, 404, 407 to 413 or of at least 8 consecutive nucleotides of these nucleic acid sequences and/or the nucleic acid sequences complementary thereto (nucleotide sequences corresponding to the various variable sequences of the CRISPR2 locus of the serotype Rosenberg);
- set S77-1, comprising or constituted of at least one probe comprising or constituted of at least 8 consecutive nucleotides of the nucleic acid sequence SEQ ID NO: 917 and/or the nucleic acid sequence complementary thereto (nucleotide sequences corresponding to the variable sequence of the CRISPR1 locus of the serotype Rubislaw);
- set S77-2, comprising or constituted of at least one probe comprising or constituted of at least 8 consecutive nucleotides of the nucleic acid sequence SEQ ID NO : 225 and/or the nucleic acid sequence complementary thereto;
- set S78-1, comprising or constituted of the probes of nucleic acid sequences SEQ ID NO : 1525, 1609 to 1621 or of at least 8 consecutive nucleotides of these nucleic acid sequences and/or the nucleic acid sequences complementary thereto;
- set S78-2, comprising or constituted of the probes of nucleic acid sequences SEQ ID NO : 225 to 227, 229 to 231 or of at least 8 consecutive nucleotides of these nucleic acid sequences and/or the nucleic acid sequences complementary thereto (nucleotide sequences corresponding to the various variable sequences of the CRISPR2 locus of the serotype Sandiego);
- set S79-1, comprising or constituted of the probes of nucleic acid sequences SEQ ID NO: 1106, 1456, 1917 to 1924 or of at least 8 consecutive nucleotides of these nucleic acid sequences and/or the nucleic acid sequences complementary thereto (nucleotide sequences corresponding to the various variable sequences of the CRISPR1 locus of the serotype Stourbridge);
- set S79-2 comprising or constituted of the probes of nucleic acid sequences SEQ ID NO : 918, 1093, 1925 to 1936 or of at least 8 consecutive nucleotides of these nucleic acid sequences and/or the nucleic acid sequences complementary thereto (nucleotide sequences corresponding to the various variable sequences of the CRISPR2 locus of the serotype Stourbridge);
- set S80-1 comprising or constituted of the probes of nucleic acid sequences SEQ ID NO : 1961 to 1963 or of at least 8 consecutive nucleotides of these nucleic acid sequences and/or the nucleic acid sequences complementary thereto (nucleotide sequences corresponding to the various variable sequences of the CRISPR1 locus of the serotype Tallahassee);
- set S80-2 comprising or constituted of the probes of nucleic acid sequences SEQ ID NO : 25, 603, 2127, 2133, 1964 to 1966 or of at least 8 consecutive nucleotides of these nucleic acid sequences and/or the nucleic acid sequences complementary thereto (nucleotide sequences corresponding to the various variable sequences of the CRISPR2 locus of the serotype Tallahassee);
- set S81-1 comprising or constituted of the probes of nucleic acid sequences SEQ ID NO : 67, 929, 1967 to 2005 or of at least 8 consecutive nucleotides of these nucleic acid sequences and/or the nucleic acid sequences complementary thereto;
- set S81-2 comprising or constituted of the probes of nucleic acid sequences SEQ ID NO : 24, 769, 1550, 1659, 2006 to 2022 or of at least 8 consecutive nucleotides of these nucleic acid sequences and/or the nucleic acid sequences complementary thereto (nucleotide sequences corresponding to the various variable sequences of the CRISPR2 locus of the serotype Tennessee);
- set S82-1, comprising or constituted of at least one probe comprising or constituted of at least 8 consecutive nucleotides of the nucleic acid sequence SEQ ID NO : 916 and/or the nucleic acid sequence complementary thereto (nucleotide sequences corresponding to the variable sequence of the CRISPR1 locus of the serotype Urbana);
- set S82-2 comprising or constituted of the probes of nucleic acid sequences SEQ ID NO : 1697, 1698, 1800 or of at least 8 consecutive nucleotides of these nucleic acid sequences and/or the nucleic acid sequences complementary thereto (nucleotide sequences corresponding to the various variable sequences of the CRISPR2 locus of the serotype Urbana);
- set S83-1 comprising or constituted of the probes of nucleic acid sequences SEQ ID NO : 948, 1120, 1123, 1325, 2039 to 2078 or of at least 8 consecutive nucleotides of these nucleic acid sequences and/or the nucleic acid sequences complementary thereto (nucleotide sequences corresponding to the various variable sequences of the CRISPR1 locus of the serotype Weltevreden);
- set S83-2 comprising or constituted of the probes of nucleic acid sequences SEQ ID NO : 254 to 256, 2079 to 2108 or of at least 8 consecutive nucleotides of these nucleic acid sequences and/or the nucleic acid sequences complementary thereto (nucleotide sequences corresponding to the various variable sequences of the CRISPR2 locus of the serotype Weltevreden);
- set S84-1 comprising or constituted of the probes of nucleic acid sequences SEQ ID NO: 1312, 1690, 2141, 2142 or of at least 8 consecutive nucleotides of these nucleic acid sequences and/or the nucleic acid sequences complementary thereto (nucleotide sequences corresponding to the various variable sequences of the CRISPR1 locus of the serotype Zaiman);
- set S84-2 comprising or constituted of the probes of nucleic acid sequences SEQ ID NO: 1697, 1698, 1800, 1924, 2143, 2144 or of at least 8 consecutive nucleotides of these nucleic acid sequences and/or the nucleic acid sequences complementary thereto (nucleotide sequences corresponding to the various variable sequences of the CRISPR2 locus of the serotype Zaiman);

19. A set of primers, capable of amplifying a fragment of the genomic sequence of a bacterium of the *Salmonella* genus, **characterised in that** it is selected from the group constituted of:
- the set of primers constituted of the primer of sequence SEQ ID No: 1326, combined with at least one of the primers of sequence SEQ ID Nos: 1327, 1328, 1329 2151 and 2152,
- the set of primers constituted of the primer of sequence SEQ ID No: 1330, combined with at least one of the primers of sequence SEQ ID Nos: 1331 and 1332, and
- the set of primers constituted of the primer of sequence SEQ ID No: 1326, combined with at least one of the two primers of sequence 1331 and 1332.

20. A DNA chip, **characterised in that** it comprises the probes included in at least one of the sets of probes as claimed in claim 18.

21. A kit or pack for detecting or identifying the molecular type and subtype of a bacterium of the *Salmonella* genus, **characterised in that** it comprises at least one set of probes as defined in claim 18 and a set of primers as claimed in claim 19 and, optionally, a DNA chip as claimed in claim 20.

22. The use of a set of probes as defined in claim 18, of a set of primers as claimed in claim 19 or of a DNA chip as claimed in claim 20, for detecting or identifying the molecular type and/or subtype of a bacterium of the *Salmonella* genus.
